(19) Europäisches Patentamt
European Patent Office
Office européen des brevets

(11) EP 2 663 645 B1

(12) **EUROPEAN PATENT SPECIFICATION**

(45) Date of publication and mention of the grant of the patent:
**17.12.2014 Bulletin 2014/51**

(51) Int Cl.:
*C12P 7/06* (2006.01) *C12N 1/19* (2006.01)
*C12N 9/02* (2006.01) *C12N 9/04* (2006.01)

(21) Application number: **11788234.0**

(22) Date of filing: **18.11.2011**

(86) International application number:
**PCT/NL2011/050787**

(87) International publication number:
**WO 2012/067510 (24.05.2012 Gazette 2012/21)**

(54) **YEAST STRAINS ENGINEERED TO PRODUCE ETHANOL FROM GLYCEROL**

HEFESTÄMME ZUR HERSTELLUNG VON ETHANOL AUS GLYCEROL

SOUCHES DE LEVURES MODIFIÉES POUR PRODUIRE DE L'ÉTHANOL À PARTIR DE GLYCÉROL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2010 EP 10191736**
**18.11.2010 US 415054 P**
**05.04.2011 US 201161471836 P**

(43) Date of publication of application:
**20.11.2013 Bulletin 2013/47**

(73) Proprietor: **DSM IP Assets B.V.**
**6411 TE Heerlen (NL)**

(72) Inventors:
- **DE BONT, Johannes Adrianus Maria**
**NL-6707 AK Wageningen (NL)**
- **TEUNISSEN, Aloysius Wilhelmus Rudolphus Hubertus**
**NL-3052 HP Rotterdam (NL)**

(74) Representative: **Kleiborn, Paul Erik**
**DSM Intellectual Property**
**P.O. Box 4**
**6100 AA Echt (NL)**

(56) References cited:
**WO-A1-2010/019882 WO-A2-2009/143495**

- **YU K O ET AL: "Engineering of glycerol utilization pathway for ethanol production by Saccharomyces cerevisiae", BIORESOURCE TECHNOLOGY, ELSEVIER BV, GB, vol. 101, no. 11, 1 June 2010 (2010-06-01) , pages 4157-4161, XP026917458, ISSN: 0960-8524, DOI: DOI: 10.1016/J.BIORTECH.2010.01.066 [retrieved on 2010-02-09] cited in the application**
- **WON-KYUNG HONG ET AL: "Enhanced production of ethanol from glycerol by engineered Hansenula polymorpha expressing pyruvate decarboxylase and aldehyde dehydrogenase genes from Zymomonas mobilis", BIOTECHNOLOGY LETTERS, SPRINGER NETHERLANDS, DORDRECHT, vol. 32, no. 8, 31 March 2010 (2010-03-31) , pages 1077-1082, XP019813441, ISSN: 1573-6776**
- **YU K O ET AL: "Reduction of glycerol production to improve ethanol yield in an engineered Saccharomyces cerevisiae using glycerol as a substrate", JOURNAL OF BIOTECHNOLOGY, ELSEVIER SCIENCE PUBLISHERS, AMSTERDAM, NL, vol. 150, no. 2, 15 October 2010 (2010-10-15), pages 209-214, XP027424705, ISSN: 0168-1656, DOI: DOI:10.1016/J.JBIOTEC. 2010.09.932 [retrieved on 2010-10-15] cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 663 645 B1

- **MEDINA VICTOR GUADALUPE ET AL: "Elimination of Glycerol Production in Anaerobic Cultures of a Saccharomyces cerevisiae Strain Engineered To Use Acetic Acid as an Electron Acceptor", APPLIED AND ENVIRONMENTAL MICROBIOLOGY, AMERICAN SOCIETY FOR MICROBIOLOGY, US, vol. 76, no. 1, 1 January 2010 (2010-01-01), pages 190-195, XP002603125, ISSN: 0099-2240, DOI: DOI:10.1128/AEM. 01772-09 [retrieved on 2009-11-13]**
- **SHAMS YAZDANI S ET AL: "Engineering Escherichia coli for the efficient conversion of glycerol to ethanol and co-products", METABOLIC ENGINEERING, ACADEMIC PRESS, US, vol. 10, no. 6, 1 November 2008 (2008-11-01), pages 340-351, XP025744625, ISSN: 1096-7176, DOI: DOI:10.1016/J.YMBEN.2008.08.005 [retrieved on 2008-09-09]**
- **NEVOIGT E: "Progress in metabolic engineering of Saccharomyces cerevisiae", MICROBIOLOGY AND MOLECULAR BIOLOGY REVIEWS, AMERICAN SOCIETY FOR MICROBIOLOGY, US, vol. 72, no. 3, 1 September 2008 (2008-09-01), pages 379-412, XP008100901, ISSN: 1092-2172, DOI: 10.1128/MMBR.00025-07**

## Description

### Field of the invention

The present invention relates to metabolic engineering in microorganisms such as yeast. In particular the invention relates to yeast strains that have been engineered to produce ethanol from glycerol. These strains have retained their natural ability to produce ethanol from hexoses (glucose, fructose, galactose, etc) and comprise an engineered ability to produce ethanol from pentoses like xylose. The invention further relates to the processes wherein the engineered strains of the invention produce ethanol from glycerol, either as main fermentation feedstock, or concomitantly with one or more of hexoses and pentoses.

### Background of the invention

Both the formation and the degradation of glycerol in the yeast *Saccharomyces cerevisiae* are important processes that have been studied for more than a century. During the last decades, the emphasis has been on (1) suppression of the formation of glycerol during ethanol production. In the beginning of the previous century, (2) the optimization of glycerol production from sugars has been studied. Both of these aspects have profound economic impacts. More recently, a fully new aspect has been introduced in this glycerol field. It deals with (3) the anaerobic fermentation of glycerol into ethanol.

(1) Suppression of glycerol formation during ethanol production.

Bioethanol is produced by *Saccharomyces cerevisiae* from a range of substrates including lignocellulosic hydrolysates of non-food feedstocks (e.g. energy crops and agricultural residues). One problem of yeast-based ethanol production is that during anaerobic ethanolic fermentation of sugar feedstocks, substantial amounts of glycerol are invariably formed as a by-product. Sugar dissimilation during anaerobic growth of *S. cerevisiae* occurs via alcoholic fermentation. In this process, the NADH formed in glycolysis is reoxidised via a $NAD^+$ dependent alcohol dehydrogenase which converts acetaldehyde (formed by decarboxylation of pyruvate) to ethanol. This dissimilatory pathway is redox-neutral and can therefore not compensate a net reduction of $NAD^+$ to NADH occurring elsewhere in metabolism. Such net reduction of $NAD^+$ to NADH occurs in assimilation when yeast biomass is synthesized under anaerobic conditions from sugars and e.g. ammonia. Under anaerobic conditions, NADH reoxidation in *S. cerevisiae* to compensate for the assimilatory-associated NADH-formation is mainly dependent on reduction of at least part of the sugar carbon source to glycerol, resulting in a lower ethanol yield. To address this problem, several metabolic engineering approaches have been explored to reduce or eliminate glycerol production in anaerobically grown *S. cerevisiae.* Two examples are:

Nissen et al (2000) disclose an approach in which the enzyme NADPH-dependent glutamate dehydrogenase was deleted. Glutamine synthetase and glutamate synthase were overexpressed. The resulting strain no longer synthesized glutamate from ammonium and 2-oxoglutarate via a NADPH-requiring route, but rather via a NADH- and ATP-requiring pathway. The resulting strain had a 10% higher yield in ethanol and a 38% lower glycerol yield than the wild type strain.

Guadalupe Medina et al. (2009, Appl. Environ. Microbiol., 76: 190-195) disclose a *S. cerevisiae* strain wherein production of the by-product glycerol is eliminated by disruption of the endogenous NAD-dependent glycerol 3-phosphate dehydrogenase genes *(GPD1* and *GPD2).* Expression of the *E. coli mhpF* gene, encoding the acetylating NAD-dependent acetaldehyde dehydrogenase restored the ability of the GPD-disrupted strain to grow anaerobically. However, the GPD-disrupted strain could only grow anaerobically if the medium is supplemented with acetic acid.

(2) Optimization of glycerol production from sugars.

Production of glycerol from sugars, as opposed to minimizing its production during ethanol formation, has been an economically important process during World War 1 in Germany. In recent years, a renewed interest in this process is seen, as witnessed for instance by the work of Overkamp et al (2002, Appl. Environ. Microbiol. 68:2814-21).

(3) Fermentation of glycerol into ethanol.

The fermentation of glycerol into ethanol is not feasible in normal *S. cerevisiae* and under fully anoxic cultivation conditions due to an unbalanced balance for NADH. Glycerol is more reduced than ethanol and hence the organism cannot dispose of its excess NADH by reoxidation under normal anoxic conditions. Some initial work has been done in the field of fermentation of glycerol into ethanol: Yu et al. (2010, Bioresour. Technol. 101(11):4157-61. Epub 2010 Feb 9) disclose *S. cerevisiae* strains metabolically engineered for improved ethanol production from glycerol by simultaneous

overexpression of glycerol dehydrogenase (GCY), dihydroxyacetone kinase *(DAK)* and the glycerol uptake protein *(GUP1).* In a later communication, Yu et al (2010, J. Biotechnol. doi:10.1016/j.jbiotec.2010.09.932) disclose an optimisation of their strain for ethanol production from glycerol by deleting two glycerol production genes, *FPS1* and *GPD2.* It was shown that ethanol production from glycerol is possible in their strains. However, the increase in yield was dependent on micro aerobic conditions, which can be explained by the requirement for NADH oxidation. The excess of NADH produced in the path from glycerol to ethanol apparently could only be reoxidised via an oxygen-dependent reaction.

Waks and Silver (2009, Appl. Environ. Microbiol., 75:1867-1875) disclose a synthetic dual-organism system for biohydrogen production. In a first step formate is produced by an engineered *S. cerevisiae* strain wherein a formate-overproducing pathway has been implemented. In a second step the formate produced by the engineered yeast is processed into hydrogen by *Escherichia coli.* The *S. cerevisiae* strain was engineered to produce formate by expressing the anaerobic enzyme pyruvate formate lyase (PFL) from *E. coli.* Formate production was further increased by also introducing expression of a downstream enzyme, the AdhE of *E. coli,* the bifunctional enzyme that reduces acetyl-CoA generated by PFL into ethanol.

WO 2010/019882 discloses genetically engineered yeast that are able to uptake glycerol and convert the glycerol into ethanol.

Won-Kyung HONG et al, biotechnology letters vol. 32, no. 8, 2010, pages 1077-1082 discloses enhanced production of ethanol from glycerol by engineered *Hansenula polymorpha* expressing pyruvate decarboxylase and aldehyde dehydrogenase genes from *Zymomonas mobilis.*

It is an object of the present invention to provide for yeasts that are capable of producing ethanol from glycerol while retaining their abilities of fermenting hexoses (glucose, fructose, galactose, etc) as well as pentoses like xylose, as well as processes wherein these strains are used for the production of ethanol and/or other fermentation products.

## Description of the invention

### Definitions

Sequence identity is herein defined as a relationship between two or more amino acid (polypeptide or protein) sequences or two or more nucleic acid (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods. The terms "sequence identity" or "sequence similarity" means that two (poly)peptide or two nucleotide sequences, when optimally aligned, preferably over the entire length (of at least the shortest sequence in the comparison) and maximizing the number of matches and minimizes the number of gaps such as by the programs ClustalW (1.83), GAP or BESTFIT using default parameters, share at least a certain percentage of sequence identity as defined elsewhere herein. GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). A preferred multiple alignment program for aligning protein sequences of the invention is ClustalW (1.83) using a blosum matrix and default settings (Gap opening penalty: 10; Gap extension penalty: 0.05). It is clear than when RNA sequences are said to be essentially similar or have a certain degree of sequence identity with DNA sequences, thymine (T) in the DNA sequence is considered equal to uracil (U) in the RNA sequence. Sequence alignments and scores for percentage sequence identity may be determined using computer programs, such as the GCG Wisconsin Package, Version 10.3, available from Accelrys Inc., 9685 Scranton Road, San Diego, CA 92121-3752 USA or the open-source software Emboss for Windows (current version 2.10.0-0.8). Alternatively percent similarity or identity may be determined by searching against databases such as FASTA, BLAST, etc.

A variant of a nucleotide or amino acid sequence disclosed herein may also be defined as a nucleotide or amino acid sequence having one or several substitutions, insertions and/or deletions as compared to the nucleotide or amino acid sequence specifically disclosed herein (e.g. in de the sequence listing).

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side

chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

Nucleotide sequences of the invention may also be defined by their capability to hybridise with parts of specific nucleotide sequences disclosed herein, respectively, under moderate, or preferably under stringent hybridisation conditions. Stringent hybridisation conditions are herein defined as conditions that allow a nucleic acid sequence of at least about 25, preferably about 50 nucleotides, 75 or 100 and most preferably of about 200 or more nucleotides, to hybridise at a temperature of about 65°C in a solution comprising about 1 M salt, preferably 6 x SSC or any other solution having a comparable ionic strength, and washing at 65°C in a solution comprising about 0.1 M salt, or less, preferably 0.2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity.

Moderate conditions are herein defined as conditions that allow a nucleic acid sequences of at least 50 nucleotides, preferably of about 200 or more nucleotides, to hybridise at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 90%.

A "nucleic acid construct" or "nucleic acid vector" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules. The terms "expression vector" or expression construct" refer to nucleotide sequences that are capable of affecting expression of a gene in host cells or host organisms compatible with such sequences. These expression vectors typically include at least suitable transcription regulatory sequences and optionally, 3' transcription termination signals. Additional factors necessary or helpful in effecting expression may also be present, such as expression enhancer elements. The expression vector will be introduced into a suitable host cell and be able to effect expression of the coding sequence in an in vitro cell culture of the host cell. The expression vector will be suitable for replication in the host cell or organism of the invention.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer.

The term "selectable marker" is a term familiar to one of ordinary skill in the art and is used herein to describe any genetic entity which, when expressed, can be used to select for a cell or cells containing the selectable marker. The term "reporter" may be used interchangeably with marker, although it is mainly used to refer to visible markers, such as green fluorescent protein (GFP). Selectable markers may be dominant or recessive or bidirectional.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.

The terms "protein" or "polypeptide" are used interchangeably and refer to molecules consisting of a chain of amino acids, without reference to a specific mode of action, size, 3-dimensional structure or origin.

"Fungi" (singular fungus) are herein understood as heterotrophic eukaryotic microorganism that digest their food externally, absorbing nutrient molecules into their cells. Fungi are a separate kingdom of eukaryotic organisms and include yeasts, molds, and mushrooms. The terms fungi, fungus and fungal as used herein thus expressly includes

yeasts as well as filamentous fungi.

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'nontranslated sequence (3'end) comprising a polyadenylation site. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence and, if applicable, another (heterologous) secretory signal sequence and/or terminator sequence than in its natural environment. It is understood that the regulatory sequences, signal sequences, terminator sequences, etc. may also be homologous to the host cell. In this context, the use of only "homologous" sequence elements allows the construction of "self-cloned" genetically modified organisms (GMO's) (self-cloning is defined herein as in European Directive 98/81/EC Annex II). When used to indicate the relatedness of two nucleic acid sequences the term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later.

The terms "heterologous" and "exogenous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous and exogenous nucleic acids or proteins are not endogenous to the cell into which it is introduced, but have been obtained from another cell or synthetically or recombinantly produced. Generally, though not necessarily, such nucleic acids encode proteins, i.e. exogenous proteins, that are not normally produced by the cell in which the DNA is transcribed or expressed. Similarly exogenous RNA encodes for proteins not normally expressed in the cell in which the exogenous RNA is present. Heterologous/exogenous nucleic acids and proteins may also be referred to as foreign nucleic acids or proteins. Any nucleic acid or protein that one of skill in the art would recognize as foreign to the cell in which it is expressed is herein encompassed by the term heterologous or exogenous nucleic acid or protein. The terms heterologous and exogenous also apply to non-natural combinations of nucleic acid or amino acid sequences, i.e. combinations where at least two of the combined sequences are foreign with respect to each other.

The "specific activity" of an enzyme is herein understood to mean the amount of activity of a particular enzyme per amount of total host cell protein, usually expressed in units of enzyme activity per mg total host cell protein. In the context of the present invention, the specific activity of a particular enzyme may be increased or decreased as compared to the specific activity of that enzyme in an (otherwise identical) wild type host cell.

"Anaerobic conditions" or an anaerobic fermentation process is herein defined as conditions or a fermentation process run in the absence of oxygen or in which substantially no oxygen is consumed, preferably less than 5, 2.5 or 1 mmol/L/h, more preferably 0 mmol/L/h is consumed (i.e. oxygen consumption is not detectable), and wherein organic molecules serve as both electron donor and electron acceptors.

<u>Detailed description of the invention</u>

Biodiesel is having and will continue to have a substantial impact in the area of biofuels. It can be produced from a variety of sources including algae, rape and palm. A comprehensive list of potential feedstock sources is available from "Feedstock and Biodiesel Characteristics Report, "Renewable Energy Group, Inc., www.regfuel.com (2009)". The vegetable oils from which the biodiesel is produced by interesterification inevitably result in large byproduct streams of glycerol. Furthermore, the plant material from which the oil is extracted will give rise to lignocellulosic byproduct. The latter by-product will contain cellulose and hemicelluloses in varying relative concentrations. These polymeric sugars can be hydrolyzed into glucose and pentoses such as xylose, respectively, by known processes that are still optimized. Hence, two by-product streams may result from the production of biodiesel. The present invention describes a method for the concomitant fermentation of both glycerol and the sugars into ethanol. This process can be applied for various feedstocks. As an example, the situation in the palm oil industry is presented in some detail below.

The palm oil industry produces huge amounts of by-products. The oil consists of only 10% of the total biomass produced in the plantation. The remainder consists of lignocellulosic materials such as oil palm fronds, trunks and empty fruit bunches (EFB) (See e.g. http://umpir.ump.edu.my/697/1/Kamarul_Azlan_Abd_Hamid.pdf). These lignocellulosic materials can be used for the production of value added products such as ethanol that may be used as biofuel. The

lignocellulosic materials may be hydrolysed by known methods into streams containing hexoses and pentose such as glucose and xylose, which can then be fermented to ethanol by engineered yeast strains as e.g. described by Kuypers et al (2005, FEMS Yeast Res. 5:399-409). However, hexose- and pentose-containing streams obtained from hydrolysis of this type of lignocellulosic materials are relatively dilute, and will therefore yield relatively low ethanol concentrations of no more than about 5% (v/v).

At the same time, large amounts of concentrated glycerol are generated as a by-product in biodiesel production from transesterification reactions using vegetable oils such as palm oil and alcohols. The availability of crude glycerol is predicted to increase over the next years as a result of the growth in biodiesel production worldwide. Consequently large amounts of concentrated glycerol will be available at low cost near palm oil plantations. This offers the possibility to increase the carbon concentration of diluted streams obtained in the above-described hydrolysis of lignocellulosic materials by mixing in the available highly concentrated glycerol. Utilisation of such mixed streams would require engineered yeast strains which can ferment not only hexoses and pentose but also glycerol to ethanol. However, anaerobic consumption of large amount of glycerol would produce a redox imbalance in yeast. The present invention addresses this problem by engineering the yeast to produce formic acid, in addition to ethanol. The present invention therefore provides yeast strains engineered to produce ethanol and formic acid from carbon sources containing one or more of glycerol, hexose and pentose, as well as processes wherein these strains are used to produce ethanol and formic acid from these carbon sources.

In a first aspect the invention relates to a fungal host cell comprising an exogenous gene coding for a enzyme with the ability to convert pyruvate and coenzyme-A into formate and acetyl-CoA. An enzyme with the ability to convert pyruvate and coenzyme-A into formate and acetyl-CoA katalyses the reaction (EC 2.3.1.54):

pyruvate + coenzyme A (CoA) ↔ acetyl-CoA + formate.

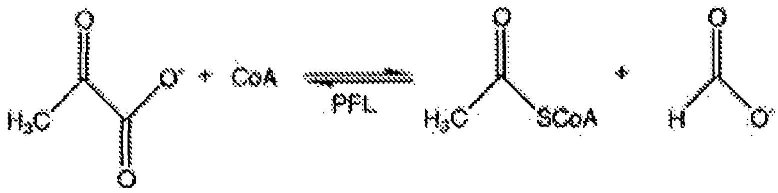


Such an enzyme is herein understood as an enzyme having pyruvate formate lyase activity and is referred to as a pyruvate formate lyase (PFL) or formate C-acetyltransferase.

A suitable exogenous gene coding for an enzyme with pyruvate formate lyase activity is e.g. a prokaryotic pyruvate formate lyase, such as the pyruvate formate lyase from *E. coli.* The *E. coli* pyruvate formate lyase is a dimer of PflB (encoded by *pflB*), whose maturation requires the activating enzyme PflAE (encoded by *pflA*), radical S-adenosylmethionine, and a single electron donor, which in the case of *E. coli* is flavodoxin (Buis and Broderick, 2005, Arch. Biochem. Biophys. 433:288-296; Sawers and Watson, 1998, Mol. Microbiol. 29:945-954). However, Waks and Silver (*supra*) have shown that for activation of the pyruvate formate lyase in yeast, only co-expression of an activating enzyme is required but expression of flavodoxin is not necessary.

The exogenous gene coding for an enzyme with pyruvate formate lyase activity preferably comprises a nucleotide sequence coding for an amino acid sequence with at least 45, 50, 60, 65, 70, 75, 80, 85, 90, 95, 98, 99% amino acid sequence identity with SEQ ID NO: 1. Suitable examples of organisms comprising an enzyme with pyruvate formate lyase activity are provided in Table 1. Further examples of such organisms are listed by Lehtiö and Goldman (2004, Prot. Engin. Design & Selection, 17:545-552). The amino acid sequences of these enzymes are available in public databases and can be used by the skilled person to design codon-optimised nucleotide sequences coding for the corresponding enzyme with pyruvate formate lyase activity (see e.g. SEQ ID NO: 2). The exogenous gene coding for an enzyme with pyruvate formate lyase activity may also comprises a nucleotide sequence coding for an amino acid sequence having one or several substitutions, insertions and/or deletions as compared to the amino acid sequence of SEQ ID NO: 1. Preferably the amino acid sequence has no more than 420, 380, 300, 250, 200, 150, 100, 75, 50, 40, 30, 20, 10 or 5 amino acid substitutions, insertions and/or deletions as compared to SEQ ID NO: 1.

Table 1: Enzymes with pyruvate formate lyase activity related to *E.coli* pflB

| Organism | Amino acid identity (%) |
|---|---|
| *Escherichia coli* str. K12 substr. MG1655 | 100% |

(continued)

| Organism | Amino acid identity (%) |
|---|---|
| *Shigella boydii* | 100% |
| *Escherichia albertii* TW07627 | 99% |
| *Salmonella enterica* | 97% |
| *Citrobacter rodentium* ICC168 | 97% |
| *Klebsiella pneumoniae* NTUH-K2044 | 96% |
| *Yersinia aldovae* ATCC 35236 | 91% |
| *Proteus mirabilis* HI4320 | 87% |
| *Haemophilus influenzae* Rd KW20 | 86% |
| *Actinobacillus succinogenes* 130Z | 83% |
| *Piromyces* sp. E2 | 57% |

The host cell of the invention further preferably comprises an exogenous gene coding for the PflAE activating enzyme for activation of the pyruvate formate lyase. The pyruvate formate lyase activating enzyme is herein understood as an enzyme that katalyses the reaction:

S-adenosyl-L-methionine + dihydroflavodoxin + [pyruvate formate lyase]-glycine

↕

5'-deoxyadenosine + L-methionine + flavodoxin semiquinone +

[formate C-acetyltransferase]-glycin-2-yl radical

The exogenous gene coding for the pyruvate formate lyase activating enzyme preferably comprises a nucleotide sequence coding for an amino acid sequence with at least 45, 50, 60, 65, 70, 75, 80, 85, 90, 95, 98, 99% amino acid sequence identity with SEQ ID NO: 3. Suitable examples of organisms comprising an enzyme with pyruvate formate lyase activity are provided in Table 2. The amino acid sequences of these enzymes are available in public databases and can be used by the skilled person to design codon-optimised nucleotide sequences coding for the corresponding enzyme with pyruvate formate lyase activity (see e.g. SEQ ID NO: 4). The exogenous gene coding for the pyruvate formate lyase activating enzyme may also comprises a nucleotide sequence coding for an amino acid sequence having one or several substitutions, insertions and/or deletions as compared to the amino acid sequence of SEQ ID NO: 3. Preferably the amino acid sequence has no more than 135, 125, 100, 75, 50, 25, 20, 15, 10, 8, 5 or 2 amino acid substitutions, insertions and/or deletions as compared to SEQ ID NO: 3.

Table 2: Pyruvate formate lyase activating enzymes related to *E.coli* pflA

| Organism | Amino acid identity (%) |
|---|---|
| *Escherichia coli* str. K12 substr. MG1655 | 100% |
| *Shigella boydii* | 100% |
| *Escherichia albertii* TW07627 | 99% |
| *Salmonella enterica* | 98% |
| *Citrobacter rodentium* ICC168 | 98% |
| *Klebsiella pneumoniae* NTUH-K2044 | 97% |
| *Yersinia rohdei* ATCC 43380 | 89% |
| *Proteus penneri* ATCC 35198 | 85% |
| *Haemophilus parasuis* 29755 | 70% |

In a preferred host cell of the invention, the exogenous genes coding for the enzyme with pyruvate formate lyase activity and the pyruvate formate lyase activating enzyme are from the same donor organism, i.e. be homologous to each other. However, the exogenous genes coding for the enzyme with pyruvate formate lyase activity and the pyruvate formate lyase activating enzyme may also be from different donor organisms, i.e. be heterologous to each other.

In one aspect the invention relates to methods for preparing or constructing the yeast cells of the invention. For this purpose standard genetic and molecular biology techniques are used that are generally known in the art and have e.g. been described by Sambrook and Russell (2001, "Molecular cloning: a laboratory manual" (3rd edition), Cold Spring Harbor Laboratory, Cold Spring Harbor Laboratory Press) and Ausubel et al. (1987, eds., "Current protocols in molecular biology", Green Publishing and Wiley Interscience, New York). Furthermore, the construction of mutated host yeast strains is carried out by genetic crosses, sporulation of the resulting diploids, tetrad dissection of the haploid spores containing the desired auxotrophic markers, and colony purification of such haploid host yeasts in the appropriate selection medium. All of these methods are standard yeast genetic methods known to those in the art. See, for example, Sherman et al., Methods Yeast Genetics, Cold Spring Harbor Laboratory, NY (1978) and Guthrie et al. (Eds.) Guide To Yeast Genetics and Molecular Biology Vol. 194, Academic Press, San Diego (1991).

The exogenous genes coding for the enzyme having pyruvate formate lyase activity and the pyruvate formate lyase activating enzyme, preferably are expression constructs comprising the nucleotide sequence coding for the enzymes operably linked to suitable expression regulatory regions/sequences to ensure expression of the enzymes upon transformation of the expression constructs into the host cell of the invention. Thus, the gene or expression construct will at least comprise a promoter that is functional in the host cell operably linked to the coding sequence. The gene or construct may further comprise a 5' leader sequence upstream of the coding region and a 3'-nontranslated sequence (3'end) comprising a polyadenylation site and a transcription termination site downstream of the coding sequence. It is understood that the nucleotide sequences coding for the enzyme having pyruvate formate lyase activity and the pyruvate formate lyase activating enzyme may be present together on a single expression construct, or each enzyme may be present on a separate expression construct.

Suitable promoters for expression of the nucleotide sequences coding for the enzyme having pyruvate formate lyase activity and the pyruvate formate lyase activating enzyme (as well as other enzymes of the invention; see below) include promoters that are preferably insensitive to catabolite (glucose) repression, that are active under anaerobic conditions and/or that preferably do not require xylose or arabinose for induction. Promoters having these characteristics are widely available and known to the skilled person. Suitable examples of such promoters include e.g. promoters from glycolytic genes such as the phosphofructokinase (*PPK*), triose phosphate isomerase (*TPI*), glyceraldehyde-3-phosphate dehydrogenase (*GPD, TDH3* or *GAPDH*), pyruvate kinase (*PYK*), phosphoglycerate kinase (*PGK*), glucose-6-phosphate isomerase promoter (*PGI1*) promoters from yeasts. More details about such promoters from yeast may be found in (WO 93/03159). Other useful promoters are ribosomal protein encoding gene promoters (*TEF1*), the lactase gene promoter (*LAC4*), alcohol dehydrogenase promoters (*ADH1, ADH4,* and the like), the enolase promoter (*ENO*) and the hexose(glucose) transporter promoter (*HXT7*). Alternatively, the nucleotide sequences encoding the enzyme having pyruvate formate lyase activity and the PflAE activating enzyme are expressed under anaerobic conditions by using an anoxic promoter such as e.g. the *S. cerevisiae ANB1* promoter (SEQ ID NO: 24). Other promoters, both constitutive and inducible, and enhancers or upstream activating sequences will be known to those of skill in the art. Preferably the promoter that is operably linked to nucleotide sequence as defined above is homologous to the host cell. Suitable terminator sequences are e.g. obtainable from the cytochrome c1 (*CYC1*) gene or an alcohol dehydrogenase gene (e.g. *ADH1*).

To increase the likelihood that the enzyme having pyruvate formate lyase activity is expressed at sufficient levels and in active form in the transformed host cells of the invention, the nucleotide sequence encoding these enzymes, as well as the pyruvate formate lyase activating enzyme and other enzymes of the invention (see below), are preferably adapted to optimise their codon usage to that of the host cell in question. The adaptiveness of a nucleotide sequence encoding an enzyme to the codon usage of a host cell may be expressed as codon adaptation index (CAI). The codon adaptation index is herein defined as a measurement of the relative adaptiveness of the codon usage of a gene towards the codon usage of highly expressed genes in a particular host cell or organism. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li, 1987, Nucleic Acids Research 15: 1281-1295; also see: Jansen et al., 2003, Nucleic Acids Res. 31(8):2242-51). An adapted nucleotide sequence preferably has a CAI of at least 0.2, 0.3, 0.4, 0.5, 0.6, 0.7, 0.8 or 0.9. Most preferred are the sequences which have been codon optimised for expression in the fungal host cell in question such as e.g. *S. cerevisiae* cells.

The fungal host cell to be transformed with a nucleic acid construct comprising a nucleotide sequence encoding an enzyme with pyruvate formate lyase activity preferably is a yeast host cell. Preferably the host cell is a cultured cell. The host cell of the invention, preferably is a host capable of active or passive pentose (xylose and preferably also

arabinose) transport into the cell. The host cell preferably contains active glycolysis. The host cell may further preferably contains an endogenous pentose phosphate pathway and may contain endogenous xylulose kinase activity so that xylulose isomerised from xylose may be metabolised to pyruvate. The host further preferably contains enzymes for conversion of a pentose (preferably through pyruvate) to a desired fermentation product such as ethanol, lactic acid, 3-hydroxy-propionic acid, acrylic acid, 1,3-propane-diol, butanols (1-butanol, 2-butanol, isobutanol) and isoprenoid-derived products. A particularly preferred host cell is a yeast cell that is naturally capable of alcoholic fermentation, preferably, anaerobic alcoholic fermentation. The yeast host cell further preferably has a high tolerance to ethanol, a high tolerance to low pH (i.e. capable of growth at a pH lower than 5, 4, or 3) and towards organic acids like lactic acid, acetic acid or formic acid and sugar degradation products such as furfural and hydroxy-methylfurfural, and a high tolerance to elevated temperatures. Any of these characteristics or activities of the host cell may be naturally present in the host cell or may be introduced or modified by genetic modification, preferably by self cloning or by the methods of the invention described below. A suitable cell is a cultured cell, a cell that may be cultured in fermentation process e.g. in submerged or solid state fermentation. Particularly suitable host cells are eukaryotic microorganism like e.g. fungi, however, most suitable for use in the present inventions are yeasts.

Yeasts are herein defined as eukaryotic microorganisms and include all species of the subdivision Eumycotina (Yeasts: characteristics and identification, J.A. Barnett, R.W. Payne, D. Yarrow, 2000, 3rd ed., Cambridge University Press, Cambridge UK; and, The yeasts, a taxonomic study, C.P. Kurtzman and J.W. Fell (eds) 1998, 4th ed., Elsevier Science Publ. B.V., Amsterdam, The Netherlands) that predominantly grow in unicellular form. Yeasts may either grow by budding of a unicellular thallus or may grow by fission of the organism. Preferred yeasts cells for use in the present invention belong to the genera *Saccharomyces, Kluyveromyces, Candida, Pichia, Schizosaccharomyces, Hansenula, Kloeckera, Schwanniomyces,* and *Yarrowia.* Preferably the yeast is capable of anaerobic fermentation, more preferably anaerobic alcoholic fermentation. Over the years suggestions have been made for the introduction of various organisms for the production of bio-ethanol from crop sugars. In practice, however, all major bio-ethanol production processes have continued to use the yeasts of the genus *Saccharomyces* as ethanol producer. This is due to the many attractive features of *Saccharomyces* species for industrial processes, i.e., a high acid-, ethanol- and osmo-tolerance, capability of anaerobic growth, and of course its high alcoholic fermentative capacity. Preferred yeast species as host cells include *S. cerevisiae, S. exiguus, S. bayanus, K. lactis, K. marxianus* and *Schizosaccharomyces pombe.*

In a further embodiment, the host cell of the invention further comprises a genetic modification that reduces specific $NAD^+$-dependent formate dehydrogenase activity in the cell. $NAD^+$-dependent formate dehydrogenases (FDH; EC 1.2.1.2) katalyse the oxidation of formate to bicarbonate, donating the electrons to $NAD^+$. In the cells of the invention, the specific formate dehydrogenase activity is preferably reduced by at least a factor 0.8, 0.5, 0.3, 0.1, 0.05 or 0.01 as compared to a strain which is genetically identical except for the genetic modification causing the reduction in specific activity, preferably under anaerobic conditions. Formate dehydrogenase activity may be determined as described by Overkamp et al. (2002, Yeast 19:509-520).

Preferably, formate dehydrogenase activity is reduced in the host cell by one or more genetic modifications that reduce the expression of or inactivates a gene encoding an formate dehydrogenase. Preferably, the genetic modifications reduce or inactivate the expression of each endogenous copy of the gene encoding a specific formate dehydrogenase in the cell's genome. A given cell may comprise multiple copies of the gene encoding a specific formate dehydrogenase with one and the same amino acid sequence as a result of di-, poly- or aneu-ploidy. In such instances preferably the expression of each copy of the specific gene that encodes the formate dehydrogenase is reduced or inactivated. Alternatively, a cell may contain several different (iso)enzymes with formate dehydrogenase activity that differ in amino acid sequence and that are each encoded by a different gene. In such instances, in some embodiments of the invention it may be preferred that only certain types of the isoenzymes are reduced or inactivated while other types remain unaffected. Preferably, however, expression of all copies of genes encoding (iso)enzymes with formate dehydrogenase activity is reduced or inactivated.

Preferably, a gene encoding formate dehydrogenase activity is inactivated by deletion of at least part of the gene or by disruption of the gene, whereby in this context the term gene also includes any non-coding sequence up- or down-stream of the coding sequence, the (partial) deletion or inactivation of which results in a reduction of expression of formate dehydrogenase activity in the host cell.

A preferred gene encoding a formate dehydrogenase whose activity is to be reduced or inactivated in the cell of the invention is the *S. cerevisiae FDH1* as described by van den Berg and Steensma (1997, Yeast 13:551-559), encoding the amino acid sequence of SEQ ID NO: 5 and orthologues thereof in other species. Therefore a gene encoding a formate dehydrogenase whose activity is to be reduced or inactivated in the cell of the invention preferably is a gene encoding a formate dehydrogenase having an amino acid sequence with at least 45, 50, 60, 65, 70, 75, 80, 85, 90, 95, 98 or 99% sequence identity to SEQ ID NO: 5 or a gene encoding a formate dehydrogenase having an amino acid sequence having one or several substitutions, insertions and/or deletions as compared to the amino acid sequence of SEQ ID NO: 5.

However, in some strains of *S. cerevisiae* a second gene encoding a formate dehydrogenase is active, i.e. the

*FDH2,* see e.g. Overkamp et al. (2002, *supra*). Another preferred gene encoding a formate dehydrogenase whose activity is to be reduced or inactivated in the cell of the invention therefore is an *S. cerevisiae FDH2* as described by Overkamp et al. (2002, *supra*), encoding the amino acid sequence of SEQ ID NO: 6 and orthologues thereof in other species. Therefore a gene encoding a formate dehydrogenase whose activity is to be reduced or inactivated in the cell of the invention preferably is a gene encoding a formate dehydrogenase having an amino acid sequence with at least 45, 50, 60, 65, 70, 75, 80, 85, 90, 95, 98 or 99% sequence identity to SEQ ID NO: 6 or a gene encoding a formate dehydrogenase having an amino acid sequence having one or several substitutions, insertions and/or deletions as compared to the amino acid sequence of SEQ ID NO: 6.

In a further embodiment, the activity of all the genes in the host cell encoding a formate dehydrogenase is reduced or inactivated. In such cells preferably all copies of endogenous genes encoding a formate dehydrogenase having an amino acid sequence with at least 45, 50, 60, 65, 70, 75, 80, 85, 90, 95, 98 or 99% sequence identity to SEQ ID NO: 5 or 6 (or having an amino acid sequence having one or several substitutions, insertions and/or deletions as compared to the amino acid sequences of SEQ ID NO: 5 or 6) are inactivated or at least reduced in expression.

In a preferred embodiment, the host cell of the invention further comprises an exogenous gene coding for a enzyme with the ability to reduce acetylCoA into acetaldehyde, which gene confers to the cell the ability to convert acetylCoA (and/or acetic acid) into ethanol. An enzyme with the ability to reduce acetylCoA into acetaldehyde is herein understood as an enzyme which catalyze the reaction (ACDH; EC 1.2.1.10):

$$\text{acetaldehyde} + NAD^+ + \text{Coenzyme A} \leftrightarrow \text{acetyl-Coenzyme A} + NADH + H^+.$$

Thus, the enzyme catalyzes the conversion of acetylCoA into acetaldehyde (and *vice versa*) and is also referred to as an (acetylating NAD-dependent) acetaldehyde dehydrogenase or an acetyl-CoA reductase. The enzyme may be a bifunctional enzyme which further catalyzes the conversion of acetaldehyde into ethanol (and *vice versa*; see below). For convenience we shall refer herein to an enzyme having at least the ability to reduce acetylCoA into either acetaldehyde or ethanol as an "acetaldehyde dehydrogenase". It is further understood herein that the host cell has endogenous alcohol dehydrogenase activities which allow the cell, being provided with acetaldehyde dehydrogenase activity, to complete the conversion of acetyl-CoA into ethanol. It is further also preferred that the host cell has endogenous acetyl-CoA synthetase which allow the cell, being provided with acetaldehyde dehydrogenase activity, to complete the conversion of acetic acid (via acetyl-CoA) into ethanol.

The exogenous gene may encode for a monofunctional enzyme having only acetaldehyde dehydrogenase activity (i.e. an enzyme only having the ability to reduce acetylCoA into acetaldehyde) such as e.g. the acetaldehyde dehydrogenase encoded by the *E.coli* mhpF gene. A suitable exogenous gene coding for an enzyme with acetaldehyde dehydrogenase activity comprises a nucleotide sequence coding for an amino acid sequence with at least 45, 50, 60, 65, 70, 75, 80, 85, 90, 95, 98, 99% amino acid sequence identity with SEQ ID NO: 7. Suitable examples of prokaryotes comprising monofunctional enzymes with acetaldehyde dehydrogenase activity are provided in Table 3. The amino acid sequences of these monofunctional enzymes are available in public databases and can be used by the skilled person to design codon-optimised nucleotide sequences coding for the corresponding monofunctional enzyme (see e.g. SEQ ID NO: 8). The exogenous gene coding for the monofunctional enzyme having only acetaldehyde dehydrogenase activity may also comprises a nucleotide sequence coding for an amino acid sequence having one or several substitutions, insertions and/or deletions as compared to SEQ ID NO: 7.

Table 3: Enzyme with acetaldehyde dehydrogenase activity related to *E.coli* mhpF

| **Organism** | **Amino acid identity (%)** |
|---|---|
| *Escherichia coli* str. K12 substr. MG1655 | 100% |
| *Shigella sonnei* | 100% |
| *Escherichia coli* IAI39 | 99% |
| *Citrobacter youngae* ATCC 29220 | 93% |
| *Citrobacter sp.* 30_2 | 92% |
| *Klebsiella pneumoniae* 342) | 87% |
| *Klebsiella variicola* | 87% |
| *Pseudomonas putida* | 81% |
| *Ralstonia eutropha* JMP134 | 82% |
| *Burkholderia sp.* H160 | 81% |

(continued)

| Organism | Amino acid identity (%) |
|---|---|
| *Azotobacter vinelandii* DJ | 79% |
| *Ralstonia metallidurans* CH34 | 70% |
| *Xanthobacter autotrophicus* Py2 | 67% |
| *Burkholderia cenocepacia* J2315 | 68% |
| *Frankia sp.* EAN1pec | 67% |
| *Polaromonas sp.* JS666 | 68% |
| *Burkholderia phytofirmans* PsJN | 70% |
| *Rhodococcus opacus* B4 | 64% |

Preferably, the host cell comprises an exogenous gene coding for a bifunctional enzyme with acetaldehyde dehydrogenase and alcohol dehydrogenase activity, which gene confers to the cell the ability to convert acetylCoA into ethanol. The advantage of using a bifunctional enzyme with acetaldehyde dehydrogenase and alcohol dehydrogenase activities as opposed to separate enzymes for each of the acetaldehyde dehydrogenase and alcohol dehydrogenase activities, is that it allows for direct channelling of the intermediate between enzymes that catalyze consecutive reactions in a pathway offers the possibility of an efficient, exclusive, and protected means of metabolite delivery. Substrate channelling thus decreases transit time of intermediates, prevents loss of intermediates by diffusion, protects labile intermediates from solvent, and forestalls entrance of intermediates into competing metabolic pathways. The bifunctional enzyme therefore allows for a more efficient conversion of acetylCoA into ethanol as compared to the separate acetaldehyde dehydrogenase and alcohol dehydrogenase enzymes. A further advantage of using the bifunctional enzyme is that it may also be used in host cells having little or no alcohol dehydrogenase activity under the condition used, such as e.g. anaerobic conditions and/or conditions of catabolite repression.

Bifunctional enzymes with acetaldehyde dehydrogenase and alcohol dehydrogenase activity are known in the art prokaryotes and protozoans, including e.g. the bifunctional enzymes encoded by the *Escherichia coli* adhE and *Entamoeba histolytica* ADH2 genes (see e.g. Bruchaus and Tannich, 1994, J. Biochem. 303: 743-748; Burdette and Zeikus, 1994, J. Biochem. 302: 163-170; Koo et al., 2005, Biotechnol. Lett. 27: 505-510; Yong et al., 1996, Proc Natl Acad Sci USA, 93: 6464-6469). Bifunctional enzymes with acetaldehyde dehydrogenase and alcohol dehydrogenase activity are larger proteins consisting of around 900 amino acids and they are bifunctional in that they exhibit both acetaldehyde dehydrogenase (ACDH; EC 1.2.1.10) and alcohol dehydrogenase activity (ADH; EC 1.1.1.1). The *E. coli* adhE and *Entamoeba histolytica* ADH2 show 45% amino acid identity. Therefore, in one embodiment of the invention, a suitable exogenous gene coding for a bifunctional enzyme with acetaldehyde dehydrogenase and alcohol dehydrogenase activity comprises a nucleotide sequence coding for an amino acid sequence with at least 45, 50, 60, 65, 70, 75, 80, 85, 90, 95, 98, 99% amino acid sequence identity with at least one of SEQ ID NO: 9 and 11. Suitable examples of prokaryotes comprising bifunctional enzymes with acetaldehyde dehydrogenase and alcohol dehydrogenase activity are provided in Tables 4 and 5. The amino acid sequences of these bifunctional enzymes are available in public databases and can be used by the skilled person to design codon-optimised nucleotide sequences coding for the corresponding bifunctional enzyme (see e.g. SEQ ID NO: 10 or 12). The exogenous gene coding for the a bifunctional enzyme with acetaldehyde dehydrogenase and alcohol dehydrogenase activity may also comprises a nucleotide sequence coding for an amino acid sequence having one or several substitutions, insertions and/or deletions as compared to at least one of SEQ ID NO: 9 and 11.

Table 4: Bifunctional enzymes with acetaldehyde dehydrogenase and alcohol dehydrogenase activity related to *E.coli* adhE

| Organism | Amino acid identity (%) |
|---|---|
| *Escherichia coli* O157:H7 str. Sakai | 100% |
| *Shigella sonnei* | 100% |
| *Shigella dysenteriae* 1012 | 99% |
| *Klebsiella pneumoniae 342* | 97% |
| *Enterobacter* sp. 638 | 94% |

(continued)

| Organism | Amino acid identity (%) |
|---|---|
| *Yersinia pestis biovar* Microtus str. 91001 | 90% |
| *Serratia proteamaculans* 568 | 90% |
| *Pectobacterium carotovorum* WPP14 | 90% |
| *Sodalis glossinidius* str. 'morsitans' | 87% |
| *Erwinia tasmaniensis* Et1/99 | 86% |
| *Aeromonas hydrophila* ATCC 7966 | 81% |
| *Vibrio vulnificus* YJ016] | 76% |

Table 5: Bifunctional enzymes with acetaldehyde dehydrogenase and alcohol dehydrogenase activity related to *Entamoeba histolytica* ADH2

| Organism | Amino acid identity (%) |
|---|---|
| *Entamoeba histolytica* HM-1:IMSS | 99% |
| *Entamoeba dispar* SAW760 | 98% |
| *Mollicutes bacterium* D7 | 65% |
| *Fusobacterium mortiferum* ATCC 9817 | 64% |
| *Actinobacillus succinogenes* 130Z | 63% |
| *Pasteurella multocida* Pm70 | 62% |
| *Mannheimia succiniciproducens* MBEL55E | 61% |
| *Streptococcus* sp. 2_1_36FAA] | 61% |

For expression of the nucleotide sequence encoding the bifunctional enzyme having acetaldehyde dehydrogenase and alcohol dehydrogenase activities, or the enzyme having acetaldehyde dehydrogenase activity, the nucleotide sequence (to be expressed) is placed in an expression construct wherein it is operably linked to suitable expression regulatory regions/sequences to ensure expression of the enzyme upon transformation of the expression construct into the host cell of the invention (see above). Suitable promoters for expression of the nucleotide sequence coding for the enzyme having the bifunctional enzyme having acetaldehyde dehydrogenase and alcohol dehydrogenase activities, or the enzyme having acetaldehyde dehydrogenase activity include promoters that are preferably insensitive to catabolite (glucose) repression, that are active under anaerobic conditions and/or that preferably do not require xylose or arabinose for induction. Examples of such promoters are given above.

Preferably, the nucleotide sequence encoding the bifunctional enzyme having acetaldehyde dehydrogenase and alcohol dehydrogenase activities, or the enzyme having acetaldehyde dehydrogenase activity is adapted to optimise its codon usage to that of the host cell in question (as described above).

The enzyme having acetaldehyde dehydrogenase and optionally alcohol dehydrogenase activities preferably is expressed in active form in the transformed host cell. Thus, expression of the nucleotide sequence in the host cell produces an acetaldehyde dehydrogenase with a specific activity of at least 0.005, 0.010, 0.020, 0.050 or 0.10 μmol $min^{-1}$ $(mg\ protein)^{-1}$, determined as acetyl-CoA dependent rate of NADH reduction in cell extracts of the transformed host cell at 30 °C as described in the Examples herein.

In a further embodiment, the host cell of the invention further comprises a genetic modification that increases at least one of: i) the specific activity of glycerol dehydrogenase; ii) the specific activity of dihydroxyacetone kinase; and, iii) transport of glycerol into the cell.

Preferably, the genetic modification that increases the specific activity of at least one of glycerol dehydrogenase and dihydroxyacetone kinase is overexpression of a nucleotide sequence encoding at least one of a glycerol dehydrogenase and dihydroxyacetone kinase. However, alternatively, the specific activity of the glycerol dehydrogenase and/or dihydroxyacetone kinase may be increased by expressing an enzyme having increased activity as compared to the endogenous wild type enzyme of the host cell, in addition to, or as a replacement for the wild type enzyme.

A glycerol dehydrogenase is herein understood as an enzyme that catalyzes the chemical reaction (EC 1.1.1.6):

glycerol + $NAD^+$ ↔ glycerone + NADH + $H^+$

Other names in common use include glycerin dehydrogenase, $NAD^+$-linked glycerol dehydrogenase and glycerol:NAD+ 2-oxidoreductase. Preferably the genetic modification causes overexpression of a glycerol dehydrogenase, e.g. by overexpression of a nucleotide sequence encoding a glycerol dehydrogenase. The nucleotide sequence encoding the glycerol dehydrogenase may be endogenous to the cell or may be a glycerol dehydrogenase that is heterologous to the cell. Nucleotide sequences that may be used for overexpression of glycerol dehydrogenase in the cells of the invention are e.g. the glycerol dehydrogenase gene from *S. cerevisiae* (*GCY1*) as e.g. described by Oechsner et al. (1988, FEBS Lett. 238: 123-128) or Voss et al. (1997, Yeast 13: 655-672). Preferably, the nucleotide sequence encoding the glycerol dehydrogenase comprises a nucleotide sequence coding for an amino acid sequence with at least 45, 50, 60, 65, 70, 75, 80, 85, 90, 95, 98, 99% amino acid sequence identity with SEQ ID NO: 13 or a nucleotide sequence coding for an amino acid sequence having one or several substitutions, insertions and/or deletions as compared to SEQ ID NO: 13. In a preferred embodiment a codon-optimised (see above) nucleotide sequence encoding the glycerol dehydrogenase is overexpressed, such as e.g. a codon optimised nucleotide sequence encoding the glycerol dehydrogenase of SEQ ID NO: 13.

The glycerol dehydrogenase encoded by the yeast *GCY1* gene however appears to be specific for the cofactor $NADP^+$ (EC 1.1.1.72) as opposed to $NAD^+$ (EC 1.1.1.6). The yeasts such *S. cerevisiae* appear to lack $NAD^+$-dependent glycerol dehydrogenase activity (EC 1.1.1.6) (see e.g. KEGG pathway 00561). More preferred nucleotide sequences for overexpression of a heterologous glycerol dehydrogenase in the cells of the invention are therefore e.g. sequences encoding bacterial glycerol dehydrogenases which use $NAD^+$ as cofactor (EC 1.1.1.6), such as e.g. the *gldA* gene from *E. coli* described by Truniger and Boos (1994, J Bacteriol. 176(6):1796-1800), the expression of which in yeast has already been reported (Lee and Dasilva, 2006, Metab Eng. 8(1):58-65). Preferably, the nucleotide sequence encoding a heterologous glycerol dehydrogenase comprises a nucleotide sequence coding for an amino acid sequence with at least 45, 50, 60, 65, 70, 75, 80, 85, 90, 95, 98, 99% amino acid sequence identity with SEQ ID NO: 49 or a nucleotide sequence coding for an amino acid sequence having one or several substitutions, insertions and/or deletions as compared to SEQ ID NO: 49. In a preferred embodiment a codon-optimised (see above) nucleotide sequence encoding the heterologous glycerol dehydrogenase is overexpressed, such as e.g. a codon-optimised nucleotide sequence encoding the amino acid sequence of the glycerol dehydrogenase of SEQ ID NO: 49. Such a codon-optimised nucleotide sequence is e.g. provided in SEQ ID NO: 50 (positions 10 - 1113; CAI = 0.976).

For overexpression of the nucleotide sequence encoding the glycerol dehydrogenase, the nucleotide sequence (to be overexpressed) can be placed in an expression construct wherein it is operably linked to suitable expression regulatory regions/sequences to ensure overexpression of the glycerol dehydrogenase enzyme upon transformation of the expression construct into the host cell of the invention (see above). Suitable promoters for (over)expression of the nucleotide sequence coding for the enzyme having glycerol dehydrogenase activity include promoters that are preferably insensitive to catabolite (glucose) repression, that are active under anaerobic conditions and/or that preferably do not require xylose or arabinose for induction. Examples of such promoters are given above. In the cells of the invention, a glycerol dehydrogenase to be overexpressed is preferably overexpressed by at least a factor 1.1, 1.2, 1.5, 2, 5, 10 or 20 as compared to a strain which is genetically identical except for the genetic modification causing the overexpression. Preferably, the glycerol dehydrogenase is overexpressed under anaerobic conditions by at least a factor 1.1, 1.2, 1.5, 2, 5, 10 or 20 as compared to a strain which is genetically identical except for the genetic modification causing the overexpression. It is to be understood that these levels of overexpression may apply to the steady state level of the enzyme's activity (specific activity in the cell), the steady state level of the enzyme's protein as well as to the steady state level of the transcript coding for the enzyme in the cell. Overexpression of the nucleotide sequence in the host cell produces a specific glycerol dehydrogenase activity of at least 0.2, 0.5, 1.0, 2.0, or 5.0 U $min^{-1}$ (mg protein)$^{-1}$, determined in cell extracts of the transformed host cells at 30 °C as described in the Examples herein.

A dihydroxyacetone kinase is herein understood as an enzyme that catalyzes the chemical reaction ((EC 2.7.1.29):

ATP + glycerone ↔ ADP + glycerone phosphate

Other names in common use include glycerone kinase, ATP:glycerone phosphotransferase and (phosphorylating) acetol kinase. It is understood that glycerone and dihydroxyacetone are the same molecule. Preferably the genetic modification causes overexpression of a dihydroxyacetone kinase, e.g. by overexpression of a nucleotide sequence encoding a dihydroxyacetone kinase. The nucleotide sequence encoding the dihydroxyacetone kinase may be endogenous to the cell or may be a dihydroxyacetone kinase that is heterologous to the cell. Nucleotide sequences that may be used for overexpression of dihydroxyacetone kinase in the cells of the invention are e.g. the dihydroxyacetone kinase genes from *S. cerevisiae* (*DAK1*) and (*DAK2*) as e.g. described by Molin et al. (2003, J. Biol. Chem.

278:1415-1423). Preferably, the nucleotide sequence encoding the dihydroxyacetone kinase comprises an amino acid sequence with at least 45, 50, 60, 65, 70, 75, 80, 85, 90, 95, 98, 99% amino acid sequence identity with at least one of SEQ ID NO's: 14 and 15 or a nucleotide sequence coding for an amino acid sequence having one or several substitutions, insertions and/or deletions as compared to at least one of SEQ ID NO's: 14 and 15. In a preferred embodiment a codon-optimised (see above) nucleotide sequence encoding the dihydroxyacetone kinase is overexpressed, such as e.g. a codon optimised nucleotide sequence encoding the dihydroxyacetone kinase of SEQ ID NO: 14 or a codon optimised nucleotide sequence encoding the dihydroxyacetone kinase of SEQ ID NO: 15. A preferred nucleotide sequence for overexpression of a dihydroxyacetone kinase is a nucleotide sequence encoding a dihydroxyacetone kinase comprises an amino acid sequence with at least 45, 50, 60, 65, 70, 75, 80, 85, 90, 95, 98, 99% amino acid sequence identity with SEQ ID NO's: 14 (*S. cerevisiae* (*DAK1*), having one or several substitutions, insertions and/or deletions as compared to SEQ ID NO: 14.

Nucleotide sequences that may be used for overexpression of a heterologous dihydroxyacetone kinase in the cells of the invention are e.g. sequences encoding bacterial dihydroxyacetone kinases such as the *dhaK* gene from *Citrobacter freundii* e.g. described by Daniel et al. (1995, J. Bacteriol. 177:4392-4401). Preferably, the nucleotide sequence encoding a heterologous dihydroxyacetone kinase comprises a nucleotide sequence coding for an amino acid sequence with at least 45, 50, 60, 65, 70, 75, 80, 85, 90, 95, 98, 99% amino acid sequence identity with SEQ ID NO: 52 or a nucleotide sequence coding for an amino acid sequence having one or several substitutions, insertions and/or deletions as compared to SEQ ID NO: 52. In a preferred embodiment a codon-optimised (see above) nucleotide sequence encoding the heterologous dihydroxyacetone kinase is overexpressed, such as e.g. a codon optimised nucleotide sequence encoding the amino acid sequence of the dihydroxyacetone kinase of SEQ ID NO: 52. Such a codon-optimised nucleotide sequence is e.g. provided in SEQ ID NO: 53 (positions 10 - 1668).

For overexpression of the nucleotide sequence encoding the dihydroxyacetone kinase, the nucleotide sequence (to be overexpressed) can be placed in an expression construct wherein it is operably linked to suitable expression regulatory regions/sequences to ensure overexpression of the dihydroxyacetone kinase enzyme upon transformation of the expression construct into the host cell of the invention (see above). Suitable promoters for (over)expression of the nucleotide sequence coding for the enzyme having dihydroxyacetone kinase activity include promoters that are preferably insensitive to catabolite (glucose) repression, that are active under anaerobic conditions and/or that preferably do not require xylose or arabinose for induction. Examples of such promoters are given above. In the cells of the invention, a dihydroxyacetone kinase to be overexpressed is preferably overexpressed by at least a factor 1.1, 1.2, 1.5, 2, 5, 10 or 20 as compared to a strain which is genetically identical except for the genetic modification causing the overexpression. Preferably, the dihydroxyacetone kinase is overexpressed under anaerobic conditions by at least a factor 1.1, 1.2, 1.5, 2, 5, 10 or 20 as compared to a strain which is genetically identical except for the genetic modification causing the overexpression. It is to be understood that these levels of overexpression may apply to the steady state level of the enzyme's activity (specific activity in the cell), the steady state level of the enzyme's protein as well as to the steady state level of the transcript coding for the enzyme in the cell. Overexpression of the nucleotide sequence in the host cell produces a specific dihydroxyacetone kinase activity of at least 0.002, 0.005, 0.01, 0.02 or 0.05 U $min^{-1}$ (mg $protein)^{-1}$, determined in cell extracts of the transformed host cells at 30 °C as described in the Examples herein.

Preferably, the genetic modification that increases transport of glycerol into the cell preferably is a genetic modification that causes overexpression of a nucleotide sequence encoding at least one of a glycerol uptake protein and a glycerol channel.

A glycerol uptake protein is herein understood as a multimembrane-spanning protein that belongs to the included in the membrane bound O-acyltransferases (MBOAT) superfamily including e.g. the *S. cerevisiae* glycerol uptake proteins encoded by the *GUP1* and *GUP2* genes. Preferably the genetic modification causes overexpression of a glycerol uptake protein, e.g. by overexpression of a nucleotide sequence encoding a glycerol uptake protein. The nucleotide sequence encoding the glycerol uptake protein may be endogenous to the cell or may be a glycerol uptake protein that is heterologous to the cell. Nucleotide sequences that may be used for overexpression of glycerol uptake protein in the cells of the invention are e.g. the glycerol uptake protein genes from *S. cerevisiae* (*GUP1*) and (*GUP2*) and orthologues thereof as e.g. described by Neves et al. (2004, FEMS Yeast Res. 5:51-62). Preferably, the nucleotide sequence encoding the glycerol uptake protein comprises a nucleotide sequence coding for an amino acid sequence with at least 45, 50, 60, 65, 70, 75, 80, 85, 90, 95, 98, 99% amino acid sequence identity with at least one of SEQ ID NO's: 16 (Gup1p) and 17 (Gup2p) or a nucleotide sequence coding for an amino acid sequence having one or several substitutions, insertions and/or deletions as compared to at least one of SEQ ID NO's: 16 and 17. In a preferred embodiment a codon-optimised (see above) nucleotide sequence encoding the glycerol uptake protein is overexpressed, such as e.g. a codon optimised nucleotide sequence encoding the glycerol uptake protein SEQ ID NO: 16 or a codon optimised nucleotide sequence encoding the glycerol uptake protein of SEQ ID NO: 17. Although the exact nature of the influence of GUP1 on glycerol transport is not yet clear, Yu et al. (2010, *supra*) have shown that overexpression of GUP1 in *S. cerevisiae* improves ethanol production on glycerol grown cells. A preferred nucleotide sequence for overexpression of a glycerol uptake protein is therefore a nucleotide sequence encoding a glycerol uptake protein that is capable of rescuing salt stress-associated phenotype of a S. *cerevisiae gup1Δ* mutant by complementation as described by Neves et al. (2004, *supra).* Such complementing orthologues of *S. cerevisiae GUP1* include nucleotide sequences encoding amino acid sequences having at least 60, 68, 72, 75, 80, 85, 90, 95, 98, 99% identity with the amino acid sequence of SEQ ID NO: 16 and may be obtained from yeast species belonging to the genera of *Saccharomyces, Zygosaccharomyces, Kluyveromyces, Candida, Pichia, Hansenula, Kloeckera, Schwanniomyces,* and *Yarrowia.*

A glycerol channel is herein understood as a member of the MIP family of channel proteins reviewed by Reizer et al. (1993, CRC Crit. Rev. Biochem. Mol. Biol., 28: 235-257), which channel proteins comprise a 250 -280 amino acid transmembrane domain consisting of six membrane-spanning domains and have at least 30, 35, 40, 45, 50, 60, 70, 80, 90, 95, 98 or 99 % amino acid identity, or at least 55, 60, 65, 70, 80, 90, 95, 98 or 99% amino acid similarity with the amino acid sequence between amino acids 250 and 530 of SEQ ID NO: 18, the S. *cerevisiae* FPS1 aquaglyceroporin. Alternatively the channel protein comprises a 250 -280 amino acid transmembrane domain consisting of six membrane-spanning domains, and having one or several substitutions, insertions and/or deletions as compared to the amino acid sequence between amino acids 250 and 530 of SEQ ID NO: 18.

Nucleotide sequences that may be used for overexpression of a glycerol channel in the cells of the invention include nucleotide sequences encoding the yeast aquaglyceroporin FPS1 gene from e.g. *S. cerevisiae* (Van Aelst et al., 1991, EMBO J. 10:2095-2104) and orthologues thereof from other yeasts including *Kluyveromyces lactis, Kluyveromyces marxianus* and *Zygosaccharomyces rouxii* as e.g. described by Neves et al. (2004, *supra).* However, the use of bacterial or plant glycerol channels is not excluded as e.g. Luyten et al. (1995, EMBO J. 14:1360-1371) have shown that the *E.coli* glycerol facilitator, having only 30% sequence identity with the amino acid sequence between amino acids 250 and 530 of the S. *cerevisiae* FPS1 aquaglyceroporin, can complement glycerol uptake in a S. *cerevisiae fps1Δ* mutant. The nucleotide sequence encoding the glycerol channel may be endogenous to the cell or may be a glycerol channel that is heterologous to the cell. In a preferred embodiment a codon-optimised (see above) nucleotide sequence encoding the glycerol channel is overexpressed, such as e.g. a codon optimised nucleotide sequence encoding the aquaglyceroporin of SEQ ID NO: 18.

For overexpression of the nucleotide sequence encoding the glycerol uptake protein and/or the glycerol channel protein, the nucleotide sequence (to be overexpressed) can be placed in an expression construct wherein it is operably linked to suitable expression regulatory regions/sequences to ensure overexpression of the glycerol uptake protein and/or the glycerol channel protein upon transformation of the expression construct into the host cell of the invention (see above). Suitable promoters for (over)expression of the nucleotide sequence coding for the glycerol uptake protein and/or the glycerol channel protein include promoters that are preferably insensitive to catabolite (glucose) repression, that are active under anaerobic conditions and/or that preferably do not require xylose or arabinose for induction. Examples of such promoters are given above. In the cells of the invention, a glycerol uptake protein and/or a glycerol channel protein to be overexpressed are preferably overexpressed by at least a factor 1.1, 1.2, 1.5, 2, 5, 10 or 20 as compared to a strain which is genetically identical except for the genetic modification causing the overexpression. Preferably, the glycerol uptake protein and/or the glycerol channel protein are overexpressed under anaerobic conditions by at least a factor 1.1, 1.2, 1.5, 2, 5, 10 or 20 as compared to a strain which is genetically identical except for the genetic modification causing the overexpression. It is to be understood that these levels of overexpression may apply to the steady state level of the enzyme's activity (specific activity in the cell), the steady state level of the enzyme's protein as well as to the steady state level of the transcript coding for the enzyme in the cell.

In a further embodiment, the host cell of the invention further comprises a genetic modification that increases the specific acetyl-CoA synthetase activity in the cell, preferably under anaerobic conditions as this activity is rate-limiting under these conditions. Acetyl-CoA synthetase or acetate-CoA ligase (EC 6.2.1.1) is herein understood as an enzyme that catalyzes the formation of a new chemical bond between acetate and coenzyme A (CoA). Preferably the genetic modification causes overexpression of a acetyl-CoA synthetase, e.g. by overexpression of a nucleotide sequence encoding a acetyl-CoA synthetase. The nucleotide sequence encoding the acetyl-CoA synthetase may be endogenous to the cell or may be a acetyl-CoA synthetase that is heterologous to the cell. Nucleotide sequences that may be used for overexpression of acetyl-CoA synthetase in the cells of the invention are e.g. the acetyl-CoA synthetase genes from *S. cerevisiae* (*ACS1* and *ACS2)* as e.g. described by de Jong-Gubbels et al. (1998, FEMS Microbiol Lett. 165: 15-20). Preferably, the nucleotide sequence encoding the acetyl-CoA synthetase comprises an amino acid sequence with at least 45, 50, 60, 65, 70, 75, 80, 85, 90, 95, 98, 99% amino acid sequence identity with at least one of SEQ ID NO's: 19 and 20, or a nucleotide sequence coding for an amino acid sequence having one or several substitutions, insertions and/or deletions as compared to at least one of SEQ ID NO's: 19 and 20.

In one embodiment, the nucleotide sequence that is overexpressed encodes an acetyl-CoA synthetase with a high affinity for acetate. Use of an acetyl-CoA synthetase with a high affinity for acetate is preferred for conditions under which there is a relatively low concentration of acetic acid in the culture medium, e.g. no more than 2 g acetic acid/L culture medium. An acetyl-CoA synthetase with a high affinity for acetate is herein defined as an acetyl-CoA synthetase with a higher affinity for acetate than the acetyl-CoA synthetase encoded by the *S. cerevisiae ACS2* (SEQ ID NO: 20). Preferably, an acetyl-CoA synthetase with a high affinity for acetate has a Km for acetate of no more than 10, 5, 2, 1,

0.5, 0.2 or 0.1 mM, such e.g. the acetyl-CoA synthetase encoded by the *S. cerevisiae ACS1* gene. More preferably a codon-optimised (see above) nucleotide sequence encoding the amino acid sequence of SEQ ID NO: 19 is overexpressed.

In another embodiment, the nucleotide sequence that is overexpressed encodes an acetyl-CoA synthetase with a high maximum rate ($v_{max}$). Use of an acetyl-CoA synthetase with a high maximum rate is preferred for condition under which there is a relatively high concentration of acetic acid in the culture medium, e.g. at least 2, 3, 4 or 5 g acetic acid/L culture medium. An acetyl-CoA synthetase with a high maximum rate is herein defined as an acetyl-CoA synthetase with a higher maximum rate than the acetyl-CoA synthetase encoded by the *S. cerevisiae ACS1.* Preferably, the acetyl-CoA synthetase with a high maximum rate is the acetyl-CoA synthetase encoded by the S. *cerevisiae ACS2* gene. More preferably a codon-optimised (see above) nucleotide sequence encoding the amino acid sequence of SEQ ID NO: 20 is overexpressed.

For overexpression of the nucleotide sequence encoding the acetyl-CoA synthetase (to be overexpressed) can be placed in an expression construct wherein it is operably linked to suitable expression regulatory regions/sequences to ensure overexpression of the acetyl-CoA synthetase enzyme upon transformation of the expression construct into the host cell of the invention (see above). Suitable promoters for (over)expression of the nucleotide sequence coding for the enzyme having acetyl-CoA synthetase activity include promoters that are preferably insensitive to catabolite (glucose) repression, that are active under anaerobic conditions and/or that preferably do not require xylose or arabinose for induction. Examples of such promoters are given above. In the cells of the invention, an acetyl-CoA synthetase to be overexpressed is overexpressed by at least a factor 1.1, 1.2, 1.5, 2, 5, 10 or 20 as compared to a strain which is genetically identical except for the genetic modification causing the overexpression. Preferably, the acetyl-CoA synthetase is overexpressed under anaerobic conditions by at least a factor 2, 5, 10, 20, 50, or 100 as compared to a strain which is genetically identical except for the genetic modification causing the overexpression. It is to be understood that these levels of overexpression may apply to the steady state level of the enzyme's activity (specific activity), the steady state level of the enzyme's protein as well as to the steady state level of the transcript coding for the enzyme.

In a further embodiment, the host cell of the invention further comprises a genetic modification that reduces specific $NAD^+$-dependent glycerol 3-phosphate dehydrogenase activity in the cell. Glycerol 3-phosphate dehydrogenase or glycerolphosphate dehydrogenase (EC 1.1.1.8) katalyses the reduction of dihydroxyacetone phosphate to sn-glycerol 3-phosphate while oxidising NADH to $NAD^+$. In the cells of the invention, the specific glycerolphosphate dehydrogenase activity is preferably reduced by at least a factor 0.8, 0.5, 0.3, 0.1, 0.05 or 0.01 as compared to a strain which is genetically identical except for the genetic modification causing the overexpression, preferably under anaerobic conditions.

Preferably, glycerolphosphate dehydrogenase activity is reduced in the host cell by one or more genetic modifications that reduce the expression of or inactivates a gene encoding an glycerolphosphate dehydrogenase. Preferably, the genetic modifications reduce or inactivate the expression of each endogenous copy of the gene encoding a specific glycerolphosphate dehydrogenase in the cell's genome. A given cell may comprise multiple copies of the gene encoding a specific glycerolphosphate dehydrogenase with one and the same amino acid sequence as a result of di-, poly- or aneu-ploidy. In such instances preferably the expression of each copy of the specific gene that encodes the glycerolphosphate dehydrogenase is reduced or inactivated. Alternatively, a cell may contain several different (iso)enzymes with glycerolphosphate dehydrogenase activity that differ in amino acid sequence and that are each encoded by a different gene. In such instances, in some embodiments of the invention it is preferred that only certain types of the isoenzymes are reduced or inactivated while other types remain unaffected (see below). Preferably, the gene is inactivated by deletion of at least part of the gene or by disruption of the gene, whereby in this context the term gene also includes any non-coding sequence up- or down-stream of the coding sequence, the (partial) deletion or inactivation of which results in a reduction of expression of glycerolphosphate dehydrogenase activity in the host cell.

A preferred gene encoding a glycerolphosphate dehydrogenase whose activity is to be reduced or inactivated in the cell of the invention is the *S. cerevisiae GPD2* gene as described by Eriksson et al. (1995, Mol. Microbiol. 17: 95-107), encoding the amino acid sequence of SEQ ID NO: 21 and orthologues thereof in other species. Therefore a gene encoding a glycerolphosphate dehydrogenase whose activity is to be reduced or inactivated in the cell of the invention preferably is a gene encoding a glycerolphosphate dehydrogenase having an amino acid sequence with at least 70, 75, 80, 85, 90, 95, 98 or 99% sequence identity to SEQ ID NO: 21 or an amino acid sequence having one or several substitutions, insertions and/or deletions as compared to SEQ ID NO: 21.

In a preferred embodiment of the invention, the host cell of the invention comprises a functional high-osmolarity glycerol response pathway. Preferably therefore, only the activity of the gene(s) encoding a glycerolphosphate dehydrogenase having an amino acid sequence with at least 70% sequence identity to SEQ ID NO: 21 are reduced or inactivated, while at least one endogenous gene encoding a glycerolphosphate dehydrogenase having an amino acid sequence with at least 70, 75, 80, 85, 90, 95, 98 or 99% sequence identity to SEQ ID NO: 22 is functional. SEQ ID NO: 22 depicts the amino acid sequence encoded by the *S. cerevisiae GPD1* gene as described by Albertyn et al. (1994, Mol. Cell. Biol. 14: 4135-4144), which has 69% amino acid identity with the *S. cerevisiae GPD2* glycerolphosphate dehydrogenase. The *S. cerevisiae GPD1* gene is the stress-induced glycerolphosphate dehydrogenase of *S. cerevisiae,*

which is important for growth under osmotic stress as may occur under industrial fermentations conditions. Its expression is *inter alia* regulated by the high-osmolarity glycerol response pathway. It is therefore advantageous that a host cell of the invention has at least one functional copy of a endogenous gene encoding a glycerolphosphate dehydrogenase having an amino acid sequence with at least 70, 75, 80, 85, 90, 95, 98 or 99% sequence identity to SEQ ID NO: 22 or an amino acid sequence having one or several substitutions, insertions and/or deletions as compared to SEQ ID NO: 22.

In a further embodiment, the activity of all the genes in the host cell encoding a glycerolphosphate dehydrogenase is reduced or inactivated. In such cells preferably all copies of endogenous genes encoding a glycerolphosphate dehydrogenase having an amino acid sequence with at least 70, 75, 80, 85, 90, 95, 98 or 99% sequence identity to SEQ ID NO: 21 or 22 (or having an amino acid sequence having one or several substitutions, insertions and/or deletions as compared to at least one of SEQ ID NO's: 21 and 22) are inactivated or at least reduced in expression.

In a further preferred embodiment, the host cell of the invention has at least one of: a) the ability of isomerising xylose to xylulose; and, b) the ability to convert L-arabinose into D-xylulose 5-phosphate. For a) the cell preferably has a functional exogenous xylose isomerase gene, which gene confers to the cell the ability to isomerise xylose into xylulose. For b) the cell preferably has functional exogenous genes coding for a L-arabinose isomerase, a L-ribulokinase and a L-ribulose-5-phosphate 4-epimerase, which genes together confers to the cell the ability to isomerise convert L-arabinose into D-xylulose 5-phosphate.

Fungal host cells having the ability of isomerising xylose to xylulose as e.g. described in WO 03/0624430 and in WO 06/009434. The ability of isomerising xylose to xylulose is preferably conferred to the cell by transformation with a nucleic acid construct comprising a nucleotide sequence encoding a xylose isomerase. Preferably the cell thus acquires the ability to directly isomerise xylose into xylulose. More preferably the cell thus acquires the ability to grow aerobically and/or anaerobically on xylose as sole energy and/or carbon source though direct isomerisation of xylose into xylulose (and further metabolism of xylulose). It is herein understood that the direct isomerisation of xylose into xylulose occurs in a single reaction catalysed by a xylose isomerase, as opposed to the two step conversion of xylose into xylulose via a xylitol intermediate as catalysed by xylose reductase and xylitol dehydrogenase, respectively.

Several xylose isomerases (and their amino acid and coding nucleotide sequences) that may be successfully used to confer to the cell of the invention the ability to directly isomerise xylose into xylulose have been described in the art. These include the xylose isomerases of *Piromyces* sp. and of other anaerobic fungi that belongs to the families *Neocallimastix, Caecomyces, Piromyces* or *Ruminomyces* (WO 03/0624430), *Cyllamyces aberensis* (US 20060234364), *Orpinomyces* (Madhavan et al., 2008, DOI 10.1007/s00253-008-1794-6), the xylose isomerase of the bacterial genus *Bacteroides,* including e.g. *B. thetaiotaomicron* (WO 06/009434), *B. fragilis,* and *B. uniformis* (WO 09/109633), the xylose isomerase of the anaerobic bacterium *Clostridium phytofermentans* (Brat et al., 2009, Appl. Environ. Microbiol. 75: 2304-2311), and the xylose isomerases of *Clostridium difficile, Ciona intestinales* and *Fusobacterium mortiferum* (WO 10/074577).

Fungal host cells having the ability to convert L-arabinose into D-xylulose 5-phosphate as e.g. described in Wisselink et al. (2007, AEM Accepts, published online ahead of print on 1 June 2007; Appl. Environ. Microbiol. doi:10.1128/AEM.00177-07) and in EP 1 499 708. The ability of to converting L-arabinose into D-xylulose 5-phosphate is preferably conferred to the cell by transformation with a nucleic acid construct(s) comprising nucleotide sequences encoding a) an arabinose isomerase; b) a ribulokinase, preferably a L-ribulokinase a xylose isomerase; and c) a ribulose-5-P-4-epimerase, preferably a L-ribulose-5-P-4-epimerase. Preferably, in the cells of the invention, the ability to convert L-arabinose into D-xylulose 5-phosphate is the ability to convert L-arabinose into D-xylulose 5-phosphate through the subsequent reactions of 1) isomerisation of arabinose into ribulose; 2) phosphorylation of ribulose to ribulose 5-phosphate; and, 3) epimerisation of ribulose 5-phosphate into D-xylulose 5-phosphate. Suitable nucleotide sequences encoding arabinose isomerases, a ribulokinases and ribulose-5-P-4-epimerases may be obtained from *Bacillus subtilis, Escherichia coli* (see e.g. EP 1 499 708), *Lactobacilli,* e.g. *Lactobacillus plantarum* (see e.g. Wisselink et al. *supra),* or species of *Clavibacter, Arthrobacter* and *Gramella,* of which preferably *Clavibacter michiganensis, Arthrobacter aurescens* and *Gramella forsetii* (see WO2009/011591).

The transformed host cell of the invention further preferably comprises xylulose kinase activity so that xylulose isomerised from xylose may be metabolised to pyruvate. Preferably, the cell contains endogenous xylulose kinase activity. More preferably, a cell of the invention comprises a genetic modification that increases the specific xylulose kinase activity. Preferably the genetic modification causes overexpression of a xylulose kinase, e.g. by overexpression of a nucleotide sequence encoding a xylulose kinase. The gene encoding the xylulose kinase may be endogenous to the cell or may be a xylulose kinase that is heterologous to the cell. A nucleotide sequence that may be used for overexpression of xylulose kinase in the cells of the invention is e.g. the xylulose kinase gene from *S. cerevisiae (XKS1)* as described by Deng and Ho (1990, Appl. Biochem. Biotechnol. 24-25: 193-199). Another preferred xylulose kinase is a xylose kinase that is related to the xylulose kinase from *Piromyces (xylB;* see WO 03/0624430). This *Piromyces* xylulose kinase is actually more related to prokaryotic kinase than to all of the known eukaryotic kinases such as the yeast kinase. The eukaryotic xylulose kinases have been indicated as non-specific sugar kinases, which have a broad substrate range that includes xylulose. In contrast, the prokaryotic xylulose kinases, to which the *Piromyces* kinase is

most closely related, have been indicated to be more specific kinases for xylulose, i.e. having a narrower substrate range. In the cells of the invention, a xylulose kinase to be overexpressed is overexpressed by at least a factor 1.1, 1.2, 1.5, 2, 5, 10 or 20 as compared to a strain which is genetically identical except for the genetic modification causing the overexpression. It is to be understood that these levels of overexpression may apply to the steady state level of the enzyme's activity, the steady state level of the enzyme's protein as well as to the steady state level of the transcript coding for the enzyme.

A cell of the invention further preferably comprises a genetic modification that increases the flux of the pentose phosphate pathway as described in WO 06/009434. In particular, the genetic modification causes an increased flux of the non-oxidative part pentose phosphate pathway. A genetic modification that causes an increased flux of the non-oxidative part of the pentose phosphate pathway is herein understood to mean a modification that increases the flux by at least a factor 1.1, 1.2, 1.5, 2, 5, 10 or 20 as compared to the flux in a strain which is genetically identical except for the genetic modification causing the increased flux. The flux of the non-oxidative part of the pentose phosphate pathway may be measured as described in WO 06/009434.

Genetic modifications that increase the flux of the pentose phosphate pathway may be introduced in the cells of the invention in various ways. These including e.g. achieving higher steady state activity levels of xylulose kinase and/or one or more of the enzymes of the non-oxidative part pentose phosphate pathway and/or a reduced steady state level of unspecific aldose reductase activity. These changes in steady state activity levels may be effected by selection of mutants (spontaneous or induced by chemicals or radiation) and/or by recombinant DNA technology e.g. by overexpression or inactivation, respectively, of genes encoding the enzymes or factors regulating these genes.

In a preferred cell of the invention, the genetic modification comprises overexpression of at least one enzyme of the (non-oxidative part) pentose phosphate pathway. Preferably the enzyme is selected from the group consisting of the enzymes encoding for ribulose-5-phosphate isomerase, ribulose-5-phosphate 3-epimerase, transketolase and transaldolase. Various combinations of enzymes of the (non-oxidative part) pentose phosphate pathway may be overexpressed. E.g. the enzymes that are overexpressed may be at least the enzymes ribulose-5-phosphate isomerase and ribulose-5-phosphate 3-epimerase; or at least the enzymes ribulose-5-phosphate isomerase and transketolase; or at least the enzymes ribulose-5-phosphate isomerase and transaldolase; or at least the enzymes ribulose-5-phosphate 3-epimerase and transketolase; or at least the enzymes ribulose-5-phosphate 3-epimerase and transaldolase; or at least the enzymes transketolase and transaldolase; or at least the enzymes ribulose-5-phosphate 3-epimerase, transketolase and transaldolase; or at least the enzymes ribulose-5-phosphate isomerase, transketolase and transaldolase; or at least the enzymes ribulose-5-phosphate isomerase, ribulose-5-phosphate 3-epimerase, and transaldolase; or at least the enzymes ribulose-5-phosphate isomerase, ribulose-5-phosphate 3-epimerase, and transketolase. In one embodiment of the invention each of the enzymes ribulose-5-phosphate isomerase, ribulose-5-phosphate 3-epimerase, transketolase and transaldolase are overexpressed in the cell of the invention. Preferred is a cell in which the genetic modification comprises at least overexpression of the enzyme transaldolase. More preferred is a cell in which the genetic modification comprises at least overexpression of both the enzymes transketolase and transaldolase as such a host cell is already capable of anaerobic growth on xylose. In fact, under some conditions we have found that cells overexpressing only the transketolase and the transaldolase already have the same anaerobic growth rate on xylose as do cells that overexpress all four of the enzymes, i.e. the ribulose-5-phosphate isomerase, ribulose-5-phosphate 3-epimerase, transketolase and transaldolase. Moreover, cells of the invention overexpressing both of the enzymes ribulose-5-phosphate isomerase and ribulose-5-phosphate 3-epimerase are preferred over cells overexpressing only the isomerase or only the 3-epimerase as overexpression of only one of these enzymes may produce metabolic imbalances.

There are various means available in the art for overexpression of enzymes in the host cells of the invention. In particular, an enzyme may be overexpressed by increasing the copynumber of the gene coding for the enzyme in the cell, e.g. by integrating additional copies of the gene in the cell's genome, by expressing the gene from an episomal multicopy expression vector or by introducing a episomal expression vector that comprises multiple copies of the gene. The coding sequence used for overexpression of the enzymes preferably is homologous to the host cell of the invention. However, coding sequences that are heterologous to the host cell of the invention may likewise be applied. Alternatively overexpression of enzymes in the cells of the invention may be achieved by using a promoter that is not native to the sequence coding for the enzyme to be overexpressed, i.e. a promoter that is heterologous to the coding sequence to which it is operably linked. Although the promoter preferably is heterologous to the coding sequence to which it is operably linked, it is also preferred that the promoter is homologous, i.e. endogenous to the cell of the invention. Preferably the heterologous promoter is capable of producing a higher steady state level of the transcript comprising the coding sequence (or is capable of producing more transcript molecules, i.e. mRNA molecules, per unit of time) than is the promoter that is native to the coding sequence, preferably under conditions where one or more of xylose, arabinose and glucose are available as carbon sources, more preferably as major carbon sources (i.e. more than 50% of the available carbon source consists of one or more of xylose, arabinose and glucose), most preferably as sole carbon sources.

A further preferred cell of the invention comprises a genetic modification that reduces unspecific aldose reductase activity in the cell. Preferably, unspecific aldose reductase activity is reduced in the host cell by one or more genetic

modifications that reduce the expression of or inactivates a gene encoding an unspecific aldose reductase. Preferably, the genetic modifications reduce or inactivate the expression of each endogenous copy of a gene encoding an unspecific aldose reductase that is capable of reducing an aldopentose, including, xylose, xylulose and arabinose, in the cell's genome. A given cell may comprise multiple copies of genes encoding unspecific aldose reductases as a result of di-, poly- or aneu-ploidy, and/or a cell may contain several different (iso)enzymes with aldose reductase activity that differ in amino acid sequence and that are each encoded by a different gene. Also in such instances preferably the expression of each gene that encodes an unspecific aldose reductase is reduced or inactivated. Preferably, the gene is inactivated by deletion of at least part of the gene or by disruption of the gene, whereby in this context the term gene also includes any non-coding sequence up- or down-stream of the coding sequence, the (partial) deletion or inactivation of which results in a reduction of expression of unspecific aldose reductase activity in the host cell. A nucleotide sequence encoding an aldose reductase whose activity is to be reduced in the cell of the invention and amino acid sequences of such aldose reductases are described in WO 06/009434 and include e.g. the (unspecific) aldose reductase genes of *S. cerevisiae GRE3* gene (Träff et al., 2001, Appl. Environm. Microbiol. 67: 5668-5674) and orthologues thereof in other species.

A further preferred transformed host cell according to the invention may comprises further genetic modifications that result in one or more of the characteristics selected from the group consisting of (a) increased transport of xylose and/or arabinose into the cell; (b) decreased sensitivity to catabolite repression; (c) increased tolerance to ethanol, osmolarity or organic acids; and, (d) reduced production of by-products. By-products are understood to mean carbon-containing molecules other than the desired fermentation product and include e.g. xylitol, arabinitol, glycerol and/or acetic acid. Any genetic modification described herein may be introduced by classical mutagenesis and screening and/or selection for the desired mutant, or simply by screening and/or selection for the spontaneous mutants with the desired characteristics. Alternatively, the genetic modifications may consist of overexpression of endogenous genes and/or the inactivation of endogenous genes. Genes the overexpression of which is desired for increased transport of arabinose and/or xylose into the cell are preferably chosen form genes encoding a hexose or pentose transporter. In S. *cerevisiae* and other yeasts these genes include *HXT1, HXT2, HXT3, HXT4, HXT5*, *HXT7* and *GAL2,* of which *HXT7, HXT5* and *GAL2* are most preferred (see Sedlack and Ho, Yeast 2004; 21: 671-684). Another preferred transporter for expression in yeast is the glucose transporter encoded by the *P. stipitis SUT1* gene (Katahira et al., 2008, Enzyme Microb. Technol. 43: 115-119). Similarly orthologues of these transporter genes in other species may be overexpressed. Other genes that may be overexpressed in the cells of the invention include genes coding for glycolytic enzymes and/or ethanologenic enzymes such as alcohol dehydrogenases. Preferred endogenous genes for inactivation include hexose kinase genes e.g. the *S. cerevisiae HXK2* gene (see Diderich et al., 2001, Appl. Environ. Microbiol. 67: 1587-1593); the *S. cerevisiae MIG1* or *MIG2* genes; genes coding for enzymes involved in glycerol metabolism such as the *S. cerevisiae* glycerolphosphate dehydrogenase 1 and/or 2 genes; or (hybridising) orthologues of these genes in other species. Other preferred further modifications of host cells for xylose fermentation are described in van Maris et al. (2006, Antonie van Leeuwenhoek 90:391-418), WO2006/009434, WO2005/023998, WO2005/111214, and WO2005/091733. Any of the genetic modifications of the cells of the invention as described herein are, in as far as possible, preferably introduced or modified by self cloning genetic modification.

A preferred host cell according to the invention has the ability to grow on at least one of xylose and arabinose as carbon/energy source, preferably as sole carbon/energy source, and preferably under anaerobic conditions, i.e. conditions as defined herein below for anaerobic fermentation process. Preferably, when grown on xylose as carbon/energy source the host cell produces essentially no xylitol, e.g. the xylitol produced is below the detection limit or e.g. less than 5, 2, 1, 0.5, or 0.3% of the carbon consumed on a molar basis. Preferably, when grown on arabinose as carbon/energy source, the cell produces essentially no arabinitol, e.g. the arabinitol produced is below the detection limit or e.g. less than 5, 2, 1, 0.5, or 0.3 % of the carbon consumed on a molar basis.

A preferred host cell of the invention has the ability to grow on at least one of a hexose, a pentose, glycerol, acetic acid and combinations thereof at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under anaerobic conditions. Therefore, preferably the host cell has the ability to grow on at least one of xylose and arabinose as sole carbon/energy source at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under anaerobic conditions. More preferably, the host cell has the ability to grow on a mixture of a hexose (e.g. glucose) and at least one of xylose and arabinose (in a 1:1 weight ratio) as sole carbon/energy source at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under anaerobic conditions. Most preferably, the host cell has the ability to grow on a mixture of a hexose (e.g. glucose), at least one of xylose and arabinose and glycerol (in a 1:1:1 weight ratio) as sole carbon/energy source at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under anaerobic conditions.

In a one aspect, the invention relates to the use of a yeast cell according to the invention for the preparation of a fermentation product selected from the group consisting of ethanol, lactic acid, 3-hydroxy-propionic acid, acrylic

acid, 1,3-propanediol, butanols and isoprenoid-derived products.

In another aspect the invention relates to a process for producing a fermentation product selected from the group consisting of ethanol, lactic acid, 3-hydroxy-propionic acid, acrylic acid, 1,3-propane-diol, butanols (1-butanol, 2-butanol, isobutanol) and isoprenoid-derived products. The process can be a process wherein formate is produced in addition to the fermentation product. The process preferably comprises the step of: a) fermenting a medium with a yeast cell, whereby the medium contains or is fed with: a) a source of at least one of a hexose, a pentose and glycerol and whereby the yeast cell ferments the at least one of a hexose, pentose and glycerol to the fermentation product, and optionally to formate. The yeast cell preferably is a (host) cell as herein defined above. The process preferably comprise one or more further steps wherein the fermentation product and/or the formate are recovered. The process may be a batch process, a fed-batch process or a continuous process as are well known in the art.

In a preferred process the source of at least one of a hexose, a pentose and glycerol comprises or consist of: hexose and pentose; hexose and glycerol; pentose and glycerol; hexose, pentose and glycerol. In a further preferred process, the source of at least one of a hexose, a pentose and glycerol further comprises acetic acid.

In a preferred process the source of hexose comprises or consists of glucose. Preferably the source of pentose comprises or consists of at least one of xylose and arabinose, of which xylose is preferred. Preferably, the medium fermented by the cells of the invention comprises or is fed with (fractions of) hydrolysed biomass comprising at least one at least one of a hexose and a pentose such as glucose, xylose and/or arabinose. The (fractions of) hydrolysed biomass comprising the hexoses and pentose will usually also comprise acetic acid (or a salt thereof). An example of hydrolysed biomass to be fermented in the processes of the invention is e.g. hydrolysed lignocellulosic biomass. Lignocellulosic biomass is herein understood as plant biomass that is composed of cellulose, hemicellulose, and lignin. The carbohydrate polymers (cellulose and hemicelluloses) are tightly bound to the lignin. Examples of lignocellulosic biomass to be hydrolysed for use in the present invention include agricultural residues (including e.g. empty fruit bunches (EFB) of oil palm, corn stover and sugarcane bagasse), wood residues (including sawmill and paper mill discards and (municipal) paper waste. Methods for hydrolysis of biomass such as lignocelluloses are known in the art per se and include e.g. acids, such as sulphuric acid and enzymes such as cellulases and hemicellulases.

In the process of the invention, the sources of xylose, glucose and arabinose may be xylose, glucose and arabinose as such (i.e. as monomeric sugars) or they may be in the form of any carbohydrate oligo- or polymer comprising xylose, glucose and/or arabinose units, such as e.g. lignocellulose, arabinans, xylans, cellulose, starch and the like. For release of xylose, glucose and/or arabinose units from such carbohydrates, appropriate carbohydrases (such as arabinases, xylanases, glucanases, amylases, cellulases, glucanases and the like) may be added to the fermentation medium or may be produced by the modified host cell. In the latter case the modified host cell may be genetically engineered to produce and excrete such carbohydrases. An additional advantage of using oligo- or polymeric sources of glucose is that it enables to maintain a low(er) concentration of free glucose during the fermentation, e.g. by using rate-limiting amounts of the carbohydrases preferably during the fermentation. This, in turn, will prevent repression of systems required for metabolism and transport of non-glucose sugars such as xylose and arabinose. In a preferred process the modified host cell ferments both the glucose and at least one of xylose and arabinose, preferably simultaneously in which case preferably a modified host cell is used which is insensitive to glucose repression to prevent diauxic growth. In addition to a source of at least one of a hexose, a pentose and glycerol, as carbon source, the fermentation medium will further comprise the appropriate ingredients required for growth of the modified host cell. Compositions of fermentation media for growth of eukaryotic microorganisms such as yeasts are well known in the art.

In the process of the invention, the medium further preferably comprises and/or is fed a source of glycerol. Glycerol for use in the process of the present invention may advantageously be glycerol that is generated as a by-product in biodiesel production from transesterification reactions using vegetable oils, such as palm oil, or animal fats and an alcohol.

The fermentation process may be an aerobic or an anaerobic fermentation process. An anaerobic fermentation process is herein defined as a fermentation process run in the absence of oxygen or in which substantially no oxygen is consumed, preferably less than 5, 2.5 or 1 mmol/L/h, more preferably 0 mmol/L/h is consumed (i.e. oxygen consumption is not detectable), and wherein organic molecules serve as both electron donor and electron acceptors. In the absence of oxygen, NADH produced in glycolysis and biomass formation, cannot be oxidised by oxidative phosphorylation. To solve this problem many microorganisms use pyruvate or one of its derivatives as an electron and hydrogen acceptor thereby regenerating $NAD^+$. Thus, in a preferred anaerobic fermentation process pyruvate is used as an electron (and hydrogen acceptor) and is reduced to fermentation products such as ethanol, as well as non-ethanol fermentation products such as lactic acid, 3-hydroxy-propionic acid, acrylic acid, 1,3-propane-diol, butanols (1-butanol, 2-butanol, isobutanol) and isoprenoid-derived products, preferably under concomitant production of formate. Anaerobic processes of the invention are preferred over aerobic processes because anaerobic processes do not require investments and energy for aeration and in addition, anaerobic processes produce higher product yields than aerobic processes. Alternatively, the fermentation process of the invention may be run under aerobic oxygen-limited conditions. Preferably, in an aerobic process under oxygen-limited conditions, the rate of oxygen consumption is at least 5.5, more preferably at

least 6 and even more preferably at least 7 mmol/L/h.

The fermentation process is preferably run at a temperature that is optimal for the modified cells of the invention. Thus, for most yeasts cells, the fermentation process is performed at a temperature which is less than 42°C, preferably less than 38°C. For yeast cells, the fermentation process is preferably performed at a temperature which is lower than 35, 33, 30 or 28°C and at a temperature which is higher than 20, 22, or 25°C.

Because undissociated formic acid is more toxic to the host cells of the invention as compared to the dissociated acid ion, the fermentation process is preferably run at a pH that is higher than the pKa of formic acid, which is 3.75. The fermentation process is therefore preferably run at a pH of at least 3.8, 4.0, 4.5, 5.0, 5.5, 6.0, 6.5, 7.0, 7.5 or 8.0. Preferably the fermentation process is run at at least the aforementioned pHs for the entire duration of the process. Alternatively the pH may be regulated so as to maintain the concentration of undissociated formic acid below a given concentration. Preferably the pH of the medium is regulated during the fermentation process to maintain a concentration of undissociated formic acid that is not higher than 50.0, 30.0, 20.0, 18.1, 15.0, 10.0, 5.0, 2.0 or 1.0 mM.

A preferred fermentation process according to the invention is a process for the production of ethanol and optionally formate, whereby the process comprises the step of fermenting a medium with a yeast cell, whereby the medium contains or is fed with a source of at least one of a hexose, a pentose and glycerol and whereby the yeast cell ferments the at least one of a hexose, pentose and glycerol to ethanol and optionally formate. Optionally the process comprises the step of recovery of at least one of ethanol and optionally formate. The fermentation may further be performed as described above. In the process the volumetric ethanol productivity is preferably at least 0.5, 1.0, 1.5, 2.0, 2.5, 3.0, 5.0 or 10.0 g ethanol per litre per hour. The ethanol yield on hexose and/or pentose and/or glycerol (and/or acetate) in the process preferably is at least 50, 60, 70, 80, 90, 95 or 98%. The ethanol yield is herein defined as a percentage of the theoretical maximum yield, which, for xylose, glucose and arabinose is 0.51 g. ethanol per g. hexose or pentose. For glycerol the theoretical maximum yield is 0.50 g. ethanol per g. glycerol and for acetic acid the theoretical maximum yield is 0.77 g. ethanol per g. acetic acid.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

## Examples

### 1. Enzyme activity assays

Cell extracts for activity assays were prepared from exponentially growing aerobic or anaerobic batch cultures and analysed for protein content as described by Abbot et al., (2009, Appl. Environ. Microbiol. 75: 2320-2325).

$NAD^+$-dependent acetaldehyde dehydrogenase (EC 1.2.1.10) activity was measured at 30 °C by monitoring the oxidation of NADH at 340 nm. The reaction mixture (total volume 1 ml) contained 50 mM potassium phosphate buffer (pH 7.5), 0.15 mM NADH and cell extract. The reaction was started by addition of 0.5 mM acetyl-Coenzyme A.

For glycerol 3-phosphate dehydrogenase (EC 1.1.1.8) activity determination, cell extracts were prepared as described above except that the phosphate buffer was replaced by triethanolamine buffer (10 mM, pH 5). Glycerol-3-phosphate dehydrogenase activities were assayed in cell extracts at 30 °C as described previously (Blomberg and Adler, 1989, J. Bacteriol. 171: 1087-1092.9). Reaction rates were proportional to the amounts of cell extract added.

Acetyl-CoA synthase (EC 6.2.1.1) activity was measured as described by Frenkel and Kitchens (1977, J. Biol. Chem. 252: 504-507) which is a modification of the method of Webster (Webster, 1969, Methods Enzymol. 13: 375-381). NADH formation measured is spectrophotometrically when the acetyl-CoA produced is coupled with citrate synthase and malate dehydrogenase reactions. The assay system contained 100 mM Tris-Cl(pH 7.6), 10 mM $MgCl_2$, 6 mM ATP, 5 mM malate, 1 mM $NAD^+$, 0.1 mM NADH, 2.5 mM dithiothreitol or 2-mercaptoethanol, 0.2 mM coenzyme A, 25 μg citrate synthase (80 units/mg), 20 μg malate dehydrogenase (1000 units/mg), and 10 mM acetate and the reaction was measured rate was measured at 340 nm and calculated from the extinction coefficient of NADH (6.22 x $10^6$ $cm^2$/mol).

The activity of glycerol dehydrogenase and dihydroxyacetone kinase are measured at 30 °C in cell extracts, essentially as previously described (Gonzalez et al., 2008, Metab. Eng. 10, 234-245). Enzyme activities of glycerol dehydrogenase and dihydroxyacetone kinase are reported as μmoles of substrate/min/mg of cell protein.

2. Strain construction

All modifications start with the xylose fermenting strain RN1041. RN1041 is a CEN.PK based strain with the following genotype:

*Mat a, ura3-52, leu2-112, his3::loxP, gre3::loxP, loxP-Ptpi::TAL1, loxP-Ptpi::RK11, loxP-Ptpi-TKL1, loxP-Ptpi-RPE1, delta::Padh1XKS1Tcyc1-LEU2, delta::URA3-Ptpi-xylA-Tcyc1*
*Mat a* = mating type a
*ura3-52, leu2-112, his3::loxP* mutations in the genes ura3, leu2 and his3, the ura3 is complemented by the *Piromyces* xylA overexpression construct, leu2 is complemented by the XKS1 overexpression construct. his3 could be used for selection of additional plasmids, RN1041 needs histidine in the medium for growth.
*gre3::loxP* = deletion of the gre3 gene encoding xylose reductase, loxP site is left after marker removal.
*loxP-Ptpi* .....= overexpression of het pentose phosphate pathway, loxP site upstream of constitutive promoter is left after marker removal
*delta::* = integration of the construct after recombination on the long terminal repeats of the Ty1 retrotransposon.

Constructs for expression of the *E.coli pflA* and pflB genes in yeast

The *E.coli* pflA and pflB genes were obtained as codon optimized synthetic genes.
The pflA expression construct by ligating a TEF1 promoter-fragment (cut with the restriction enzymes *Sal*I and *Hin*dIII; from plasmid collection Royal Nedalco), a pflA synthetic ORF (cut with *Hin*dIII and *Bss*HII) and an ADH1 terminator-fragment (cut with *Bss*HII and *Bsi*WI; from plasmid collection Royal Nedalco) together into pCRII blunt (Invitrogen) to yield pRN613.
The pflB expression construct by ligating a PGK1 promoter-fragment (cut with the restriction enzymes *Spe*I and *Pst*I; from plasmid collection Royal Nedalco), a pflB synthetic ORF (cut with *Pst*I and *Sal*I) and an PGI1 terminator-fragment (cut with *XhoI* and *BsiWI;* from plasmid collection Royal Nedalco) together into pCRII blunt (Invitrogen) to yield pRN614.
The LEU2 marker from plasmid pRS305 (Sikorski and Hieter, 1989, Genetics 122:19-27) is exchanged for the ZeoMX marker (*zeoMX* = resistance to phleomycin due to expression of the Tn5 ble gene with a TEF1 promoter and terminator sequence of *Ashbya gossypii*). Yeast transformants are selected on media containing Zeomycin, pRS305 is cut with *Bst*BI and *Bsr*GI and a ZeoMX fragment (from plasmid collection Royal Nedalco) is cut with the same restriction enzymes. Ligation gives plasmid pRS30z.
A 2µORI is introduced by cutting pRS30z with *Afl*II en *Mfe*I and cutting a 2µ fragment (from plasmid collection Royal Nedalco) with *Afl*II and *Eco*RI*.* The fragments are ligated to yield pRN615 (6341bp)
For the final construction pRN615 is cut with *Xho*I and *Spe*I*,* this vector is combined with the inserts from pRN613 cut with *Sal*I and *Bsi*WI (1510bp) and pRN614cut with *Spe*I and *Bsi*WI (3381bp) to produce pRN616 (SEQ ID NO: 31).
For expression of the *E.coli* pflA gene only, pRN613 is cut with *Sal*I and *Bsi*WI and this fragment is ligated into pRN615 cut with *Acc*65I and *Xho*I to produce pRN619 (SEQ ID NO: 32).
For expression of the *E.coli* pflB gene only, pRN614 is cut with *Xho*I and *Bsi*WI and this fragment is ligated into pRN615 cut with *Acc*65I and *Xho*I to produce pRN620 (SEQ ID NO: 33).
Plasmid pRN616, pRN619 and pRN620 are used to transform the yeast strain RN1041 together with the other plasmids as indicated in Table 7 to generate the strains as indicated in Table 7.

Constructs for overexpression of GCY1, gldA, DAK1 dhaK, and GUP1

GCY1

PCR is performed on genomic DNA of *S. cerevisiae* with primers introducing a *PstI* site 5' of the ATG and an *Afl*II site 3' of the TAA to produce the fragment of SEQ ID NO: 25. A DNA fragment comprising the *S. cerevisiae* Actin1 promoter is ligated upstream of the GCY1 ORF and DNA fragment comprising the *S. cerevisiae* ADH1 terminator fragment is ligated downstream of the GCY1 ORF.

*gldA*

The construct for expression in yeast of the *E.coli gldA* was made by ligating a yeast ACT1 promoter fragment (cut with the restriction enzymes *SpeI* and *PstI),* a synthetic ORF (SEQ ID NO:50), encoding the *E.coli gldA,* (cut with *PstI en BssHII)* and a yeast CYC1 terminator fragment (cut with *BssHII and BsiWI)* together into pCRII blunt (Invitrogen)

to yield pRNgldA (SEQ ID NO:51).

DAK1

PCR is performed on genomic DNA *of S. cerevisiae* with primers introducing a *XbaI* site 5' of the ATG and a *Sal*I site 3' of the TAA to produce the fragment as contained in SEQ ID NO: 26. A DNA fragment comprising the *S. cerevisiae* TPI1 promoter is ligated upstream of the DAK1 ORF and DNA fragment comprising the *S. cerevisiae* PGI1 terminator fragment is ligated downstream of the DAK1 ORF to produce the expression construct of SEQ ID NO: 26.

*dhaK*

The construct for expression in yeast of the *Citrobacter freundii dhaK* was made by ligating the yeast TPI1 promoter fragment (cut with the restriction enzymes *XhoI* and *Xba*I), a synthetic ORF (SEQ ID NO:53), encoding the *C. freundii dhaK,* (cut with *Xba*I and *Sal*I) and a yeast PGI1 terminator fragment (cut with *XhoI* and *Bsi*WI) together into pCRII blunt (Invitrogen) to yield pRNdhaK (SEQ ID NO:54).

GUP1

PCR is performed on genomic DNA of *S. cerevisiae* with primers introducing a *Hind*III site 5' of the ATG and a *Bam*HI site 3' of the TAA to produce the fragment as contained in SEQ ID NO: 27. A DNA fragment comprising the *S. cerevisiae* TDH3 promoter is ligated upstream of the GUP1 ORF and DNA fragment comprising the *S. cerevisiae* CYC1 terminator fragment is ligated downstream of the GUP1 ORF to produce the expression construct of SEQ ID NO: 27.

Strains overexpressing GCY1, DAK1 and GUP1

The above-described expression constructs of GCY1, DAK1 and GUP1 are combined into a pRS315-based centromeric plasmid having the hygromycine resistance gene as selectable marker to produce pRN605 (SEQ ID NO: 29). Plasmid pRN605 is used to transform the yeast strain RN1041 together with the other plasmids as indicated in Table 7 to generate the strains as indicated in Table 7.

The above-described expression constructs of GCY1 and DAK1 are combined into a pRS315-based centromeric plasmid having the hygromycine resistance gene as selectable marker to produce pRN608 (SEQ ID NO: 30). is used to transform the yeast strain RN1041 together with the other plasmids as indicated in Table 7 to generate the strain RN1098, as indicated in Table 7.

Strains expressing *E.coli* gldA with or without expression of *C.freundii* dhaK

The *E.coli* gldA expression construct is cut from plasmid pRNgldA with the restriction enzymes *SpeI* and *Bsi*WI. The *C. freundii* dhaK expression construct is cut from plasmid pRNdhaK with the restriction enzymes *Bsi*WI and *Xho*I. These fragments are ligated into plasmid pRN595 cut with the restriction enzymes *Spe*I and *Sal*I to yield pRN957 (SEQ ID NO: 55). Plasmid pRN958 (SEQ ID NO: 56) is subsequently made from plasmid pRN957 by deleting the *C.freundii* dhaK expression construct. To this end plasmid pRN957 is cut with the restriction enzymes PspOMI and *Avr*II, the single stranded parts are filled in with the DNA polymerase phusion (Finnzymes) and the plasmid is religated.

The expression constructs of DAK1 and GUP1 are combined into a pRS315-based centromeric plasmid having the hygromycine resistance gene as selectable marker to produce pRN607 (SEQ ID NO: 57).

Plasmids pRN957, pRN958 and pRN607 used to transform yeast together with the other plasmids indicated in Table 7 to produce strain RN1194, RN1195 and RN1196.

Construct for overexpression of FPS1

PCR is performed on genomic DNA of *S. cerevisiae* with primers introducing a *NsiI* site 5' of the ATG and a *Bam*HI site 3' of the TAA to produce the fragment as contained in SEQ ID NO: 28. A DNA fragment comprising the *S. cerevisiae* ADH1(medium) promoter is ligated upstream of the FSP1 ORF and DNA fragment comprising the *S. cerevisiae* CYC1 terminator fragment is ligated downstream of the FSP1 ORF to produce the expression construct of SEQ ID NO: 28. Ligation of these fragments in pCRII blunt produces pRN617. The insert of pRN617 is cut with *Bsi*WI and *Xho*I and cloned in pRN616 cut with *Bsr*GI and *Sal*I to produce pRN618 (SEQ ID NO: 34). Plasmid pRN618 is used to transform the yeast strain RN1041 together with the other plasmids as indicated in Table 7 to generate the strains as indicated in Table 7.

Constructs for deletion of FDH1 and FDH2

Primers FDHuf, FDHur, FDHdf and FDHdr are used for amplification of genomic sequences fragments upstream and downstream of both FDH1 and FDH2 genes for their inactivation. The same primers are used for both FDH1 and FDH2 alleles.

For inactivation of the FDH1 allele, a 423bp upstream PCR fragment with a *BspEI* site at the 3'-end for ligation to the MX marker, and a *BbsI* site at the 5'-end for isolation of the deletion construct is amplified using FDHuf and FDHur and cloned in pCRII (blunt) using topo-cloning (Invitrogen), which results in an additional *Bst*BI site at the 5'-end.
FDHuf: TCGAAGACTCCGAATGAAAAAGACATGCCAG (SEQ ID NO: 35)
FDHur: TCCGGATACCAAGTTCATTTTCAATACACCCCA (SEQ ID NO: 36)

A 378bp downstream PCR fragment with a *Nsi*I and a *Sph*I site at the 5'-end for ligation to the MX marker, and a *Bbs*I site at the 3'-end for isolation of the deletion construct is amplified using FDHdf and FDHdr.
FDHdf: ATGCATGCAGAATGGTTCTTATGCCAC (SEQ ID NO: 37)
FDHdr: GAAGACAGTTCTGTTATTAACGACGAGCCA (SEQ ID NO: 38)

For the final construction the downstream fragment cut with *Xho*I and *Nsi*I is ligated to the patMX marker (collection Royal Nedalco) cut with *BspE*I and *Nsi*I in the plasmid containing the upstream-fragment cut with *BspE*I and *Xho*I. The final plasmid, pRN621 (SEQ ID NO: 39), is cut with *Bbs*I prior to yeast transformation. Transformants are selected for phosphinotricin resistance. Correct integration of the disruption fragment is verified by PCR with specific primers.

FDH2 is found as a pseudogene in some strains (e.g. S288C, YPL275W and YPL276W). Two mutations restore the ORF homology with FDH1 (T436C and del A476). Sequence analysis shows that in the CEN.PK background (RN1041) FDH2 is one ORF homologous to FDH1.

For the final construction the downstream fragment cut with *Xho*I and *Sph*I is ligated to the kanMX marker (collection Royal Nedalco) cut with *BspE*I and *Sph*I into the plasmid containing the upstream fragment cut with *Bsp*EI and *Xho*I. The final plasmid, pRN622 (SEQ ID NO: 40), is cut with *Bbs*I prior to yeast transformation. Transformants are selected for G418 resistance. Correct integration of the disruption fragment is verified by PCR with specific primers.

Disruption fragments from pRN621 and pRN622 are used to transform the yeast strain RN1041 together with the other plasmids as indicated in Table 7 to generate the strains as indicated in Table 7.

Constructs for expression of the *E.coli* mhpF, adhE or the *Entamoebe histolytica* adh2 genes in yeast

For expression of the *E.coli* mhpF gene, a yeast PGK1 promoter fragment *(Spe*I-*Pst*I*)* and an ADH1 terminator fragment *(Afl*II-*Not*I*)* (both from the Nedalco plasmid collection) were ligated onto the codon-optimized synthetic fragment encoding the *E.coli* mhpF (SEQ ID NO: 8).

pRS 303 with 2μ ori (= pRN347, Royal Nedalco plasmid collection) was cut with *Spe*I and *Not*I and the mhpF expression construct was cloned into this vector to produce pRN558 (SEQ ID NO: 41). pRN558 is used to transform the yeast strain RN1041 (selection on medium lacking histidine) together with the other plasmids as indicated in Table 7 to generate the strains as indicated in Table 7. Overexpression of mhpF is verified by qPCR.

For expression of the *E.coli* adhE gene, a codon optimized synthetic fragment encoding the *E.coli* adhE (SEQ ID NO: 10) is cut with *Xba*I and *Afl*II and ligated into pRN558 cut with *Xba*I and *Afl*II (replacing the *E.coli* mhpF gene in pRN558) to produce pRN595 (SEQ ID NO: 42), pRN595 is used to transform the yeast strain RN1041 (selection on medium lacking histidine) together with the other plasmids as indicated in Table 7 to generate the strains as indicated in Table 7. Overexpression of adhE is verified by qPCR.

For expression of the *Entamoebe histolytica* adh2, a codon optimized synthetic fragment encoding the *E. histolytica* adh2 (SEQ ID NO: 12) is cut *with Xba*I and *Afl*II and ligated into pRN558 cut with *Xba*I and *Afl*II (replacing the *E.coli* mhpF gene in pRN558) to produce pRN596 (SEQ ID NO: 43). pRN596 is used to transform the yeast strain RN1041 (selection on medium lacking histidine) together with the other plasmids as indicated in Table 7 to generate the strains as indicated in Table 7. Overexpression of adh2 is verified by qPCR.

Constructs for deletion of GPD2

Primers GPD2uf, GPD2ur, GPD2df and GPD2dr are used for amplification of genomic sequences fragments upstream and downstream of the GPD2 gene for its inactivation. A 407bp upstream PCR fragment with an *Afl*II site at the 3'-end (derived from the GPD2 sequence) and a *Bgl*II site at the 5'-end (for isolation of the deletion construct) is amplified using GPD2uf, GPD2ur and cloned in pCR2.1 (topo T/A, Invitrogen).
GPD2uf: GGTACCAGATCTTTTGCGGCGAGGTGCCG (SEQ ID NO: 44)
GPD2ur: TCTAGACTTAAGGAATGTGTATCTTGTTAATCTTCTGACAGC (SEQ ID NO: 45)

A 417bp downstream PCR fragment with a *Xho*I site at the 5'-end and a *Bgl*II site at the 3'-end is amplified

using GPD2df and GPD2dr.
GPD2df: CTCGAGATAGTCTACAACAACGTCCGCA (SEQ ID NO: 46)
GPD2dr: CCATGGAGATCTGCAGTGAAAAAGCTCGAAGAAACAGCT (SEQ ID NO: 47)

For the final construction the plasmid containing the upstream fragment is cut with *Afl*II and *Kpn,* the downstream fragment is cut with *Xho*I en *Nco*I and the natMX marker (plasmid collection Royal Nedalco) is cut *with Afl*II en *Xho*I and the fragments are ligated to produce plasmid pRN594 (SEQ ID NO: 48). pRN594 is cut with *Bgl*II prior to yeast transformation. pRN594 is used to transform the yeast strain RN1041 (selection for nourseotricin resistance) together with the other plasmids as indicated in Table 7 to generate the strains as indicated in Table 7. Correct integration is verified by PCR using specific primers.

3. Fermentations with the constructed strains

3.1 Experimental set-up

Strains RN1041, RN1088, RN1089, RN1090, RN1091, RN1092, RN1093, RN1094, RN1095, RN1096, RN1097, RN1098, RN1099, RN1194, RN1195 and RN1196. are all tested for their ability to grow under both anoxic conditions (no oxygen present) at the expense of glycerol as carbon source. At the same time, formation of ethanol and formic acid is monitored. In several instances it is confirmed by experiments that the glycerol-consuming strains also retain their ability to ferment glucose and xylose. Precultures of strains are prepared by inoculating a frozen glycerol stock culture of the yeast in an YP (Yeast extract Peptone) medium with addition of the sugar glucose (2.5% w/v) at 32°C and pH 5.5. After 24 h incubation, this culture is used to inoculate the fermenter cultures. Cells are harvested by centrifugation and washed with cold $dH_2O$. Yeast inoculation used is 2 gram dry matter yeast per litre of fermentation medium.

Fermentations are performed as chemostat cultivation at 32 °C in 1-liter fermenters with a working volume of 800 ml. The dilution rate is set at 0.1 $h^{-1}$. These anoxic batch fermentations are performed in YP medium to which glycerol in combination with xylose and/or glucose is added. The culture pH is kept at pH = 7.0 by automatic addition of 2M KOH. Cultures were stirred at 300 rpm.

Samples for analysis of glucose, xylose, ethanol, glycerol and formic acid production are taken at steady state situations which are obtained after 5 volume changes of the fermentation vessel. Ethanol, glycerol and formic acid concentrations are monitored by HPLC analysis. Glucose and xylose are determined by HPAEC (Dionex) analysis.

3.2 Results

Strain RN1041 (Tables 6 and 7) is a strain that is derived from strain RN1001 (RN1001 his3::loxP). Strain RN1001 has been described previously. If either glucose (25 mM) or xylose (25 mM) are added to the YP medium, then the anoxic fermentations under the conditions described above, leads to a full consumption of either glucose or xylose by this strain. The concentrations of ethanol in the glucose and xylose media, respectively, are 40 mM and 33 mM at steady state. Small amounts of glycerol are formed both for glucose and xylose (1.8 and 1.5 mM, respectively). No formic acid formation is observed. In the absence of sugars in the YP medium, no ethanol nor glycerol or formic acid is formed. If glycerol was added to the medium at 50 mM concentration, then the organism did not produce additional ethanol or formic acid as compared to the situations with glucose or xylose alone.

Strain RN1088 was derived from strain RN1041 by

1. upregulating DAK1, GCY1 and GUP1 allowing for enhanced uptake and consumption of glycerol under anoxic conditions
2. heterologous expression of pflA + pflB allowing for the conversion of pyruvate into formic acid and acetylCoA
3. heterologous expression of adhE2 allowing the conversion of acetylCoA into ethanol
4. deleting FDH1 and FDH2 preventing the oxidation of formic acid by this strain.

The organism is tested for growth and product formation in the same way as RN1041 as described above. With either glucose or xylose, similar results are obtained as for RN1041 in terms of ethanol production. However, the formation of glycerol was slightly lower and traces of formic acid are observed. If glycerol is added to either the xylose- or glucose-containing medium at 50 mM concentration, then the organism does not consume more glycerol or produce additional ethanol and furthermore no additional formic acid is produced. Strains RN1089 and RN1090 have a similar genetic background as RN1088. The only difference is that adhE is not expressed in these organisms. Instead adh2 or mhpF are expressed. The characteristics of these strains during glycerol fermentations are similar to the characteristics of strain RN1088. Both strains showed similar characteristics as RN1088 for both glucose or xylose fermentation as strain RN1088.

Strains RN1091, RN1092 and RN1093 have the same genetic background as strain RN1088 with the exception of formic acid dehydrogenases. These strains have either 1 or 2 genes still present encoding for a formate dehydrogenase. Neither of the 3 organisms is able to perform good fermentations on glycerol. Only traces of either ethanol and formic acid were seen during fermentation experiments. The organisms do ferment both glucose or xylose at rates comparable to strain RN1088.

Strains RN1094, RN1095 and RN1096 have the same genetic background as strain RN1088 with the exception of the expression of either pflA or pflB. These strains lack either both these genes or either of the genes. Neither of the 3 organisms is able to perform good fermentations on glycerol. No additional ethanol or formic acid are seen as based on the addition of 50 mM glycerol to xylose-containing medium (25 mM) during fermentation experiments. The organisms do ferment both glucose or xylose at rates comparable to strain RN1088.

Strain RN1097 is derived from strain RN1088 by deleting GPD2. In doing so, a partial operation of a futile cycle might be prevented at the level of glycerol consumption/production. With glucose or xylose, the ethanol concentration at steady states are 38mM and 31 mM, respectively. Glycerol accumulates at 0.9mM and 0.5mM, respectively and formic acid had accumulates at 1.0 mM and 0.8 mM respectively. If glycerol is added to either the xylose- or glucose-containing medium at 50 mM concentration, then the organism does not consume more glycerol or produce additional ethanol and furthermore no additional formic acid is produced.

Strains RN1098 and RN1099 resemble strain RN1088 but differ genetically at the level of the glycerol transporter(s). Strain RN1098 has FPS1 overexpressed instead of GUP1, whereas strain RN1099 has both transporter genes expressed. The organisms are tested for growth and product formation in the same way as RN1041 as described above. With either glucose or xylose, similar results are obtained as for RN1041 in terms of ethanol production. However, the formation of glycerol was slightly lower and traces of formic acid were observed. If glycerol was added to either the xylose- or glucose-containing medium at 50 mM concentration, then the RN1098 and RN1099 organisms did not consume more glycerol or produce additional ethanol and furthermore no additional formic acid was produced.

RN1194 is tested for growth and product formation in the same way as RN1041 as described above. With either glucose or xylose, similar results are obtained as for RN1041 in terms of ethanol production. However, the formation of glycerol is slightly lower and traces of formic acid were observed. If glycerol is added to either the xylose- or glucose-containing medium at 50 mM concentration, then the organism produces additional ethanol and furthermore formic acid is produced. The concentration of ethanol at steady state in the glycerol/glucose medium is 75 mM and the formic acid concentration is 32 mM. The remaining glycerol level is 7 mM. The concentration of ethanol at steady state in the glycerol/xylose medium is 66 mM and the formic acid concentration is 30 mM. The remaining glycerol level is 8 mM. No attempts are made to test for any other fermentation products that might have been produced on the basis of glycerol. RN1196 produces similar results as RN1194 although glycerol consumption and production of ethanol and formic acid are somewhat reduced compared to RN 1194.

Strain RN1195 is derived from strain RN1194 by deleting GPD2. In doing so, a partial operation of a futile cycle might be prevented at the level of glycerol consumption/production. With glucose or xylose, the ethanol concentration at steady states are 38mM and 31 mM, respectively. Glycerol accumulates at 0.9mM and 0.5mM, respectively and formic acid accumulates at 1.0 mM and 0.8 mM, respectively. If glycerol is added to the xylose-medium (25 mM) at 50 mM concentration, then the organism additionally produces both ethanol and formic acid. The final concentration of ethanol at steady state was 72 mM, for glycerol 2 mM, and for formic acid 37 mM. No attempts were made to test for any other fermentation products that might have been produced on the basis of glycerol. These results clearly indicate that deletion of the GPD2 gene has a beneficial effect on the fermentation of glycerol in strain RN1195 as compared to strain RN 1194.

Table 6

| Gene | GPD2 | adhE | ade2 | mphF | DAK1 | GCY1 | GUP1 | FPS1 | FDH1 | FDH2 | pflA+pflB | E.cgldA | C.f. dhaK |
|---|---|---|---|---|---|---|---|---|---|---|---|---|---|
| Strain / marker | natMX | HIS3 | HIS3 | HIS3 | hphMX | hphMX | hphMX | zeoMX | patMX | kanMX | zeoMX | HIS3 | HIS3 |
| RN1041 | wt | absent | absent | absent | wt | wt | wt | wt | wt | wt | absent/absent | absent | absent |
| RN1088 | wt | expr | absent | absent | up | up | up | wt | del | del | expr/expr | absent | absent |
| RN1089 | wt | absent | expr | absent | up | up | up | wt | del | del | expr/expr | absent | absent |
| RN1090 | wt | absent | absent | expr | up | up | up | wt | del | del | expr/expr | absent | absent |
| RN1091 | wt | expr | absent | absent | up | up | up | wt | wt | wt | expr/expr | absent | absent |
| RN1092 | wt | expr | absent | absent | up | up | up | wt | del | wt | expr/expr | absent | absent |
| RN1093 | wt | expr | absent | absent | up | up | up | wt | wt | del | expr/expr | absent | absent |
| RN1094 | wt | expr | absent | absent | up | up | up | wt | del | del | absent/absent | absent | absent |
| RN1095 | wt | expr | absent | absent | up | up | up | wt | del | del | expr/absent | absent | absent |
| RN1096 | wt | expr | absent | absent | up | up | up | wt | del | del | absent/expr | absent | absent |
| RN1097 | del | expr | absent | absent | up | up | up | wt | del | del | expr/expr | absent | absent |
| RN1098 | wt | expr | absent | absent | up | up | wt | up | del | del | expr/expr | absent | absent |
| RN1099 | wt | expr | absent | absent | up | up | up | up | del | del | expr/expr | absent | absent |
| RN1194 | wt | expr | absent | absent | wt | wt | wt | wt | del | del | expr/expr | expr | expr |
| RN1195 | del | expr | absent | absent | wt | wt | wt | wt | del | del | expr/expr | expr | expr |
| RN1196 | wt | expr | absent | absent | up | wt | up | wt | del | del | expr/expr | expr | absent |

Table 7

| | plasmids | | | | | |
|---|---|---|---|---|---|---|
| selection: | HIS3 | hph | loxPNatMXlox P | nat | kan | zeo |
| Strain | | | | | | |
| RN1041 | pRN347 | | | | | |
| RN1088 | pRN595 | pRN605 | | pRN621 | pRN622 | pRN616 |
| RN1089 | pRN596 | pRN605 | | pRN621 | pRN622 | pRN616 |
| RN1090 | pRN558 | pRN605 | | pRN621 | pRN622 | pRN616 |
| RN1091 | pRN595 | pRN605 | | | | pRN616 |
| RN1092 | pRN595 | pRN605 | | pRN621 | | pRN616 |
| RN1093 | pRN595 | pRN605 | | | pRN622 | pRN616 |
| RN1094 | pRN595 | pRN605 | | pRN621 | pRN622 | |
| RN1095 | pRN595 | pRN605 | | pRN621 | pRN622 | pRN619 |
| RN1096 | pRN595 | pRN605 | | pRN621 | pRN622 | pRN620 |
| RN1097 | pRN595 | pRN605 | pRN594 | pRN621 | pRN622 | pRN616 |
| RN1098 | pRN595 | pRN608 | | pRN621 | pRN622 | pRN618 |
| RN1099 | pRN595 | pRN605 | | pRN621 | pRN622 | pRN618 |
| RN1194 | pRN957 | | | pRN621 | pRN622 | pRN616 |
| RN1195 | pRN957 | | pRN594 | pRN621 | pRN622 | pRN616 |
| RN1196 | pRN958 | pRN607 | | pRN621 | pRN622 | pRN616 |
| pRN347 = pRS303+2μORI | | | | | | |

## Claims

**1.** A yeast cell comprising:

a) an exogenous gene coding for a enzyme with the ability to convert pyruvate and coenzyme-A into formate and acetyl-CoA;
b) a genetic modification that reduces specific $NAD^+$-dependent formate dehydrogenase activity in the cell;
c) an exogenous gene coding for an enzyme with acetaldehyde dehydrogenase activity, which gene confers to the cell the ability to reduce acetyl-CoA into acetaldehyde; and,
d) a genetic modification that increases the specific activity of $NAD^+$-linked glycerol dehydrogenase.

**2.** A yeast cell according to claim 1, wherein the cell further comprises a genetic modification selected from the group consisting of:

a) a genetic modification that increases the specific activity of dihydroxyacetone kinase;
b) a genetic modification that increases transport of glycerol into the cell;
an exogenous gene coding for an enzyme for activation of the pyruvate formate lyase;
c) a genetic modification that increases the specific acetyl-CoA synthetase activity in the cell; and,
d) a genetic modification that reduces specific $NAD^+$-dependent glycerol 3-phosphate dehydrogenase activity in the cell, while the yeast cell comprises a functional high-osmolarity glycerol response pathway.

**3.** A yeast cell according to claim 1 or 2, wherein the cell comprises at least one of:

i) an exogenous xylose isomerase gene, which gene confers to the cell the ability to isomerise xylose into xylulose; and,
ii) an exogenous genes coding for a L-arabinose isomerase, a L-ribulokinase and a L-ribulose-5-phosphate 4-epimerase, which genes together confer to the cell the ability to convert L-arabinose into D-xylulose 5-phosphate.

**4.** A yeast cell according to any one of claims 1 - 3, wherein at least one of:

a) the genetic modification that reduces specific $NAD^+$-dependent formate dehydrogenase activity in the cell comprises the inactivation of at least one endogenous copy of a gene encoding a formate dehydrogenase in the cell's genome;
b) the genetic modification that increases the specific activity of the $NAD^+$-linked glycerol dehydrogenase is expression of a heterologous gene encoding an $NAD^+$-linked glycerol dehydrogenase;
c) the genetic modification that increases transport of glycerol into the cell is overexpression of a nucleotide sequence encoding at least one of a glycerol uptake protein and a glycerol channel; and,
d) the genetic modification that reduces specific $NAD^+$-dependent glycerol 3-phosphate dehydrogenase activity in the cell comprises the inactivation of at least one endogenous copy of a gene encoding a glycerol 3-phosphate dehydrogenase in the cell's genome and,
e) the genetic modification that increases the specific acetyl-CoA synthetase activity is overexpression of a nucleotide sequence encoding an acetyl-CoA synthetase; and,
f) the genetic modification that increases the specific activity of dihydroxyacetone kinase is overexpression of a nucleotide sequence encoding a dihydroxyacetone kinase.

**5.** A yeast cell according to any one of claims 1 - 4, wherein:

a) the exogenous gene coding for an enzyme with pyruvate formate lyase activity comprises a nucleotide sequence coding for an amino acid sequence with at least 57% amino acid sequence identity with SEQ ID NO: 1;
b) the exogenous gene coding for the pyruvate formate lyase activating enzyme comprises a nucleotide sequence coding for an amino acid sequence with at least 70% amino acid sequence identity with SEQ ID NO: 3;
c) the gene encoding a formate dehydrogenase whose activity is to be reduced or inactivated in the cell of the invention is a gene encoding a formate dehydrogenase having an amino acid sequence with at least 70% sequence identity to at least one of SEQ ID NO: 5 and 6;
d) the exogenous gene coding for the enzyme with acetaldehyde dehydrogenase activity comprises a nucleotide sequence coding for an amino acid sequence with at least one of:

i) at least 64% amino acid sequence identity with SEQ ID NO: 7,
ii) at least 76% amino acid sequence identity with SEQ ID NO: 9 and
iii) at least 61% amino acid sequence identity with SEQ ID NO: 11;

e) the nucleotide sequence encoding the $NAD^+$-linked glycerol dehydrogenase comprises a nucleotide sequence

coding for an amino acid sequence with at least 50% amino acid sequence identity with SEQ ID NO: 49,
f) the nucleotide sequence encoding the dihydroxyacetone kinase comprises a nucleotide sequence coding for an amino acid sequence with at least 50% amino acid sequence identity with at least one of SEQ ID NO: 14, 15 and 52;
g) the nucleotide sequence encoding the glycerol uptake protein comprises a nucleotide sequence coding for an amino acid sequence with at least 50% amino acid sequence identity with at least one of SEQ ID NO: 16 and 17, and wherein the nucleotide sequence encoding the glycerol channel comprises a nucleotide sequence coding for an amino acid sequence with at least 30% amino acid sequence identity with the amino acid sequence between amino acids 250 and 530 of SEQ ID NO: 18;
h) the nucleotide sequence encoding the acetyl-CoA synthetase comprises an amino acid sequence with at least 70% amino acid sequence identity with at least one of SEQ ID NO's: 19 and 20; and,
i) the gene encoding a glycerolphosphate dehydrogenase whose activity is to be reduced or inactivated in the cell of the invention is a gene encoding a glycerolphosphate dehydrogenase having an amino acid sequence with at least 70% sequence identity to SEQ ID NO: 21, while the cell has at least one functional copy of a endogenous gene encoding a glycerolphosphate dehydrogenase having an amino acid sequence with at least 70% sequence identity to SEQ ID NO: 22.

**6.** A yeast cell according to any one of claims 1 - 5, wherein the yeast cell comprises at least one further genetic modification that results in a characteristic selected from the group consisting of:

a) increased xylulose kinase specific activity;
b) increased flux of the pentose phosphate pathway
c) reduced unspecific aldose reductase specific activity
d) increased transport of at least one of xylose and arabinose into the host cell;
e) decreased sensitivity to catabolite repression;
f) increased tolerance to ethanol, osmolarity or organic acids; and,
g) reduced production of by-products.

**7.** A yeast cell according to any one of claims 1 - 6, wherein the yeast cell is of a genus selected from the group consisting of *Saccharomyces, Kluyveromyces, Candida, Pichia, Schizosaccharomyces, Hansenula, Kloeckera, Schwanniomyces,* and *Yarrowia.*

**8.** A yeast cell according to claim 7, wherein the yeast cell belongs to a species selected from the group consisting of *S. cerevisiae, S. exiguus, S. bayanus, K. lactis, K. marxianus* and *Schizosaccharomyces pombe.*

**9.** A yeast cell according any one of the preceding claims wherein at least one of the nucleotide sequences coding for the enzyme with pyruvate formate lyase activity, the pyruvate formate lyase activating enzyme, the enzyme with acetaldehyde dehydrogenase activity, the xylose isomerase, the L-arabinose isomerase, the L-ribulokinase and the L-ribulose-5-phosphate 4-epimerase is a nucleotide sequence the codon usage of which has been optimised for expression in the yeast cell.

**10.** A process for producing a fermentation product selected from the group consisting of ethanol, lactic acid, 3-hydroxy-propionic acid, acrylic acid, 1,3-propanediol, a butanol and an isoprenoid-derived product, whereby the process comprises the steps of:

a) fermenting a medium with a yeast cell as defined in any one of claim 1 - 9, whereby the medium contains or is fed with a source of glycerol and with a source of at least one of a hexose and a pentose, whereby the yeast cell ferments the glycerol and the at least one of the hexose and pentose to the fermentation product and formate; and optionally,
b) recovery of at least one of the fermentation product and formate.

**11.** A process according to claim 11, wherein the fermentation product is ethanol.

**12.** A process according to claim 10 or 11, wherein the medium contains or is fed with a lignocellulosic hydrolysate.

**13.** A process according to any one of the claims 10 - 12, wherein the yeast cell ferments under anaerobic conditions.

**14.** A process according to any one of the claims 10 - 12, wherein the pH of the medium is regulated during the

fermentation process to maintain a concentration of undissociated formic acid that is not higher than 20.0 mM.

## Patentansprüche

**1.** Hefezelle, umfassend:

a) ein für ein Enzym mit der Fähigkeit, Pyruvat und Coenzym-A in Formiat und Acetyl-CoA umzuwandeln, codierendes exogenes Gen;
b) eine genetische Modifikation, durch die spezifische $NAD^+$-abhängige Formiat-Dehydrogenase-Aktivität in der Zelle reduziert wird;
c) ein für ein Enzym mit Acetaldehyd-Dehydrogenase-Aktivität codierendes exogenes Gen, das der Zelle die Fähigkeit verleiht, Acetyl-CoA zu Acetaldehyd zu reduzieren; und
d) eine genetische Modifikation, durch die die spezifische Aktivität von $NAD^+$-verknüpfter Glycerin-Dehydrogenase erhöht wird.

**2.** Hefezelle nach Anspruch 1, wobei die Zelle ferner eine aus der aus

a) einer genetischen Modifikation, durch die die spezifische Aktivität von Dihydroxyaceton-Kinase erhöht wird;
b) einer genetischen Modifikation, durch die der Transport von Glycerin in die Zelle gesteigert wird;
einem für ein Enzym zur Aktivierung der Pyruvat-Formiat-Lyase codierenden exogenen Gen;
c) einer genetischen Modifikation, durch die die spezifische Acetyl-CoA-Synthetase-Aktivität in der Zelle erhöht wird; und
d) einer genetischen Modifikation, durch die spezifische $NAD^+$-abhängige Glycerin-3-Phosphat-Dehydrogenase-Aktivität in der Zelle reduziert wird, während die Hefezelle einen funktionellen HOG(High-Osmolarity Glycerol Response)-Weg umfasst,

bestehenden Gruppe ausgewählte genetische Modifikation umfasst.

**3.** Hefezelle nach Anspruch 1 oder 2, wobei die Zelle:

i) ein exogenes Xylose-Isomerase-Gen, das der Zelle die Fähigkeit verleiht, Xylose zu Xylulose zu isomerisieren; oder/und
ii) für eine L-Arabinose-Isomerase, eine L-Ribulokinase und eine L-Ribulose-5-phosphat-4-Epimerase codierende exogene Gene, die zusammen der Zelle die Fähigkeit verleihen, L-Arabinose in D-Xylulose-5-phosphat umzuwandeln,

umfasst.

**4.** Hefezelle nach einem der Ansprüche 1 - 3, wobei wenigstens:

a) die genetische Modifikation, durch die spezifische $NAD^+$-abhängige Formiat-Dehydrogenase-Aktivität in der Zelle reduziert wird, die Inaktivierung wenigstens einer endogenen Kopie eines eine Formiat-Dehydrogenase codierenden Gens im Genom der Zelle umfasst; oder
b) die genetische Modifikation, durch die die spezifische Aktivität der $NAD^+$-verknüpften Glycerin-Dehydrogenase erhöht wird, die Expression eines eine $NAD^+$-verknüpfte Glycerin-Dehydrogenase codierenden heterologen Gens bedeutet; oder
c) die genetische Modifikation, durch die der Transport von Glycerin in die Zelle gesteigert wird, die Überexpression einer ein Glycerinaufnahme-Protein oder/und einen Glycerin-Kanal codierenden Nukleotidsequenz bedeutet; oder
d) die genetische Modifikation, durch die spezifische $NAD^+$-abhängige Glycerin-3-Phosphat-Dehydrogenase-Aktivität in der Zelle reduziert wird, die Inaktivierung wenigstens einer endogenen Kopie eines eine Glycerin-3-Phosphat-Dehydrogenase codierenden Gens im Genom der Zelle umfasst oder
e) die genetische Modifikation, durch die die spezifische Acetyl-CoA-Synthetase-Aktivität erhöht wird, die Überexpression einer eine Acetyl-CoA-Synthetase codierenden Nukleotidsequenz bedeutet; oder
f) die genetische Modifikation, durch die die spezifische Aktivität von Dihydroxyaceton-Kinase erhöht wird, die Überexpression einer eine Dihydroxyaceton-Kinase codierenden Nukleotidsequenz bedeutet.

**5.** Hefezelle nach einem der Ansprüche 1 - 4, wobei:

a) das für ein Enzym mit Pyruvat-Formiat-Lyase-Aktivität codierende exogene Gen eine für eine Aminosäuresequenz mit wenigstens 57% Aminosäuresequenzidentität mit SEQ ID NO: 1 codierende Nukleotidsequenz umfasst;
b) das für das Pyruvat-Formiat-Lyase aktivierende Enzym codierende exogene Gen eine für eine Aminosäuresequenz mit wenigstens 70% Aminosäuresequenzidentität mit SEQ ID NO: 3 codierende Nukleotidsequenz umfasst;
c) es sich bei dem eine Formiat-Dehydrogenase, deren Aktivität in der erfindungsgemäßen Zelle reduziert oder inaktiviert werden soll, codierenden Gen um ein eine Formiat-Dehydrogenase mit einer Aminosäuresequenz mit wenigstens 70% Sequenzidentität mit wenigstens einer der Sequenzen SEQ ID NO: 5 und 6 codierendes Gen handelt;
d) das für das Enzym mit Acetaldehyd-Dehydrogenase-Aktivität codierende exogene Gen eine für eine Aminosäuresequenz mit wenigstens einer Aminosäuresequenzidentität aus:

i) wenigstens 64% Aminosäuresequenzidentität mit SEQ ID NO: 7,
ii) wenigstens 76% Aminosäuresequenzidentität mit SEQ ID NO: 9 und
iii) wenigstens 61% Aminosäuresequenzidentität mit SEQ ID NO: 11;

codierende Nukleotidsequenz umfasst;
e) die die $NAD^+$-verknüpfte Glycerin-Dehydrogenase codierende Nukleotidsequenz eine für eine Aminosäuresequenz mit wenigstens 50% Aminosäuresequenzidentität mit SEQ ID NO: 49 codierende Nukleotidsequenz umfasst,
f) die die Dihydroxyaceton-Kinase codierende Nukleotidsequenz eine für eine Aminosäuresequenz mit wenigstens 50% Aminosäuresequenzidentität mit wenigstens einer der Sequenzen SEQ ID NO: 14, 15 und 52 codierende Nukleotidsequenz umfasst;
g) die das Glycerinaufnahme-Protein codierende Nukleotidsequenz eine für eine Aminosäuresequenz mit wenigstens 50% Aminosäuresequenzidentität mit wenigstens einer der Sequenzen SEQ ID NO: 16 und 17 codierende Nukleotidsequenz umfasst und wobei die den Glycerin-Kanal codierende Nukleotidsequenz eine für eine Aminosäuresequenz mit wenigstens 30% Aminosäuresequenzidentität mit der Aminosäuresequenz zwischen Aminosäure 250 und 530 der SEQ ID NO: 18 codierende Nukleotidsequenz umfasst;
h) die die Acetyl-CoA-Synthetase codierende Nukleotidsequenz eine Aminosäuresequenz mit wenigstens 70% Aminosäuresequenzidentität mit wenigstens einer der Sequenzen SEQ ID NO: 19 und 20 umfasst; und
i) es sich bei dem eine Glycerinphosphat-Dehydrogenase, deren Aktivität in der erfindungsgemäßen Zelle reduziert oder inaktiviert werden soll, codierenden Gen um ein eine Glycerinphosphat-Dehydrogenase mit einer Aminosäuresequenz mit wenigstens 70% Sequenzidentität mit SEQ ID NO: 21 codierendes Gen handelt, wobei die Zelle wenigstens eine funktionelle Kopie eines eine Glycerinphosphat-Dehydrogenase mit einer Aminosäuresequenz mit wenigstens 70% Sequenzidentität mit SEQ ID NO: 22 codierenden endogenen Gens aufweist.

**6.** Hefezelle nach einem der Ansprüche 1 - 5, wobei die Hefezelle wenigstens eine weitere genetische Modifikation umfasst, die zu einer Charakteristik führt, die ausgewählt ist aus der Gruppe bestehend aus:

a) erhöhter spezifischer Aktivität der Xylulosekinase;
b) erhöhtem Flux des Pentosephosphat-Wegs;
c) reduzierter spezifischer Aktivität der unspezifischen Aldose-Reduktase;
d) erhöhtem Transport von wenigstens entweder Xylose oder Arabinose in die Wirtszelle;
e) verminderter Empfindlichkeit gegenüber Katabolitenrepression;
f) erhöhter Toleranz gegenüber Ethanol, Osmolarität oder organischen Säuren; und
g) verringerter Produktion von Nebenprodukten.

**7.** Hefezelle nach einem der Ansprüche 1 - 5, wobei die Hefezelle aus einer aus der aus *Saccharomyces, Kluyveromyces, Candida, Pichia, Schizosaccharomyces, Hansenula, Kloeckera, Schwanniomyces* und *Yarrowia* bestehenden Gruppe ausgewählten Gattung stammt.

**8.** Hefezelle nach Anspruch 7, wobei die Hefezelle zu einer aus der aus *S. cerevisiae, S. exiguus, S. bayanus, K. lactis, K. marxianus* und *Schizosaccharomyces pombe* bestehenden Gruppe ausgewählten Spezies gehört.

**9.** Hefezelle nach einem der vorhergehenden Ansprüche, wobei es sich bei wenigstens einer der für das Enzym mit

Pyruvat-Formiat-Lyase-Aktivität, das Pyruvat-Formiat-Lyase aktivierende Enzym, das Enzym mit Acetaldehyd-Dehydrogenase-Aktivität, die Xylose-Isomerase, die L-Arabinose-Isomerase, die L-Ribulokinase und die L-Ribulose-5-phosphat-4-Epimerase codierenden Nukleotidsequenzen um eine Nukleotidsequenz handelt, deren Codonverwendung für die Expression in der Hefezelle optimiert wurde.

**10.** Verfahren zur Herstellung eines aus der aus Ethanol, Milchsäure, 3-Hydroxypropionsäure, Acrylsäure, 1,3-Propandiol, einem Butanol und einem von Isoprenoid abgeleiteten Produkt bestehenden Gruppe ausgewählten Fermentationsprodukts, wobei das Verfahren die Schritte umfasst:

a) Fermentieren eines Mediums mit einer Hefezelle mit der in einem der Ansprüche 1 - 9 angegebenen Bedeutung, wobei das Medium eine Quelle für Glycerin und eine Quelle für wenigstens entweder eine Hexose oder eine Pentose enthält oder damit gespeist wird, wobei die Hefezelle das Glycerin sowie entweder die Hexose oder die Pentose zum Fermentationsprodukt und Formiat vergärt; und gegebenenfalls
b) Gewinnung wenigstens entweder des Fermentationsprodukts oder des Formiats.

**11.** Verfahren nach Anspruch 11, wobei es sich bei dem Fermentationsprodukt um Ethanol handelt.

**12.** Verfahren nach Anspruch 10 oder 11, wobei das Medium ein Lignocellulose-Hydrolysat enthält oder damit gespeist wird.

**13.** Verfahren nach einem der Ansprüche 10 - 12, wobei die Hefezelle unter anaeroben Bedingungen vergärt.

**14.** Verfahren nach einem der Ansprüche 10 - 12, wobei der pH-Wert des Mediums während des Fermentationsverfahrens reguliert wird, so dass eine Konzentration undissoziierter Ameisensäure beibehalten wird, die nicht höher als 20,0 mM liegt.

## Revendications

**1.** Cellule de levure comprenant :

a) un gène exogène codant pour une enzyme ayant la capacité de convertir le pyruvate et le coenzyme-A en formiate et en acétyl-CoA ;
b) une modification génétique qui réduit l'activité spécifique formiate déshydrogénase $NAD^+$-dépendante dans la cellule ;
c) un gène exogène codant pour une enzyme ayant une activité acétaldéhyde déshydrogénase, ledit gène conférant à la cellule la capacité de réduire l'acétyl-CoA en acétaldéhyde ; et,
d) une modification génétique qui augmente l'activité spécifique de la glycérol déshydrogénase $NAD^+$-liée.

**2.** Cellule de levure selon la revendication 1, la cellule comprenant en outre une modification génétique choisie dans le groupe constitué de :

a) une modification génétique qui augmente l'activité spécifique de dihydroxyacétone kinase ;
b) une modification génétique qui augmente le transport de glycérol dans la cellule ;
un gène exogène codant pour une enzyme pour activation de la pyruvate formiate lyase ;
c) une modification génétique qui augmente l'activité spécifique acétyl-CoA synthétase dans la cellule ; et,
d) une modification génétique qui réduit l'activité spécifique glycérol 3-phosphate déshydrogénase $NAD^+$-dépendante dans la cellule, tandis que la cellule de levure comprend une voie de réponse au glycérol à osmolarité élevée fonctionnelle.

**3.** Cellule de levure selon la revendication 1 ou 2, la cellule comprenant au moins l'un de :

i) un gène xylose isomérase exogène, ledit gène conférant à la cellule la capacité d'isomériser le xylose en xylulose ; et,
ii) des gènes exogènes codant pour une L-arabinose isomérase, une L-ribulokinase et une L-ribulose-5-phosphate 4-épimérase, lesdits gènes conférant conjointement à la cellule la capacité de convertir le L-arabinose en D-xylulose 5-phosphate.

**4.** Cellule de levure selon l'une quelconque des revendications 1 à 3, dans laquelle au moins l'un de :

a) la modification génétique qui réduit l'activité spécifique formiate déshydrogénase $NAD^+$-dépendante dans la cellule comprend l'inactivation d'au moins une copie endogène d'un gène codant pour une formiate déshydrogénase dans le génome de la cellule ;
b) la modification génétique qui augmente l'activité spécifique de la glycérol déshydrogénase $NAD^+$-liée est l'expression d'un gène hétérologue codant pour une glycérol déshydrogénase $NAD^+$-liée ;
c) la modification génétique qui augmente le transport de glycérol dans la cellule est la surexpression d'une séquence nucléotidique codant pour au moins l'un d'une protéine d'absorption de glycérol et un canal glycérol ; et,
d) la modification génétique qui réduit l'activité spécifique glycérol 3-phosphate déshydrogénase $NAD^+$-dépendante dans la cellule comprend l'inactivation d'au moins une copie endogène d'un gène codant pour une glycérol 3-phosphate déshydrogénase dans le génome de la cellule et,
e) la modification génétique qui augmente l'activité spécifique acétyl-CoA synthétase est la surexpression d'une séquence nucléotidique codant pour une acétyl-CoA synthétase ; et,
f) la modification génétique qui augmente l'activité spécifique de dihydroxyacétone kinase est la surexpression d'une séquence nucléotidique codant pour une dihydroxyacétone kinase.

**5.** Cellule de levure selon l'une quelconque des revendications 1 à 4, dans laquelle :

a) le gène exogène codant pour une enzyme ayant une activité pyruvate formiate lyase comprend une séquence nucléotidique codant pour une séquence d'acides aminés ayant au moins 57 % d'identité de séquence d'acides aminés avec SEQ ID NO: 1 ;
b) le gène exogène codant pour l'enzyme activant la pyruvate formiate lyase comprend une séquence nucléotidique codant pour une séquence d'acides aminés ayant au moins 70 % d'identité de séquence d'acides aminés avec SEQ ID NO: 3 ;
c) le gène codant pour une formiate déshydrogénase dont l'activité doit être réduite ou inactivée dans la cellule de l'invention est un gène codant pour une formiate déshydrogénase ayant une séquence d'acides aminés ayant au moins 70 % d'identité de séquence avec au moins l'un de SEQ ID NO: 5 et 6 ;
d) le gène exogène codant pour l'enzyme ayant une activité acétaldéhyde déshydrogénase comprend une séquence nucléotidique codant pour une séquence d'acides aminés ayant au moins l'un de :

i) au moins 64 % d'identité de séquence d'acides aminés avec SEQ ID NO: 7,
ii) au moins 76 % d'identité de séquence d'acides aminés avec SEQ ID NO: 9 et
iii) au moins 61 % d'identité de séquence d'acides aminés avec SEQ ID NO: 11 ;

e) la séquence nucléotidique codant pour la glycérol déshydrogénase $NAD^+$-liée comprend une séquence nucléotidique codant pour une séquence d'acides aminés ayant au moins 50 % d'identité de séquence d'acides aminés avec SEQ ID NO: 49,
f) la séquence nucléotidique codant pour la dihydroxyacétone kinase comprend une séquence nucléotidique codant pour une séquence d'acides aminés ayant au moins 50 % d'identité de séquence d'acides aminés avec au moins l'un de SEQ ID NO: 14, 15 et 52 ;
g) la séquence nucléotidique codant pour la protéine d'absorption de glycérol comprend une séquence nucléotidique codant pour une séquence d'acides aminés ayant au moins 50 % d'identité de séquence d'acides aminés avec au moins l'un de SEQ ID NO: 16 et 17, et où la séquence nucléotidique codant pour le canal glycérol comprend une séquence nucléotidique codant pour une séquence d'acides aminés ayant au moins 30 % d'identité de séquence d'acides aminés avec la séquence d'acides aminés entre les acides aminés 250 et 530 de SEQ ID NO: 18 ;
h) la séquence nucléotidique codant pour l'acétyl-CoA synthétase comprend une séquence d'acides aminés ayant au moins 70 % d'identité de séquence d'acides aminés avec au moins l'un de SEQ ID NO : 19 et 20 ; et,
i) le gène codant pour une glycérolphosphate déshydrogénase dont l'activité doit être réduite ou inactivée dans la cellule de l'invention est un gène codant pour une glycérolphosphate déshydrogénase ayant une séquence d'acides aminés ayant au moins 70 % d'identité de séquence avec SEQ ID NO: 21, tandis que la cellule a au moins une copie fonctionnelle d'un gène endogène codant pour une glycérolphosphate déshydrogénase ayant une séquence d'acides aminés ayant au moins 70 % d'identité de séquence avec SEQ ID NO: 22.

**6.** Cellule de levure selon l'une quelconque des revendications 1 à 5, la cellule de levure comprenant au moins une modification génétique supplémentaire qui conduit à une caractéristique choisie dans le groupe constitué de :

a) une activité spécifique xylulose kinase augmentée ;
b) un flux augmenté de la voie pentose phosphate ;
c) une activité spécifique aldose réductase non spécifique réduite ;
d) un transport augmenté d'au moins l'un du xylose et de l'arabinose dans la cellule hôte ;
e) une sensibilité réduite à la répression par catabolite ;
f) une tolérance augmentée à l'éthanol, l'osmolarité ou les acides organiques ; et,
g) une production réduite de sous-produits.

**7.** Cellule de levure selon l'une quelconque des revendications 1 à 6, la cellule de levure étant d'un genre choisi dans le groupe constitué de *Saccharomyces, Kluyveromyces, Candida, Pichia, Schizosaccharomyces, Hansenula, Kloeckera, Schwanniomyces,* et *Yarrowia.*

**8.** Cellule de levure selon la revendication 7, la cellule de levure appartenant à une espèce choisie dans le groupe constitué de *S. cerevisiae, S. exiguus, S. bayanus, K. lactis, K. marxianus* et *Schizosaccharomyces pombe*.

**9.** Cellule de levure selon l'une quelconque des revendications précédentes dans laquelle au moins l'une des séquences nucléotidiques codant pour l'enzyme ayant une activité pyruvate formiate lyase, l'enzyme activant la pyruvate formiate lyase, l'enzyme ayant une activité acétaldéhyde déshydrogénase, la xylose isomérase, la L-arabinose isomérase, la L-ribulokinase et la L-ribulose-5-phosphate 4-épimerase est une séquence nucléotidique dont l'usage de codon a été optimisé pour l'expression dans la cellule de levure.

**10.** Procédé de production d'un produit de fermentation choisi dans le groupe constitué de l'éthanol, l'acide lactique, l'acide 3-hydroxy-propionique, l'acide acrylique, le 1,3-propane-diol, un butanol et un produit dérivé d'isoprénoïde, le procédé comprenant les étapes de :

a) fermentation d'un milieu avec une cellule de levure telle que définie dans l'une quelconque des revendications 1 à 9, où le milieu contient ou est approvisionné avec une source de glycérol et avec une source d'au moins l'un d'un hexose et un pentose, de sorte que la cellule de levure fermente le glycérol et l'au moins l'un de l'hexose et du pentose en produit de fermentation et en formiate ; et facultativement,
b) récupération d'au moins l'un du produit de fermentation et du formiate.

**11.** Procédé selon la revendication 11, dans lequel le produit de fermentation et l'éthanol.

**12.** Procédé selon la revendication 10 ou 11, dans lequel le milieu contient ou est approvisionné en hydrolysat lignocellulosique.

**13.** Procédé selon l'une quelconque des revendications 10 à 12, dans lequel la cellule de levure fermente dans des conditions anaérobies.

**14.** Procédé selon l'une quelconque des revendications 10 à 12, dans lequel le pH du milieu est régulé pendant le processus de fermentation pour maintenir une concentration d'acide formique non dissocié qui n'est pas supérieure à 20,0 mM.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010019882 A
- WO 9303159 A
- WO 030624430 A
- WO 06009434 A
- US 20060234364 A
- WO 09109633 A
- WO 10074577 A
- EP 1499708 A
- WO 2009011591 A
- WO 2006009434 A
- WO 2005023998 A
- WO 2005111214 A
- WO 2005091733 A
- EP 10191736 A
- US 61415054 B
- US 61471836 B


### Non-patent literature cited in the description


- **GUADALUPE MEDINA et al.** *Appl. Environ. Microbiol.,* 2009, vol. 76, 190-195
- **OVERKAMP et al.** *Appl. Environ. Microbiol.,* 2002, vol. 68, 2814-21
- **YU et al.** *Bioresour. Technol.,* 09 February 2010, vol. 101 (11), 4157-61
- **YU et al.** *J. Biotechnol.,* 2010
- **WAKS ; SILVER.** *Appl. Environ. Microbiol.,* 2009, vol. 75, 1867-1875
- **WON-KYUNG HONG et al.** *biotechnology letters,* 2010, vol. 32 (8), 1077-1082
- **HENIKOFF ; HENIKOFF.** *PNAS,* 1992, vol. 89, 915-919
- Feedstock and Biodiesel Characteristics Report. Renewable Energy Group, Inc, 2009
- **KUYPERS et al.** *FEMS Yeast Res.,* 2005, vol. 5, 399-409
- **BUIS ; BRODERICK.** *Arch. Biochem. Biophys.,* 2005, vol. 433, 288-296
- **SAWERS ; WATSON.** *Mol. Microbiol.,* 1998, vol. 29, 945-954
- **LEHTIÖ ; GOLDMAN.** *Prot. Engin. Design & Selection,* 2004, vol. 17, 545-552
- **SAMBROOK ; RUSSELL.** Molecular cloning: a laboratory manual. Cold Spring Harbor Laboratory Press, 2001
- **AUSUBEL et al.** Current protocols in molecular biology. Green Publishing and Wiley Interscience, 1987
- **SHERMAN et al.** Methods Yeast Genetics. Cold Spring Harbor Laboratory, 1978
- Guide To Yeast Genetics and Molecular Biology. Academic Press, 1991, vol. 194
- **SHARP ; LI.** *Nucleic Acids Research,* 1987, vol. 15, 1281-1295
- **JANSEN et al.** *Nucleic Acids Res.,* 2003, vol. 31 (8), 2242-51
- **J.A. BARNETT ; R.W. PAYNE ; D. YARROW.** Yeasts: characteristics and identification. Cambridge University Press, 2000
- The yeasts, a taxonomic study. Elsevier Science Publ. B.V, 1998
- **OVERKAMP et al.** *Yeast,* 2002, vol. 19, 509-520
- **VAN DEN BERG ; STEENSMA.** *Yeast,* 1997, vol. 13, 551-559
- **BRUCHAUS ; TANNICH.** *J. Biochem.,* 1994, vol. 303, 743-748
- **BURDETTE ; ZEIKUS.** *J. Biochem.,* 1994, vol. 302, 163-170
- **KOO et al.** *Biotechnol. Lett.,* 2005, vol. 27, 505-510
- **YONG et al.** *Proc Natl Acad Sci USA,* 1996, vol. 93, 6464-6469
- **OECHSNER et al.** *FEBS Lett.,* 1988, vol. 238, 123-128
- **VOSS et al.** *Yeast,* 1997, vol. 13, 655-672
- **TRUNIGER ; BOOS.** *J Bacteriol.,* 1994, vol. 176 (6), 1796-1800
- **LEE ; DASILVA.** *Metab Eng.,* 2006, vol. 8 (1), 58-65
- **MOLIN et al.** *J. Biol. Chem.,* 2003, vol. 278, 1415-1423
- **DANIEL et al.** *J. Bacteriol.,* 1995, vol. 177, 4392-4401
- **NEVES et al.** *FEMS Yeast Res.,* 2004, vol. 5, 51-62
- **REIZER et al.** *CRC Crit. Rev. Biochem. Mol. Biol.,* 1993, vol. 28, 235-257
- **VAN AELST et al.** *EMBO J.,* 1991, vol. 10, 2095-2104
- **LUYTEN et al.** *EMBO J.,* 1995, vol. 14, 1360-1371

- **DE JONG-GUBBELS et al.** *FEMS Microbiol Lett.,* 1998, vol. 165, 15-20
- **ERIKSSON et al.** *Mol. Microbiol.,* 1995, vol. 17, 95-107
- **ALBERTYN et al.** *Mol. Cell. Biol.,* 1994, vol. 14, 4135-4144
- **BRAT et al.** *Appl. Environ. Microbiol.,* 2009, vol. 75, 2304-2311
- **WISSELINK et al.** *AEM Accepts,* 01 June 2007
- *Appl. Environ. Microbiol.*
- **DENG ; HO.** *Appl. Biochem. Biotechnol.,* 1990, vol. 24-25, 193-199
- **TRÄFF et al.** *Appl. Environm. Microbiol.,* 2001, vol. 67, 5668-5674
- **SEDLACK ; HO.** *Yeast,* 2004, vol. 21, 671-684
- **KATAHIRA et al.** *Enzyme Microb. Technol.,* 2008, vol. 43, 115-119
- **DIDERICH et al.** *Appl. Environ. Microbiol.,* 2001, vol. 67, 1587-1593
- **VAN MARIS et al.** *Antonie van Leeuwenhoek,* 2006, vol. 90, 391-418
- **ABBOT et al.** *Appl. Environ. Microbiol.,* 2009, vol. 75, 2320-2325
- **BLOMBERG ; ADLER.** *J. Bacteriol.,* 1989, vol. 171, 1087-1092.9
- **FRENKEL ; KITCHENS.** *J. Biol. Chem.,* 1977, vol. 252, 504-507
- **WEBSTER.** *Methods Enzymol.,* 1969, vol. 13, 375-381
- **GONZALEZ et al.** *Metab. Eng.,* 2008, vol. 10, 234-245
- **SIKORSKI ; HIETER.** *Genetics,* 1989, vol. 122, 19-27

SEQUENCE LISTING

<110> Royal Nedalco B.V.
C5 Yeast Company B.V.

<120> Yeast strains engineered to produce ethanol and formate

<130> P6031691PCT

<150> EP10191736.7
<151> 2010-11-18

<150> US61/415,054
<151> 2010-11-18

<150> US61/471,836
<151> 2011-04-05

<160> 57

<170> PatentIn version 3.3

<210> 1

<211> 760
<212> PRT
<213> Escherichia coli

<400> 1

```
Met Ser Glu Leu Asn Glu Lys Leu Ala Thr Ala Trp Glu Gly Phe Thr
1               5                   10                  15

Lys Gly Asp Trp Gln Asn Glu Val Asn Val Arg Asp Phe Ile Gln Lys
            20                  25                  30

Asn Tyr Thr Pro Tyr Glu Gly Asp Glu Ser Phe Leu Ala Gly Ala Thr
        35                  40                  45

Glu Ala Thr Thr Thr Leu Trp Asp Lys Val Met Glu Gly Val Lys Leu
    50                  55                  60

Glu Asn Arg Thr His Ala Pro Val Asp Phe Asp Thr Ala Val Ala Ser
65                  70                  75                  80

Thr Ile Thr Ser His Asp Ala Gly Tyr Ile Asn Lys Gln Leu Glu Lys
                85                  90                  95

Ile Val Gly Leu Gln Thr Glu Ala Pro Leu Lys Arg Ala Leu Ile Pro
            100                 105                 110

Phe Gly Gly Ile Lys Met Ile Glu Gly Ser Cys Lys Ala Tyr Asn Arg
        115                 120                 125

Glu Leu Asp Pro Met Ile Lys Lys Ile Phe Thr Glu Tyr Arg Lys Thr
    130                 135                 140
```

```
His Asn Gln Gly Val Phe Asp Val Tyr Thr Pro Asp Ile Leu Arg Cys
145                 150                 155                 160

Arg Lys Ser Gly Val Leu Thr Gly Leu Pro Asp Ala Tyr Gly Arg Gly
                165                 170                 175

Arg Ile Ile Gly Asp Tyr Arg Arg Val Ala Leu Tyr Gly Ile Asp Tyr
            180                 185                 190

Leu Met Lys Asp Lys Leu Ala Gln Phe Thr Ser Leu Gln Ala Asp Leu
        195                 200                 205

Glu Asn Gly Val Asn Leu Glu Gln Thr Ile Arg Leu Arg Glu Glu Ile
    210                 215                 220

Ala Glu Gln His Arg Ala Leu Gly Gln Met Lys Glu Met Ala Ala Lys
225                 230                 235                 240

Tyr Gly Tyr Asp Ile Ser Gly Pro Ala Thr Asn Ala Gln Glu Ala Ile
                245                 250                 255

Gln Trp Thr Tyr Phe Gly Tyr Leu Ala Ala Val Lys Ser Gln Asn Gly
            260                 265                 270

Ala Ala Met Ser Phe Gly Arg Thr Ser Thr Phe Leu Asp Val Tyr Ile
        275                 280                 285

Glu Arg Asp Leu Lys Ala Gly Lys Ile Thr Glu Gln Glu Ala Gln Glu
    290                 295                 300

Met Val Asp His Leu Val Met Lys Leu Arg Met Val Arg Phe Leu Arg
305                 310                 315                 320

Thr Pro Glu Tyr Asp Glu Leu Phe Ser Gly Asp Pro Ile Trp Ala Thr
                325                 330                 335

Glu Ser Ile Gly Gly Met Gly Leu Asp Gly Arg Thr Leu Val Thr Lys
            340                 345                 350

Asn Ser Phe Arg Phe Leu Asn Thr Leu Tyr Thr Met Gly Pro Ser Pro
        355                 360                 365

Glu Pro Asn Met Thr Ile Leu Trp Ser Glu Lys Leu Pro Leu Asn Phe
    370                 375                 380

Lys Lys Phe Ala Ala Lys Val Ser Ile Asp Thr Ser Ser Leu Gln Tyr
```

385 390 395 400

Glu Asn Asp Asp Leu Met Arg Pro Asp Phe Asn Asn Asp Asp Tyr Ala
405 410 415

Ile Ala Cys Cys Val Ser Pro Met Ile Val Gly Lys Gln Met Gln Phe
420 425 430

Phe Gly Ala Arg Ala Asn Leu Ala Lys Thr Met Leu Tyr Ala Ile Asn
435 440 445

Gly Gly Val Asp Glu Lys Leu Lys Met Gln Val Gly Pro Lys Ser Glu
450 455 460

Pro Ile Lys Gly Asp Val Leu Asn Tyr Asp Glu Val Met Glu Arg Met
465 470 475 480

Asp His Phe Met Asp Trp Leu Ala Lys Gln Tyr Ile Thr Ala Leu Asn
485 490 495

Ile Ile His Tyr Met His Asp Lys Tyr Ser Tyr Glu Ala Ser Leu Met
500 505 510

Ala Leu His Asp Arg Asp Val Ile Arg Thr Met Ala Cys Gly Ile Ala
515 520 525

Gly Leu Ser Val Ala Ala Asp Ser Leu Ser Ala Ile Lys Tyr Ala Lys
530 535 540

Val Lys Pro Ile Arg Asp Glu Asp Gly Leu Ala Ile Asp Phe Glu Ile
545 550 555 560

Glu Gly Glu Tyr Pro Gln Phe Gly Asn Asn Asp Pro Arg Val Asp Asp
565 570 575

Leu Ala Val Asp Leu Val Glu Arg Phe Met Lys Lys Ile Gln Lys Leu
580 585 590

His Thr Tyr Arg Asp Ala Ile Pro Thr Gln Ser Val Leu Thr Ile Thr
595 600 605

Ser Asn Val Val Tyr Gly Lys Lys Thr Gly Asn Thr Pro Asp Gly Arg
610 615 620

Arg Ala Gly Ala Pro Phe Gly Pro Gly Ala Asn Pro Met His Gly Arg
625 630 635 640

```
Asp Gln Lys Gly Ala Val Ala Ser Leu Thr Ser Val Ala Lys Leu Pro
                645                 650                 655

Phe Ala Tyr Ala Lys Asp Gly Ile Ser Tyr Thr Phe Ser Ile Val Pro
            660                 665                 670

Asn Ala Leu Gly Lys Asp Asp Glu Val Arg Lys Thr Asn Leu Ala Gly
        675                 680                 685

Leu Met Asp Gly Tyr Phe His His Glu Ala Ser Ile Glu Gly Gly Gln
    690                 695                 700

His Leu Asn Val Asn Val Met Asn Arg Glu Met Leu Leu Asp Ala Met
705                 710                 715                 720

Glu Asn Pro Glu Lys Tyr Pro Gln Leu Thr Ile Arg Val Ser Gly Tyr
                725                 730                 735

Ala Val Arg Phe Asn Ser Leu Thr Lys Glu Gln Gln Gln Asp Val Ile
            740                 745                 750

Thr Arg Thr Phe Thr Gln Ser Met
        755                 760
```

<210> 2
<211> 2283
<212> DNA
<213> Artificial

<220>
<223> synthetic codon optimised E.coli pf1B

<400> 2

atgtctgaat tgaacgagaa gttggctacc gcttgggaag gtttcaccaa gggtgactgg 60
caaaacgaag ttaacgttag agacttcatc caaaagaact acaccccata cgaaggtgac 120
gaatctttct tggctggtgc taccgaagct accaccacct tgtgggacaa ggttatggaa 180
ggtgttaagt tggaaaacag aacccacgct ccagttgact tcgacaccgc tgttgcttct 240
accatcacct ctcacgacgc tggttacatc aacaagcaat tggaaaagat cgttggttta 300
caaaccgaag ctccattgaa gagagctttg atcccattcg gtggtatcaa gatgatcgaa 360
ggttcttgta aggcttacaa cagagaattg gacccaatga tcaagaagat tttcaccgaa 420
tacagaaaga cccacaacca aggtgttttc gacgtttaca ctccagacat cttgagatgt 480
agaaagtctg gtgttttgac tggtttgcca gacgcttacg gtagaggtag aatcatcggt 540
gactacagaa gagttgcttt gtacggtatc gactacttga tgaaggacaa gttggctcaa 600
ttcacctctt tgcaagctga cttggaaaac ggtgttaact tggaacaaac catcagattg 660

agagaagaaa tcgctgaaca acacagagct ttgggtcaaa tgaaggaaat ggctgctaag 720
tacggttacg acatctctgg tccagctacc aacgctcaag aagctatcca atggacctac 780
ttcggttact tggctgctgt taagtctcaa aacggtgctg ctatgtcttt cggtaggacc 840
tctaccttct tggacgttta catcgaaaga gacttgaagg ctggtaagat caccgaacaa 900
gaagctcaag aaatggttga ccacttggtt atgaagttga gaatggttag attcttgaga 960
accccagaat acgacgaatt gttctctggt gacccaatct gggctaccga atctatcggt 1020
ggtatgggtt tggacggtag aaccttggtt accaagaact ctttcagatt cttgaacacc 1080
ttatacacca tgggtccatc tccagaacca aacatgacca tcttgtggtc tgaaaagtta 1140
ccattgaact tcaagaagtt cgctgctaag gtttctatcg acacctcttc tttgcaatac 1200
gaaaacgacg acttgatgag accagacttc aacaacgacg actacgctat cgcttgttgt 1260
gtttctccaa tgatcgttgg taagcaaatg caattcttcg gtgctagagc taacttggct 1320
aagaccatgt tgtacgctat caacggtggt gttgacgaaa agttgaagat gcaagttggt 1380
ccaaagtctg aaccaatcaa gggtgacgtt ttgaactacg acgaagttat ggaaagaatg 1440
gaccacttca tggactggtt ggctaagcaa tacatcaccg ctttgaacat catccactac 1500
atgcacgaca agtactctta cgaagcatca ttgatggctt tgcacgacag agacgtaatc 1560
agaaccatgg cttgtggtat cgctggtttg tctgttgctg ctgactcttt gtctgctatc 1620
aagtacgcta aggttaagcc aatcagagac gaagacggtt tggctatcga cttcgaaatc 1680
gaaggtgaat accctcaatt cggtaacaac gacccaagag ttgacgactt ggctgttgac 1740
ttggttgaaa gatttatgaa gaagatccaa aagttgcaca cctacagaga cgctatccca 1800
acccaatctg ttttgactat cacatctaac gttgtttacg gtaagaagac tggtaacacc 1860
ccagacggta gaagagctgg tgctccattc ggtccaggtg ctaacccaat gcacggtaga 1920
gaccaaaagg gtgctgtagc atctttgacc tctgttgcta agttgccatt cgcttacgct 1980
aaggacggta tctcttacac cttctctatc gttccaaacg ctttgggtaa ggacgatgaa 2040
gttagaaaga ccaacttggc tggtttgatg gacggttact tccaccacga agcatctatc 2100
gaaggtggtc aacacttgaa cgtaaatgtt atgaacagag aaatgttgtt ggacgctatg 2160
gaaaacccag aaaagtaccc acaattgacc atcagagttt ctggttacgc tgttagattc 2220
aactctttga ccaaggaaca acaacaagac gttatcacca gaaccttcac ccaatctatg 2280
taa 2283

<210> 3
<211> 246
<212> PRT
<213> Escherichia coli

<400> 3

Met Ser Val Ile Gly Arg Ile His Ser Phe Glu Ser Cys Gly Thr Val
1               5                   10                  15

Asp Gly Pro Gly Ile Arg Phe Ile Thr Phe Phe Gln Gly Cys Leu Met
            20                  25                  30

Arg Cys Leu Tyr Cys His Asn Arg Asp Thr Trp Asp Thr His Gly Gly
        35                  40                  45

Lys Glu Val Thr Val Glu Asp Leu Met Lys Glu Val Val Thr Tyr Arg
    50                  55                  60

His Phe Met Asn Ala Ser Gly Gly Gly Val Thr Ala Ser Gly Gly Glu
65                  70                  75                  80

Ala Ile Leu Gln Ala Glu Phe Val Arg Asp Trp Phe Arg Ala Cys Lys
                85                  90                  95

Lys Glu Gly Ile His Thr Cys Leu Asp Thr Asn Gly Phe Val Arg Arg
            100                 105                 110

Tyr Asp Pro Val Ile Asp Glu Leu Leu Glu Val Thr Asp Leu Val Met
        115                 120                 125

Leu Asp Leu Lys Gln Met Asn Asp Glu Ile His Gln Asn Leu Val Gly
    130                 135                 140

Val Ser Asn His Arg Thr Leu Glu Phe Ala Lys Tyr Leu Ala Asn Lys
145                 150                 155                 160

Asn Val Lys Val Trp Ile Arg Tyr Val Val Val Pro Gly Trp Ser Asp
                165                 170                 175

Asp Asp Asp Ser Ala His Arg Leu Gly Glu Phe Thr Arg Asp Met Gly
            180                 185                 190

Asn Val Glu Lys Ile Glu Leu Leu Pro Tyr His Glu Leu Gly Lys His
        195                 200                 205

Lys Trp Val Ala Met Gly Glu Glu Tyr Lys Leu Asp Gly Val Lys Pro
    210                 215                 220

Pro Lys Lys Glu Thr Met Glu Arg Val Lys Gly Ile Leu Glu Gln Tyr
225                 230                 235                 240

Gly His Lys Val Met Phe
                245

<210> 4
<211> 741
<212> DNA
<213> Artificial

<220>
<223> synthetic codon optimised E.coli pflA

<400> 4

```
atgtctgtta tcggtagaat ccactctttc gaatcttgtg gtactgttga cggtccaggt      60
atcagattca tcaccttctt ccaaggttgt ttgatgagat gtttgtactg tcacaacaga     120
gacacctggg acacccacgg tggtaaggaa gttactgttg aagacttgat gaaggaagtt     180
gttacctaca gacactttat gaacgcttca ggaggtggtg ttaccgcttc tggtggtgaa     240
gctatcttgc aagctgaatt tgttagagac tggttcagag cttgtaagaa ggaaggtatc     300
cacacctgtt tggacaccaa cggtttcgtt agaagatacg acccagttat cgacgaattg     360
ttggaagtta ccgacttggt tatgttggac ttgaagcaaa tgaacgacga aatccaccaa     420
aacttggttg gtgtttctaa ccacagaacc ttggaatttg ctaagtactt ggctaacaag     480
aacgttaagg tttggatcag atacgttgtt gttccaggtt ggtctgacga cgacgactct     540
gctcacagat tgggtgagtt caccagagac atgggtaacg ttgaaaagat cgaattgttg     600
ccataccacg aattgggtaa gcacaagtgg gttgctatgg gtgaagaata caagttggac     660
ggtgttaagc caccaaagaa ggaaaccatg gaaagagtta agggtatctt ggaacaatac     720
ggtcacaagg ttatgttcta a                                               741
```

<210> 5
<211> 376
<212> PRT
<213> Saccharomyces cerevisiae

<400> 5

```
Met Ser Lys Gly Lys Val Leu Leu Val Leu Tyr Glu Gly Gly Lys His
1               5                   10                  15

Ala Glu Glu Gln Glu Lys Leu Leu Gly Cys Ile Glu Asn Glu Leu Gly
            20                  25                  30

Ile Arg Asn Phe Ile Glu Glu Gln Gly Tyr Glu Leu Val Thr Thr Ile
        35                  40                  45

Asp Lys Asp Pro Glu Pro Thr Ser Thr Val Asp Arg Glu Leu Lys Asp
    50                  55                  60

Ala Glu Ile Val Ile Thr Thr Pro Phe Phe Pro Ala Tyr Ile Ser Arg
65                  70                  75                  80
```

Asn Arg Ile Ala Glu Ala Pro Asn Leu Lys Leu Cys Val Thr Ala Gly
85 90 95

Val Gly Ser Asp His Val Asp Leu Glu Ala Ala Asn Glu Arg Lys Ile
100 105 110

Thr Val Thr Glu Val Thr Gly Ser Asn Val Val Ser Val Ala Glu His
115 120 125

Val Met Ala Thr Ile Leu Val Leu Ile Arg Asn Tyr Asn Gly Gly His
130 135 140

Gln Gln Ala Ile Asn Gly Glu Trp Asp Ile Ala Gly Val Ala Lys Asn
145 150 155 160

Glu Tyr Asp Leu Glu Asp Lys Ile Ile Ser Thr Val Gly Ala Gly Arg
165 170 175

Ile Gly Tyr Arg Val Leu Glu Arg Leu Val Ala Phe Asn Pro Lys Lys
180 185 190

Leu Leu Tyr Tyr Asp Tyr Gln Glu Leu Pro Ala Glu Ala Ile Asn Arg
195 200 205

Leu Asn Glu Ala Ser Lys Leu Phe Asn Gly Arg Gly Asp Ile Val Gln
210 215 220

Arg Val Glu Lys Leu Glu Asp Met Val Ala Gln Ser Asp Val Val Thr
225 230 235 240

Ile Asn Cys Pro Leu His Lys Asp Ser Arg Gly Leu Phe Asn Lys Lys
245 250 255

Leu Ile Ser His Met Lys Asp Gly Ala Tyr Leu Val Asn Thr Ala Arg
260 265 270

Gly Ala Ile Cys Val Ala Glu Asp Val Ala Glu Ala Val Lys Ser Gly
275 280 285

Lys Leu Ala Gly Tyr Gly Gly Asp Val Trp Asp Lys Gln Pro Ala Pro
290 295 300

Lys Asp His Pro Trp Arg Thr Met Asp Asn Lys Asp His Val Gly Asn
305 310 315 320

Ala Met Thr Val His Ile Ser Gly Thr Ser Leu Asp Ala Gln Lys Arg

325 330 335

Tyr Ala Gln Gly Val Lys Asn Ile Leu Asn Ser Tyr Phe Ser Lys Lys
340 345 350

Phe Asp Tyr Arg Pro Gln Asp Ile Ile Val Gln Asn Gly Ser Tyr Ala
355 360 365

Thr Arg Ala Tyr Gly Gln Lys Lys
370 375

<210> 6
<211> 376
<212> PRT
<213> Saccharomyces cerevisiae

<400> 6

Met Ser Lys Gly Lys Val Leu Leu Val Leu Tyr Glu Gly Gly Lys His
1 5 10 15

Ala Glu Glu Gln Glu Lys Leu Leu Gly Cys Ile Glu Asn Glu Leu Gly
20 25 30

Ile Arg Asn Phe Ile Glu Glu Gln Gly Tyr Glu Leu Val Thr Thr Ile
35 40 45

Asp Lys Asp Pro Glu Pro Thr Ser Thr Val Asp Arg Glu Leu Lys Asp
50 55 60

Ala Glu Ile Val Ile Thr Thr Pro Phe Phe Pro Ala Tyr Ile Ser Arg
65 70 75 80

Asn Arg Ile Ala Glu Ala Pro Asn Leu Lys Leu Cys Val Thr Ala Gly
85 90 95

Val Gly Ser Asp His Val Asp Leu Glu Ala Ala Asn Glu Arg Lys Ile
100 105 110

Thr Val Thr Glu Val Thr Gly Ser Asn Val Val Ser Val Ala Glu His
115 120 125

Val Met Ala Thr Ile Leu Val Leu Ile Arg Asn Tyr Asn Gly Gly His
130 135 140

Gln Gln Ala Ile Asn Gly Glu Trp Asp Ile Ala Gly Val Ala Lys Asn
145 150 155 160

Glu Tyr Asp Leu Glu Asp Lys Ile Ile Ser Thr Val Gly Ala Gly Arg

165 170 175

Ile Gly Tyr Arg Val Leu Glu Arg Leu Val Ala Phe Asn Pro Lys Lys
180 185 190

Leu Leu Tyr Tyr Asp Tyr Gln Glu Leu Pro Ala Glu Ala Ile Asn Arg
195 200 205

Leu Asn Glu Ala Ser Lys Leu Phe Asn Gly Arg Gly Asp Ile Val Gln
210 215 220

Arg Val Glu Lys Leu Glu Asp Met Val Ala Gln Ser Asp Val Val Thr
225 230 235 240

Ile Asn Cys Pro Leu His Lys Asp Ser Arg Gly Leu Phe Asn Lys Lys
245 250 255

Leu Ile Ser His Met Lys Asp Gly Ala Tyr Leu Val Asn Thr Ala Arg
260 265 270

Gly Ala Ile Cys Val Ala Glu Asp Val Ala Glu Ala Val Lys Ser Gly
275 280 285

Lys Leu Ala Gly Tyr Gly Gly Asp Val Trp Asp Lys Gln Pro Ala Pro
290 295 300

Lys Asp His Pro Trp Arg Thr Met Asp Asn Lys Asp His Val Gly Asn
305 310 315 320

Ala Met Thr Val His Ile Ser Gly Thr Ser Leu Asp Ala Gln Lys Arg
325 330 335

Tyr Ala Gln Gly Val Lys Asn Ile Leu Asn Ser Tyr Phe Ser Lys Lys
340 345 350

Phe Asp Tyr Arg Pro Gln Asp Ile Ile Val Gln Asn Gly Ser Tyr Ala
355 360 365

Thr Arg Ala Tyr Gly Gln Lys Lys
370 375

<210> 7
<211> 316
<212> PRT
<213> Escherichia coli

<400> 7

Met Ser Lys Arg Lys Val Ala Ile Ile Gly Ser Gly Asn Ile Gly Thr

1 5 10 15

Asp Leu Met Ile Lys Ile Leu Arg His Gly Gln His Leu Glu Met Ala
20 25 30

Val Met Val Gly Ile Asp Pro Gln Ser Asp Gly Leu Ala Arg Ala Arg
35 40 45

Arg Met Gly Val Ala Thr Thr His Glu Gly Val Ile Gly Leu Met Asn
50 55 60

Met Pro Glu Phe Ala Asp Ile Asp Ile Val Phe Asp Ala Thr Ser Ala
65 70 75 80

Gly Ala His Val Lys Asn Asp Ala Ala Leu Arg Glu Ala Lys Pro Asp
85 90 95

Ile Arg Leu Ile Asp Leu Thr Pro Ala Ala Ile Gly Pro Tyr Cys Val
100 105 110

Pro Val Val Asn Leu Glu Ala Asn Val Asp Gln Leu Asn Val Asn Met
115 120 125

Val Thr Cys Gly Gly Gln Ala Thr Ile Pro Met Val Ala Ala Val Ser
130 135 140

Arg Val Ala Arg Val His Tyr Ala Glu Ile Ile Ala Ser Ile Ala Ser
145 150 155 160

Lys Ser Ala Gly Pro Gly Thr Arg Ala Asn Ile Asp Glu Phe Thr Glu
165 170 175

Thr Thr Ser Arg Ala Ile Glu Val Val Gly Gly Ala Ala Lys Gly Lys
180 185 190

Ala Ile Ile Val Leu Asn Pro Ala Glu Pro Pro Leu Met Met Arg Asp
195 200 205

Thr Val Tyr Val Leu Ser Asp Glu Ala Ser Gln Asp Asp Ile Glu Ala
210 215 220

Ser Ile Asn Glu Met Ala Glu Ala Val Gln Ala Tyr Val Pro Gly Tyr
225 230 235 240

Arg Leu Lys Gln Arg Val Gln Phe Glu Val Ile Pro Gln Asp Lys Pro
245 250 255

Val Asn Leu Pro Gly Val Gly Gln Phe Ser Gly Leu Lys Thr Ala Val
260 265 270

Trp Leu Glu Val Glu Gly Ala Ala His Tyr Leu Pro Ala Tyr Ala Gly
275 280 285

Asn Leu Asp Ile Met Thr Ser Ser Ala Leu Ala Thr Ala Glu Lys Met
290 295 300

Ala Gln Ser Leu Ala Arg Lys Ala Gly Glu Ala Ala
305 310 315

<210> 8
<211> 968
<212> DNA
<213> Artificial

<220>
<223> synthetic codon optimised E.coli mhpF

<400> 8

ctgcagtcta gatgtctaag agaaaggttg ctatcatcgg ttctggtaac atcggtactg 60

acttgatgat caagatccta agacacggtc aacacttgga aatggctgtt atggttggta 120

tcgacccaca atctgacggt ttggctagag ctagaagaat gggtgttgct accacccacg 180

aaggtgttat cggtttgatg aacatgccag aattcgctga catcgacatc gttttcgacg 240

ctacctctgc tggtgctcac gttaagaacg acgctgcttt gagagaagct aagccagaca 300

tcagattgat cgacttgacc ccagctgcta tcggtccata ctgtgttcca gttgttaact 360

tggaagctaa cgttgaccaa ttaaacgtta acatggttac ctgtggtggt caagctacca 420

tcccaatggt tgctgctgtt tcaagagttg ctagagttca ctacgctgaa atcatcgctt 480

ctatcgcttc taagtctgct ggtccaggta ccagagctaa catcgacgaa ttcaccgaaa 540

ccacctctag ggctatcgaa gttgttggtg gtgctgctaa gggtaaggct atcatcgttt 600

tgaacccagc tgaaccacca ttgatgatga gagacaccgt ttacgttttg tctgacgaag 660

catctcaaga cgacatcgaa gcttcaatca acgaaatggc tgaagctgtt caagcatacg 720

ttccaggtta cagattgaag caaagagttc aattcgaagt tatcccacaa gacaagccag 780

ttaacttgcc aggtgttggt caattctctg gtttgaagac cgctgtttgg ttggaagttg 840

aaggtgctgc tcactacttg ccagcttacg ctggtaactt ggacattatg acctcttctg 900

ctttggctac cgctgaaaag atggctcaat ctttggctag aaaggctggt gaagctgctt 960

aagcgcgc 968

<210> 9

<211> 891
<212> PRT
<213> Escherichia coli

<400> 9

Met Ala Val Thr Asn Val Ala Glu Leu Asn Ala Leu Val Glu Arg Val
1 5 10 15

Lys Lys Ala Gln Arg Glu Tyr Ala Ser Phe Thr Gln Glu Gln Val Asp
20 25 30

Lys Ile Phe Arg Ala Ala Ala Leu Ala Ala Ala Asp Ala Arg Ile Pro
35 40 45

Leu Ala Lys Met Ala Val Ala Glu Ser Gly Met Gly Ile Val Glu Asp
50 55 60

Lys Val Ile Lys Asn His Phe Ala Ser Glu Tyr Ile Tyr Asn Ala Tyr
65 70 75 80

Lys Asp Glu Lys Thr Cys Gly Val Leu Ser Glu Asp Asp Thr Phe Gly
85 90 95

Thr Ile Thr Ile Ala Glu Pro Ile Gly Ile Ile Cys Gly Ile Val Pro
100 105 110

Thr Thr Asn Pro Thr Ser Thr Ala Ile Phe Lys Ser Leu Ile Ser Leu
115 120 125

Lys Thr Arg Asn Ala Ile Ile Phe Ser Pro His Pro Arg Ala Lys Asp
130 135 140

Ala Thr Asn Lys Ala Ala Asp Ile Val Leu Gln Ala Ala Ile Ala Ala
145 150 155 160

Gly Ala Pro Lys Asp Leu Ile Gly Trp Ile Asp Gln Pro Ser Val Glu
165 170 175

Leu Ser Asn Ala Leu Met His His Pro Asp Ile Asn Leu Ile Leu Ala
180 185 190

Thr Gly Gly Pro Gly Met Val Lys Ala Ala Tyr Ser Ser Gly Lys Pro
195 200 205

Ala Ile Gly Val Gly Ala Gly Asn Thr Pro Val Val Ile Asp Glu Thr
210 215 220

Ala Asp Ile Lys Arg Ala Val Ala Ser Val Leu Met Ser Lys Thr Phe
225 230 235 240

Asp Asn Gly Val Ile Cys Ala Ser Glu Gln Ser Val Val Val Val Asp
245 250 255

Ser Val Tyr Asp Ala Val Arg Glu Arg Phe Ala Thr His Gly Gly Tyr
260 265 270

Leu Leu Gln Gly Lys Glu Leu Lys Ala Val Gln Asp Val Ile Leu Lys
275 280 285

Asn Gly Ala Leu Asn Ala Ala Ile Val Gly Gln Pro Ala Tyr Lys Ile
290 295 300

Ala Glu Leu Ala Gly Phe Ser Val Pro Glu Asn Thr Lys Ile Leu Ile
305 310 315 320

Gly Glu Val Thr Val Val Asp Glu Ser Glu Pro Phe Ala His Glu Lys
325 330 335

Leu Ser Pro Thr Leu Ala Met Tyr Arg Ala Lys Asp Phe Glu Asp Ala
340 345 350

Val Glu Lys Ala Glu Lys Leu Val Ala Met Gly Gly Ile Gly His Thr
355 360 365

Ser Cys Leu Tyr Thr Asp Gln Asp Asn Gln Pro Ala Arg Val Ser Tyr
370 375 380

Phe Gly Gln Lys Met Lys Thr Ala Arg Ile Leu Ile Asn Thr Pro Ala
385 390 395 400

Ser Gln Gly Gly Ile Gly Asp Leu Tyr Asn Phe Lys Leu Ala Pro Ser
405 410 415

Leu Thr Leu Gly Cys Gly Ser Trp Gly Gly Asn Ser Ile Ser Glu Asn
420 425 430

Val Gly Pro Lys His Leu Ile Asn Lys Lys Thr Val Ala Lys Arg Ala
435 440 445

Glu Asn Met Leu Trp His Lys Leu Pro Lys Ser Ile Tyr Phe Arg Arg
450 455 460

Gly Ser Leu Pro Ile Ala Leu Asp Glu Val Ile Thr Asp Gly His Lys
465 470 475 480

Arg Ala Leu Ile Val Thr Asp Arg Phe Leu Phe Asn Asn Gly Tyr Ala
485 490 495

Asp Gln Ile Thr Ser Val Leu Lys Ala Ala Gly Val Glu Thr Glu Val
500 505 510

Phe Phe Glu Val Glu Ala Asp Pro Thr Leu Ser Ile Val Arg Lys Gly
515 520 525

Ala Glu Leu Ala Asn Ser Phe Lys Pro Asp Val Ile Ile Ala Leu Gly
530 535 540

Gly Gly Ser Pro Met Asp Ala Ala Lys Ile Met Trp Val Met Tyr Glu
545 550 555 560

His Pro Glu Thr His Phe Glu Glu Leu Ala Leu Arg Phe Met Asp Ile
565 570 575

Arg Lys Arg Ile Tyr Lys Phe Pro Lys Met Gly Val Lys Ala Lys Met
580 585 590

Ile Ala Val Thr Thr Thr Ser Gly Thr Gly Ser Glu Val Thr Pro Phe
595 600 605

Ala Val Val Thr Asp Asp Ala Thr Gly Gln Lys Tyr Pro Leu Ala Asp
610 615 620

Tyr Ala Leu Thr Pro Asp Met Ala Ile Val Asp Ala Asn Leu Val Met
625 630 635 640

Asp Met Pro Lys Ser Leu Cys Ala Phe Gly Gly Leu Asp Ala Val Thr
645 650 655

His Ala Met Glu Ala Tyr Val Ser Val Leu Ala Ser Glu Phe Ser Asp
660 665 670

Gly Gln Ala Leu Gln Ala Leu Lys Leu Leu Lys Glu Tyr Leu Pro Ala
675 680 685

Ser Tyr His Glu Gly Ser Lys Asn Pro Val Ala Arg Glu Arg Val His
690 695 700

Ser Ala Ala Thr Ile Ala Gly Ile Ala Phe Ala Asn Ala Phe Leu Gly
705 710 715 720

Val Cys His Ser Met Ala His Lys Leu Gly Ser Gln Phe His Ile Pro
725 730 735

His Gly Leu Ala Asn Ala Leu Leu Ile Cys Asn Val Ile Arg Tyr Asn

740 745 750

Ala Asn Asp Asn Pro Thr Lys Gln Thr Ala Phe Ser Gln Tyr Asp Arg
755 760 765

Pro Gln Ala Arg Arg Arg Tyr Ala Glu Ile Ala Asp His Leu Gly Leu
770 775 780

Ser Ala Pro Gly Asp Arg Thr Ala Ala Lys Ile Glu Lys Leu Leu Ala
785 790 795 800

Trp Leu Glu Thr Leu Lys Ala Glu Leu Gly Ile Pro Lys Ser Ile Arg
805 810 815

Glu Ala Gly Val Gln Glu Ala Asp Phe Leu Ala Asn Val Asp Lys Leu
820 825 830

Ser Glu Asp Ala Phe Asp Asp Gln Cys Thr Gly Ala Asn Pro Arg Tyr
835 840 845

Pro Leu Ile Ser Glu Leu Lys Gln Ile Leu Leu Asp Thr Tyr Tyr Gly
850 855 860

Arg Asp Tyr Val Glu Gly Glu Thr Ala Ala Lys Lys Glu Ala Ala Pro
865 870 875 880

Ala Lys Ala Glu Lys Lys Ala Lys Lys Ser Ala
885 890

<210> 10
<211> 2690
<212> DNA
<213> Artificial

<220>
<223> synthetic codon optimised E.coli adhE

<400> 10

```
tctagaaaat ggctgttacc aacgttgctg aattgaacgc tttggttgaa agggttaaga     60
aggctcaaag agaatacgct tctttcaccc aagaacaagt tgacaagatc ttcagagctg    120
ctgctttggc tgctgctgac gctagaatcc cattggctaa gatggctgtt gctgaatctg    180
gtatgggtat cgttgaagac aaggttatca agaaccactt cgcttctgaa tacatctaca    240
acgcttacaa ggacgaaaag acctgtggtg ttttgtcaga agacgacacc ttcggtacca    300
tcaccatcgc tgaaccaatc ggtatcatct gtggtatcgt tccaaccacc aacccaacct    360
ctaccgctat cttcaagtct ttgatctctt tgaagaccag aaacgctatc atcttctctc    420
cacacccaag agctaaagac gctaccaaca aggctgctga catcgttttg caagctgcta    480
```

tcgctgctgg tgctccaaag gacttgatcg gttggatcga ccaaccatct gttgaattgt 540

ctaacgcttt gatgcaccac ccagacatca acttgatctt ggctaccggt ggtccaggta 600

tggttaaggc tgcttactct tctggtaagc cagctatcgg tgttggtgct ggtaacaccc 660

cagttgttat cgacgaaacc gctgacatca agagagctgt tgcttctgtt ttgatgtcta 720

agaccttcga caacggtgtt atctgtgctt ctgaacaatc tgttgttgtt gttgactctg 780

tttacgacgc tgttagagaa agattcgcta cccacggtgg ttacttgttg caaggtaagg 840

aattgaaggc tgttcaagac gttatcttga agaacggtgc tttgaacgct gctatcgttg 900

gtcaaccagc ttacaagatc gctgaattag ctggtttctc tgttccagaa aacaccaaga 960

tcttgatcgg tgaagttacc gttgttgacg aatctgaacc attcgctcac gaaaagttgt 1020

ctccaacctt ggctatgtac agagctaagg acttcgaaga cgctgttgaa aaagctgaaa 1080

agttggttgc tatgggtggt attggtcaca cctcttgttt gtacaccgac caagacaacc 1140

aaccagctag agtttcttac ttcggtcaaa agatgaagac cgctagaatc ttgatcaaca 1200

ccccagcttc tcaaggtggt atcggtgact tgtacaactt caagttggct ccatctttga 1260

ccttgggttg tggttcttgg ggtggtaact ctatctctga aaacgttggt ccaaagcact 1320

tgatcaacaa gaagaccgtt gctaagagag ctgaaaacat gttgtggcac aagttgccaa 1380

aatctatcta cttcagaaga ggttctttgc caatcgcttt ggacgaagtt atcaccgacg 1440

gtcacaagag agctttgatc gttaccgaca gattcttgtt caacaacggt tacgctgacc 1500

aaatcacctc tgttttgaag gctgctggtg ttgaaaccga agttttcttc gaagttgaag 1560

ctgacccaac cttgtctatc gttagaaagg gtgctgaatt ggctaactct ttcaagccag 1620

acgttatcat cgctttgggt ggtggttctc caatggacgc tgctaagatc atgtgggtta 1680

tgtacgaaca cccagaaacc cacttcgaag aattggcttt gagattcatg gacatcagaa 1740

agagaatcta caagttccca aagatgggtg ttaaggctaa gatgatcgct gttaccacca 1800

cctctggtac cggttctgaa gttaccccat tcgctgttgt taccgacgac gctaccggtc 1860

aaaagtaccc attggctgac tacgctttga ccccagacat ggctatcgtt gacgctaact 1920

tggttatgga catgccaaag tctttgtgtg ctttcggtgg tttggacgct gttacccacg 1980

ctatggaagc ttacgtttct gttttggctt ctgaattctc tgacggtcaa gctttgcaag 2040

ctttgaagtt gttgaaggaa tacttgccag cttcttacca cgaaggttct aagaacccag 2100

ttgctagaga aagagttcac tctgctgcta ccatcgctgg tatcgctttc gctaacgctt 2160

tcttgggtgt ttgtcactct atggctcaca agttgggttc tcaattccac atcccacacg 2220

gtttggctaa cgctttgttg atctgtaacg ttatcagata caacgctaac gacaacccaa 2280

ccaagcaaac cgctttctct caatacgaca gaccacaagc tagaagaaga tacgctgaaa 2340

```
tcgctgacca cttgggtttg tctgctccag gtgacagaac cgctgcaaag atcgaaaagt   2400
tgttggcttg gttggaaacc ttgaaggctg aattgggtat cccaaagtct atcagagaag   2460
ctggtgttca agaagctgac ttcttggcta acgttgacaa gttgtctgaa gacgctttcg   2520
acgaccaatg taccggtgct aacccaagat acccattgat ctctgaattg aagcaaatct   2580
tgttggacac ctactacggt agagactacg ttgaaggtga aaccgctgct aagaaggaag   2640
ctgctccagc taaggctgaa aagaaggcta agaagtctgc ttagcttaag               2690
```

<210> 11
<211> 870
<212> PRT
<213> Entamoeba histolytica

<400> 11

```
Met Ser Thr Gln Gln Thr Met Thr Val Asp Glu His Ile Asn Gln Leu
1               5                   10                  15

Val Arg Lys Ala Gln Val Ala Leu Lys Glu Tyr Leu Lys Pro Glu Tyr
            20                  25                  30

Thr Gln Glu Lys Ile Asp Tyr Ile Val Lys Lys Ala Ser Val Ala Ala
        35                  40                  45

Leu Asp Gln His Cys Ala Leu Ala Ala Ala Ala Val Glu Glu Thr Gly
    50                  55                  60

Arg Gly Ile Phe Glu Asp Lys Ala Thr Lys Asn Ile Phe Ala Cys Glu
65                  70                  75                  80

His Val Thr His Glu Met Arg His Ala Lys Thr Val Gly Ile Ile Asn
                85                  90                  95

Val Asp Pro Leu Tyr Gly Ile Thr Glu Ile Ala Glu Pro Val Gly Val
            100                 105                 110

Val Cys Gly Val Thr Pro Val Thr Asn Pro Thr Ser Thr Ala Ile Phe
        115                 120                 125

Lys Ser Leu Ile Ser Ile Lys Thr Arg Asn Pro Ile Val Phe Ser Phe
    130                 135                 140

His Pro Ser Ala Leu Lys Cys Ser Ile Met Ala Ala Lys Ile Val Arg
145                 150                 155                 160

Asp Ala Ala Ile Ala Ala Gly Ala Pro Glu Asn Cys Ile Gln Trp Ile
                165                 170                 175
```

Glu Phe Gly Gly Ile Glu Ala Ser Asn Lys Leu Met Asn His Pro Gly
180 185 190

Val Ala Thr Ile Leu Ala Thr Gly Gly Asn Ala Met Val Lys Ala Ala
195 200 205

Tyr Ser Ser Gly Lys Pro Ala Leu Gly Val Gly Ala Gly Asn Val Pro
210 215 220

Thr Tyr Ile Glu Lys Thr Cys Asn Ile Lys Gln Ala Ala Asn Asp Val
225 230 235 240

Val Met Ser Lys Ser Phe Asp Asn Gly Met Ile Cys Ala Ser Glu Gln
245 250 255

Ala Ala Ile Ile Asp Lys Glu Ile Tyr Asp Gln Val Val Glu Glu Met
260 265 270

Lys Thr Leu Gly Ala Tyr Phe Ile Asn Glu Glu Glu Lys Ala Lys Leu
275 280 285

Glu Lys Phe Met Phe Gly Val Asn Ala Tyr Ser Ala Asp Val Asn Asn
290 295 300

Ala Arg Leu Asn Pro Lys Cys Pro Gly Met Ser Pro Gln Trp Phe Ala
305 310 315 320

Glu Gln Val Gly Ile Lys Val Pro Glu Asp Cys Asn Ile Ile Cys Ala
325 330 335

Val Cys Lys Glu Val Gly Pro Asn Glu Pro Leu Thr Arg Glu Lys Leu
340 345 350

Ser Pro Val Leu Ala Ile Leu Lys Ala Glu Asn Thr Gln Asp Gly Ile
355 360 365

Asp Lys Ala Glu Ala Met Val Glu Phe Asn Gly Arg Gly His Ser Ala
370 375 380

Ala Ile His Ser Asn Asp Lys Ala Val Val Glu Lys Tyr Ala Leu Thr
385 390 395 400

Met Lys Ala Cys Arg Ile Leu His Asn Thr Pro Ser Ser Gln Gly Gly
405 410 415

Ile Gly Ser Ile Tyr Asn Tyr Ile Trp Pro Ser Phe Thr Leu Gly Cys
420 425 430

Gly Ser Tyr Gly Gly Asn Ser Val Ser Ala Asn Val Thr Tyr His Asn
435 440 445

Leu Leu Asn Ile Lys Arg Leu Ala Asp Arg Arg Asn Asn Leu Gln Trp
450 455 460

Phe Arg Val Pro Pro Lys Ile Phe Phe Glu Pro His Ser Ile Arg Tyr
465 470 475 480

Leu Ala Glu Leu Lys Glu Leu Ser Lys Ile Phe Ile Val Ser Asp Arg
485 490 495

Met Met Tyr Lys Leu Gly Tyr Val Asp Arg Val Met Asp Val Leu Lys
500 505 510

Arg Arg Ser Asn Glu Val Glu Ile Glu Ile Phe Ile Asp Val Glu Pro
515 520 525

Asp Pro Ser Ile Gln Thr Val Gln Lys Gly Leu Ala Val Met Asn Thr
530 535 540

Phe Gly Pro Asp Asn Ile Ile Ala Ile Gly Gly Gly Ser Ala Met Asp
545 550 555 560

Ala Ala Lys Ile Met Trp Leu Leu Tyr Glu His Pro Glu Ala Asp Phe
565 570 575

Phe Ala Met Lys Gln Lys Phe Ile Asp Leu Arg Lys Arg Ala Phe Lys
580 585 590

Phe Pro Thr Met Gly Lys Lys Ala Arg Leu Ile Cys Ile Pro Thr Thr
595 600 605

Ser Gly Thr Gly Ser Glu Val Thr Pro Phe Ala Val Ile Ser Asp His
610 615 620

Glu Thr Gly Lys Lys Tyr Pro Leu Ala Asp Tyr Ser Leu Thr Pro Ser
625 630 635 640

Val Ala Ile Val Asp Pro Met Phe Thr Met Ser Leu Pro Lys Arg Ala
645 650 655

Ile Ala Asp Thr Gly Leu Asp Val Leu Val His Ala Thr Glu Ala Tyr
660 665 670

Val Ser Val Met Ala Asn Glu Tyr Thr Asp Gly Leu Ala Arg Glu Ala

675 680 685

Val Lys Leu Val Phe Glu Asn Leu Leu Lys Ser Tyr Asn Gly Asp Leu
690 695 700

Glu Ala Arg Glu Lys Met His Asn Ala Ala Thr Ile Ala Gly Met Ala
705 710 715 720

Phe Ala Ser Ala Phe Leu Gly Met Asp His Ser Met Ala His Lys Val
725 730 735

Gly Ala Ala Phe His Leu Pro His Gly Arg Cys Val Ala Val Leu Leu
740 745 750

Pro His Val Ile Arg Tyr Asn Gly Gln Lys Pro Arg Lys Leu Ala Met
755 760 765

Trp Pro Lys Tyr Asn Phe Tyr Lys Ala Asp Gln Arg Tyr Met Glu Leu
770 775 780

Ala Gln Met Val Gly Leu Lys Cys Asn Thr Pro Ala Glu Gly Val Glu
785 790 795 800

Ala Phe Ala Lys Ala Cys Glu Glu Leu Met Lys Ala Thr Glu Thr Ile
805 810 815

Thr Gly Phe Lys Lys Ala Asn Ile Asp Glu Ala Ala Trp Met Ser Lys
820 825 830

Val Pro Glu Met Ala Leu Leu Ala Phe Glu Asp Gln Cys Ser Pro Ala
835 840 845

Asn Pro Arg Val Pro Met Val Lys Asp Met Glu Lys Ile Leu Lys Ala
850 855 860

Ala Tyr Tyr Pro Ile Ala
865 870

<210> 12
<211> 2627
<212> DNA
<213> Artificial

<220>
<223> synthetic codon optimised E. histolytica ADH2

<400> 12

tctagaaaat gtctacccaa caaaccatga ccgttgacga acacatcaac caattagtta 60

gaaaggctca agttgctttg aaggaatact tgaagccaga atacacccaa gaaaagatcg 120

```
actacatcgt taagaaggct tctgttgctg ctttggacca acactgtgct ttggctgctg    180
ctgctgttga agaaaccggt agaggtatct tcgaagacaa ggctaccaag aacatcttcg    240
cttgtgaaca cgttacccac gaaatgagac acgctaagac cgttggtatc atcaacgttg    300
acccattgta cggtatcacc gaaatcgctg aaccagttgg tgttgtttgt ggtgttaccc    360
cagttaccaa cccaacctct accgctatct tcaagtcttt gatctctatc aagaccagaa    420
acccaatcgt tttctctttc cacccatctg ctttgaagtg ttctattatg gctgctaaaa    480
tcgttagaga cgctgctatc gctgctggtg ctccagaaaa ctgtatccaa tggatcgaat    540
tcggtggtat cgaagcttct aacaagttga tgaaccaccc aggtgttgct actatcttgg    600
ctaccggtgg taacgctatg gttaaggctg catactcttc tggtaagcca gctttgggtg    660
ttggtgctgg taacgttcca acctacatcg aaaagacctg taacatcaag caagctgcta    720
acgacgttgt tatgtctaag tctttcgaca acggtatgat ctgtgcttct gaacaagctg    780
ctatcatcga caaggaaatc tacgaccaag ttgttgaaga aatgaagacc ttgggtgctt    840
acttcatcaa cgaagaagaa aaggctaagt tggaaaagtt catgttcggt gttaacgctt    900
actctgctga cgttaacaac gctagattga acccaaagtg tccaggtatg tctccacaat    960
ggttcgctga acaagttggt atcaaggtac cagaagactg taacatcatc tgtgctgttt   1020
gtaaggaagt tggtccaaac gaaccattga ccagagaaaa gttgtctcca gttttggcta   1080
tcttgaaagc tgaaaacacc caagacggta tcgacaaggc tgaagctatg gttgaattta   1140
acggtagagg tcactctgct gctatccact ctaacgacaa ggctgttgtt gaaaagtacg   1200
ctttgaccat gaaggcttgt agaatcttgc acaacacccc atcttctcaa ggtggtatcg   1260
gttctatcta caactacatc tggccatctt tcaccttggg ttgtggttct tacggtggta   1320
actctgtttc tgctaacgtt acctaccaca acttgttgaa catcaagaga ttggctgaca   1380
gaagaaacaa cttgcaatgg ttcagagttc caccaaagat cttcttcgaa ccacactcta   1440
tcagatactt ggctgaattg aaggaattgt ctaagatctt catcgtttct gacagaatga   1500
tgtacaagtt gggttacgtt gacagagtta tggacgtttt gaagagaaga tctaacgaag   1560
ttgaaatcga aatcttcatc gacgttgaac cagacccatc tatccaaacc gttcaaaagg   1620
gtttggctgt tatgaacacc ttcggtccag acaacatcat cgctatcggt ggtggttctg   1680
ctatggacgc tgctaagatc atgtggttgt tgtacgaaca cccagaagct gacttcttcg   1740
ctatgaagca aaagttcatc gacttgagaa agagagcttt caagttccca accatgggta   1800
agaaggctag attgatctgt atcccaacca cctctggtac cggttctgaa gttaccccat   1860
tcgctgttat ctctgaccac gaaaccggta agaagtaccc attggctgac tactctttga   1920
ccccatctgt tgctatcgtt gacccaatgt tcaccatgtc tttgccaaag agagctatcg   1980
```

ctgacaccgg tttggacgtt ttggttcacg ctaccgaagc ttacgtttct gttatggcta 2040

acgaatacac cgacggtttg gctagagaag ctgttaagtt ggtttttgaa aacttgttga 2100

agtcttacaa cggtgacttg gaagctagag aaaagatgca caacgctgct accatcgctg 2160

gtatggcttt cgcttctgct ttcttgggta tggaccactc tatggctcac aaggttggtg 2220

ctgctttcca cttgccacac ggtagatgtg ttgctgtttt gttgccacac gttatcagat 2280

acaacggtca aaagccaaga aagttggcta tgtggccaaa gtacaacttc tacaaggctg 2340

accaaagata catggaattg gctcaaatgg ttggtttgaa gtgtaacacc ccagctgaag 2400

gtgttgaagc tttcgctaag gcttgtgaag aattgatgaa ggctaccgaa accatcaccg 2460

gtttcaagaa ggctaacatc gacgaagctg cttggatgtc taaggttcca gaaatggctt 2520

tgttggcttt cgaagaccaa tgttctccag ctaacccaag agttccaatg gttaaggaca 2580

tggaaaagat cttgaaggct gcttactacc caatcgctta gcttaag 2627

<210> 13
<211> 312
<212> PRT
<213> Saccharomyces cerevisiae

<400> 13

Met Pro Ala Thr Leu His Asp Ser Thr Lys Ile Leu Ser Leu Asn Thr
1               5                   10                  15

Gly Ala Gln Ile Pro Gln Ile Gly Leu Gly Thr Trp Gln Ser Lys Glu
            20                  25                  30

Asn Asp Ala Tyr Lys Ala Val Leu Thr Ala Leu Lys Asp Gly Tyr Arg
        35                  40                  45

His Ile Asp Thr Ala Ala Ile Tyr Arg Asn Glu Asp Gln Val Gly Gln
    50                  55                  60

Ala Ile Lys Asp Ser Gly Val Pro Arg Glu Glu Ile Phe Val Thr Thr
65                  70                  75                  80

Lys Leu Trp Cys Thr Gln His His Glu Pro Glu Val Ala Leu Asp Gln
                85                  90                  95

Ser Leu Lys Arg Leu Gly Leu Asp Tyr Val Asp Leu Tyr Leu Met His
            100                 105                 110

Trp Pro Ala Arg Leu Asp Pro Ala Tyr Ile Lys Asn Glu Asp Ile Leu
        115                 120                 125

Ser Val Pro Thr Lys Lys Asp Gly Ser Arg Ala Val Asp Ile Thr Asn

130 135 140

Trp Asn Phe Ile Lys Thr Trp Glu Leu Met Gln Glu Leu Pro Lys Thr
145 150 155 160

Gly Lys Thr Lys Ala Val Gly Val Ser Asn Phe Ser Ile Asn Asn Leu
165 170 175

Lys Asp Leu Leu Ala Ser Gln Gly Asn Lys Leu Thr Pro Ala Ala Asn
180 185 190

Gln Val Glu Ile His Pro Leu Leu Pro Gln Asp Glu Leu Ile Asn Phe
195 200 205

Cys Lys Ser Lys Gly Ile Val Val Glu Ala Tyr Ser Pro Leu Gly Ser
210 215 220

Thr Asp Ala Pro Leu Leu Lys Glu Pro Val Ile Leu Glu Ile Ala Lys
225 230 235 240

Lys Asn Asn Val Gln Pro Gly His Val Val Ile Ser Trp His Val Gln
245 250 255

Arg Gly Tyr Val Val Leu Pro Lys Ser Val Asn Pro Asp Arg Ile Lys
260 265 270

Thr Asn Arg Lys Ile Phe Thr Leu Ser Thr Glu Asp Phe Glu Ala Ile
275 280 285

Asn Asn Ile Ser Lys Glu Lys Gly Glu Lys Arg Val Val His Pro Asn
290 295 300

Trp Ser Pro Phe Glu Val Phe Lys
305 310

<210> 14
<211> 584
<212> PRT
<213> Saccharomyces cerevisiae

<400> 14

Met Ser Ala Lys Ser Phe Glu Val Thr Asp Pro Val Asn Ser Ser Leu
1 5 10 15

Lys Gly Phe Ala Leu Ala Asn Pro Ser Ile Thr Leu Val Pro Glu Glu
20 25 30

Lys Ile Leu Phe Arg Lys Thr Asp Ser Asp Lys Ile Ala Leu Ile Ser

35 40 45

Gly Gly Gly Ser Gly His Glu Pro Thr His Ala Gly Phe Ile Gly Lys
50 55 60

Gly Met Leu Ser Gly Ala Val Val Gly Glu Ile Phe Ala Ser Pro Ser
65 70 75 80

Thr Lys Gln Ile Leu Asn Ala Ile Arg Leu Val Asn Glu Asn Ala Ser
85 90 95

Gly Val Leu Leu Ile Val Lys Asn Tyr Thr Gly Asp Val Leu His Phe
100 105 110

Gly Leu Ser Ala Glu Arg Ala Arg Ala Leu Gly Ile Asn Cys Arg Val
115 120 125

Ala Val Ile Gly Asp Asp Val Ala Val Gly Arg Glu Lys Gly Gly Met
130 135 140

Val Gly Arg Arg Ala Leu Ala Gly Thr Val Leu Val His Lys Ile Val
145 150 155 160

Gly Ala Phe Ala Glu Glu Tyr Ser Ser Lys Tyr Gly Leu Asp Gly Thr
165 170 175

Ala Lys Val Ala Lys Ile Ile Asn Asp Asn Leu Val Thr Ile Gly Ser
180 185 190

Ser Leu Asp His Cys Lys Val Pro Gly Arg Lys Phe Glu Ser Glu Leu
195 200 205

Asn Glu Lys Gln Met Glu Leu Gly Met Gly Ile His Asn Glu Pro Gly
210 215 220

Val Lys Val Leu Asp Pro Ile Pro Ser Thr Glu Asp Leu Ile Ser Lys
225 230 235 240

Tyr Met Leu Pro Lys Leu Leu Asp Pro Asn Asp Lys Asp Arg Ala Phe
245 250 255

Val Lys Phe Asp Glu Asp Asp Glu Val Val Leu Leu Val Asn Asn Leu
260 265 270

Gly Gly Val Ser Asn Phe Val Ile Ser Ser Ile Thr Ser Lys Thr Thr
275 280 285

Asp Phe Leu Lys Glu Asn Tyr Asn Ile Thr Pro Val Gln Thr Ile Ala
290 295 300

Gly Thr Leu Met Thr Ser Phe Asn Gly Asn Gly Phe Ser Ile Thr Leu
305 310 315 320

Leu Asn Ala Thr Lys Ala Thr Lys Ala Leu Gln Ser Asp Phe Glu Glu
325 330 335

Ile Lys Ser Val Leu Asp Leu Leu Asn Ala Phe Thr Asn Ala Pro Gly
340 345 350

Trp Pro Ile Ala Asp Phe Glu Lys Thr Ser Ala Pro Ser Val Asn Asp
355 360 365

Asp Leu Leu His Asn Glu Val Thr Ala Lys Ala Val Gly Thr Tyr Asp
370 375 380

Phe Asp Lys Phe Ala Glu Trp Met Lys Ser Gly Ala Glu Gln Val Ile
385 390 395 400

Lys Ser Glu Pro His Ile Thr Glu Leu Asp Asn Gln Val Gly Asp Gly
405 410 415

Asp Cys Gly Tyr Thr Leu Val Ala Gly Val Lys Gly Ile Thr Glu Asn
420 425 430

Leu Asp Lys Leu Ser Lys Asp Ser Leu Ser Gln Ala Val Ala Gln Ile
435 440 445

Ser Asp Phe Ile Glu Gly Ser Met Gly Gly Thr Ser Gly Gly Leu Tyr
450 455 460

Ser Ile Leu Leu Ser Gly Phe Ser His Gly Leu Ile Gln Val Cys Lys
465 470 475 480

Ser Lys Asp Glu Pro Val Thr Lys Glu Ile Val Ala Lys Ser Leu Gly
485 490 495

Ile Ala Leu Asp Thr Leu Tyr Lys Tyr Thr Lys Ala Arg Lys Gly Ser
500 505 510

Ser Thr Met Ile Asp Ala Leu Glu Pro Phe Val Lys Glu Phe Thr Ala
515 520 525

Ser Lys Asp Phe Asn Lys Ala Val Lys Ala Ala Glu Glu Gly Ala Lys
530 535 540

Ser Thr Ala Thr Phe Glu Ala Lys Phe Gly Arg Ala Ser Tyr Val Gly
545 550 555 560

Asp Ser Ser Gln Val Glu Asp Pro Gly Ala Val Gly Leu Cys Glu Phe
565 570 575

Leu Lys Gly Val Gln Ser Ala Leu
580

<210> 15
<211> 591
<212> PRT
<213> Saccharomyces cerevisiae

<400> 15

Met Ser His Lys Gln Phe Lys Ser Asp Gly Asn Ile Val Thr Pro Tyr
1 5 10 15

Leu Leu Gly Leu Ala Arg Ser Asn Pro Gly Leu Thr Val Ile Lys His
20 25 30

Asp Arg Val Val Phe Arg Thr Ala Ser Ala Pro Asn Ser Gly Asn Pro
35 40 45

Pro Lys Val Ser Leu Val Ser Gly Gly Gly Ser Gly His Glu Pro Thr
50 55 60

His Ala Gly Phe Val Gly Glu Gly Ala Leu Asp Ala Ile Ala Ala Gly
65 70 75 80

Ala Ile Phe Ala Ser Pro Ser Thr Lys Gln Ile Tyr Ser Ala Ile Lys
85 90 95

Ala Val Glu Ser Pro Lys Gly Thr Leu Ile Ile Val Lys Asn Tyr Thr
100 105 110

Gly Asp Ile Ile His Phe Gly Leu Ala Ala Glu Arg Ala Lys Ala Ala
115 120 125

Gly Met Lys Val Glu Leu Val Ala Val Gly Asp Asp Val Ser Val Gly
130 135 140

Lys Lys Lys Gly Ser Leu Val Gly Arg Arg Gly Leu Gly Ala Thr Val
145 150 155 160

Leu Val His Lys Ile Ala Gly Ala Ala Ala Ser His Gly Leu Glu Leu
165 170 175

```
Ala Glu Val Ala Glu Val Ala Gln Ser Val Val Asp Asn Ser Val Thr
            180                 185                 190

Ile Ala Ala Ser Leu Asp His Cys Thr Val Pro Gly His Lys Pro Glu
        195                 200                 205

Ala Ile Leu Gly Glu Asn Glu Tyr Glu Ile Gly Met Gly Ile His Asn
    210                 215                 220

Glu Ser Gly Thr Tyr Lys Ser Ser Pro Leu Pro Ser Ile Ser Glu Leu
225                 230                 235                 240

Val Ser Gln Met Leu Pro Leu Leu Leu Asp Glu Asp Glu Asp Arg Ser
                245                 250                 255

Tyr Val Lys Phe Glu Pro Lys Glu Asp Val Val Leu Met Val Asn Asn
            260                 265                 270

Met Gly Gly Met Ser Asn Leu Glu Leu Gly Tyr Ala Ala Glu Val Ile
        275                 280                 285

Ser Glu Gln Leu Ile Asp Lys Tyr Gln Ile Val Pro Lys Arg Thr Ile
    290                 295                 300

Thr Gly Ala Phe Ile Thr Ala Leu Asn Gly Pro Gly Phe Gly Ile Thr
305                 310                 315                 320

Leu Met Asn Ala Ser Lys Ala Gly Gly Asp Ile Leu Lys Tyr Phe Asp
                325                 330                 335

Tyr Pro Thr Thr Ala Ser Gly Trp Asn Gln Met Tyr His Ser Ala Lys
            340                 345                 350

Asp Trp Glu Val Leu Ala Lys Gly Gln Val Pro Thr Ala Pro Ser Leu
        355                 360                 365

Lys Thr Leu Arg Asn Glu Lys Gly Ser Gly Val Lys Ala Asp Tyr Asp
    370                 375                 380

Thr Phe Ala Lys Ile Leu Leu Ala Gly Ile Ala Lys Ile Asn Glu Val
385                 390                 395                 400

Glu Pro Lys Val Thr Trp Tyr Asp Thr Ile Ala Gly Asp Gly Asp Cys
                405                 410                 415

Gly Thr Thr Leu Val Ser Gly Gly Glu Ala Leu Glu Glu Ala Ile Lys
            420                 425                 430
```

Asn His Thr Leu Arg Leu Glu Asp Ala Ala Leu Gly Ile Glu Asp Ile
435 440 445

Ala Tyr Met Val Glu Asp Ser Met Gly Gly Thr Ser Gly Gly Leu Tyr
450 455 460

Ser Ile Tyr Leu Ser Ala Leu Ala Gln Gly Val Arg Asp Ser Gly Asp
465 470 475 480

Lys Glu Leu Thr Ala Glu Thr Phe Lys Lys Ala Ser Asn Val Ala Leu
485 490 495

Asp Ala Leu Tyr Lys Tyr Thr Arg Ala Arg Pro Gly Tyr Arg Thr Leu
500 505 510

Ile Asp Ala Leu Gln Pro Phe Val Glu Ala Leu Lys Ala Gly Lys Gly
515 520 525

Pro Arg Ala Ala Ala Gln Ala Ala Tyr Asp Gly Ala Glu Lys Thr Arg
530 535 540

Lys Met Asp Ala Leu Val Gly Arg Ala Ser Tyr Val Ala Lys Glu Glu
545 550 555 560

Leu Arg Lys Leu Asp Ser Glu Gly Gly Leu Pro Asp Pro Gly Ala Val
565 570 575

Gly Leu Ala Ala Leu Leu Asp Gly Phe Val Thr Ala Ala Gly Tyr
580 585 590

<210> 16
<211> 560
<212> PRT
<213> Saccharomyces cerevisiae

<400> 16

Met Ser Leu Ile Ser Ile Leu Ser Pro Leu Ile Thr Ser Glu Gly Leu
1 5 10 15

Asp Ser Arg Ile Lys Pro Ser Pro Lys Lys Asp Ala Ser Thr Thr Thr
20 25 30

Lys Pro Ser Leu Trp Lys Thr Thr Glu Phe Lys Phe Tyr Tyr Ile Ala
35 40 45

Phe Leu Val Val Val Pro Leu Met Phe Tyr Ala Gly Leu Gln Ala Ser
50 55 60

```
Ser Pro Glu Asn Pro Asn Tyr Ala Arg Tyr Glu Arg Leu Leu Ser Gln
65                  70                  75                  80

Gly Trp Leu Phe Gly Arg Lys Val Asp Asn Ser Asp Ser Gln Tyr Arg
                85                  90                  95

Phe Phe Arg Asp Asn Phe Ala Leu Leu Ser Val Leu Met Leu Val His
            100                 105                 110

Thr Ser Ile Lys Arg Ile Val Leu Tyr Ser Thr Asn Ile Thr Lys Leu
        115                 120                 125

Arg Phe Asp Leu Ile Phe Gly Leu Ile Phe Leu Val Ala Ala His Gly
    130                 135                 140

Val Asn Ser Ile Arg Ile Leu Ala His Met Leu Ile Leu Tyr Ala Ile
145                 150                 155                 160

Ala His Val Leu Lys Asn Phe Arg Arg Ile Ala Thr Ile Ser Ile Trp
                165                 170                 175

Ile Tyr Gly Ile Ser Thr Leu Phe Ile Asn Asp Asn Phe Arg Ala Tyr
            180                 185                 190

Pro Phe Gly Asn Ile Cys Ser Phe Leu Ser Pro Leu Asp His Trp Tyr
        195                 200                 205

Arg Gly Ile Ile Pro Arg Trp Asp Val Phe Phe Asn Phe Thr Leu Leu
    210                 215                 220

Arg Val Leu Ser Tyr Asn Leu Asp Phe Leu Glu Arg Trp Glu Asn Leu
225                 230                 235                 240

Gln Lys Lys Lys Ser Pro Ser Tyr Glu Ser Lys Glu Ala Lys Ser Ala
                245                 250                 255

Ile Leu Leu Asn Glu Arg Ala Arg Leu Thr Ala Ala His Pro Ile Gln
            260                 265                 270

Asp Tyr Ser Leu Met Asn Tyr Ile Ala Tyr Val Thr Tyr Thr Pro Leu
        275                 280                 285

Phe Ile Ala Gly Pro Ile Ile Thr Phe Asn Asp Tyr Val Tyr Gln Ser
    290                 295                 300

Lys His Thr Leu Pro Ser Ile Asn Phe Lys Phe Ile Phe Tyr Tyr Ala
```

305 310 315 320

Val Arg Phe Val Ile Ala Leu Leu Ser Met Glu Phe Ile Leu His Phe
325 330 335

Leu His Val Val Ala Ile Ser Lys Thr Lys Ala Trp Glu Asn Asp Thr
340 345 350

Pro Phe Gln Ile Ser Met Ile Gly Leu Phe Asn Leu Asn Ile Ile Trp
355 360 365

Leu Lys Leu Leu Ile Pro Trp Arg Leu Phe Arg Leu Trp Ala Leu Leu
370 375 380

Asp Gly Ile Asp Thr Pro Glu Asn Met Ile Arg Cys Val Asp Asn Asn
385 390 395 400

Tyr Ser Ser Leu Ala Phe Trp Arg Ala Trp His Arg Ser Tyr Asn Lys
405 410 415

Trp Val Val Arg Tyr Ile Tyr Ile Pro Leu Gly Gly Ser Lys Asn Arg
420 425 430

Val Leu Thr Ser Leu Ala Val Phe Ser Phe Val Ala Ile Trp His Asp
435 440 445

Ile Glu Leu Lys Leu Leu Leu Trp Gly Trp Leu Ile Val Leu Phe Leu
450 455 460

Leu Pro Glu Ile Phe Ala Thr Gln Ile Phe Ser His Tyr Thr Asp Ala
465 470 475 480

Val Trp Tyr Arg His Val Cys Ala Val Gly Ala Val Phe Asn Ile Trp
485 490 495

Val Met Met Ile Ala Asn Leu Phe Gly Phe Cys Leu Gly Ser Asp Gly
500 505 510

Thr Lys Lys Leu Leu Ser Asp Met Phe Cys Thr Val Ser Gly Phe Lys
515 520 525

Phe Val Ile Leu Ala Ser Val Ser Leu Phe Ile Ala Val Gln Ile Met
530 535 540

Phe Glu Ile Arg Glu Glu Glu Lys Arg His Gly Ile Tyr Leu Lys Cys
545 550 555 560

<210> 17
<211> 609
<212> PRT
<213> Saccharomyces cerevisiae

<400> 17

```
Met Ser Met Leu Arg Ile Trp Ser Cys Ile Val His Phe Phe Ser Val
1               5                   10                  15

Gln Ala Leu Asp Ser Arg Ile Lys Pro Asp Ile Glu Phe Lys Arg Arg
            20                  25                  30

Gln Arg Ile Phe Ile Asn Ser Ser Lys Glu Glu Asn Gly Ser Ser Ser
        35                  40                  45

Ser Ala Val Thr Val Thr Arg Asn Pro Val Leu Ser Ser Asn Ser Pro
    50                  55                  60

Ser Pro Pro Leu Trp Asn Thr Trp Glu Phe Arg Leu Tyr Tyr Leu Ala
65                  70                  75                  80

Phe Thr Val Val Val Pro Phe Met Ile Lys Ala Ala Leu Ala Thr Ser
                85                  90                  95

Ser Glu Ser Asn Pro Asn Tyr Tyr Lys Phe Ser Gly Leu Leu Ala His
            100                 105                 110

Gly Trp Ile Leu Gly Arg Lys Val Asp Asn Ser Asp Pro Gln Tyr Arg
        115                 120                 125

Phe Phe Arg Ser Asn Phe Phe Leu Leu Ala Ile Leu Ile Leu Leu Gln
    130                 135                 140

Ile Ile Leu Lys Lys Val Phe Val Lys Phe Ser Lys Ile Pro Lys Thr
145                 150                 155                 160

Lys Phe Asp Phe Ala Cys Gly Leu Val Phe Val Cys Phe Met Tyr Gly
                165                 170                 175

Ile Asn Ser Val Lys Leu Phe Thr His Ala Phe Ile Phe Phe Thr Leu
            180                 185                 190

Ala His Ser Leu Lys Arg Lys Arg Leu Ile Ala Ala Phe Ala Ile Trp
        195                 200                 205

Ser Tyr Gly Ile Phe Thr Leu Phe Ile Asn Gln Lys Met Lys Asn Leu
    210                 215                 220
```

Pro Phe Asn Asn Ile Ala Ile Ile Leu Ser Pro Met Asp Gln Trp Tyr
225 230 235 240

Lys Gly Ile Val Pro Arg Trp Asp Phe Phe Phe Asn Phe Thr Leu Leu
245 250 255

Arg Leu Leu Ser Tyr Ser Met Asp Phe Leu Glu Arg Trp His Glu Gln
260 265 270

Leu Ser Arg Gln Pro Ser Ile Asp Tyr Asp Asp Arg Arg Pro Glu Phe
275 280 285

Arg Lys Ser Leu Ser Gly Ser Thr Leu Gln Thr Ile Tyr Glu Ser Gly
290 295 300

Lys Asn Val Leu Glu Glu Lys Glu Arg Leu Val Ala Glu His His Ile
305 310 315 320

Gln Asp Tyr Asn Phe Ile Asn Phe Ile Ala Tyr Ile Thr Tyr Ala Pro
325 330 335

Leu Phe Leu Val Gly Pro Ile Ile Thr Phe Asn Asp Tyr Leu Tyr Gln
340 345 350

Ser Glu Asn Lys Leu Pro Ser Leu Thr Lys Lys Asn Ile Gly Phe Tyr
355 360 365

Ala Leu Lys Val Phe Ser Ser Leu Leu Leu Met Glu Ile Ile Leu His
370 375 380

Tyr Ile Tyr Val Gly Ala Ile Ala Arg Thr Lys Ala Trp Asn Asn Asp
385 390 395 400

Thr Pro Leu Gln Gln Ala Met Ile Ala Leu Phe Asn Leu Asn Ile Met
405 410 415

Tyr Leu Lys Leu Leu Ile Pro Trp Arg Leu Phe Arg Leu Trp Ala Met
420 425 430

Val Asp Gly Ile Asp Ala Pro Glu Asn Met Leu Arg Cys Val Asp Asn
435 440 445

Asn Tyr Ser Thr Val Gly Phe Trp Arg Ala Trp His Thr Ser Phe Asn
450 455 460

Lys Trp Val Ile Arg Tyr Ile Tyr Val Pro Phe Gly Gly Ser Asn Asn
465 470 475 480

Lys Ile Leu Thr Ser Phe Ala Val Phe Ser Phe Val Ala Ile Trp His
485 490 495

Asp Ile Gln Leu Arg Val Leu Phe Trp Gly Trp Leu Thr Val Leu Leu
500 505 510

Leu Leu Gly Glu Thr Tyr Ile Thr Asn Cys Phe Ser Arg Tyr Arg Phe
515 520 525

Arg Ser Trp Tyr Arg Phe Val Cys Gly Ile Gly Ala Ala Ile Asn Ile
530 535 540

Cys Met Met Met Ile Ile Asn Val Tyr Gly Phe Cys Leu Gly Ala Glu
545 550 555 560

Gly Thr Lys Leu Leu Leu Lys Gly Ile Phe Asn Asn Ser His Ser Pro
565 570 575

Glu Phe Leu Thr Ala Val Met Val Ser Leu Phe Ile Ala Val Gln Val
580 585 590

Met Phe Glu Ile Arg Glu Glu Glu Lys Arg His Gly Ile Asn Leu Lys
595 600 605

Cys

<210> 18
<211> 669
<212> PRT
<213> Saccharomyces cerevisiae

<400> 18

Met Ser Asn Pro Gln Lys Ala Leu Asn Asp Phe Leu Ser Ser Glu Ser
1 5 10 15

Val His Thr His Asp Ser Ser Arg Lys Gln Ser Asn Lys Gln Ser Ser
20 25 30

Asp Glu Gly Arg Ser Ser Ser Gln Pro Ser His His His Ser Gly Gly
35 40 45

Thr Asn Asn Asn Asn Asn Asn Asn Asn Asn Asn Asn Asn Ser Asn Asn
50 55 60

Asn Asn Asn Gly Asn Asp Gly Gly Asn Asp Asp Asp Tyr Asp Tyr Glu
65 70 75 80

```
Met Gln Asp Tyr Arg Pro Ser Pro Gln Ser Ala Arg Pro Thr Pro Thr
                85                  90                  95

Tyr Val Pro Gln Tyr Ser Val Glu Ser Gly Thr Ala Phe Pro Ile Gln
            100                 105                 110

Glu Val Ile Pro Ser Ala Tyr Ile Asn Thr Gln Asp Ile Asn His Lys
        115                 120                 125

Asp Asn Gly Pro Pro Ser Ala Ser Ser Asn Arg Ala Phe Arg Pro Arg
    130                 135                 140

Gly Gln Thr Thr Val Ser Ala Asn Val Leu Asn Ile Glu Asp Phe Tyr
145                 150                 155                 160

Lys Asn Ala Asp Asp Ala His Thr Ile Pro Glu Ser His Leu Ser Arg
                165                 170                 175

Arg Arg Ser Arg Ser Arg Ala Thr Ser Asn Ala Gly His Ser Ala Asn
            180                 185                 190

Thr Gly Ala Thr Asn Gly Arg Thr Thr Gly Ala Gln Thr Asn Met Glu
        195                 200                 205

Ser Asn Glu Ser Pro Arg Asn Val Pro Ile Met Val Lys Pro Lys Thr
    210                 215                 220

Leu Tyr Gln Asn Pro Gln Thr Pro Thr Val Leu Pro Ser Thr Tyr His
225                 230                 235                 240

Pro Ile Asn Lys Trp Ser Ser Val Lys Asn Thr Tyr Leu Lys Glu Phe
                245                 250                 255

Leu Ala Glu Phe Met Gly Thr Met Val Met Ile Ile Phe Gly Ser Ala
            260                 265                 270

Val Val Cys Gln Val Asn Val Ala Gly Lys Ile Gln Gln Asp Asn Phe
        275                 280                 285

Asn Val Ala Leu Asp Asn Leu Asn Val Thr Gly Ser Ser Ala Glu Thr
    290                 295                 300

Ile Asp Ala Met Lys Ser Leu Thr Ser Leu Val Ser Ser Val Ala Gly
305                 310                 315                 320

Gly Thr Phe Asp Asp Val Ala Leu Gly Trp Ala Ala Ala Val Val Met
                325                 330                 335
```

Gly Tyr Phe Cys Ala Gly Gly Ser Ala Ile Ser Gly Ala His Leu Asn
340 345 350

Pro Ser Ile Thr Leu Ala Asn Leu Val Tyr Arg Gly Phe Pro Leu Lys
355 360 365

Lys Val Pro Tyr Tyr Phe Ala Gly Gln Leu Ile Gly Ala Phe Thr Gly
370 375 380

Ala Leu Ile Leu Phe Ile Trp Tyr Lys Arg Val Leu Gln Glu Ala Tyr
385 390 395 400

Ser Asp Trp Trp Met Asn Glu Ser Val Ala Gly Met Phe Cys Val Phe
405 410 415

Pro Lys Pro Tyr Leu Ser Ser Gly Arg Gln Phe Phe Ser Glu Phe Leu
420 425 430

Cys Gly Ala Met Leu Gln Ala Gly Thr Phe Ala Leu Thr Asp Pro Tyr
435 440 445

Thr Cys Leu Ser Ser Asp Val Phe Pro Leu Met Met Phe Ile Leu Ile
450 455 460

Phe Ile Ile Asn Ala Ser Met Ala Tyr Gln Thr Gly Thr Ala Met Asn
465 470 475 480

Leu Ala Arg Asp Leu Gly Pro Arg Leu Ala Leu Tyr Ala Val Gly Phe
485 490 495

Asp His Lys Met Leu Trp Val His His His His Phe Phe Trp Val Pro
500 505 510

Met Val Gly Pro Phe Ile Gly Ala Leu Met Gly Gly Leu Val Tyr Asp
515 520 525

Val Cys Ile Tyr Gln Gly His Glu Ser Pro Val Asn Trp Ser Leu Pro
530 535 540

Val Tyr Lys Glu Met Ile Met Arg Ala Trp Phe Arg Arg Pro Gly Trp
545 550 555 560

Lys Lys Arg Asn Arg Ala Arg Arg Thr Ser Asp Leu Ser Asp Phe Ser
565 570 575

Tyr Asn Asn Asp Asp Asp Glu Glu Phe Gly Glu Arg Met Ala Leu Gln

580 585 590

Lys Thr Lys Thr Lys Ser Ser Ile Ser Asp Asn Glu Asn Glu Ala Gly
595 600 605

Glu Lys Lys Val Gln Phe Lys Ser Val Gln Arg Gly Lys Arg Thr Phe
610 615 620

Gly Gly Ile Pro Thr Ile Leu Glu Glu Glu Asp Ser Ile Glu Thr Ala
625 630 635 640

Ser Leu Gly Ala Thr Thr Thr Asp Ser Ile Gly Leu Ser Asp Thr Ser
645 650 655

Ser Glu Asp Ser His Tyr Gly Asn Ala Lys Lys Val Thr
660 665

<210> 19
<211> 713
<212> PRT
<213> Saccharomyces cerevisiae

<400> 19

```
Met Ser Pro Ser Ala Val Gln Ser Ser Lys Leu Glu Glu Gln Ser Ser
1               5                   10                  15

Glu Ile Asp Lys Leu Lys Ala Lys Met Ser Gln Ser Ala Ala Thr Ala
            20                  25                  30

Gln Gln Lys Lys Glu His Glu Tyr Glu His Leu Thr Ser Val Lys Ile
        35                  40                  45

Val Pro Gln Arg Pro Ile Ser Asp Arg Leu Gln Pro Ala Ile Ala Thr
    50                  55                  60

His Tyr Ser Pro His Leu Asp Gly Leu Gln Asp Tyr Gln Arg Leu His
65                  70                  75                  80

Lys Glu Ser Ile Glu Asp Pro Ala Lys Phe Phe Gly Ser Lys Ala Thr
                85                  90                  95

Gln Phe Leu Asn Trp Ser Lys Pro Phe Asp Lys Val Phe Ile Pro Asp
            100                 105                 110

Pro Lys Thr Gly Arg Pro Ser Phe Gln Asn Asn Ala Trp Phe Leu Asn
        115                 120                 125

Gly Gln Leu Asn Ala Cys Tyr Asn Cys Val Asp Arg His Ala Leu Lys
```

```
        130                 135                 140

Thr Pro Asn Lys Lys Ala Ile Ile Phe Glu Gly Asp Glu Pro Gly Gln
145                 150                 155                 160

Gly Tyr Ser Ile Thr Tyr Lys Glu Leu Leu Glu Glu Val Cys Gln Val
                165                 170                 175

Ala Gln Val Leu Thr Tyr Ser Met Gly Val Arg Lys Gly Asp Thr Val
            180                 185                 190

Ala Val Tyr Met Pro Met Val Pro Glu Ala Ile Ile Thr Leu Leu Ala
        195                 200                 205

Ile Ser Arg Ile Gly Ala Ile His Ser Val Val Phe Ala Gly Phe Ser
    210                 215                 220

Ser Asn Ser Leu Arg Asp Arg Ile Asn Asp Gly Asp Ser Lys Val Val
225                 230                 235                 240

Ile Thr Thr Asp Glu Ser Asn Arg Gly Gly Lys Val Ile Glu Thr Lys
                245                 250                 255

Arg Ile Val Asp Asp Ala Leu Arg Glu Thr Pro Gly Val Arg His Val
            260                 265                 270

Leu Val Tyr Arg Lys Thr Asn Asn Pro Ser Val Ala Phe His Ala Pro
        275                 280                 285

Arg Asp Leu Asp Trp Ala Thr Glu Lys Lys Lys Tyr Lys Thr Tyr Tyr
    290                 295                 300

Pro Cys Thr Pro Val Asp Ser Glu Asp Pro Leu Phe Leu Leu Tyr Thr
305                 310                 315                 320

Ser Gly Ser Thr Gly Ala Pro Lys Gly Val Gln His Ser Thr Ala Gly
                325                 330                 335

Tyr Leu Leu Gly Ala Leu Leu Thr Met Arg Tyr Thr Phe Asp Thr His
            340                 345                 350

Gln Glu Asp Val Phe Phe Thr Ala Gly Asp Ile Gly Trp Ile Thr Gly
        355                 360                 365

His Thr Tyr Val Val Tyr Gly Pro Leu Leu Tyr Gly Cys Ala Thr Leu
    370                 375                 380
```

Val Phe Glu Gly Thr Pro Ala Tyr Pro Asn Tyr Ser Arg Tyr Trp Asp
385 390 395 400

Ile Ile Asp Glu His Lys Val Thr Gln Phe Tyr Val Ala Pro Thr Ala
405 410 415

Leu Arg Leu Leu Lys Arg Ala Gly Asp Ser Tyr Ile Glu Asn His Ser
420 425 430

Leu Lys Ser Leu Arg Cys Leu Gly Ser Val Gly Glu Pro Ile Ala Ala
435 440 445

Glu Val Trp Glu Trp Tyr Ser Glu Lys Ile Gly Lys Asn Glu Ile Pro
450 455 460

Ile Val Asp Thr Tyr Trp Gln Thr Glu Ser Gly Ser His Leu Val Thr
465 470 475 480

Pro Leu Ala Gly Gly Val Thr Pro Met Lys Pro Gly Ser Ala Ser Phe
485 490 495

Pro Phe Phe Gly Ile Asp Ala Val Val Leu Asp Pro Asn Thr Gly Glu
500 505 510

Glu Leu Asn Thr Ser His Ala Glu Gly Val Leu Ala Val Lys Ala Ala
515 520 525

Trp Pro Ser Phe Ala Arg Thr Ile Trp Lys Asn His Asp Arg Tyr Leu
530 535 540

Asp Thr Tyr Leu Asn Pro Tyr Pro Gly Tyr Tyr Phe Thr Gly Asp Gly
545 550 555 560

Ala Ala Lys Asp Lys Asp Gly Tyr Ile Trp Ile Leu Gly Arg Val Asp
565 570 575

Asp Val Val Asn Val Ser Gly His Arg Leu Ser Thr Ala Glu Ile Glu
580 585 590

Ala Ala Ile Ile Glu Asp Pro Ile Val Ala Glu Cys Ala Val Val Gly
595 600 605

Phe Asn Asp Asp Leu Thr Gly Gln Ala Val Ala Ala Phe Val Val Leu
610 615 620

Lys Asn Lys Ser Ser Trp Ser Thr Ala Thr Asp Asp Glu Leu Gln Asp
625 630 635 640

Ile Lys Lys His Leu Val Phe Thr Val Arg Lys Asp Ile Gly Pro Phe
645 650 655

Ala Ala Pro Lys Leu Ile Ile Leu Val Asp Asp Leu Pro Lys Thr Arg
660 665 670

Ser Gly Lys Ile Met Arg Arg Ile Leu Arg Lys Ile Leu Ala Gly Glu
675 680 685

Ser Asp Gln Leu Gly Asp Val Ser Thr Leu Ser Asn Pro Gly Ile Val
690 695 700

Arg His Leu Ile Asp Ser Val Lys Leu
705 710

<210> 20
<211> 683
<212> PRT
<213> Saccharomyces cerevisiae

<400> 20

Met Thr Ile Lys Glu His Lys Val Val Tyr Glu Ala His Asn Val Lys
1 5 10 15

Ala Leu Lys Ala Pro Gln His Phe Tyr Asn Ser Gln Pro Gly Lys Gly
20 25 30

Tyr Val Thr Asp Met Gln His Tyr Gln Glu Met Tyr Gln Gln Ser Ile
35 40 45

Asn Glu Pro Glu Lys Phe Phe Asp Lys Met Ala Lys Glu Tyr Leu His
50 55 60

Trp Asp Ala Pro Tyr Thr Lys Val Gln Ser Gly Ser Leu Asn Asn Gly
65 70 75 80

Asp Val Ala Trp Phe Leu Asn Gly Lys Leu Asn Ala Ser Tyr Asn Cys
85 90 95

Val Asp Arg His Ala Phe Ala Asn Pro Asp Lys Pro Ala Leu Ile Tyr
100 105 110

Glu Ala Asp Asp Glu Ser Asp Asn Lys Ile Ile Thr Phe Gly Glu Leu
115 120 125

Leu Arg Lys Val Ser Gln Ile Ala Gly Val Leu Lys Ser Trp Gly Val
130 135 140

```
Lys Lys Gly Asp Thr Val Ala Ile Tyr Leu Pro Met Ile Pro Glu Ala
145                 150                 155                 160

Val Ile Ala Met Leu Ala Val Ala Arg Ile Gly Ala Ile His Ser Val
                165                 170                 175

Val Phe Ala Gly Phe Ser Ala Gly Ser Leu Lys Asp Arg Val Val Asp
            180                 185                 190

Ala Asn Ser Lys Val Val Ile Thr Cys Asp Glu Gly Lys Arg Gly Gly
        195                 200                 205

Lys Thr Ile Asn Thr Lys Lys Ile Val Asp Glu Gly Leu Asn Gly Val
    210                 215                 220

Asp Leu Val Ser Arg Ile Leu Val Phe Gln Arg Thr Gly Thr Glu Gly
225                 230                 235                 240

Ile Pro Met Lys Ala Gly Arg Asp Tyr Trp Trp His Glu Glu Ala Ala
                245                 250                 255

Lys Gln Arg Thr Tyr Leu Pro Pro Val Ser Cys Asp Ala Glu Asp Pro
            260                 265                 270

Leu Phe Leu Leu Tyr Thr Ser Gly Ser Thr Gly Ser Pro Lys Gly Val
        275                 280                 285

Val His Thr Thr Gly Gly Tyr Leu Leu Gly Ala Ala Leu Thr Thr Arg
    290                 295                 300

Tyr Val Phe Asp Ile His Pro Glu Asp Val Leu Phe Thr Ala Gly Asp
305                 310                 315                 320

Val Gly Trp Ile Thr Gly His Thr Tyr Ala Leu Tyr Gly Pro Leu Thr
                325                 330                 335

Leu Gly Thr Ala Ser Ile Ile Phe Glu Ser Thr Pro Ala Tyr Pro Asp
            340                 345                 350

Tyr Gly Arg Tyr Trp Arg Ile Ile Gln Arg His Lys Ala Thr His Phe
        355                 360                 365

Tyr Val Ala Pro Thr Ala Leu Arg Leu Ile Lys Arg Val Gly Glu Ala
    370                 375                 380

Glu Ile Ala Lys Tyr Asp Thr Ser Ser Leu Arg Val Leu Gly Ser Val
385                 390                 395                 400
```

```
Gly Glu Pro Ile Ser Pro Asp Leu Trp Glu Trp Tyr His Glu Lys Val
                405                 410                 415

Gly Asn Lys Asn Cys Val Ile Cys Asp Thr Met Trp Gln Thr Glu Ser
            420                 425                 430

Gly Ser His Leu Ile Ala Pro Leu Ala Gly Ala Val Pro Thr Lys Pro
        435                 440                 445

Gly Ser Ala Thr Val Pro Phe Phe Gly Ile Asn Ala Cys Ile Ile Asp
    450                 455                 460

Pro Val Thr Gly Val Glu Leu Glu Gly Asn Asp Val Glu Gly Val Leu
465                 470                 475                 480

Ala Val Lys Ser Pro Trp Pro Ser Met Ala Arg Ser Val Trp Asn His
                485                 490                 495

His Asp Arg Tyr Met Asp Thr Tyr Leu Lys Pro Tyr Pro Gly His Tyr
            500                 505                 510

Phe Thr Gly Asp Gly Ala Gly Arg Asp His Asp Gly Tyr Tyr Trp Ile
        515                 520                 525

Arg Gly Arg Val Asp Asp Val Val Asn Val Ser Gly His Arg Leu Ser
    530                 535                 540

Thr Ser Glu Ile Glu Ala Ser Ile Ser Asn His Glu Asn Val Ser Glu
545                 550                 555                 560

Ala Ala Val Val Gly Ile Pro Asp Glu Leu Thr Gly Gln Thr Val Val
                565                 570                 575

Ala Tyr Val Ser Leu Lys Asp Gly Tyr Leu Gln Asn Asn Ala Thr Glu
            580                 585                 590

Gly Asp Ala Glu His Ile Thr Pro Asp Asn Leu Arg Arg Glu Leu Ile
        595                 600                 605

Leu Gln Val Arg Gly Glu Ile Gly Pro Phe Ala Ser Pro Lys Thr Ile
    610                 615                 620

Ile Leu Val Arg Asp Leu Pro Arg Thr Arg Ser Gly Lys Ile Met Arg
625                 630                 635                 640

Arg Val Leu Arg Lys Val Ala Ser Asn Glu Ala Glu Gln Leu Gly Asp
```

645 650 655

Leu Thr Thr Leu Ala Asn Pro Glu Val Val Pro Ala Ile Ile Ser Ala
660 665 670

Val Glu Asn Gln Phe Phe Ser Gln Lys Lys Lys
675 680

<210> 21
<211> 440
<212> PRT
<213> Saccharomyces cerevisiae

<400> 21

```
Met Leu Ala Val Arg Arg Leu Thr Arg Tyr Thr Phe Leu Lys Arg Thr
1               5                   10                  15

His Pro Val Leu Tyr Thr Arg Arg Ala Tyr Lys Ile Leu Pro Ser Arg
            20                  25                  30

Ser Thr Phe Leu Arg Arg Ser Leu Leu Gln Thr Gln Leu His Ser Lys
        35                  40                  45

Met Thr Ala His Thr Asn Ile Lys Gln His Lys His Cys His Glu Asp
    50                  55                  60

His Pro Ile Arg Arg Ser Asp Ser Ala Val Ser Ile Val His Leu Lys
65                  70                  75                  80

Arg Ala Pro Phe Lys Val Thr Val Ile Gly Ser Gly Asn Trp Gly Thr
                85                  90                  95

Thr Ile Ala Lys Val Ile Ala Glu Asn Thr Glu Leu His Ser His Ile
            100                 105                 110

Phe Glu Pro Glu Val Arg Met Trp Val Phe Asp Glu Lys Ile Gly Asp
        115                 120                 125

Glu Asn Leu Thr Asp Ile Ile Asn Thr Arg His Gln Asn Val Lys Tyr
    130                 135                 140

Leu Pro Asn Ile Asp Leu Pro His Asn Leu Val Ala Asp Pro Asp Leu
145                 150                 155                 160

Leu His Ser Ile Lys Gly Ala Asp Ile Leu Val Phe Asn Ile Pro His
                165                 170                 175

Gln Phe Leu Pro Asn Ile Val Lys Gln Leu Gln Gly His Val Ala Pro
```

180 185 190

His Val Arg Ala Ile Ser Cys Leu Lys Gly Phe Glu Leu Gly Ser Lys
195 200 205

Gly Val Gln Leu Leu Ser Ser Tyr Val Thr Asp Glu Leu Gly Ile Gln
210 215 220

Cys Gly Ala Leu Ser Gly Ala Asn Leu Ala Pro Glu Val Ala Lys Glu
225 230 235 240

His Trp Ser Glu Thr Thr Val Ala Tyr Gln Leu Pro Lys Asp Tyr Gln
245 250 255

Gly Asp Gly Lys Asp Val Asp His Lys Ile Leu Lys Leu Leu Phe His
260 265 270

Arg Pro Tyr Phe His Val Asn Val Ile Asp Asp Val Ala Gly Ile Ser
275 280 285

Ile Ala Gly Ala Leu Lys Asn Val Val Ala Leu Ala Cys Gly Phe Val
290 295 300

Glu Gly Met Gly Trp Gly Asn Asn Ala Ser Ala Ala Ile Gln Arg Leu
305 310 315 320

Gly Leu Gly Glu Ile Ile Lys Phe Gly Arg Met Phe Phe Pro Glu Ser
325 330 335

Lys Val Glu Thr Tyr Tyr Gln Glu Ser Ala Gly Val Ala Asp Leu Ile
340 345 350

Thr Thr Cys Ser Gly Gly Arg Asn Val Lys Val Ala Thr Tyr Met Ala
355 360 365

Lys Thr Gly Lys Ser Ala Leu Glu Ala Glu Lys Glu Leu Leu Asn Gly
370 375 380

Gln Ser Ala Gln Gly Ile Ile Thr Cys Arg Glu Val His Glu Trp Leu
385 390 395 400

Gln Thr Cys Glu Leu Thr Gln Glu Phe Pro Leu Phe Glu Ala Val Tyr
405 410 415

Gln Ile Val Tyr Asn Asn Val Arg Met Glu Asp Leu Pro Glu Met Ile
420 425 430

Glu Glu Leu Asp Ile Asp Asp Glu
435 440

<210> 22
<211> 391
<212> PRT
<213> Saccharomyces cerevisiae

<400> 22

Met Ser Ala Ala Ala Asp Arg Leu Asn Leu Thr Ser Gly His Leu Asn
1 5 10 15

Ala Gly Arg Lys Arg Ser Ser Ser Ser Val Ser Leu Lys Ala Ala Glu
20 25 30

Lys Pro Phe Lys Val Thr Val Ile Gly Ser Gly Asn Trp Gly Thr Thr
35 40 45

Ile Ala Lys Val Val Ala Glu Asn Cys Lys Gly Tyr Pro Glu Val Phe
50 55 60

Ala Pro Ile Val Gln Met Trp Val Phe Glu Glu Glu Ile Asn Gly Glu
65 70 75 80

Lys Leu Thr Glu Ile Ile Asn Thr Arg His Gln Asn Val Lys Tyr Leu
85 90 95

Pro Gly Ile Thr Leu Pro Asp Asn Leu Val Ala Asn Pro Asp Leu Ile
100 105 110

Asp Ser Val Lys Asp Val Asp Ile Ile Val Phe Asn Ile Pro His Gln
115 120 125

Phe Leu Pro Arg Ile Cys Ser Gln Leu Lys Gly His Val Asp Ser His
130 135 140

Val Arg Ala Ile Ser Cys Leu Lys Gly Phe Glu Val Gly Ala Lys Gly
145 150 155 160

Val Gln Leu Leu Ser Ser Tyr Ile Thr Glu Glu Leu Gly Ile Gln Cys
165 170 175

Gly Ala Leu Ser Gly Ala Asn Ile Ala Thr Glu Val Ala Gln Glu His
180 185 190

Trp Ser Glu Thr Thr Val Ala Tyr His Ile Pro Lys Asp Phe Arg Gly
195 200 205

```
Glu Gly Lys Asp Val Asp His Lys Val Leu Lys Ala Leu Phe His Arg
    210                 215                 220

Pro Tyr Phe His Val Ser Val Ile Glu Asp Val Ala Gly Ile Ser Ile
225                 230                 235                 240

Cys Gly Ala Leu Lys Asn Val Val Ala Leu Gly Cys Gly Phe Val Glu
                245                 250                 255

Gly Leu Gly Trp Gly Asn Asn Ala Ser Ala Ala Ile Gln Arg Val Gly
            260                 265                 270

Leu Gly Glu Ile Ile Arg Phe Gly Gln Met Phe Phe Pro Glu Ser Arg
        275                 280                 285

Glu Glu Thr Tyr Tyr Gln Glu Ser Ala Gly Val Ala Asp Leu Ile Thr
    290                 295                 300

Thr Cys Ala Gly Gly Arg Asn Val Lys Val Ala Arg Leu Met Ala Thr
305                 310                 315                 320

Ser Gly Lys Asp Ala Trp Glu Cys Glu Lys Glu Leu Leu Asn Gly Gln
                325                 330                 335

Ser Ala Gln Gly Leu Ile Thr Cys Lys Glu Val His Glu Trp Leu Glu
            340                 345                 350

Thr Cys Gly Ser Val Glu Asp Phe Pro Leu Phe Glu Ala Val Tyr Gln
        355                 360                 365

Ile Val Tyr Asn Asn Tyr Pro Met Lys Asn Leu Pro Asp Met Ile Glu
    370                 375                 380

Glu Leu Asp Leu His Glu Asp
385                 390
```

<210> 23
<211> 2058
<212> DNA
<213> Artificial

<220>
<223> GPD2 disruption

<400> 23

agatcttttg cggcgaggtg ccgatgggtt gctgagggga agagtgttta gcttacggac 60
ctattgccat tgttattccg attaatctat tgttcagcag ctcttctcta ccctgtcatt 120
ctagtatttt tttttttttt ttttggtttt actttttttt cttcttgcct ttttttcttg 180

ttactttttt tctagttttt tttccttcca ctaagctttt tccttgattt atccttgggt 240
tcttctttct actcctttag attttttttt tatatattaa tttttaagtt tatgtatttt 300
ggtagattca attctctttc cctttccttt tccttcgctc cccttcctta tcaatgcttg 360
ctgtcagaag attaacaaga tacacattcc ttaaggcctc gtccccgccg ggtcacccgg 420
ccagcgacat ggaggcccag aataccctcc ttgacagtct tgacgtgcgc agctcagggg 480
catgatgtga ctgtcgcccg tacatttagc ccatacatcc ccatgtataa tcatttgcat 540
ccatacattt tgatggccgc acggcgcgaa gcaaaaatta cggctcctcg ctgcagacct 600
gcgagcaggg aaacgctccc ctcacagacg cgttgaattg tccccacgcc gcgcccctgt 660
agagaaatat aaaaggttag gatttgccac tgaggttctt ctttcatata cttcctttta 720
aaatcttgct aggatacagt tctcacatca catccgaaca taaacaacca tgtaaaatga 780
ccactcttga cgacacggct taccggtacc gcaccagtgt cccgggggac gccgaggcca 840
tcgaggcact ggatgggtcc ttcaccaccg acaccgtctt ccgcgtcacc gccaccgggg 900
acggcttcac cctgcgggag gtgccggtgg acccgcccct gaccaaggtg ttccccgacg 960
acgaatcgga cgacgaatcg gacgccgggg aggacggcga cccggactcc cggacgttcg 1020
tcgcgtacgg ggacgacggc gacctggcgg gcttcgtggt cgtctcgtac tccggctgga 1080
accgccggct gaccgtcgag gacatcgagg tcgccccgga gcaccggggg cacggggtcg 1140
ggcgcgcgtt gatggggctc gcgacggagt tcgcccgcga gcggggcgcc gggcacctct 1200
ggctggaggt caccaacgtc aacgcaccgg cgatccacgc gtaccggcgg atggggttca 1260
ccctctgcgg cctggacacc gccctgtacg acggcaccgc ctcggacggc gagcaggcgc 1320
tctacatgag catgccctgc ccctagtact gacaataaaa agattcttgt tttcaagaac 1380
ttgtcatttg tatagttttt ttatattgta gttgttctat tttaatcaaa tgttagcgtg 1440
atttatattt tttttcgcct cgacatcatc tgcccagatg cgaagttaag tgcgcagaaa 1500
gtaatatcat gcgtcaatcg tatgtgaatg ctggtcgcta tactgctgtc gattcgatac 1560
taacgccgcc atccagtgtc gacggatcct aggtgtacag ggcccaaaag ggcgaattct 1620
gcagatatcc atcacactgg cggccgctcg aggatagtct acaacaacgt ccgcatggaa 1680
gacctaccgg agatgattga agagctagac atcgatgacg aatagacact ctcccccccc 1740
ctcccccctct gatctttcct gttgcctctt tttcccccaa ccaatttatc attatacaca 1800
agttctacaa ctactactag taacattact acagttatta taattttcta ttctcttttt 1860
ctttaagaat ctatcattaa cgttaatttc tatatataca taactaccat tatacacgct 1920
attatcgttt acatatcaca tcaccgttaa tgaaagatac gacaccctgt acactaacac 1980
aattaaataa tcgccataac cttttctgtt atctatagcc cttaaagctg tttcttcgag 2040
ctttttcact gcagatct 2058

<210> 24
<211> 816
<212> DNA
<213> Saccharomyces cerevisiae

<400> 24

```
ccatatgatc atgtgtcgtc gcacacatat atatatgcct gtatgtgtca gcactaaagt      60
tgcctggcca tccacgctat atatacacgc ctggcggatc tgctcgagga ttgcctacgc     120
gtgggcttga tccaccaacc aacgctcgcc aaatgaactg gcgctttggt cttctgccat     180
cgtccgtaaa ccccggccaa agagaccgga aagatcggtg aaaacatctt gatcttgctc     240
ccgggaattt tagattcagg taggaaattg attacatcaa tactgttacc ctgaatcata     300
ttcgacgatg tcgtctcaca cggaaatata attcatttct tggttttcca aaaaaatttt     360
catttttttt cacttttttg tttcgtcctc cttttttttt tttttttttt attttttttc     420
ctgtgttcac cttttttttt ttcagttgac atctttctgc attcttttct gtgttttttt     480
tttttttttt cgtttttcca ttgttcgttc gttgcctgtt ttttcgccct attgttctcg     540
agcctaaaaa tttttttcctt tcctgctttc ctttcttcgt tcaaagtttc ctattccatt     600
gttctctttg gtaaactcat tgttgtcgga actcagatat attcaggtca atttactgta     660
cttcaattga ctttttcttt gaaatttcaa cttgcctttt caacttgttc ttcttttttta     720
atcttattct acactttagt tcccttacct tgttcctaat tattgtctag caaaaagaaa     780
acatacacct atttcattca cacactgcag aaaatg                               816
```

<210> 25
<211> 954
<212> DNA
<213> Artificial

<220>
<223> GCY1 PCR fragment

<400> 25

```
ctgcagaaaa tgcctgctac tttacatgat tctacgaaaa tcctttctct aaatactgga      60
gcccaaatcc ctcaaatagg tttaggtacg tggcagtcga aagagaacga tgcttataag     120
gctgttttaa ccgctttgaa agatggctac cgacacattg atactgctgc tatttaccgt     180
aatgaagacc aagtcggtca agccatcaag gattcaggtg ttcctcggga agaaatcttt     240
gttactacaa agttatggtg tacacaacac cacgaacctg aagtagcgct ggatcaatca     300
ctaaagaggt taggattgga ctacgtagac ttatatttga tgcattggcc tgccagatta     360
gatccagcct acatcaaaaa tgaagacatc ttgagtgtgc caacaaagaa ggatggttct     420
cgtgcagtgg atatcaccaa ttggaatttc atcaaaacct gggaattaat gcaggaacta     480
ccaaagactg gtaaaactaa ggccgttgga gtctccaact tttctataaa taacctgaaa     540

gatctattag catctcaagg taataagctt acgccagctg ctaaccaagt cgaaatacat     600
ccattactac ctcaagacga attgattaat ttttgtaaaa gtaaaggcat tgtggttgaa     660
gcttattctc cgttaggtag taccgatgct ccactattga aggaaccggt tatccttgaa     720
attgcgaaga aaaataacgt tcaacccgga cacgttgtta ttagctggca cgtccaaaga     780
ggttatgttg tcttgccaaa atctgtgaat cccgatcgaa tcaaaacgaa caggaaaata     840
tttactttgt ctactgagga ctttgaagct atcaataaca tatcgaagga aaagggcgaa     900
aaaagggttg tacatccaaa ttggtctcct ttcgaagtat tcaagtaact taag           954
```

<210> 26
<211> 2986
<212> DNA
<213> Artificial

<220>
<223> DAK1 PCR fragment

<400> 26

```
ctcgagtacc ggtttcttct tcagattccc tcatggagaa agtgcggcag atgtatatga     60
cagagtcgcc agtttccaag agactttatt caggcacttc catgataggc aagagagaag    120
acccagagat gttgttgtcc tagttacaca tggtatttat tccagagtat tcctgatgaa    180
atggtttaga tggacatacg aagagtttga atcgtttacc aatgttccta acgggagcgt    240
aatggtgatg gaactggacg aatccatcaa tagatacgtc ctgaggaccg tgctacccaa    300
atggactgat tgtgagggag acctaactac atagtgttta aagattacgg atatttaact    360
tacttagaat aatgccattt ttttgagtta taataatcct acgttagtgt gagcgggatt    420
taaactgtga ggaccttaat acattcagac acttctgcgg tatcacccta cttattccct    480
tcgagattat atctaggaac ccatcaggtt ggtggaagat tacccgttct aagacttttc    540
agcttcctct attgatgtta cacctggaca ccccttttct ggcatccagt ttttaatctt    600
cagtggcatg tgagattctc cgaaattaat taaagcaatc acacaattct ctcggatacc    660
acctcggttg aaactgacag gtggtttgtt acgcatgcta atgcaaagga gcctatatac    720
ctttggctcg gctgctgtaa cagggaatat aaagggcagc ataatttagg agtttagtga    780
acttgcaaca tttactattt tcccttctta cgtaaatatt tttcttttta attctaaatc    840
aatcttttc aatttttgt ttgtattctt ttcttgctta aatctataac tacaaaaaac      900
acatacataa atctagaaaa tgtccgctaa atcgtttgaa gtcacagatc cagtcaattc    960
aagtctcaaa gggtttgccc ttgctaaccc ctccattacg ctggtccctg aagaaaaaat   1020
tctcttcaga aagaccgatt ccgacaagat cgcattaatt tctggtggtg gtagtggaca   1080
tgaacctaca cacgccggtt tcattggtaa gggtatgttg agtggcgccg tggttggcga   1140
```

aatttttgca tcccccttcaa caaaacagat tttaaatgca atccgtttag tcaatgaaaa 1200

tgcgtctggc gttttattga ttgtgaagaa ctacacaggt gatgttttgc attttggtct 1260

gtccgctgag agagcaagag ccttgggtat taactgccgc gttgctgtca taggtgatga 1320

tgttgcagtt ggcagagaaa agggtggtat ggttggtaga agagcattgg caggtaccgt 1380

tttggttcat aagattgtag gtgccttcgc agaagaatat tctagtaagt atggcttaga 1440

cggtacagct aaagtggcta aaattatcaa cgacaatttg gtgaccattg gatcttcttt 1500

agaccattgt aaagttcctg gcaggaaatt cgaaagtgaa ttaaacgaaa aacaaatgga 1560

attgggtatg ggtattcata acgaacctgg tgtgaaagtt ttagacccta ttccttctac 1620

cgaagacttg atctccaagt atatgctacc aaaactattg gatccaaacg ataaggatag 1680

agcttttgta aagtttgatg aagatgatga agttgtcttg ttagttaaca atctcggcgg 1740

tgtttctaat tttgttatta gttctatcac ttccaaaact acggatttct taaaggaaaa 1800

ttacaacata accccggttc aaacaattgc tggcacattg atgacctcct tcaatggtaa 1860

tgggttcagt atcacattac taaacgccac taaggctaca aaggctttgc aatctgattt 1920

tgaggagatc aaatcagtac tagacttgtt gaacgcattt acgaacgcac cgggctggcc 1980

aattgcagat tttgaaaaga cttctgcccc atctgttaac gatgacttgt tacataatga 2040

agtaacagca aaggccgtcg gtacctatga ctttgacaag tttgctgagt ggatgaagag 2100

tggtgctgaa caagttatca agagcgaacc gcacattacg gaactagaca atcaagttgg 2160

tgatggtgat tgtggttaca ctttagtggc aggagttaaa ggcatcaccg aaaaccttga 2220

caagctgtcg aaggactcat tatctcaggc ggttgcccaa atttcagatt tcattgaagg 2280

ctcaatggga ggtacttctg gtggtttata ttctattctt ttgtcgggtt tttcacacgg 2340

attaattcag gtttgtaaat caaaggatga acccgtcact aaggaaattg tggctaagtc 2400

actcggaatt gcattggata ctttatacaa atatacaaag gcaaggaagg gatcatccac 2460

catgattgat gctttagaac cattcgttaa agaatttact gcatctaagg atttcaataa 2520

ggcggtaaaa gctgcagagg aaggtgctaa atccactgct acattcgagg ccaaatttgg 2580

cagagcttcg tatgtcggcg attcatctca agtagaagat cctggtgcag taggcctatg 2640

tgagtttttg aaggggggttc aaagcgcctt gtaagtcgag acaaatcgct cttaaatata 2700

tacctaaaga acattaaagc tatattataa gcaaagatac gtaaattttg cttatattat 2760

tatacacata tcatatttct atatttttaa gatttggtta tataatgtac gtaatgcaaa 2820

ggaaataaat tttatacatt attgaacagc gtccaagtaa ctacattatg tgcactaata 2880

gtttagcgtc gtgaagactt tattgtgtcg cgaaaagtaa aaattttaaa aattagagca 2940

ccttgaactt gcgaaaaagg ttctcatcaa ctgtttaaaa cgtacg 2986

<210> 27
<211> 2651
<212> DNA
<213> Artificial

<220>
<223> GUP1 PCR fragment

<400> 27

ggtacctagg accggtttat cattatcaat actgccattt caaagaatac gtaaataatt 60
aatagtagtg attttcctaa ctttatttag tcaaaaaatt agccttttaa ttctgctgta 120
acccgtacat gcccaaaata gggggcgggt tacacagaat atataacatc gtaggtgtct 180
gggtgaacag tttattcctg gcatccacta aatataatgg agcccgcttt ttaagctggc 240
atccagaaaa aaaaagaatc ccagcaccaa aatattgttt tcttcaccaa ccatcagttc 300
ataggtccat tctcttagcg caactacaga gaacaggggc acaaacaggc aaaaaacggg 360
cacaacctca atggagtgat gcaacctgcc tggagtaaat gatgacacaa ggcaattgac 420
ccacgcatgt atctatctca ttttcttaca ccttctatta ccttctgctc tctctgattt 480
ggaaaaagct gaaaaaaaag gttgaaacca gttccctgaa attattcccc tacttgacta 540
ataagtatat aaagacggta ggtattgatt gtaattctgt aaatctattt cttaaacttc 600
ttaaattcta cttttatagt tagtcttttt tttagtttta aaacaccaag aacttagttt 660
cgaataaaca cacataaaga attcgaaaat gtcgctgatc agcatcctgt ctcccctaat 720
tacttccgag ggcttagatt caagaatcaa accttcacca aaaaaggatg cctctactac 780
cactaagcca tcactatgga aaactactga gttcaaattc tactacattg catttctggt 840
cgtggttccc ttgatgttct atgctgggtt acaagctagt tcgcccgaaa atccaaacta 900
tgcaagatac gaacgtctcc tatctcaagg ttggttattt ggcagaaaag tagacaatag 960
tgattctcaa tataggtttt tcagggacaa ttttgcgcta ttgtcagttt taatgctagt 1020
ccacacttct ataaaacgca ttgtacttta ttcaacaaat atcactaaat tgaggtttga 1080
tctgatattt ggtttgatct ttttagtggc cgctcatggt gtcaattcga taagaatttt 1140
agcccatatg ctaattttat atgccatcgc ccatgtacta aagaacttta gaagaatagc 1200
caccatcagc atttggattt atggtatttc tacgcttttt attaacgaca acttcagagc 1260
atatccattt ggtaatattt gctctttttt aagcccattg gaccattggt atagaggtat 1320
cattccaaga tgggatgtct tttcaattt tactcttttg agagtcttaa gttacaactt 1380
ggacttctta gagaggtggg agaatttaca aaagaagaaa agtccatcct atgaatcaaa 1440
agaagctaaa tcagccattt tgctcaatga acgtgctaga ttaactgctg cacaccccat 1500
acaggactac agcttaatga attatattgc atatgttact tacacgccac ttttcattgc 1560
cggccccatt ataacattca atgattatgt ttaccaatcg aaacatacct tgccatcaat 1620

aaatttcaaa ttcatttttt actatgcggt gagattcgtt attgctctct tatctatgga 1680
gttcatttta cactttctcc acgttgtggc aatctcaaaa accaaagcgt gggaaaatga 1740
cacacctttc cagatttcca tgattggctt atttaatttg aatattattt ggctaaaact 1800
actgattccg tggaggctgt ttaggctgtg ggctttgcta gacggaatcg atacacctga 1860
aaatatgatc aggtgtgttg ataacaatta cagttcacta gcattctgga gagcttggca 1920
tagaagctac aataagtggg ttgtccgtta catatatatt cctctaggtg gttcaaaaaa 1980
tagagttttg acatcactag cagtcttttc cttcgtagct atatggcatg acatcgaact 2040
aaagttatta ttatggggtt ggctaatagt tttgttcctc ttaccagaaa tttttgctac 2100
ccaaattttc tctcattata ccgacgcagt ctggtacaga cacgtttgcg ctgtcggtgc 2160
tgttttcaac atatgggtta tgatgatcgc taatcttttt ggattctgct tgggctctga 2220
cggtactaaa aaattactaa gcgatatgtt ctgtaccgta tctggtttca aatttgtaat 2280
tttggcaagc gttagtttat tcatcgcagt acaaataatg tttgaaatca gagaagaaga 2340
aaagaggcac ggaatttacc taaaatgctg aggatcccct tttcctttgt cgatatcatg 2400
taattagtta tgtcacgctt acattcacgc cctcctccca catccgctct aaccgaaaag 2460
gaaggagtta gacaacctga agtctaggtc cctatttatt tttttaata gttatgttag 2520
tattaagaac gttatttata tttcaaattt ttcttttttt tctgtacaaa cgcgtgtacg 2580
catgtaacat tatactgaaa accttgcttg agaaggtttt gggacgctcg aaggcttcct 2640
aggctcgagt t 2651

<210> 28
<211> 3037
<212> DNA
<213> Artificial

<220>
<223> FPS1 PCR fragment

<400> 28

```
aagagctccg gactagtcgt acgaattcta tccttttgtt gtttccgggt gtacaatatg     60
gacttcctct tttctggcaa ccaaacccat acatcgggat tcctataata ccttcgttgg    120
tctccctaac atgtaggtgg cggaggggag atatacaata gaacagatac cagacaagac    180
ataatgggct aaacaagact acaccaatta cactgcctca ttgatggtgg tacataacga    240
actaatactg tagccctaga cttgatagcc atcatcatat cgaagtttca ctacccttttt   300
tccatttgcc atctattgaa gtaataatag gcgcatgcaa cttcttttct tttttttct    360
tttctctctc ccccgttgtt gtctcaccat atccgcaatg acaaaaaaat gatggaagac    420
actaaaggaa aaaattaacg acaaagacag caccaacaga tgtcgttgtt ccagagctga    480
tgaggggtat ctcgaagcac acgaaacttt ttccttcctt cattcacgca cactactctc    540
```

taatgagcaa cggtatacgg ccttccttcc agttacttga atttgaaata aaaaaagttt 600
gctgtcttgc tatcaagtat aaatagacct gcaattatta atcttttgtt tcctcgtcat 660
tgttctcgtt ccctttcttc cttgtttctt tttctgcaca atatttcaag ctataccaag 720
catacaatca actccagctg cattaaaatg agtaatcctc aaaaagctct aaacgacttt 780
ctgtccagtg aatctgttca tacacatgat agttctagga aacaatctaa taagcagtca 840
tccgacgaag gacgctcttc atcacaacct tcacatcatc actctggtgg tactaacaac 900
aataataaca ataataataa taataataac agtaacaaca acaacaacgg caacgatggg 960
ggaaatgatg acgactatga ttatgaaatg caagattata gaccttctcc gcaaagtgcg 1020
cggcctactc ccacgtatgt tccacaatat tctgtagaaa gtgggactgc tttcccgatt 1080
caagaggtta ttcctagcgc atacattaac acacaagata taaaccataa agataacggt 1140
ccgccgagtg caagcagtaa tagagcattc aggcctagag ggcagaccac agtgtcggcc 1200
aacgtgctta acattgaaga tttttacaaa aatgcagacg atgcgcatac catcccggag 1260
tcacatttat cgagaaggag aagtaggtcg agggctacga gtaatgctgg gcacagtgcc 1320
aatacaggcg ccacgaatgg caggactact ggtgcccaaa ctaatatgga aagcaatgaa 1380
tcaccacgta acgtccccat tatggtgaag ccaaagacat tataccagaa ccctcaaaca 1440
cctacagtct tgccctccac ataccatcca attaataaat ggtcttccgt caaaaacact 1500
tatttgaagg aatttttagc cgagtttatg ggaacaatgg ttatgattat tttcggtagt 1560
gctgttgttt gtcaggtcaa tgttgctggg aaaatacagc aggacaattt caacgtggct 1620
ttggataacc ttaacgttac cgggtcttct gcagaaacga tagacgctat gaagagttta 1680
acatccttgg tttcatccgt tgcgggcggt acctttgatg atgtggcatt gggctgggct 1740
gctgccgtgg tgatgggcta tttctgcgct ggtggtagtg ccatctcagg tgctcatttg 1800
aatccgtcta ttacattagc caatttggtg tatagaggtt ttcccctgaa gaaagttcct 1860
tattactttg ctggacaatt gatcggtgcc ttcacaggcg ctttgatctt gtttatttgg 1920
tacaaaaggg tgttacaaga ggcatatagc gattggtgga tgaatgaaag tgttgcggga 1980
atgttttgcg tttttccaaa gccttatcta agttcaggac ggcaattttt ttccgaattt 2040
ttatgtggag ctatgttaca agcaggaaca tttgcgctga ccgatcctta tacgtgtttg 2100
tcctctgatg ttttcccatt gatgatgttt attttgattt tcattatcaa tgcttccatg 2160
gcttatcaga caggtacagc aatgaatttg gctcgtgatc tgggcccacg tcttgcacta 2220
tatgcagttg gatttgatca taaaatgctt tgggtgcatc atcatcattt cttttgggtt 2280
cccatggtag gcccatttat tggtgcgtta atgggggggt tggtttacga tgtctgtatt 2340
tatcagggtc atgaatctcc agtcaactgg tctttaccag tttataagga aatgattatg 2400

agagcctggt ttagaaggcc tggttggaag aagagaaata gagcaagaag aacatcggac 2460
ctgagtgact tctcatacaa taacgatgat gatgaggaat ttggagaaag aatggctctt 2520
caaaagacaa agaccaagtc atctatttca gacaacgaaa atgaagcagg agaaaagaaa 2580
gtgcaattta aatctgttca gcgcggcaaa agaacgtttg gtggtatacc aacaattctt 2640
gaagaagaag attccattga aactgcttcg ctaggtgcga cgacgactga ttctattggg 2700
ttatccgaca catcatcaga agattcgcat tatggtaatg ctaagaaggt aacatgagga 2760
tcccctttc ctttgtcgat atcatgtaat tagttatgtc acgcttacat tcacgccctc 2820
ctcccacatc cgctctaacc gaaaaggaag gagttagaca acctgaagtc taggtcccta 2880
tttatttttt ttaatagtta tgttagtatt aagaacgtta tttatatttc aaatttttct 2940
ttttttttctg tacaaacgcg tgtacgcatg taacattata ctgaaaacct tgcttgagaa 3000
ggttttggga cgctcgaagg cttcctaggc tcgagtt 3037

<210> 29
<211> 12954
<212> DNA
<213> Artificial

<220>
<223> pRN605 plasmid for overexpression of GCY1, DAK1 and GUP1

<400> 29

```
tacgccaagc tcggaattaa ccctcactaa agggaacaaa agctgggtac cgggcccccc     60
ctcgagccta ggaagccttc gagcgtccca aaaccttctc aagcaaggtt ttcagtataa    120
tgttacatgc gtacacgcgt ttgtacagaa aaaaaagaaa aatttgaaat ataaataacg    180
ttcttaatac taacataact attaaaaaaa ataaataggg acctagactt caggttgtct    240
aactccttcc ttttcggtta gagcggatgt gggaggaggg cgtgaatgta agcgtgacat    300
aactaattac atgatatcga caaaggaaaa ggggatcctc agcattttag gtaaattccg    360
tgcctctttt cttcttctct gatttcaaac attatttgta ctgcgatgaa taaactaacg    420
cttgccaaaa ttacaaattt gaaaccagat acggtacaga acatatcgct tagtaatttt    480
ttagtaccgt cagagcccaa gcagaatcca aaaagattag cgatcatcat aacccatatg    540
ttgaaaacag caccgacagc gcaaacgtgt ctgtaccaga ctgcgtcggt ataatgagag    600
aaaatttggg tagcaaaaat ttctggtaag aggaacaaaa ctattagcca accccataat    660
aataacttta gttcgatgtc atgccatata gctacgaagg aaaagactgc tagtgatgtc    720
aaaactctat tttttgaacc acctagagga atatatatgt aacggacaac ccacttattg    780
tagcttctat gccaagctct ccagaatgct agtgaactgt aattgttatc aacacacctg    840
atcatatttt caggtgtatc gattccgtct agcaaagccc acagcctaaa cagcctccac    900
ggaatcagta gttttagcca aataatattc aaattaaata agccaatcat ggaaatctgg    960
```

aaaggtgtgt cattttccca cgctttggtt tttgagattg ccacaacgtg gagaaagtgt 1020

aaaatgaact ccatagataa gagagcaata acgaatctca ccgcatagta aaaaatgaat 1080

ttgaaattta ttgatggcaa ggtatgtttc gattggtaaa cataatcatt gaatgttata 1140

atggggccgg caatgaaaag tggcgtgtaa gtaacatatg caatataatt cattaagctg 1200

tagtcctgta tggggtgtgc agcagttaat ctagcacgtt cattgagcaa aatggctgat 1260

ttagcttctt ttgattcata ggatggactt ttcttctttt gtaaattctc ccacctctct 1320

aagaagtcca agttgtaact taagactctc aaaagagtaa aattgaaaaa gacatcccat 1380

cttggaatga tacctctata ccaatggtcc aatgggctta aaaaagagca aatattacca 1440

aatggatatg ctctgaagtt gtcgttaata aaaagcgtag aaataccata aatccaaatg 1500

ctgatggtgg ctattcttct aaagttcttt agtacatggg cgatggcata taaaattagc 1560

atatgggcta aaattcttat cgaattgaca ccatgagcgg ccactaaaaa gatcaaacca 1620

aatatcagat caaacctcaa tttagtgata tttgttgaat aaagtacaat gcgttttata 1680

gaagtgtgga ctagcattaa aactgacaat agcgcaaaat tgtccctgaa aaacctatat 1740

tgagaatcac tattgtctac ttttctgcca aataaccaac cttgagatag gagacgttcg 1800

tatcttgcat agtttggatt ttcgggcgaa ctagcttgta acccagcata gaacatcaag 1860

ggaaccacga ccagaaatgc aatgtagtag aatttgaact cagtagtttt ccatagtgat 1920

ggcttagtgg tagtagaggc atcctttttt ggtgaaggtt tgattcttga atctaagccc 1980

tcggaagtaa ttaggggaga caggatgctg atcagcgaca ttttgaattc tttatgtgtg 2040

tttattcgaa actaagttct tggtgtttta aaactaaaaa aaagactaac tataaaagta 2100

gaatttaaga agtttaagaa atagatttac agaattacaa tcaataccta ccgtctttat 2160

atacttatta gtcaagtagg ggaataattt cagggaactg gtttcaacct tttttttcag 2220

ctttttccaa atcagagaga gcagaaggta atagaaggtg taagaaaatg agatagatac 2280

atgcgtgggt caattgcctt gtgtcatcat ttactccagg caggttgcat cactccattg 2340

aggttgtgcc cgtttttttgc ctgtttgtgc ccctgttctc tgtagttgcg ctaagagaat 2400

ggacctatga actgatggtt ggtgaagaaa acaatatttt ggtgctggga ttcttttttt 2460

ttctggatgc cagcttaaaa agcgggctcc attatattta gtggatgcca ggaataaact 2520

gttcacccag acacctacga tgttatatat tctgtgtaac ccgcccccta ttttgggcat 2580

gtacgggtta cagcagaatt aaaaggctaa ttttttgact aaataaagtt aggaaaatca 2640

ctactattaa ttatttacgt attctttgaa atggcagtat tgataatgat aaaccggttt 2700

cttcttcaga ttccctcatg gagaaagtgc ggcagatgta tatgacagag tcgccagttt 2760

ccaagagact ttattcaggc acttccatga taggcaagag agaagaccca gagatgttgt 2820

tgtcctagtt acacatggta tttattccag agtattcctg atgaaatggt ttagatggac 2880
atacgaagag tttgaatcgt ttaccaatgt tcctaacggg agcgtaatgg tgatggaact 2940
ggacgaatcc atcaatagat acgtcctgag gaccgtgcta cccaaatgga ctgattgtga 3000
gggagaccta actacatagt gtttaaagat tacggatatt taacttactt agaataatgc 3060
catttttttg agttataata atcctacgtt agtgtgagcg ggatttaaac tgtgaggacc 3120
ttaatacatt cagacacttc tgcggtatca ccctacttat tcccttcgag attatatcta 3180
ggaacccatc aggttggtgg aagattaccc gttctaagac ttttcagctt cctctattga 3240
tgttacacct ggacacccct tttctggcat ccagttttta atcttcagtg gcatgtgaga 3300
ttctccgaaa ttaattaaag caatcacaca attctctcgg ataccacctc ggttgaaact 3360
gacaggtggt ttgttacgca tgctaatgca aaggagccta tatacctttg gctcggctgc 3420
tgtaacaggg aatataaagg gcagcataat ttaggagttt agtgaacttg caacatttac 3480
tattttccct tcttacgtaa atatttttct ttttaattct aaatcaatct ttttcaattt 3540
tttgtttgta ttcttttctt gcttaaatct ataactacaa aaaacacata cataaatcta 3600
gaaaatgtcc gctaaatcgt ttgaagtcac agatccagtc aattcaagtc tcaaagggtt 3660
tgcccttgct aacccctcca ttacgctggt ccctgaagaa aaaattctct tcagaaagac 3720
cgattccgac aagatcgcat taatttctgg tggtggtagt ggacatgaac ctacacacgc 3780
cggtttcatt ggtaagggta tgttgagtgg cgccgtggtt ggcgaaattt ttgcatcccc 3840
ttcaacaaaa cagattttaa atgcaatccg tttagtcaat gaaaatgcgt ctggcgtttt 3900
attgattgtg aagaactaca caggtgatgt tttgcatttt ggtctgtccg ctgagagagc 3960
aagagccttg ggtattaact gccgcgttgc tgtcataggt gatgatgttg cagttggcag 4020
agaaaagggt ggtatggttg gtagaagagc attggcaggt accgttttgg ttcataagat 4080
tgtaggtgcc ttcgcagaag aatattctag taagtatggc ttagacggta cagctaaagt 4140
ggctaaaatt atcaacgaca atttggtgac cattggatct tctttagacc attgtaaagt 4200
tcctggcagg aaattcgaaa gtgaattaaa cgaaaaacaa atggaattgg gtatgggtat 4260
tcataacgaa cctggtgtga aagttttaga ccctattcct tctaccgaag acttgatctc 4320
caagtatatg ctaccaaaac tattggatcc aaacgataag gatagagctt ttgtaaagtt 4380
tgatgaagat gatgaagttg tcttgttagt taacaatctc ggcggtgttt ctaattttgt 4440
tattagttct atcacttcca aaactacgga tttcttaaag gaaaattaca acataacccc 4500
ggttcaaaca attgctggca cattgatgac ctccttcaat ggtaatgggt tcagtatcac 4560
attactaaac gccactaagg ctacaaaggc tttgcaatct gattttgagg agatcaaatc 4620
agtactagac ttgttgaacg catttacgaa cgcaccgggc tggccaattg cagattttga 4680
aaagacttct gccccatctg ttaacgatga cttgttacat aatgaagtaa cagcaaaggc 4740

cgtcggtacc tatgactttg acaagtttgc tgagtggatg aagagtggtg ctgaacaagt 4800

tatcaagagc gaaccgcaca ttacggaact agacaatcaa gttggtgatg gtgattgtgg 4860

ttacacttta gtggcaggag ttaaaggcat caccgaaaac cttgacaagc tgtcgaagga 4920

ctcattatct caggcggttg cccaaatttc agatttcatt gaaggctcaa tgggaggtac 4980

ttctggtggt ttatattcta ttcttttgtc gggtttttca cacggattaa ttcaggtttg 5040

taaatcaaag gatgaacccg tcactaagga aattgtggct aagtcactcg gaattgcatt 5100

ggatacttta tacaaatata caaaggcaag gaagggatca tccaccatga ttgatgcttt 5160

agaaccattc gttaaagaat ttactgcatc taaggatttc aataaggcgg taaaagctgc 5220

agaggaaggt gctaaatcca ctgctacatt cgaggccaaa tttggcagag cttcgtatgt 5280

cggcgattca tctcaagtag aagatcctgg tgcagtaggc ctatgtgagt ttttgaaggg 5340

ggttcaaagc gccttgtaag tcgagacaaa tcgctcttaa atatatacct aaagaacatt 5400

aaagctatat tataagcaaa gatacgtaaa ttttgcttat attattatac acatatcata 5460

tttctatatt tttaagattt ggttatataa tgtacgtaat gcaaaggaaa taaattttat 5520

acattattga acagcgtcca agtaactaca ttatgtgcac taatagttta gcgtcgtgaa 5580

gactttattg tgtcgcgaaa agtaaaaatt ttaaaaatta gagcaccttg aacttgcgaa 5640

aaaggttctc atcaactgtt taaaacgtac gtgtggaaga acgattacaa caggtgttgt 5700

cctctgagga cataaaatac acaccgagat tcatcaactc attgctggag ttagcatatc 5760

tacaattggg tgaaatgggg agcgatttgc aggcatttgc tcggcatgcc ggtagaggtg 5820

tggtcaataa gagcgacctc atgctatacc tgagaaagca acctgaccta caggaaagag 5880

ttactcaaga ataagaattt tcgttttaaa acctaagagt cactttaaaa tttgtataca 5940

cttatttttt ttataactta tttaataata aaaatcataa atcataagaa attcgcgcgc 6000

ttacttgaat acttcgaaag gagaccaatt tggatgtaca accctttttt cgcccttttc 6060

cttcgatatg ttattgatag cttcaaagtc ctcagtagac aaagtaaata ttttcctgtt 6120

cgttttgatt cgatcgggat tcacagattt tggcaagaca acataacctc tttggacgtg 6180

ccagctaata acaacgtgtc cgggttgaac gttatttttc ttcgcaattt caaggataac 6240

cggttccttc aatagtggag catcggtact acctaacgga gaataagctt caaccacaat 6300

gcctttactt ttacaaaaat taatcaattc gtcttgaggt agtaatggat gtatttcgac 6360

ttggttagca gctggcgtaa gcttattacc ttgagatgct aatagatctt tcaggttatt 6420

tatagaaaag ttggagactc caacggcctt agttttacca gtctttggta gttcctgcat 6480

taattcccag gttttgatga aattccaatt ggtgatatcc actgcacgag aaccatcctt 6540

ctttgttggc acactcaaga tgtcttcatt tttgatgtag gctggatcta atctggcagg 6600

ccaatgcatc aaatataagt ctacgtagtc caatcctaac ctctttagtg attgatccag 6660
cgctacttca ggttcgtggt gttgtgtaca ccataacttt gtagtaacaa agatttcttc 6720
ccgaggaaca cctgaatcct tgatggcttg accgacttgg tcttcattac ggtaaatagc 6780
agcagtatca atgtgtcggt agccatcttt caaagcggtt aaaacagcct tataagcatc 6840
gttctctttc gactgccacg tacctaaacc tatttgaggg atttgggctc cagtatttag 6900
agaaaggatt ttcgtagaat catgtaaagt agcaggcatt ttctgcagtt aattcagtaa 6960
attttcgatc ttgggaagaa aaaagcagta agcgtgaaaa atctaaaagc tgatgtagta 7020
gaagatccta ttctttaaca aagattgacc ttttcttttt cttcttggtt tgagtagaaa 7080
ggggaaggaa gaatacaaga gagaggaaaa aaaggaagat aaaaagagag cgtgatataa 7140
atgaatatat attaaacaag agagattggg aaggaaagga tcaaacaaac ccaaaaatat 7200
ttcaaaaagg agagagagag gcgagtttgg tttcaaaacg gtttatttat ttatgcaaga 7260
ggacgtggaa gaaaaagaag aaggaagaaa aaaatttgaa agaaaaaaac gcgtggcggg 7320
taaagaagaa aatggaaaat agaggccggg tgacagagaa atattgaggg ttaattggaa 7380
aatatgttag ggtgaggcat atgtttttaa gggttttgag gatccgataa ggaagaatgt 7440
aggttaaatg ttgtgcatta attgctgtgg cagcttaccc gcttccccac acatttacta 7500
gttctagagc ggccgccacc gcggtggagc tccaattcgc cctatagtga gtcgtattac 7560
aattcactgg ccgtcgtttt acaacgtcgt gactgggaaa accctggcgt tacccaactt 7620
aatcgccttg cagcacatcc ccccttcgcc agctggcgta atagcgaaga ggcccgcacc 7680
gatcgccctt cccaacagtt gcgcagcctg aatggcgaat ggcgcgacgc gccctgtagc 7740
ggcgcattaa gcgcggcggg tgtggtggtt acgcgcagcg tgaccgctac acttgccagc 7800
gccctagcgc ccgctccttt cgctttcttc ccttcctttc tcgccacgtt cgccggtagt 7860
gttagacctg aacaaggttt actaaaaatc cgtaaagaac ttcaattgta cgccaactta 7920
aggcctcgtc cccgccgggt cacccggcca gcgacatgga ggcccagaat accctccttg 7980
acagtcttga cgtgcgcagc tcaggggcat gatgtgactg tcgcccgtac atttagccca 8040
tacatcccca tgtataatca tttgcatcca tacattttga tggccgcacg gcgcgaagca 8100
aaaattacgg ctcctcgctg cagacctgcg agcagggaaa cgctcccctc acagacgcgt 8160
gaattgtccc cacgccgcgc ccctgtagag aaatataaaa ggttaggatt tgccactgag 8220
gttcttcttt catatacttc cttttaaaat cttgctagga tacagttctc acatcacatc 8280
cgaacataaa caaccatggg taaaaagcct gaactcaccg cgacgtctgt cgagaagttt 8340
ctgatcgaaa agttcgacag cgtctccgac ctgatgcagc tctcggaggg cgaagaatct 8400
cgtgctttca gcttcgatgt aggagggcgt ggatatgtcc tgcgggtaaa tagctgcgcc 8460
gatggtttct acaaagatcg ttatgtttat cggcactttg catcggccgc gctcccgatt 8520

ccggaagtgc ttgacattgg ggaattcagc gagagcctga cctattgcat ctcccgccgt 8580

gcacagggtg tcacgttgca agacctgcct gaaaccgaac tgcccgctgt tctgcagccg 8640

gtcgcggagg ccatggatgc gatcgctgcg gccgatctta gccagacgag cgggttcggc 8700

ccattcggac cgcaaggaat cggtcaatac actacatggc gtgatttcat atgcgcgatt 8760

gctgatcccc atgtgtatca ctggcaaact gtgatggacg acaccgtcag tgcgtccgtc 8820

gcgcaggctc tcgatgagct gatgctttgg gccgaggact gccccgaagt ccggcacctc 8880

gtgcacgcgg atttcggctc caacaatgtc ctgacggaca atggccgcat aacagcggtc 8940

attgactgga gcgaggcgat gttcggggat tcccaatacg aggtcgccaa catcttcttc 9000

tggaggccgt ggttggcttg tatggagcag cagacgcgct acttcgagcg gaggcatccg 9060

gagcttgcag gatcgccgcg gctccgggcg tatatgctcc gcattggtct tgaccaactc 9120

tatcagagct tggttgacgg caatttcgat gatgcagctt gggcgcaggg tcgatgcgac 9180

gcaatcgtcc gatccggagc cgggactgtc gggcgtacac aaatcgcccg cagaagcgcg 9240

gccgtctgga ccgatggctg tgtagaagta ctcgccgata gtggaaaccg acgccccagc 9300

actcgtccga gggcaaagga ataatcagta ctgacaataa aaagattctt gttttcaaga 9360

acttgtcatt tgtatagttt ttttatattg tagttgttct attttaatca aatgttagcg 9420

tgatttatat ttttttttcgc ctcgacatca tctgcccaga tgcgaagtta agtgcgcaga 9480

aagtaatatc atgcgtcaat cgtatgtgaa tgctggtcgc tatactgctg tcgattcgat 9540

actaacgccg ccatccagtg tcgacggatc ctaggtgtac ataaacttta taaatgaaat 9600

tcataataga aacgacacga aattacaaaa tggaatatgt tcatagggta gacgaaacta 9660

tatacgcaat ctacatacat ttatcaagaa ggagaaaaag gaggatagta aaggaataca 9720

ggtaagcaaa ttgatactaa tggctcaacg tgataaggaa aaagaattgc actttaacat 9780

taatattgac aaggaggagg gcaccacaca aaaagttagg tgtaacagaa aatcatgaaa 9840

ctacgattcc taatttgata ttggaggatt ttctctaaaa aaaaaaaaat acaacaaata 9900

aaaaacactc aatgacctga ccatttgatg gagtttaagt caataccttc ttgaaccatt 9960

tcccataatg gtgaaagttc cctcaagaat tttactctgt cagaaacggc cttacgacgt 10020

agtcgatatg gtgcactctc agtacaatct gctctgatgc cgcatagtta agccagcccc 10080

gacacccgcc aacacccgct gacgcgccct gacgggcttg tctgctcccg gcatccgctt 10140

acagacaagc tgtgaccgtc tccgggagct gcatgtgtca gaggttttca ccgtcatcac 10200

cgaaacgcgc gagacgaaag ggcctcgtga tacgcctatt tttataggtt aatgtcatga 10260

taataatggt ttcttaggac ggatcgcttg cctgtaactt acacgcgcct cgtatctttt 10320

aatgatggaa taatttggga atttactctg tgtttattta tttttatgtt ttgtatttgg 10380

attttagaaa gtaaataaag aaggtagaag agttacggaa tgaagaaaaa aaaataaaca 10440
aaggtttaaa aaatttcaac aaaaagcgta ctttacatat atatttatta gacaagaaaa 10500
gcagattaaa tagatataca ttcgattaac gataagtaaa atgtaaaatc acaggatttt 10560
cgtgtgtggt cttctacaca gacaagatga aacaattcgg cattaatacc tgagagcagg 10620
aagagcaaga taaaaggtag tatttgttgg cgatccccct agagtctttt acatcttcgg 10680
aaaacaaaaa ctattttttc tttaatttct ttttttactt tctattttta atttatatat 10740
ttatattaaa aaatttaaat tataattatt tttatagcac gtgatgaaaa ggacccaggt 10800
ggcacttttc ggggaaatgt gcgcggaacc cctatttgtt tatttttcta aatacattca 10860
aatatgtatc cgctcatgag acaataaccc tgataaatgc ttcaataata ttgaaaaagg 10920
aagagtatga gtattcaaca tttccgtgtc gcccttattc ccttttttgc ggcattttgc 10980
cttcctgttt ttgctcaccc agaaacgctg gtgaaagtaa aagatgctga agatcagttg 11040
ggtgcacgag tgggttacat cgaactggat ctcaacagcg gtaagatcct tgagagtttt 11100
cgccccgaag aacgttttcc aatgatgagc acttttaaag ttctgctatg tggcgcggta 11160
ttatcccgta ttgacgccgg gcaagagcaa ctcggtcgcc gcatacacta ttctcagaat 11220
gacttggttg agtactcacc agtcacagaa aagcatctta cggatggcat gacagtaaga 11280
gaattatgca gtgctgccat aaccatgagt gataacactg cggccaactt acttctgaca 11340
acgatcggag gaccgaagga gctaaccgct tttttcaca acatggggga tcatgtaact 11400
cgccttgatc gttgggaacc ggagctgaat gaagccatac caaacgacga gcgtgacacc 11460
acgatgcctg tagcaatggc aacaacgttg cgcaaactat taactggcga actacttact 11520
ctagcttccc ggcaacaatt aatagactgg atggaggcgg ataaagttgc aggaccactt 11580
ctgcgctcgg cccttccggc tggctggttt attgctgata aatctggagc cggtgagcgt 11640
gggtctcgcg gtatcattgc agcactgggg ccagatggta agccctcccg tatcgtagtt 11700
atctacacga cgggcagtca ggcaactatg gatgaacgaa atagacagat cgctgagata 11760
ggtgcctcac tgattaagca ttggtaactg tcagaccaag tttactcata tatactttag 11820
attgatttaa aacttcattt ttaatttaaa aggatctagg tgaagatcct ttttgataat 11880
ctcatgacca aaatccctta acgtgagttt tcgttccact gagcgtcaga ccccgtagaa 11940
aagatcaaag gatcttcttg agatcctttt tttctgcgcg taatctgctg cttgcaaaca 12000
aaaaaaccac cgctaccagc ggtggtttgt ttgccggatc aagagctacc aactcttttt 12060
ccgaaggtaa ctggcttcag cagagcgcag ataccaaata ctgtccttct agtgtagccg 12120
tagttaggcc accacttcaa gaactctgta gcaccgccta catacctcgc tctgctaatc 12180
ctgttaccag tggctgctgc cagtggcgat aagtcgtgtc ttaccgggtt ggactcaaga 12240
cgatagttac cggataaggc gcagcggtcg ggctgaacgg ggggttcgtg cacacagccc 12300

```
agcttggagc gaacgaccta caccgaactg agatacctac agcgtgagca ttgagaaagc  12360
gccacgcttc ccgaagggag aaaggcggac aggtatccgg taagcggcag ggtcggaaca  12420
ggagagcgca cgagggagct tccaggggg aacgcctggt atctttatag tcctgtcggg  12480
tttcgccacc tctgacttga gcgtcgattt ttgtgatgct cgtcaggggg gccgagccta  12540
tggaaaaacg ccagcaacgc ggccttttta cggttcctgg ccttttgctg gccttttgct  12600
cacatgttct ttcctgcgtt atcccctgat tctgtggata accgtattac cgcctttgag  12660
tgagctgata ccgctcgccg cagccgaacg accgagcgca gcgagtcagt gagcgaggaa  12720
gcggaagagc gcccaatacg caaaccgcct ctccccgcgc gttggccgat tcattaatgc  12780
agctggcacg acaggtttcc cgactggaaa gcgggcagtg agcgcaacgc aattaatgtg  12840
agttacctca ctcattaggc accccaggct ttacacttta tgcttccggc tcctatgttg  12900
tgtggaattg tgagcggata acaatttcac acaggaaaca gctatgacca tgat        12954
```

<210> 30
<211> 10329
<212> DNA
<213> Artificial

<220>
<223> pRN608 plasmid for overexpression of DAK1 and GCY1

<400> 30

```
agtcgtgtct taccgggttg gactcaagac gatagttacc ggataaggcg cagcggtcgg      60
gctgaacggg gggttcgtgc acacagccca gcttggagcg aacgacctac accgaactga     120
gatacctaca gcgtgagcat tgagaaagcg ccacgcttcc cgaagggaga aaggcggaca     180
ggtatccggt aagcggcagg gtcggaacag gagagcgcac gagggagctt ccagggggga     240
acgcctggta tctttatagt cctgtcgggt ttcgccacct ctgacttgag cgtcgatttt     300
tgtgatgctc gtcagggggg ccgagcctat ggaaaaacgc cagcaacgcg gcctttttac     360
ggttcctggc cttttgctgg ccttttgctc acatgttctt tcctgcgtta tcccctgatt     420
ctgtggataa ccgtattacc gcctttgagt gagctgatac cgctcgccgc agccgaacga     480
ccgagcgcag cgagtcagtg agcgaggaag cggaagagcg cccaatacgc aaaccgcctc     540
tccccgcgcg ttggccgatt cattaatgca gctggcacga caggtttccc gactggaaag     600
cgggcagtga gcgcaacgca attaatgtga gttacctcac tcattaggca ccccaggctt     660
tacactttat gcttccggct cctatgttgt gtggaattgt gagcggataa caatttcaca     720
caggaaacag ctatgaccat gattacgcca agctcggaat taaccctcac taaagggaac     780
aaaagctggg taccgggccc ccctcgagt accggtttct tcttcagatt ccctcatgga      840
gaaagtgcgg cagatgtata tgacagagtc gccagtttcc aagagacttt attcaggcac     900
```

ttccatgata ggcaagagag aagacccaga gatgttgttg tcctagttac acatggtatt 960
tattccagag tattcctgat gaaatggttt agatggacat acgaagagtt tgaatcgttt 1020
accaatgttc ctaacgggag cgtaatggtg atggaactgg acgaatccat caatagatac 1080
gtcctgagga ccgtgctacc caaatggact gattgtgagg gagacctaac tacatagtgt 1140
ttaaagatta cggatattta acttacttag aataatgcca tttttttgag ttataataat 1200
cctacgttag tgtgagcggg atttaaactg tgaggacctt aatacattca gacacttctg 1260
cggtatcacc ctacttattc ccttcgagat tatatctagg aacccatcag gttggtggaa 1320
gattacccgt tctaagactt ttcagcttcc tctattgatg ttacacctgg acaccccttt 1380
tctggcatcc agtttttaat cttcagtggc atgtgagatt ctccgaaatt aattaaagca 1440
atcacacaat tctctcggat accacctcgg ttgaaactga caggtggttt gttacgcatg 1500
ctaatgcaaa ggagcctata tacctttggc tcggctgctg taacagggaa tataaagggc 1560
agcataattt aggagtttag tgaacttgca acatttacta ttttcccttc ttacgtaaat 1620
atttttcttt ttaattctaa atcaatcttt ttcaattttt tgtttgtatt cttttcttgc 1680
ttaaatctat aactacaaaa aacacataca taaatctaga aaatgtccgc taaatcgttt 1740
gaagtcacag atccagtcaa ttcaagtctc aaagggtttg cccttgctaa cccctccatt 1800
acgctggtcc ctgaagaaaa aattctcttc agaaagaccg attccgacaa gatcgcatta 1860
atttctggtg gtggtagtgg acatgaacct acacacgccg gtttcattgg taagggtatg 1920
ttgagtggcg ccgtggttgg cgaaattttt gcatcccctt caacaaaaca gattttaaat 1980
gcaatccgtt tagtcaatga aaatgcgtct ggcgttttat tgattgtgaa gaactacaca 2040
ggtgatgttt tgcattttgg tctgtccgct gagagagcaa gagccttggg tattaactgc 2100
cgcgttgctg tcataggtga tgatgttgca gttggcagag aaaagggtgg tatggttggt 2160
agaagagcat tggcaggtac cgttttggtt cataagattg taggtgcctt cgcagaagaa 2220
tattctagta agtatggctt agacggtaca gctaaagtgg ctaaaattat caacgacaat 2280
ttggtgacca ttggatcttc tttagaccat tgtaaagttc ctggcaggaa attcgaaagt 2340
gaattaaacg aaaaacaaat ggaattgggt atgggtattc ataacgaacc tggtgtgaaa 2400
gttttagacc ctattccttc taccgaagac ttgatctcca agtatatgct accaaaacta 2460
ttggatccaa acgataagga tagagctttt gtaaagtttg atgaagatga tgaagttgtc 2520
ttgttagtta acaatctcgg cggtgtttct aattttgtta ttagttctat cacttccaaa 2580
actacggatt tcttaaagga aaattacaac ataaccccgg ttcaaacaat tgctggcaca 2640
ttgatgacct ccttcaatgg taatgggttc agtatcacat tactaaacgc cactaaggct 2700
acaaaggctt tgcaatctga ttttgaggag atcaaatcag tactagactt gttgaacgca 2760
tttacgaacg caccgggctg gccaattgca gattttgaaa agacttctgc cccatctgtt 2820

aacgatgact tgttacataa tgaagtaaca gcaaaggccg tcggtaccta tgactttgac 2880
aagtttgctg agtggatgaa gagtggtgct gaacaagtta tcaagagcga accgcacatt 2940
acggaactag acaatcaagt tggtgatggt gattgtggtt acactttagt ggcaggagtt 3000
aaaggcatca ccgaaaacct tgacaagctg tcgaaggact cattatctca ggcggttgcc 3060
caaatttcag atttcattga aggctcaatg ggaggtactt ctggtggttt atattctatt 3120
cttttgtcgg gtttttcaca cggattaatt caggtttgta aatcaaagga tgaacccgtc 3180
actaaggaaa ttgtggctaa gtcactcgga attgcattgg atactttata caaatataca 3240
aaggcaagga agggatcatc caccatgatt gatgctttag aaccattcgt taaagaattt 3300
actgcatcta aggatttcaa taaggcggta aaagctgcag aggaaggtgc taaatccact 3360
gctacattcg aggccaaatt tggcagagct tcgtatgtcg gcgattcatc tcaagtagaa 3420
gatcctggtg cagtaggcct atgtgagttt ttgaaggggg ttcaaagcgc cttgtaagtc 3480
gagacaaatc gctcttaaat atatacctaa agaacattaa agctatatta taagcaaaga 3540
tacgtaaatt ttgcttatat tattatacac atatcatatt tctatatttt taagatttgg 3600
ttatataatg tacgtaatgc aaaggaaata aattttatac attattgaac agcgtccaag 3660
taactacatt atgtgcacta atagtttagc gtcgtgaaga ctttattgtg tcgcgaaaag 3720
taaaaatttt aaaaattaga gcaccttgaa cttgcgaaaa aggttctcat caactgttta 3780
aaacgtacgt gtggaagaac gattacaaca ggtgttgtcc tctgaggaca taaaatacac 3840
accgagattc atcaactcat tgctggagtt agcatatcta caattgggtg aaatggggag 3900
cgatttgcag gcatttgctc ggcatgccgg tagaggtgtg gtcaataaga gcgacctcat 3960
gctatacctg agaaagcaac ctgacctaca ggaaagagtt actcaagaat aagaattttc 4020
gttttaaaac ctaagagtca ctttaaaatt tgtatacact tatttttttt ataacttatt 4080
taataataaa aatcataaat cataagaaat tcgcgcgctt acttgaatac ttcgaaagga 4140
gaccaatttg gatgtacaac cctttttttcg ccccttttcct tcgatatgtt attgatagct 4200
tcaaagtcct cagtagacaa agtaaatatt ttcctgttcg ttttgattcg atcgggattc 4260
acagattttg gcaagacaac ataacctctt tggacgtgcc agctaataac aacgtgtccg 4320
ggttgaacgt tatttttctt cgcaatttca aggataaccg gttccttcaa tagtggagca 4380
tcggtactac ctaacggaga ataagcttca accacaatgc ctttactttt acaaaaatta 4440
atcaattcgt cttgaggtag taatggatgt atttcgactt ggttagcagc tggcgtaagc 4500
ttattacctt gagatgctaa tagatctttc aggttattta tagaaaagtt ggagactcca 4560
acggccttag ttttaccagt ctttggtagt tcctgcatta attcccaggt tttgatgaaa 4620
ttccaattgg tgatatccac tgcacgagaa ccatccttct ttgttggcac actcaagatg 4680

tcttcatttt tgatgtaggc tggatctaat ctggcaggcc aatgcatcaa atataagtct 4740
acgtagtcca atcctaacct ctttagtgat tgatccagcg ctacttcagg ttcgtggtgt 4800
tgtgtacacc ataactttgt agtaacaaag atttcttccc gaggaacacc tgaatccttg 4860
atggcttgac cgacttggtc ttcattacgg taaatagcag cagtatcaat gtgtcggtag 4920
ccatctttca aagcggttaa aacagcctta taagcatcgt tctctttcga ctgccacgta 4980
cctaaaccta tttgagggat ttgggctcca gtatttagag aaaggatttt cgtagaatca 5040
tgtaaagtag caggcatttt ctgcagttaa ttcagtaaat tttcgatctt gggaagaaaa 5100
aagcagtaag cgtgaaaaat ctaaaagctg atgtagtaga agatcctatt ctttaacaaa 5160
gattgacctt ttctttttct tcttggtttg agtagaaagg ggaaggaaga atacaagaga 5220
gaggaaaaaa aggaagataa aaagagagcg tgatataaat gaatatatat taaacaagag 5280
agattgggaa ggaaaggatc aaacaaaccc aaaaatattt caaaaaggag agagagaggc 5340
gagtttggtt tcaaaacggt ttatttattt atgcaagagg acgtggaaga aaaagaagaa 5400
ggaagaaaaa aatttgaaag aaaaaaacgc gtggcgggta aagaagaaaa tggaaaatag 5460
aggccgggtg acagagaaat attgagggtt aattggaaaa tatgttaggg tgaggcatat 5520
gtttttaagg gttttgagga tccgataagg aagaatgtag gttaaatgtt gtgcattaat 5580
tgctgtggca gcttacccgc ttccccacac atttactagt tctagagcgg ccgccaccgc 5640
ggtggagctc caattcgccc tatagtgagt cgtattacaa ttcactggcc gtcgttttac 5700
aacgtcgtga ctgggaaaac cctggcgtta cccaacttaa tcgccttgca gcacatcccc 5760
ccttcgccag ctggcgtaat agcgaagagg cccgcaccga tcgcccttcc caacagttgc 5820
gcagcctgaa tggcgaatgg cgcgacgcgc cctgtagcgg cgcattaagc gcggcgggtg 5880
tggtggttac gcgcagcgtg accgctacac ttgccagcgc cctagcgccc gctcctttcg 5940
ctttcttccc ttcctttctc gccacgttcg ccggtagtgt tagacctgaa caaggtttac 6000
taaaaatccg taaagaactt caattgtacg ccaacttaag gcctcgtccc cgccgggtca 6060
cccggccagc gacatggagg cccagaatac cctccttgac agtcttgacg tgcgcagctc 6120
aggggcatga tgtgactgtc gcccgtacat ttagcccata catccccatg tataatcatt 6180
tgcatccata cattttgatg gccgcacggc gcgaagcaaa aattacggct cctcgctgca 6240
gacctgcgag cagggaaacg ctcccctcac agacgcgtga attgtcccca cgccgcgccc 6300
ctgtagagaa atataaaagg ttaggatttg ccactgaggt tcttctttca tatacttcct 6360
tttaaaatct tgctaggata cagttctcac atcacatccg aacataaaca accatgggta 6420
aaaagcctga actcaccgcg acgtctgtcg agaagtttct gatcgaaaag ttcgacagcg 6480
tctccgacct gatgcagctc tcggagggcg aagaatctcg tgctttcagc ttcgatgtag 6540
gagggcgtgg atatgtcctg cgggtaaata gctgcgccga tggtttctac aaagatcgtt 6600

atgtttatcg gcactttgca tcggccgcgc tcccgattcc ggaagtgctt gacattgggg 6660
aattcagcga gagcctgacc tattgcatct cccgccgtgc acagggtgtc acgttgcaag 6720
acctgcctga aaccgaactg cccgctgttc tgcagccggt cgcggaggcc atggatgcga 6780
tcgctgcggc cgatcttagc cagacgagcg ggttcggccc attcggaccg caaggaatcg 6840
gtcaatacac tacatggcgt gatttcatat gcgcgattgc tgatccccat gtgtatcact 6900
ggcaaactgt gatggacgac accgtcagtg cgtccgtcgc gcaggctctc gatgagctga 6960
tgctttgggc cgaggactgc cccgaagtcc ggcacctcgt gcacgcggat ttcggctcca 7020
acaatgtcct gacggacaat ggccgcataa cagcggtcat tgactggagc gaggcgatgt 7080
tcggggattc ccaatacgag gtcgccaaca tcttcttctg gaggccgtgg ttggcttgta 7140
tggagcagca gacgcgctac ttcgagcgga ggcatccgga gcttgcagga tcgccgcggc 7200
tccgggcgta tatgctccgc attggtcttg accaactcta tcagagcttg gttgacggca 7260
atttcgatga tgcagcttgg gcgcagggtc gatgcgacgc aatcgtccga tccggagccg 7320
ggactgtcgg gcgtacacaa atcgcccgca gaagcgcggc cgtctggacc gatggctgtg 7380
tagaagtact cgccgatagt ggaaaccgac gccccagcac tcgtccgagg gcaaaggaat 7440
aatcagtact gacaataaaa agattcttgt tttcaagaac ttgtcatttg tatagttttt 7500
ttatattgta gttgttctat tttaatcaaa tgttagcgtg atttatattt tttttcgcct 7560
cgacatcatc tgcccagatg cgaagttaag tgcgcagaaa gtaatatcat gcgtcaatcg 7620
tatgtgaatg ctggtcgcta tactgctgtc gattcgatac taacgccgcc atccagtgtc 7680
gacggatcct aggtgtacat aaactttata aatgaaattc ataatagaaa cgacacgaaa 7740
ttacaaaatg gaatatgttc atagggtaga cgaaactata tacgcaatct acatacattt 7800
atcaagaagg agaaaaagga ggatagtaaa ggaatacagg taagcaaatt gatactaatg 7860
gctcaacgtg ataaggaaaa agaattgcac tttaacatta atattgacaa ggaggagggc 7920
accacacaaa aagttaggtg taacagaaaa tcatgaaact acgattccta atttgatatt 7980
ggaggatttt ctctaaaaaa aaaaaaatac aacaaataaa aaacactcaa tgacctgacc 8040
atttgatgga gtttaagtca ataccttctt gaaccatttc ccataatggt gaaagttccc 8100
tcaagaattt tactctgtca gaaacggcct tacgacgtag tcgatatggt gcactctcag 8160
tacaatctgc tctgatgccg catagttaag ccagccccga cacccgccaa cacccgctga 8220
cgcgccctga cgggcttgtc tgctcccggc atccgcttac agacaagctg tgaccgtctc 8280
cgggagctgc atgtgtcaga ggttttcacc gtcatcaccg aaacgcgcga gacgaaaggg 8340
cctcgtgata cgcctatttt tataggttaa tgtcatgata ataatggttt cttaggacgg 8400
atcgcttgcc tgtaacttac acgcgcctcg tatcttttaa tgatggaata atttgggaat 8460

ttactctgtg tttatttatt tttatgtttt gtatttggat tttagaaagt aaataaagaa 8520
ggtagaagag ttacggaatg aagaaaaaaa aataaacaaa ggtttaaaaa atttcaacaa 8580
aaagcgtact ttacatatat atttattaga caagaaaagc agattaaata gatatacatt 8640
cgattaacga taagtaaaat gtaaaatcac aggattttcg tgtgtggtct tctacacaga 8700
caagatgaaa caattcggca ttaatacctg agagcaggaa gagcaagata aaaggtagta 8760
tttgttggcg atcccccctag agtcttttac atcttcggaa aacaaaaact atttttttctt 8820
taatttcttt ttttactttc tatttttaat ttatatattt atattaaaaa atttaaatta 8880
taattatttt tatagcacgt gatgaaaagg acccaggtgg cacttttcgg ggaaatgtgc 8940
gcggaacccc tatttgttta tttttctaaa tacattcaaa tatgtatccg ctcatgagac 9000
aataaccctg ataaatgctt caataatatt gaaaaaggaa gagtatgagt attcaacatt 9060
tccgtgtcgc ccttattccc ttttttgcgg cattttgcct tcctgttttt gctcacccag 9120
aaacgctggt gaaagtaaaa gatgctgaag atcagttggg tgcacgagtg ggttacatcg 9180
aactggatct caacagcggt aagatccttg agagttttcg ccccgaagaa cgttttccaa 9240
tgatgagcac ttttaaagtt ctgctatgtg gcgcggtatt atcccgtatt gacgccgggc 9300
aagagcaact cggtcgccgc atacactatt ctcagaatga cttggttgag tactcaccag 9360
tcacagaaaa gcatcttacg gatggcatga cagtaagaga attatgcagt gctgccataa 9420
ccatgagtga taacactgcg gccaacttac ttctgacaac gatcggagga ccgaaggagc 9480
taaccgcttt tttcacaac atgggggatc atgtaactcg ccttgatcgt tgggaaccgg 9540
agctgaatga agccatacca aacgacgagc gtgacaccac gatgcctgta gcaatggcaa 9600
caacgttgcg caaactatta actggcgaac tacttactct agcttcccgg caacaattaa 9660
tagactggat ggaggcggat aaagttgcag gaccacttct gcgctcggcc cttccggctg 9720
gctggtttat tgctgataaa tctggagccg gtgagcgtgg gtctcgcggt atcattgcag 9780
cactggggcc agatggtaag ccctcccgta tcgtagttat ctacacgacg ggcagtcagg 9840
caactatgga tgaacgaaat agacagatcg ctgagatagg tgcctcactg attaagcatt 9900
ggtaactgtc agaccaagtt tactcatata tactttagat tgatttaaaa cttcattttt 9960
aatttaaaag gatctaggtg aagatccttt ttgataatct catgaccaaa atcccttaac 10020
gtgagttttc gttccactga gcgtcagacc ccgtagaaaa gatcaaagga tcttcttgag 10080
atcctttttt tctgcgcgta atctgctgct tgcaaacaaa aaaaccaccg ctaccagcgg 10140
tggtttgttt gccggatcaa gagctaccaa ctcttttttcc gaaggtaact ggcttcagca 10200
gagcgcagat accaaatact gtccttctag tgtagccgta gttaggccac cacttcaaga 10260
actctgtagc accgcctaca tacctcgctc tgctaatcct gttaccagtg gctgctgcca 10320
gtggcgata 10329

<210> 31
<211> 11163
<212> DNA
<213> Artificial

<220>
<223> pRN616

<400> 31

```
tttgattaaa atagaacaac tacaatataa aaaaactata caaatgacaa gttcttgaaa      60
acaagaatct ttttattgtc agtgtgtcag tcctgctcct cggccacgaa gtgcacgcag     120
ttgccggccg ggtcgcgcag ggcgaactcc cgcccccacg gctgctcgcc gatctcggtc     180
atggccggcc cggaggcgtc ccggaagttc gtggacacga cctccgacca ctcggcgtac     240
agctcgtcca ggccgcgcac ccacacccag gccagggtgt tgtccggcac cacctggtcc     300
tggaccgcgc tgatgaacag ggtcacgtcg tcccggacca caccggcgaa gtcgtcctcc     360
acgaagtccc gggagaaccc gagccggtcg gtccagaact cgaccgctcc ggcgacgtcg     420
cgcgcggtga gcaccggaac ggcactggtc aacttggcca tggttgttta tgttcggatg     480
tgatgtgaga actgtatcct agcaagattt taaaaggaag tatatgaaag aagaacctca     540
gtggcaaatc ctaacctttt atatttctct acaggggcgc ggcgtgggga caattcaacg     600
cgtctgtgag gggagcgttt ccctgctcgc aggtctgcag cgaggagccg taatttttgc     660
ttcgcgccgt gcggccatca aaatgtatgg atgcaaatga ttatacatgg ggatgtatgg     720
gctaaatgta cgggcgacag tcacatcatg cccctgagct gcgcacgtca agactgtcaa     780
ggagggtatt ctgggcctcc atgtcgctgg ccgggtgacc cggcggggac gaggccttaa     840
gcggccgcat gctagctccg gattatcgat gataagctgt caaacatgag aattaattcc     900
acggactata gactatacct agtatactcc gtctactgta cgatacactt ccgctcaggt     960
ccttgtcctt taacgaggcc ttaccactct tttgttactc tattgatcca gctcagcaaa    1020
ggcagtgtga tctaagattc tatcttcgcg atgtagtaaa actagctaga ccgagaaaga    1080
gactagaaat gcaaaaggca cttctacaat ggctgccatc attattatcc gatgtgacgc    1140
tgcagcttct caatgatatt cgaatacgct ttgaggagat acagcctaat atccgacaaa    1200
ctgttttaca gatttacgat cgtacttgtt acccatcatt gaattttgaa catccgaacc    1260
tgggagtttt ccctgaaaca gatagtatat ttgaacctgt ataataatat atagtctagc    1320
gctttacgga agacaatgta tgtatttcgg ttcctggaga aactattgca tctattgcat    1380
aggtaatctt gcacgtcgca tccccggttc attttctgcg tttccatctt gcacttcaat    1440
agcatatctt tgttaacgaa gcatctgtgc ttcattttgt agaacaaaaa tgcaacgcga    1500
gagcgctaat ttttcaaaca aagaatctga gctgcatttt tacagaacag aaatgcaacg    1560
```

cgaaagcgct attttaccaa cgaagaatct gtgcttcatt tttgtaaaac aaaaatgcaa 1620
cgcgagagcg ctaatttttc aaacaaagaa tctgagctgc atttttacag aacagaaatg 1680
caacgcgaga gcgctatttt accaacaaag aatctatact tctttttgt tctacaaaaa 1740
tgcatcccga gagcgctatt tttctaacaa agcatcttag attacttttt ttctcctttg 1800
tgcgctctat aatgcagtct cttgataact ttttgcactg taggtccgtt aaggttagaa 1860
gaaggctact ttggtgtcta ttttctcttc cataaaaaaa gcctgactcc acttcccgcg 1920
tttactgatt actagcgaag ctgcgggtgc atttttcaa gataaaggca tccccgatta 1980
tattctatac cgatgtggat tgcgcatact ttgtgaacag aaagtgatag cgttgatgat 2040
tcttcattgg tcagaaaatt atgaacggtt tcttctattt tgtctctata tactacgtat 2100
aggaaatgtt tacattttcg tattgttttc gattcactct atgaatagtt cttactacaa 2160
tttttttgtc taaagagtaa tactagagat aaacataaaa aatgtagagg tcgagtttag 2220
atgcaagttc aaggagcgaa aggtggatgg gtaggttata tagggatata gcacagagat 2280
atatagcaaa gagatacttt tgagcaatgt ttgtggaaag cggtattcgc aatgccggca 2340
aaagggcgaa ttgattttga agagaatgtg gattttgatg taattgttgg gattccattt 2400
ttaataaggc aataatatta ggtatgtgga tatactagaa gttctcctcg accgtcgata 2460
tgcggtgtga aataccgcac agatgcgtaa ggagaaaata ccgcatcagg aaattgtaaa 2520
cgttaatatt ttgttaaaat tcgcgttaaa tttttgttaa atcagctcat tttttaacca 2580
ataggccgaa atcggcaaaa tcccttataa atcaaaagaa tagaccgaga tagggttgag 2640
tgttgttcca gtttggaaca agagtccact attaaagaac gtggactcca acgtcaaagg 2700
gcgaaaaacc gtctatcagg gcgatggccc actacgtgaa ccatcaccct aatcaagttt 2760
tttggggtcg aggtgccgta aagcactaaa tcggaaccct aaagggagcc cccgatttag 2820
agcttgacgg ggaaagccgg cgaacgtggc gagaaaggaa gggaagaaag cgaaaggagc 2880
gggcgctagg gcgctggcaa gtgtagcggt cacgctgcgc gtaaccacca cacccgccgc 2940
gcttaatgcg ccgctacagg gcgcgtcgcg ccattcgcca ttcaggctgc gcaactgttg 3000
ggaagggcga tcggtgcggg cctcttcgct attacgccag ctggcgaagg ggggatgtgc 3060
tgcaaggcga ttaagttggg taacgccagg gttttcccag tcacgacgtt gtaaaacgac 3120
ggccagtgaa ttgtaatacg actcactata gggcgaattg gagctccacc gcggtggcgg 3180
ccgctctaga actagtctcg agctcttcaa ctcaagacgc acagatatta taacatctgc 3240
ataataggca tttgcaagaa ttactcgtga gtaaggaaag agtgaggaac tatcgcatac 3300
ctgcatttaa agatgccgat ttgggcgcga atcctttatt ttggcttcac cctcatacta 3360
ttatcagggc cagaaaaagg aagtgtttcc ctccttcttg aattgatgtt accctcataa 3420
agcacgtggc ctcttatcga gaaagaaatt accgtcgctc gtgatttgtt tgcaaaaaga 3480

acaaaactga aaaaacccag acacgctcga cttcctgtct tcctattgat tgcagcttcc 3540

aatttcgtca cacaacaagg tcctagcgac ggctcacagg ttttgtaaca agcaatcgaa 3600

ggttctggaa tggcgggaaa gggtttagta ccacatgcta tgatgcccac tgtgatctcc 3660

agagcaaagt tcgttcgatc gtactgttac tctctctctt tcaaacagaa ttgtccgaat 3720

cgtgtgacaa caacagcctg ttctcacaca ctcttttctt ctaaccaagg gggtggttta 3780

gtttagtaga acctcgtgaa acttacattt acatatatat aaacttgcat aaattggtca 3840

atgcaagaaa tacatatttg gtcttttcta attcgtagtt tttcaagttc ttagatgctt 3900

tctttttctc ttttttacag atcatcaagg aagtaattat ctacttttta caacaaatat 3960

atctagactg cagaaaatgt ctgaattgaa cgagaagttg gctaccgctt gggaaggttt 4020

caccaagggt gactggcaaa acgaagttaa cgttagagac ttcatccaaa agaactacac 4080

cccatacgaa ggtgacgaat ctttcttggc tggtgctacc gaagctacca ccaccttgtg 4140

ggacaaggtt atggaaggtg ttaagttgga aaacagaacc cacgctccag ttgacttcga 4200

caccgctgtt gcttctacca tcacctctca cgacgctggt tacatcaaca agcaattgga 4260

aaagatcgtt ggtttacaaa ccgaagctcc attgaagaga gctttgatcc cattcggtgg 4320

tatcaagatg atcgaaggtt cttgtaaggc ttacaacaga gaattggacc caatgatcaa 4380

gaagattttc accgaataca gaaagaccca caaccaaggt gttttcgacg tttacactcc 4440

agacatcttg agatgtagaa agtctggtgt tttgactggt ttgccagacg cttacggtag 4500

aggtagaatc atcggtgact acagaagagt tgctttgtac ggtatcgact acttgatgaa 4560

ggacaagttg gctcaattca cctctttgca agctgacttg gaaaacggtg ttaacttgga 4620

acaaaccatc agattgagag aagaaatcgc tgaacaacac agagctttgg gtcaaatgaa 4680

ggaaatggct gctaagtacg gttacgacat ctctggtcca gctaccaacg ctcaagaagc 4740

tatccaatgg acctacttcg gttacttggc tgctgttaag tctcaaaacg gtgctgctat 4800

gtctttcggt aggacctcta ccttcttgga cgtttacatc gaaagagact tgaaggctgg 4860

taagatcacc gaacaagaag ctcaagaaat ggttgaccac ttggttatga agttgagaat 4920

ggttagattc ttgagaaccc cagaatacga cgaattgttc tctggtgacc caatctgggc 4980

taccgaatct atcggtggta tgggtttgga cggtagaacc ttggttacca agaactcttt 5040

cagattcttg aacaccttat acaccatggg tccatctcca gaaccaaaca tgaccatctt 5100

gtggtctgaa aagttaccat tgaacttcaa gaagttcgct gctaaggttt ctatcgacac 5160

ctcttctttg caatacgaaa acgacgactt gatgagacca gacttcaaca acgacgacta 5220

cgctatcgct tgttgtgttt ctccaatgat cgttggtaag caaatgcaat tcttcggtgc 5280

tagagctaac ttggctaaga ccatgttgta cgctatcaac ggtggtgttg acgaaaagtt 5340

gaagatgcaa gttggtccaa agtctgaacc aatcaagggt gacgttttga actacgacga 5400
agttatggaa agaatggacc acttcatgga ctggttggct aagcaataca tcaccgcttt 5460
gaacatcatc cactacatgc acgacaagta ctcttacgaa gcatcattga tggctttgca 5520
cgacagagac gtaatcagaa ccatggcttg tggtatcgct ggtttgtctg ttgctgctga 5580
ctctttgtct gctatcaagt acgctaaggt taagccaatc agagacgaag acggtttggc 5640
tatcgacttc gaaatcgaag gtgaataccc tcaattcggt aacaacgacc caagagttga 5700
cgacttggct gttgacttgg ttgaaagatt tatgaagaag atccaaaagt tgcacaccta 5760
cagagacgct atcccaaccc aatctgtttt gactatcaca tctaacgttg tttacggtaa 5820
gaagactggt aacaccccag acggtagaag agctggtgct ccattcggtc caggtgctaa 5880
cccaatgcac ggtagagacc aaaagggtgc tgtagcatct ttgacctctg ttgctaagtt 5940
gccattcgct tacgctaagg acggtatctc ttacaccttc tctatcgttc caaacgcttt 6000
gggtaaggac gatgaagtta gaaagaccaa cttggctggt ttgatggacg gttacttcca 6060
ccacgaagca tctatcgaag gtggtcaaca cttgaacgta aatgttatga acagagaaat 6120
gttgttggac gctatggaaa acccagaaaa gtacccacaa ttgaccatca gagtttctgg 6180
ttacgctgtt agattcaact ctttgaccaa ggaacaacaa caagacgtta tcaccagaac 6240
cttcacccaa tctatgtaag tcgagacaaa tcgctcttaa atatatacct aaagaacatt 6300
aaagctatat tataagcaaa gatacgtaaa ttttgcttat attattatac acatatcata 6360
tttctatatt tttaagattt ggttatataa tgtacgtaat gcaaaggaaa taaattttat 6420
acattattga acagcgtcca agtaactaca ttatgtgcac taatagttta gcgtcgtgaa 6480
gactttattg tgtcgcgaaa agtaaaaatt ttaaaaatta gagcaccttg aacttgcgaa 6540
aaaggttctc atcaactgtt taaaacgtac gtgtggaaga acgattacaa caggtgttgt 6600
cctctgagga cataaaatac acaccgagat tcatcaactc attgctggag ttagcatatc 6660
tacaattggg tgaaatgggg agcgatttgc aggcatttgc tcggcatgcc ggtagaggtg 6720
tggtcaataa gagcgacctc atgctatacc tgagaaagca acctgaccta caggaaagag 6780
ttactcaaga ataagaattt tcgttttaaa acctaagagt cactttaaaa tttgtataca 6840
cttatttttt ttataactta tttaataata aaaatcataa atcataagaa attcgcgcgc 6900
ttagaacata accttgtgac cgtattgttc caagataccc ttaactcttt ccatggtttc 6960
cttctttggt ggcttaacac cgtccaactt gtattcttca cccatagcaa cccacttgtg 7020
cttacccaat tcgtggtatg gcaacaattc gatcttttca acgttaccca tgtctctggt 7080
gaactcaccc aatctgtgag cagagtcgtc gtcgtcagac caacctggaa caacaacgta 7140
tctgatccaa accttaacgt tcttgttagc caagtactta gcaaattcca aggttctgtg 7200
gttagaaaca ccaaccaagt tttggtggat ttcgtcgttc atttgcttca agtccaacat 7260

aaccaagtcg gtaacttcca acaattcgtc gataactggg tcgtatcttc taacgaaacc 7320
gttggtgtcc aaacaggtgt ggataccttc cttcttacaa gctctgaacc agtctctaac 7380
aaattcagct tgcaagatag cttcaccacc agaagcggta acaccacctc ctgaagcgtt 7440
cataaagtgt ctgtaggtaa caacttcctt catcaagtct tcaacagtaa cttccttacc 7500
accgtgggtg tcccaggtgt ctctgttgtg acagtacaaa catctcatca aacaaccttg 7560
gaagaaggtg atgaatctga tacctggacc gtcaacagta ccacaagatt cgaaagagtg 7620
gattctaccg ataacagaca ttttaagctt ctgcagctta gattagattg ctatgctttc 7680
tttctaatga gcaagaagta aaaaaagttg taatagaaca agaaaaatga aactgaaact 7740
tgagaaattg aagaccgttt attaacttaa atatcaatgg gaggtcatcg aaagagaaaa 7800
aaatcaaaaa aaaaaatttt caagaaaaag aaacgtgata aaaattttta ttgccttttt 7860
cgacgaagaa aaagaaacga ggcggtctct tttttctttt ccaaaccttt agtacgggta 7920
attaacgaca ccctagagga agaaagaggg gaaatttagt atgctgtgct tgggtgtttt 7980
gaagtggtac ggcgatgcgc ggagtccgag aaaatctgga agagtaaaaa aggagtagaa 8040
acattttgaa gctatggtgt gtgggaccgg tcgagggggg gcccggtacc cagcttttgt 8100
tccctttagt gagggttaat tccgagcttg gcgtaatcat ggtcatagct gtttcctgtg 8160
tgaaattgtt atccgctcac aattccacac aacataggag ccggaagcat aaagtgtaaa 8220
gcctggggtg cctaatgagt gaggtaactc acattaattg cgttgcgctc actgcccgct 8280
ttccagtcgg gaaacctgtc gtgccagctg cattaatgaa tcggccaacg cgcggggaga 8340
ggcggtttgc gtattgggcg ctcttccgct tcctcgctca ctgactcgct gcgctcggtc 8400
gttcggctgc ggcgagcggt atcagctcac tcaaaggcgg taatacggtt atccacagaa 8460
tcaggggata acgcaggaaa gaacatgtga gcaaaaggcc agcaaaaggc caggaaccgt 8520
aaaaaggccg cgttgctggc gtttttccat aggctcggcc cccctgacga gcatcacaaa 8580
aatcgacgct caagtcagag gtggcgaaac ccgacaggac tataaagata ccaggcgttc 8640
ccccctggaa gctccctcgt gcgctctcct gttccgaccc tgccgcttac cggatacctg 8700
tccgcctttc tcccttcggg aagcgtggcg ctttctcaat gctcacgctg taggtatctc 8760
agttcggtgt aggtcgttcg ctccaagctg ggctgtgtgc acgaaccccc cgttcagccc 8820
gaccgctgcg ccttatccgg taactatcgt cttgagtcca acccggtaag acacgactta 8880
tcgccactgg cagcagccac tggtaacagg attagcagag cgaggtatgt aggcggtgct 8940
acagagttct tgaagtggtg gcctaactac ggctacacta gaaggacagt atttggtatc 9000
tgcgctctgc tgaagccagt taccttcgga aaaagagttg gtagctcttg atccggcaaa 9060
caaaccaccg ctggtagcgg tggttttttt gtttgcaagc agcagattac gcgcagaaaa 9120

aaaggatctc aagaagatcc tttgatcttt tctacggggt ctgacgctca gtggaacgaa 9180
aactcacgtt aagggatttt ggtcatgaga ttatcaaaaa ggatcttcac ctagatcctt 9240
ttaaattaaa aatgaagttt taaatcaatc taaagtatat atgagtaaac ttggtctgac 9300
agttaccaat gcttaatcag tgaggcacct atctcagcga tctgtctatt tcgttcatcc 9360
atagttgcct gactgccccgt cgtgtagata actacgatac gggagggctt accatctggc 9420
cccagtgctg caatgatacc gcgagaccca cgctcaccgg ctccagattt atcagcaata 9480
aaccagccag ccggaagggc cgagcgcaga agtggtcctg caactttatc cgcctccatc 9540
cagtctatta attgttgccg ggaagctaga gtaagtagtt cgccagttaa tagtttgcgc 9600
aacgttgttg ccattgctac aggcatcgtg gtgtcacgct cgtcgtttgg tatggcttca 9660
ttcagctccg gttcccaacg atcaaggcga gttacatgat cccccatgtt gtgaaaaaaa 9720
gcggttagct ccttcggtcc tccgatcgtt gtcagaagta agttggccgc agtgttatca 9780
ctcatggtta tggcagcact gcataattct cttactgtca tgccatccgt aagatgcttt 9840
tctgtgactg gtgagtactc aaccaagtca ttctgagaat agtgtatgcg gcgaccgagt 9900
tgctcttgcc cggcgtcaat acgggataat accgcgccac atagcagaac tttaaaagtg 9960
ctcatcattg gaaaacgttc ttcggggcga aaactctcaa ggatcttacc gctgttgaga 10020
tccagttcga tgtaacccac tcgtgcaccc aactgatctt cagcatcttt tactttcacc 10080
agcgtttctg ggtgagcaaa aacaggaagg caaaatgccg caaaaaaggg aataagggcg 10140
acacggaaat gttgaatact catactcttc ctttttcaat attattgaag catttatcag 10200
ggttattgtc tcatgagcgg atacatattt gaatgtattt agaaaaataa acaaataggg 10260
gttccgcgca catttccccg aaaagtgcca cctgacgtct aagaaaccat tattatcatg 10320
acattaacct ataaaaatag gcgtatcacg aggccctttc gtctcgcgcg tttcggtgat 10380
gacggtgaaa acctctgaca catgcagctc ccggagacgg tcacagcttg tctgtaagcg 10440
gatgccggga gcagacaagc ccgtcagggc gcgtcagcgg gtgttggcgg gtgtcggggc 10500
tggcttaact atgcggcatc agagcagatt gtactgagag tgcaccatat cgactacgtc 10560
gtaaggccgt ttctgacaga gtaaaattct tgagggaact ttcaccatta tgggaaatgg 10620
ttcaagaagg tattgactta aactccatca aatggtcagg tcattgagtg ttttttattt 10680
gttgtatttt tttttttta gagaaaatcc tccaatatca aattaggaat cgtagtttca 10740
tgattttctg ttacacctaa ctttttgtgt ggtgccctcc tccttgtcaa tattaatgtt 10800
aaagtgcaat tctttttcct tatcacgttg agccattagt atcaatttgc ttacctgtat 10860
tcctttacta tcctcctttt tctccttctt gataaatgta tgtagattgc gtatatagtt 10920
tcgtctaccc tatgaacata ttccattttg taatttcgtg tcgtttctat tatgaatttc 10980
atttataaag tttatgtaca cctaggatcc gtcgacactg gatggcggcg ttagtatcga 11040

atcgacagca gtatagcgac cagcattcac atacgattga cgcatgatat tactttctgc 11100

gcacttaact tcgcatctgg gcagatgatg tcgaggcgaa aaaaaatata aatcacgcta 11160

aca 11163

<210> 32
<211> 7830
<212> DNA
<213> Artificial

<220>
<223> pRN619

<400> 32

aataccgcgc cacatagcag aactttaaaa gtgctcatca ttggaaaacg ttcttcgggg 60
cgaaaactct caaggatctt accgctgttg agatccagtt cgatgtaacc cactcgtgca 120
cccaactgat cttcagcatc ttttactttc accagcgttt ctgggtgagc aaaaacagga 180
aggcaaaatg ccgcaaaaaa gggaataagg gcgacacgga aatgttgaat actcatactc 240
ttcctttttc aatattattg aagcatttat cagggttatt gtctcatgag cggatacata 300
tttgaatgta tttagaaaaa taaacaaata ggggttccgc gcacatttcc ccgaaaagtg 360
ccacctgacg tctaagaaac cattattatc atgacattaa cctataaaaa taggcgtatc 420
acgaggccct ttcgtctcgc gcgtttcggt gatgacggtg aaaacctctg acacatgcag 480
ctcccggaga cggtcacagc ttgtctgtaa gcggatgccg ggagcagaca agcccgtcag 540
ggcgcgtcag cgggtgttgg cgggtgtcgg ggctggctta actatgcggc atcagagcag 600
attgtactga gagtgcacca tatcgactac gtcgtaaggc cgtttctgac agagtaaaat 660
tcttgaggga actttcacca ttatgggaaa tggttcaaga aggtattgac ttaaactcca 720
tcaaatggtc aggtcattga gtgtttttta tttgttgtat tttttttttt ttagagaaaa 780
tcctccaata tcaaattagg aatcgtagtt tcatgatttt ctgttacacc taactttttg 840
tgtggtgccc tcctccttgt caatattaat gttaaagtgc aattcttttt ccttatcacg 900
ttgagccatt agtatcaatt tgcttacctg tattccttta ctatcctcct ttttctcctt 960
cttgataaat gtatgtagat tgcgtatata gtttcgtcta ccctatgaac atattccatt 1020
ttgtaatttc gtgtcgtttc tattatgaat ttcatttata aagtttatgt acacctagga 1080
tccgtcgaca ctggatggcg gcgttagtat cgaatcgaca gcagtatagc gaccagcatt 1140
cacatacgat tgacgcatga tattactttc tgcgcactta acttcgcatc tgggcagatg 1200
atgtcgaggc gaaaaaaaat ataaatcacg ctaacatttg attaaaatag aacaactaca 1260
atataaaaaa actatacaaa tgacaagttc ttgaaaacaa gaatcttttt attgtcagtg 1320
tgtcagtcct gctcctcggc cacgaagtgc acgcagttgc cggccgggtc gcgcagggcg 1380

aactcccgcc cccacggctg ctcgccgatc tcggtcatgg ccggcccgga ggcgtcccgg 1440
aagttcgtgg acacgacctc cgaccactcg gcgtacagct cgtccaggcc gcgcacccac 1500
acccaggcca gggtgttgtc cggcaccacc tggtcctgga ccgcgctgat gaacagggtc 1560
acgtcgtccc ggaccacacc ggcgaagtcg tcctccacga agtcccggga gaacccgagc 1620
cggtcggtcc agaactcgac cgctccggcg acgtcgcgcg cggtgagcac cggaacggca 1680
ctggtcaact tggccatggt tgtttatgtt cggatgtgat gtgagaactg tatcctagca 1740
agattttaaa aggaagtata tgaaagaaga acctcagtgg caaatcctaa ccttttatat 1800
ttctctacag gggcgcggcg tggggacaat tcaacgcgtc tgtgagggga gcgtttccct 1860
gctcgcaggt ctgcagcgag gagccgtaat ttttgcttcg cgccgtgcgg ccatcaaaat 1920
gtatggatgc aaatgattat acatggggat gtatgggcta aatgtacggg cgacagtcac 1980
atcatgcccc tgagctgcgc acgtcaagac tgtcaaggag ggtattctgg gcctccatgt 2040
cgctggccgg gtgacccggc ggggacgagg ccttaagcgg ccgcatgcta gctccggatt 2100
atcgatgata agctgtcaaa catgagaatt aattccacgg actatagact atacctagta 2160
tactccgtct actgtacgat acacttccgc tcaggtcctt gtcctttaac gaggccttac 2220
cactcttttg ttactctatt gatccagctc agcaaaggca gtgtgatcta agattctatc 2280
ttcgcgatgt agtaaaacta gctagaccga gaaagagact agaaatgcaa aaggcacttc 2340
tacaatggct gccatcatta ttatccgatg tgacgctgca gcttctcaat gatattcgaa 2400
tacgctttga ggagatacag cctaatatcc gacaaactgt tttacagatt tacgatcgta 2460
cttgttaccc atcattgaat tttgaacatc cgaacctggg agttttccct gaaacagata 2520
gtatatttga acctgtataa taatatatag tctagcgctt tacggaagac aatgtatgta 2580
tttcggttcc tggagaaact attgcatcta ttgcataggt aatcttgcac gtcgcatccc 2640
cggttcattt tctgcgtttc catcttgcac ttcaatagca tatctttgtt aacgaagcat 2700
ctgtgcttca ttttgtagaa caaaaatgca acgcgagagc gctaattttt caaacaaaga 2760
atctgagctg catttttaca gaacagaaat gcaacgcgaa agcgctattt taccaacgaa 2820
gaatctgtgc ttcatttttg taaaacaaaa atgcaacgcg agagcgctaa tttttcaaac 2880
aaagaatctg agctgcattt ttacagaaca gaaatgcaac gcgagagcgc tattttacca 2940
acaaagaatc tatacttctt ttttgttcta caaaaatgca tcccgagagc gctatttttc 3000
taacaaagca tcttagatta cttttttttct cctttgtgcg ctctataatg cagtctcttg 3060
ataacttttt gcactgtagg tccgttaagg ttagaagaag gctactttgg tgtctatttt 3120
ctcttccata aaaaaagcct gactccactt cccgcgttta ctgattacta gcgaagctgc 3180
gggtgcattt tttcaagata aaggcatccc cgattatatt ctataccgat gtggattgcg 3240
catactttgt gaacagaaag tgatagcgtt gatgattctt cattggtcag aaaattatga 3300

acggtttctt ctattttgtc tctatatact acgtatagga aatgtttaca ttttcgtatt 3360

gttttcgatt cactctatga atagttctta ctacaatttt tttgtctaaa gagtaatact 3420

agagataaac ataaaaaatg tagaggtcga gtttagatgc aagttcaagg agcgaaaggt 3480

ggatgggtag gttatatagg gatatagcac agagatatat agcaaagaga tacttttgag 3540

caatgtttgt ggaaagcggt attcgcaatg ccggcaaaag ggcgaattga ttttgaagag 3600

aatgtggatt ttgatgtaat tgttgggatt ccatttttaa taaggcaata atattaggta 3660

tgtggatata ctagaagttc tcctcgaccg tcgatatgcg gtgtgaaata ccgcacagat 3720

gcgtaaggag aaaataccgc atcaggaaat tgtaaacgtt aatattttgt taaaattcgc 3780

gttaaatttt tgttaaatca gctcattttt taaccaatag gccgaaatcg gcaaaatccc 3840

ttataaatca aaagaataga ccgagatagg gttgagtgtt gttccagttt ggaacaagag 3900

tccactatta aagaacgtgg actccaacgt caaagggcga aaaaccgtct atcagggcga 3960

tggcccacta cgtgaaccat caccctaatc aagttttttg gggtcgaggt gccgtaaagc 4020

actaaatcgg aaccctaaag ggagcccccg atttagagct tgacggggaa agccggcgaa 4080

cgtggcgaga aaggaaggga agaaagcgaa aggagcgggc gctagggcgc tggcaagtgt 4140

agcggtcacg ctgcgcgtaa ccaccacacc cgccgcgctt aatgcgccgc tacagggcgc 4200

gtcgcgccat tcgccattca ggctgcgcaa ctgttgggaa gggcgatcgg tgcgggcctc 4260

ttcgctatta cgccagctgg cgaagggggg atgtgctgca aggcgattaa gttgggtaac 4320

gccagggttt tcccagtcac gacgttgtaa aacgacggcc agtgaattgt aatacgactc 4380

actatagggc gaattggagc tccaccgcgg tggcggccgc tctagaacta gtggatcccc 4440

cgggctgcag gaattcgata tcaagcttat cgataccgtc gacctcgacc ggtcccacac 4500

accatagctt caaaatgttt ctactccttt tttactcttc cagattttct cggactccgc 4560

gcatcgccgt accacttcaa aacacccaag cacagcatac taaatttccc ctctttcttc 4620

ctctagggtg tcgttaatta cccgtactaa aggtttggaa aagaaaaaag agaccgcctc 4680

gtttcttttt cttcgtcgaa aaaggcaata aaaattttta tcacgtttct ttttcttgaa 4740

aatttttttt tttgattttt ttctctttcg atgacctccc attgatattt aagttaataa 4800

acggtcttca atttctcaag tttcagtttc atttttcttg ttctattaca acttttttta 4860

cttcttgctc attagaaaga aagcatagca atctaatcta agctgcagaa gcttaaaatg 4920

tctgttatcg gtagaatcca ctctttcgaa tcttgtggta ctgttgacgg tccaggtatc 4980

agattcatca ccttcttcca aggttgtttg atgagatgtt tgtactgtca caacagagac 5040

acctgggaca cccacggtgg taaggaagtt actgttgaag acttgatgaa ggaagttgtt 5100

acctacagac actttatgaa cgcttcagga ggtggtgtta ccgcttctgg tggtgaagct 5160

atcttgcaag ctgaatttgt tagagactgg ttcagagctt gtaagaagga aggtatccac 5220

acctgtttgg acaccaacgg tttcgttaga agatacgacc cagttatcga cgaattgttg 5280

gaagttaccg acttggttat gttggacttg aagcaaatga acgacgaaat ccaccaaaac 5340

ttggttggtg tttctaacca cagaaccttg gaatttgcta agtacttggc taacaagaac 5400

gttaaggttt ggatcagata cgttgttgtt ccaggttggt ctgacgacga cgactctgct 5460

cacagattgg gtgagttcac cagagacatg ggtaacgttg aaaagatcga attgttgcca 5520

taccacgaat tgggtaagca caagtgggtt gctatgggtg aagaatacaa gttggacggt 5580

gttaagccac caaagaagga aaccatggaa agagttaagg gtatcttgga acaatacggt 5640

cacaaggtta tgttctaagc gcgcgaattt cttatgattt atgattttta ttattaaata 5700

agttataaaa aaaataagtg tatacaaatt ttaaagtgac tcttaggttt taaaacgaaa 5760

attcttattc ttgagtaact ctttcctgta ggtcaggttg ctttctcagg tatagcatga 5820

ggtcgctctt attgaccaca cctctaccgg catgccgagc aaatgcctgc aaatcgctcc 5880

ccatttcacc caattgtaga tatgctaact ccagcaatga gttgatgaat ctcggtgtgt 5940

attttatgtc ctcagaggac aacacctgtt gtaatcgttc ttccacacgt acccagcttt 6000

tgttcccttt agtgagggtt aattccgagc ttggcgtaat catggtcata gctgtttcct 6060

gtgtgaaatt gttatccgct cacaattcca cacaacatag gagccggaag cataaagtgt 6120

aaagcctggg gtgcctaatg agtgaggtaa ctcacattaa ttgcgttgcg ctcactgccc 6180

gctttccagt cgggaaacct gtcgtgccag ctgcattaat gaatcggcca acgcgcgggg 6240

agaggcggtt tgcgtattgg gcgctcttcc gcttcctcgc tcactgactc gctgcgctcg 6300

gtcgttcggc tgcggcgagc ggtatcagct cactcaaagg cggtaatacg gttatccaca 6360

gaatcagggg ataacgcagg aaagaacatg tgagcaaaag gccagcaaaa ggccaggaac 6420

cgtaaaaagg ccgcgttgct ggcgtttttc cataggctcg gcccccctga cgagcatcac 6480

aaaaatcgac gctcaagtca gaggtggcga aacccgacag gactataaag ataccaggcg 6540

tttccccctg gaagctccct cgtgcgctct cctgttccga ccctgccgct taccggatac 6600

ctgtccgcct ttctcccttc gggaagcgtg gcgctttctc aatgctcacg ctgtaggtat 6660

ctcagttcgg tgtaggtcgt tcgctccaag ctgggctgtg tgcacgaacc ccccgttcag 6720

cccgaccgct gcgccttatc cggtaactat cgtcttgagt ccaacccggt aagacacgac 6780

ttatcgccac tggcagcagc cactggtaac aggattagca gagcgaggta tgtaggcggt 6840

gctacagagt tcttgaagtg gtggcctaac tacggctaca ctagaaggac agtatttggt 6900

atctgcgctc tgctgaagcc agttaccttc ggaaaaagag ttggtagctc ttgatccggc 6960

aaacaaacca ccgctggtag cggtggtttt tttgtttgca agcagcagat tacgcgcaga 7020

aaaaaaggat ctcaagaaga tcctttgatc ttttctacgg ggtctgacgc tcagtggaac 7080

gaaaactcac gttaagggat tttggtcatg agattatcaa aaaggatctt cacctagatc 7140

cttttaaatt aaaaatgaag ttttaaatca atctaaagta tatatgagta aacttggtct 7200

gacagttacc aatgcttaat cagtgaggca cctatctcag cgatctgtct atttcgttca 7260

tccatagttg cctgactgcc cgtcgtgtag ataactacga tacgggaggg cttaccatct 7320

ggccccagtg ctgcaatgat accgcgagac ccacgctcac cggctccaga tttatcagca 7380

ataaaccagc cagccggaag ggccgagcgc agaagtggtc ctgcaacttt atccgcctcc 7440

atccagtcta ttaattgttg ccgggaagct agagtaagta gttcgccagt taatagtttg 7500

cgcaacgttg ttgccattgc tacaggcatc gtggtgtcac gctcgtcgtt tggtatggct 7560

tcattcagct ccggttccca acgatcaagg cgagttacat gatcccccat gttgtgaaaa 7620

aaagcggtta gctccttcgg tcctccgatc gttgtcagaa gtaagttggc cgcagtgtta 7680

tcactcatgg ttatggcagc actgcataat tctcttactg tcatgccatc cgtaagatgc 7740

ttttctgtga ctggtgagta ctcaaccaag tcattctgag aatagtgtat gcggcgaccg 7800

agttgctctt gcccggcgtc aatacgggat 7830

<210> 33
<211> 9695
<212> DNA
<213> Artificial

<220>
<223> pRN620

<400> 33

```
tcgcgcgttt cggtgatgac ggtgaaaacc tctgacacat gcagctcccg gagacggtca     60
cagcttgtct gtaagcggat gccgggagca gacaagcccg tcagggcgcg tcagcgggtg    120
ttggcgggtg tcggggctgg cttaactatg cggcatcaga gcagattgta ctgagagtgc    180
accatatcga ctacgtcgta aggccgtttc tgacagagta aaattcttga gggaactttc    240
accattatgg gaaatggttc aagaaggtat tgacttaaac tccatcaaat ggtcaggtca    300
ttgagtgttt tttatttgtt gtattttttt tttttagag aaaatcctcc aatatcaaat     360
taggaatcgt agtttcatga ttttctgtta cacctaactt tttgtgtggt gccctcctcc    420
ttgtcaatat taatgttaaa gtgcaattct ttttccttat cacgttgagc cattagtatc    480
aatttgctta cctgtattcc tttactatcc tcctttttct ccttcttgat aaatgtatgt    540
agattgcgta tatagtttcg tctaccctat gaacatattc cattttgtaa tttcgtgtcg    600
tttctattat gaatttcatt tataaagttt atgtacacct aggatccgtc gacactggat    660
ggcggcgtta gtatcgaatc gacagcagta tagcgaccag cattcacata cgattgacgc    720
atgatattac tttctgcgca cttaacttcg catctgggca gatgatgtcg aggcgaaaaa    780
```

aaatataaat cacgctaaca tttgattaaa atagaacaac tacaatataa aaaaactata 840
caaatgacaa gttcttgaaa acaagaatct ttttattgtc agtgtgtcag tcctgctcct 900
cggccacgaa gtgcacgcag ttgccggccg ggtcgcgcag ggcgaactcc cgcccccacg 960
gctgctcgcc gatctcggtc atggccggcc cggaggcgtc ccggaagttc gtggacacga 1020
cctccgacca ctcggcgtac agctcgtcca ggccgcgcac ccacacccag gccagggtgt 1080
tgtccggcac cacctggtcc tggaccgcgc tgatgaacag ggtcacgtcg tcccggacca 1140
caccggcgaa gtcgtcctcc acgaagtccc gggagaaccc gagccggtcg gtccagaact 1200
cgaccgctcc ggcgacgtcg cgcgcggtga gcaccggaac ggcactggtc aacttggcca 1260
tggttgttta tgttcggatg tgatgtgaga actgtatcct agcaagattt taaaaggaag 1320
tatatgaaag aagaacctca gtggcaaatc ctaacctttt atatttctct acaggggcgc 1380
ggcgtgggga caattcaacg cgtctgtgag gggagcgttt ccctgctcgc aggtctgcag 1440
cgaggagccg taatttttgc ttcgcgccgt gcggccatca aaatgtatgg atgcaaatga 1500
ttatacatgg ggatgtatgg gctaaatgta cgggcgacag tcacatcatg cccctgagct 1560
gcgcacgtca agactgtcaa ggagggtatt ctgggcctcc atgtcgctgg ccgggtgacc 1620
cggcggggac gaggccttaa gcggccgcat gctagctccg gattatcgat gataagctgt 1680
caaacatgag aattaattcc acggactata gactatacct agtatactcc gtctactgta 1740
cgatacactt ccgctcaggt ccttgtcctt taacgaggcc ttaccactct tttgttactc 1800
tattgatcca gctcagcaaa ggcagtgtga tctaagattc tatcttcgcg atgtagtaaa 1860
actagctaga ccgagaaaga gactagaaat gcaaaaggca cttctacaat ggctgccatc 1920
attattatcc gatgtgacgc tgcagcttct caatgatatt cgaatacgct ttgaggagat 1980
acagcctaat atccgacaaa ctgttttaca gatttacgat cgtacttgtt acccatcatt 2040
gaattttgaa catccgaacc tgggagtttt ccctgaaaca gatagtatat ttgaacctgt 2100
ataataatat atagtctagc gctttacgga agacaatgta tgtatttcgg ttcctggaga 2160
aactattgca tctattgcat aggtaatctt gcacgtcgca tccccggttc attttctgcg 2220
tttccatctt gcacttcaat agcatatctt tgttaacgaa gcatctgtgc ttcattttgt 2280
agaacaaaaa tgcaacgcga gagcgctaat ttttcaaaca aagaatctga gctgcatttt 2340
tacagaacag aaatgcaacg cgaaagcgct attttaccaa cgaagaatct gtgcttcatt 2400
tttgtaaaac aaaaatgcaa cgcgagagcg ctaatttttc aaacaaagaa tctgagctgc 2460
atttttacag aacagaaatg caacgcgaga gcgctatttt accaacaaag aatctatact 2520
tcttttttgt tctacaaaaa tgcatcccga gagcgctatt tttctaacaa agcatcttag 2580
attacttttt ttctcctttg tgcgctctat aatgcagtct cttgataact ttttgcactg 2640
taggtccgtt aaggttagaa gaaggctact tggtgtctat ttttctcttc cataaaaaaa 2700

gcctgactcc acttcccgcg tttactgatt actagcgaag ctgcgggtgc atttttcaa 2760

gataaaggca tccccgatta tattctatac cgatgtggat tgcgcatact ttgtgaacag 2820

aaagtgatag cgttgatgat tcttcattgg tcagaaaatt atgaacggtt tcttctattt 2880

tgtctctata tactacgtat aggaaatgtt tacattttcg tattgttttc gattcactct 2940

atgaatagtt cttactacaa ttttttgtc taaagagtaa tactagagat aaacataaaa 3000

aatgtagagg tcgagtttag atgcaagttc aaggagcgaa aggtggatgg gtaggttata 3060

tagggatata gcacagagat atatagcaaa gagatacttt tgagcaatgt ttgtggaaag 3120

cggtattcgc aatgccggca aaagggcgaa ttgattttga agagaatgtg gattttgatg 3180

taattgttgg gattccattt ttaataaggc aataatatta ggtatgtgga tatactagaa 3240

gttctcctcg accgtcgata tgcggtgtga aataccgcac agatgcgtaa ggagaaaata 3300

ccgcatcagg aaattgtaaa cgttaatatt ttgttaaaat tcgcgttaaa tttttgttaa 3360

atcagctcat tttttaacca ataggccgaa atcggcaaaa tcccttataa atcaaaagaa 3420

tagaccgaga tagggttgag tgttgttcca gtttggaaca agagtccact attaaagaac 3480

gtggactcca acgtcaaagg gcgaaaaacc gtctatcagg gcgatggccc actacgtgaa 3540

ccatcaccct aatcaagttt tttggggtcg aggtgccgta aagcactaaa tcggaaccct 3600

aaagggagcc cccgatttag agcttgacgg ggaaagccgg cgaacgtggc gagaaaggaa 3660

gggaagaaag cgaaaggagc gggcgctagg gcgctggcaa gtgtagcggt cacgctgcgc 3720

gtaaccacca cacccgccgc gcttaatgcg ccgctacagg gcgcgtcgcg ccattcgcca 3780

ttcaggctgc gcaactgttg ggaagggcga tcggtgcggg cctcttcgct attacgccag 3840

ctggcgaagg ggggatgtgc tgcaaggcga ttaagttggg taacgccagg gttttcccag 3900

tcacgacgtt gtaaaacgac ggccagtgaa ttgtaatacg actcactata gggcgaattg 3960

gagctccacc gcggtggcgg ccgctctaga actagtggat cccccgggct gcaggaattc 4020

gatatcaagc ttatcgatac cgtcgacctc gagctcttca actcaagacg cacagatatt 4080

ataacatctg cataataggc atttgcaaga attactcgtg agtaaggaaa gagtgaggaa 4140

ctatcgcata cctgcattta aagatgccga tttgggcgcg aatcctttat tttggcttca 4200

ccctcatact attatcaggg ccagaaaaag gaagtgtttc cctccttctt gaattgatgt 4260

taccctcata aagcacgtgg cctcttatcg agaaagaaat taccgtcgct cgtgatttgt 4320

ttgcaaaaag aacaaaactg aaaaaaccca gacacgctcg acttcctgtc ttcctattga 4380

ttgcagcttc caatttcgtc acacaacaag gtcctagcga cggctcacag gttttgtaac 4440

aagcaatcga aggttctgga atggcgggaa agggtttagt accacatgct atgatgccca 4500

ctgtgatctc cagagcaaag ttcgttcgat cgtactgtta ctctctctct ttcaaacaga 4560

attgtccgaa tcgtgtgaca acaacagcct gttctcacac actcttttct tctaaccaag 4620
ggggtggttt agtttagtag aacctcgtga aacttacatt tacatatata taaacttgca 4680
taaattggtc aatgcaagaa atacatattt ggtcttttct aattcgtagt ttttcaagtt 4740
cttagatgct ttctttttct cttttttaca gatcatcaag gaagtaatta tctacttttt 4800
acaacaaata tatctagact gcagaaaatg tctgaattga acgagaagtt ggctaccgct 4860
tgggaaggtt tcaccaaggg tgactggcaa aacgaagtta acgttagaga cttcatccaa 4920
aagaactaca ccccatacga aggtgacgaa tctttcttgg ctggtgctac cgaagctacc 4980
accaccttgt gggacaaggt tatggaaggt gttaagttgg aaaacagaac ccacgctcca 5040
gttgacttcg acaccgctgt tgcttctacc atcacctctc acgacgctgg ttacatcaac 5100
aagcaattgg aaaagatcgt tggtttacaa accgaagctc cattgaagag agctttgatc 5160
ccattcggtg gtatcaagat gatcgaaggt tcttgtaagg cttacaacag agaattggac 5220
ccaatgatca agaagatttt caccgaatac agaaagaccc acaaccaagg tgttttcgac 5280
gtttacactc cagacatctt gagatgtaga aagtctggtg ttttgactgg tttgccagac 5340
gcttacggta gaggtagaat catcggtgac tacagaagag ttgctttgta cggtatcgac 5400
tacttgatga aggacaagtt ggctcaattc acctctttgc aagctgactt ggaaaacggt 5460
gttaacttgg aacaaaccat cagattgaga gaagaaatcg ctgaacaaca cagagctttg 5520
ggtcaaatga aggaaatggc tgctaagtac ggttacgaca tctctggtcc agctaccaac 5580
gctcaagaag ctatccaatg gacctacttc ggttacttgg ctgctgttaa gtctcaaaac 5640
ggtgctgcta tgtctttcgg taggacctct accttcttgg acgtttacat cgaaagagac 5700
ttgaaggctg gtaagatcac cgaacaagaa gctcaagaaa tggttgacca cttggttatg 5760
aagttgagaa tggttagatt cttgagaacc ccagaatacg acgaattgtt ctctggtgac 5820
ccaatctggg ctaccgaatc tatcggtggt atgggtttgg acggtagaac cttggttacc 5880
aagaactctt tcagattctt gaacacctta tacaccatgg gtccatctcc agaaccaaac 5940
atgaccatct tgtggtctga aaagttacca ttgaacttca agaagttcgc tgctaaggtt 6000
tctatcgaca cctcttcttt gcaatacgaa aacgacgact tgatgagacc agacttcaac 6060
aacgacgact acgctatcgc ttgttgtgtt tctccaatga tcgttggtaa gcaaatgcaa 6120
ttcttcggtg ctagagctaa cttggctaag accatgttgt acgctatcaa cggtggtgtt 6180
gacgaaaagt tgaagatgca agttggtcca aagtctgaac caatcaaggg tgacgttttg 6240
aactacgacg aagttatgga aagaatggac cacttcatgg actggttggc taagcaatac 6300
atcaccgctt tgaacatcat ccactacatg cacgacaagt actcttacga agcatcattg 6360
atggctttgc acgacagaga cgtaatcaga accatggctt gtggtatcgc tggtttgtct 6420
gttgctgctg actctttgtc tgctatcaag tacgctaagg ttaagccaat cagagacgaa 6480

gacggtttgg ctatcgactt cgaaatcgaa ggtgaatacc ctcaattcgg taacaacgac 6540
ccaagagttg acgacttggc tgttgacttg gttgaaagat ttatgaagaa gatccaaaag 6600
ttgcacacct acagagacgc tatcccaacc caatctgttt tgactatcac atctaacgtt 6660
gtttacggta agaagactgg taacacccca gacggtagaa gagctggtgc tccattcggt 6720
ccaggtgcta acccaatgca cggtagagac caaaagggtg ctgtagcatc tttgacctct 6780
gttgctaagt tgccattcgc ttacgctaag gacggtatct cttacacctt ctctatcgtt 6840
ccaaacgctt tgggtaagga cgatgaagtt agaaagacca acttggctgg tttgatggac 6900
ggttacttcc accacgaagc atctatcgaa ggtggtcaac acttgaacgt aaatgttatg 6960
aacagagaaa tgttgttgga cgctatggaa aacccagaaa agtacccaca attgaccatc 7020
agagtttctg gttacgctgt tagattcaac tctttgacca aggaacaaca acaagacgtt 7080
atcaccagaa ccttcaccca atctatgtaa gtcgagacaa atcgctctta aatatatacc 7140
taaagaacat taaagctata ttataagcaa agatacgtaa attttgctta tattattata 7200
cacatatcat atttctatat ttttaagatt tggttatata atgtacgtaa tgcaaaggaa 7260
ataaatttta tacattattg aacagcgtcc aagtaactac attatgtgca ctaatagttt 7320
agcgtcgtga agactttatt gtgtcgcgaa aagtaaaaat tttaaaaatt agagcacctt 7380
gaacttgcga aaaaggttct catcaactgt ttaaaacgta cccagctttt gttcccttta 7440
gtgagggtta attccgagct tggcgtaatc atggtcatag ctgtttcctg tgtgaaattg 7500
ttatccgctc acaattccac acaacatagg agccggaagc ataaagtgta aagcctgggg 7560
tgcctaatga gtgaggtaac tcacattaat tgcgttgcgc tcactgcccg ctttccagtc 7620
gggaaacctg tcgtgccagc tgcattaatg aatcggccaa cgcgcgggga gaggcggttt 7680
gcgtattggg cgctcttccg cttcctcgct cactgactcg ctgcgctcgg tcgttcggct 7740
gcggcgagcg gtatcagctc actcaaaggc ggtaatacgg ttatccacag aatcagggga 7800
taacgcagga aagaacatgt gagcaaaagg ccagcaaaag gccaggaacc gtaaaaaggc 7860
cgcgttgctg gcgtttttcc ataggctcgg cccccctgac gagcatcaca aaaatcgacg 7920
ctcaagtcag aggtggcgaa acccgacagg actataaaga taccaggcgt ttccccctgg 7980
aagctccctc gtgcgctctc ctgttccgac cctgccgctt accggatacc tgtccgcctt 8040
tctcccttcg ggaagcgtgg cgctttctca atgctcacgc tgtaggtatc tcagttcggt 8100
gtaggtcgtt cgctccaagc tgggctgtgt gcacgaaccc cccgttcagc ccgaccgctg 8160
cgccttatcc ggtaactatc gtcttgagtc caacccggta agacacgact tatcgccact 8220
ggcagcagcc actggtaaca ggattagcag agcgaggtat gtaggcggtg ctacagagtt 8280
cttgaagtgg tggcctaact acggctacac tagaaggaca gtatttggta tctgcgctct 8340

gctgaagcca gttaccttcg gaaaaagagt tggtagctct tgatccggca aacaaaccac 8400
cgctggtagc ggtggttttt ttgtttgcaa gcagcagatt acgcgcagaa aaaaaggatc 8460
tcaagaagat cctttgatct tttctacggg gtctgacgct cagtggaacg aaaactcacg 8520
ttaagggatt ttggtcatga gattatcaaa aaggatcttc acctagatcc ttttaaatta 8580
aaaatgaagt tttaaatcaa tctaaagtat atatgagtaa acttggtctg acagttacca 8640
atgcttaatc agtgaggcac ctatctcagc gatctgtcta tttcgttcat ccatagttgc 8700
ctgactgccc gtcgtgtaga taactacgat acgggagggc ttaccatctg gccccagtgc 8760
tgcaatgata ccgcgagacc cacgctcacc ggctccagat ttatcagcaa taaaccagcc 8820
agccggaagg gccgagcgca gaagtggtcc tgcaacttta tccgcctcca tccagtctat 8880
taattgttgc cgggaagcta gagtaagtag ttcgccagtt aatagtttgc gcaacgttgt 8940
tgccattgct acaggcatcg tggtgtcacg ctcgtcgttt ggtatggctt cattcagctc 9000
cggttcccaa cgatcaaggc gagttacatg atcccccatg ttgtgaaaaa aagcggttag 9060
ctccttcggt cctccgatcg ttgtcagaag taagttggcc gcagtgttat cactcatggt 9120
tatggcagca ctgcataatt ctcttactgt catgccatcc gtaagatgct tttctgtgac 9180
tggtgagtac tcaaccaagt cattctgaga atagtgtatg cggcgaccga gttgctcttg 9240
cccggcgtca atacgggata ataccgcgcc acatagcaga actttaaaag tgctcatcat 9300
tggaaaacgt tcttcggggc gaaaactctc aaggatctta ccgctgttga gatccagttc 9360
gatgtaaccc actcgtgcac ccaactgatc ttcagcatct tttactttca ccagcgtttc 9420
tgggtgagca aaaacaggaa ggcaaaatgc cgcaaaaaag ggaataaggg cgacacggaa 9480
atgttgaata ctcatactct tcctttttca atattattga agcatttatc agggttattg 9540
tctcatgagc ggatacatat ttgaatgtat ttagaaaaat aaacaaatag gggttccgcg 9600
cacatttccc cgaaaagtgc cacctgacgt ctaagaaacc attattatca tgacattaac 9660
ctataaaaat aggcgtatca cgaggccctt tcgtc 9695

<210> 34
<211> 14159
<212> DNA
<213> Artificial

<220>
<223> pRN618

<400> 34

```
tggtatacca acaattcttg aagaagaaga ttccattgaa actgcttcgc taggtgcgac     60
gacgactgat tctattgggt tatccgacac atcatcagaa gattcgcatt atggtaatgc    120
taagaaggta acatgaggat ccccttttcc tttgtcgata tcatgtaatt agttatgtca    180
cgcttacatt cacgccctcc tcccacatcc gctctaaccg aaaaggaagg agttagacaa    240
```

cctgaagtct aggtccctat ttattttttt taatagttat gttagtatta agaacgttat 300
ttatatttca aatttttctt ttttttctgt acaaacgcgt gtacgcatgt aacattatac 360
tgaaaacctt gcttgagaag gttttgggac gctcgaaggc ttcctaggct cgacactgga 420
tggcggcgtt agtatcgaat cgacagcagt atagcgacca gcattcacat acgattgacg 480
catgatatta ctttctgcgc acttaacttc gcatctgggc agatgatgtc gaggcgaaaa 540
aaaatataaa tcacgctaac atttgattaa aatagaacaa ctacaatata aaaaaactat 600
acaaatgaca agttcttgaa aacaagaatc tttttattgt cagtgtgtca gtcctgctcc 660
tcggccacga agtgcacgca gttgccggcc gggtcgcgca gggcgaactc ccgcccccac 720
ggctgctcgc cgatctcggt catggccggc ccggaggcgt cccggaagtt cgtggacacg 780
acctccgacc actcggcgta cagctcgtcc aggccgcgca cccacaccca ggccagggtg 840
ttgtccggca ccacctggtc ctggaccgcg ctgatgaaca gggtcacgtc gtcccggacc 900
acaccggcga agtcgtcctc cacgaagtcc cgggagaacc cgagccggtc ggtccagaac 960
tcgaccgctc cggcgacgtc gcgcgcggtg agcaccggaa cggcactggt caacttggcc 1020
atggttgttt atgttcggat gtgatgtgag aactgtatcc tagcaagatt ttaaaaggaa 1080
gtatatgaaa gaagaacctc agtggcaaat cctaaccttt tatatttctc tacaggggcg 1140
cggcgtgggg acaattcaac gcgtctgtga ggggagcgtt tccctgctcg caggtctgca 1200
gcgaggagcc gtaatttttg cttcgcgccg tgcggccatc aaaatgtatg gatgcaaatg 1260
attatacatg gggatgtatg ggctaaatgt acgggcgaca gtcacatcat gcccctgagc 1320
tgcgcacgtc aagactgtca aggagggtat tctgggcctc catgtcgctg gccgggtgac 1380
ccggcgggga cgaggcctta agcggccgca tgctagctcc ggattatcga tgataagctg 1440
tcaaacatga gaattaattc cacggactat agactatacc tagtatactc cgtctactgt 1500
acgatacact tccgctcagg tccttgtcct ttaacgaggc cttaccactc ttttgttact 1560
ctattgatcc agctcagcaa aggcagtgtg atctaagatt ctatcttcgc gatgtagtaa 1620
aactagctag accgagaaag agactagaaa tgcaaaaggc acttctacaa tggctgccat 1680
cattattatc cgatgtgacg ctgcagcttc tcaatgatat tcgaatacgc tttgaggaga 1740
tacagcctaa tatccgacaa actgttttac agatttacga tcgtacttgt tacccatcat 1800
tgaattttga acatccgaac ctgggagttt tccctgaaac agatagtata tttgaacctg 1860
tataataata tatagtctag cgctttacgg aagacaatgt atgtatttcg gttcctggag 1920
aaactattgc atctattgca taggtaatct tgcacgtcgc atccccggtt cattttctgc 1980
gtttccatct tgcacttcaa tagcatatct ttgttaacga agcatctgtg cttcattttg 2040
tagaacaaaa atgcaacgcg agagcgctaa tttttcaaac aaagaatctg agctgcattt 2100

ttacagaaca gaaatgcaac gcgaaagcgc tattttacca acgaagaatc tgtgcttcat 2160
ttttgtaaaa caaaaatgca acgcgagagc gctaattttt caaacaaaga atctgagctg 2220
catttttaca gaacagaaat gcaacgcgag agcgctattt taccaacaaa gaatctatac 2280
ttcttttttg ttctacaaaa atgcatcccg agagcgctat ttttctaaca aagcatctta 2340
gattactttt tttctccttt gtgcgctcta taatgcagtc tcttgataac tttttgcact 2400
gtaggtccgt taaggttaga agaaggctac tttggtgtct attttctctt ccataaaaaa 2460
agcctgactc cacttcccgc gtttactgat tactagcgaa gctgcgggtg catttttttca 2520
agataaaggc atccccgatt atattctata ccgatgtgga ttgcgcatac tttgtgaaca 2580
gaaagtgata gcgttgatga ttcttcattg gtcagaaaat tatgaacggt ttcttctatt 2640
ttgtctctat atactacgta taggaaatgt ttacattttc gtattgtttt cgattcactc 2700
tatgaatagt tcttactaca atttttttgt ctaaagagta atactagaga taaacataaa 2760
aaatgtagag gtcgagttta gatgcaagtt caaggagcga aaggtggatg ggtaggttat 2820
atagggatat agcacagaga tatatagcaa agagatactt ttgagcaatg tttgtggaaa 2880
gcggtattcg caatgccggc aaaagggcga attgattttg aagagaatgt ggattttgat 2940
gtaattgttg ggattccatt tttaataagg caataatatt aggtatgtgg atatactaga 3000
agttctcctc gaccgtcgat atgcggtgtg aaataccgca cagatgcgta aggagaaaat 3060
accgcatcag gaaattgtaa acgttaatat tttgttaaaa ttcgcgttaa atttttgtta 3120
aatcagctca ttttttaacc aataggccga aatcggcaaa atcccttata aatcaaaaga 3180
atagaccgag atagggttga gtgttgttcc agtttggaac aagagtccac tattaaagaa 3240
cgtggactcc aacgtcaaag ggcgaaaaac cgtctatcag ggcgatggcc cactacgtga 3300
accatcaccc taatcaagtt ttttggggtc gaggtgccgt aaagcactaa atcggaaccc 3360
taaagggagc ccccgattta gagcttgacg gggaaagccg gcgaacgtgg cgagaaagga 3420
agggaagaaa gcgaaaggag cgggcgctag ggcgctggca agtgtagcgg tcacgctgcg 3480
cgtaaccacc acacccgccg cgcttaatgc gccgctacag ggcgcgtcgc gccattcgcc 3540
attcaggctg cgcaactgtt gggaagggcg atcggtgcgg gcctcttcgc tattacgcca 3600
gctggcgaag gggggatgtg ctgcaaggcg attaagttgg gtaacgccag ggttttccca 3660
gtcacgacgt tgtaaaacga cggccagtga attgtaatac gactcactat agggcgaatt 3720
ggagctccac cgcggtggcg gccgctctag aactagtctc gagctcttca actcaagacg 3780
cacagatatt ataacatctg cataataggc atttgcaaga attactcgtg agtaaggaaa 3840
gagtgaggaa ctatcgcata cctgcattta aagatgccga tttgggcgcg aatcctttat 3900
tttggcttca ccctcatact attatcaggg ccagaaaaag gaagtgtttc cctccttctt 3960
gaattgatgt taccctcata aagcacgtgg cctcttatcg agaaagaaat taccgtcgct 4020

cgtgatttgt ttgcaaaaag aacaaaactg aaaaaaccca gacacgctcg acttcctgtc 4080
ttcctattga ttgcagcttc caatttcgtc acacaacaag gtcctagcga cggctcacag 4140
gttttgtaac aagcaatcga aggttctgga atggcgggaa agggtttagt accacatgct 4200
atgatgccca ctgtgatctc cagagcaaag ttcgttcgat cgtactgtta ctctctctct 4260
ttcaaacaga attgtccgaa tcgtgtgaca acaacagcct gttctcacac actcttttct 4320
tctaaccaag gggtggtttt agtttagtag aacctcgtga aacttacatt tacatatata 4380
taaacttgca taaattggtc aatgcaagaa atacatattt ggtcttttct aattcgtagt 4440
ttttcaagtt cttagatgct ttctttttct ctttttttaca gatcatcaag gaagtaatta 4500
tctacttttt acaacaaata tatctagact gcagaaaatg tctgaattga acgagaagtt 4560
ggctaccgct tgggaaggtt tcaccaaggg tgactggcaa aacgaagtta acgttagaga 4620
cttcatccaa aagaactaca ccccatacga aggtgacgaa tctttcttgg ctggtgctac 4680
cgaagctacc accaccttgt gggacaaggt tatggaaggt gttaagttgg aaaacagaac 4740
ccacgctcca gttgacttcg acaccgctgt tgcttctacc atcacctctc acgacgctgg 4800
ttacatcaac aagcaattgg aaaagatcgt tggtttacaa accgaagctc cattgaagag 4860
agctttgatc ccattcggtg gtatcaagat gatcgaaggt tcttgtaagg cttacaacag 4920
agaattggac ccaatgatca agaagatttt caccgaatac agaaagaccc acaaccaagg 4980
tgttttcgac gtttacactc cagacatctt gagatgtaga aagtctggtg ttttgactgg 5040
tttgccagac gcttacggta gaggtagaat catcggtgac tacagaagag ttgctttgta 5100
cggtatcgac tacttgatga aggacaagtt ggctcaattc acctctttgc aagctgactt 5160
ggaaaacggt gttaacttgg aacaaaccat cagattgaga gaagaaatcg ctgaacaaca 5220
cagagctttg ggtcaaatga aggaaatggc tgctaagtac ggttacgaca tctctggtcc 5280
agctaccaac gctcaagaag ctatccaatg gacctacttc ggttacttgg ctgctgttaa 5340
gtctcaaaac ggtgctgcta tgtctttcgg taggacctct accttcttgg acgtttacat 5400
cgaaagagac ttgaaggctg gtaagatcac cgaacaagaa gctcaagaaa tggttgacca 5460
cttggttatg aagttgagaa tggttagatt cttgagaacc ccagaatacg acgaattgtt 5520
ctctggtgac ccaatctggg ctaccgaatc tatcggtggt atgggtttgg acggtagaac 5580
cttggttacc aagaactctt tcagattctt gaacacctta tacaccatgg gtccatctcc 5640
agaaccaaac atgaccatct tgtggtctga aaagttacca ttgaacttca agaagttcgc 5700
tgctaaggtt tctatcgaca cctcttcttt gcaatacgaa aacgacgact tgatgagacc 5760
agacttcaac aacgacgact acgctatcgc ttgttgtgtt tctccaatga tcgttggtaa 5820
gcaaatgcaa ttcttcggtg ctagagctaa cttggctaag accatgttgt acgctatcaa 5880

cggtggtgtt gacgaaaagt tgaagatgca agttggtcca aagtctgaac caatcaaggg 5940
tgacgttttg aactacgacg aagttatgga aagaatggac cacttcatgg actggttggc 6000
taagcaatac atcaccgctt tgaacatcat ccactacatg cacgacaagt actcttacga 6060
agcatcattg atggctttgc acgacagaga cgtaatcaga accatggctt gtggtatcgc 6120
tggtttgtct gttgctgctg actctttgtc tgctatcaag tacgctaagg ttaagccaat 6180
cagagacgaa gacggtttgg ctatcgactt cgaaatcgaa ggtgaatacc ctcaattcgg 6240
taacaacgac ccaagagttg acgacttggc tgttgacttg gttgaaagat ttatgaagaa 6300
gatccaaaag ttgcacacct acagagacgc tatcccaacc caatctgttt tgactatcac 6360
atctaacgtt gtttacggta agaagactgg taacacccca gacggtagaa gagctggtgc 6420
tccattcggt ccaggtgcta acccaatgca cggtagagac caaaagggtg ctgtagcatc 6480
tttgacctct gttgctaagt tgccattcgc ttacgctaag gacggtatct cttacacctt 6540
ctctatcgtt ccaaacgctt tgggtaagga cgatgaagtt agaaagacca acttggctgg 6600
tttgatggac ggttacttcc accacgaagc atctatcgaa ggtggtcaac acttgaacgt 6660
aaatgttatg aacagagaaa tgttgttgga cgctatggaa aacccagaaa agtacccaca 6720
attgaccatc agagtttctg gttacgctgt tagattcaac tctttgacca aggaacaaca 6780
acaagacgtt atcaccagaa ccttcaccca atctatgtaa gtcgagacaa atcgctctta 6840
aatatatacc taaagaacat taaagctata ttataagcaa agatacgtaa attttgctta 6900
tattattata cacatatcat atttctatat ttttaagatt tggttatata atgtacgtaa 6960
tgcaaaggaa ataaatttta tacattattg aacagcgtcc aagtaactac attatgtgca 7020
ctaatagttt agcgtcgtga agactttatt gtgtcgcgaa aagtaaaaat tttaaaaatt 7080
agagcacctt gaacttgcga aaaaggttct catcaactgt ttaaaacgta cgtgtggaag 7140
aacgattaca acaggtgttg tcctctgagg acataaaata cacaccgaga ttcatcaact 7200
cattgctgga gttagcatat ctacaattgg gtgaaatggg gagcgatttg caggcatttg 7260
ctcggcatgc cggtagaggt gtggtcaata agagcgacct catgctatac ctgagaaagc 7320
aacctgacct acaggaaaga gttactcaag aataagaatt ttcgttttaa aacctaagag 7380
tcactttaaa atttgtatac acttattttt tttataactt atttaataat aaaaatcata 7440
aatcataaga aattcgcgcg cttagaacat aaccttgtga ccgtattgtt ccaagatacc 7500
cttaactctt tccatggttt ccttctttgg tggcttaaca ccgtccaact tgtattcttc 7560
acccatagca acccacttgt gcttacccaa ttcgtggtat ggcaacaatt cgatcttttc 7620
aacgttaccc atgtctctgg tgaactcacc caatctgtga gcagagtcgt cgtcgtcaga 7680
ccaacctgga acaacaacgt atctgatcca aaccttaacg ttcttgttag ccaagtactt 7740
agcaaattcc aaggttctgt ggttagaaac accaaccaag ttttggtgga tttcgtcgtt 7800

catttgcttc aagtccaaca taaccaagtc ggtaacttcc aacaattcgt cgataactgg 7860
gtcgtatctt ctaacgaaac cgttggtgtc caaacaggtg tggatacctt ccttcttaca 7920
agctctgaac cagtctctaa caaattcagc ttgcaagata gcttcaccac cagaagcggt 7980
aacaccacct cctgaagcgt tcataaagtg tctgtaggta acaacttcct tcatcaagtc 8040
ttcaacagta acttccttac caccgtgggt gtcccaggtg tctctgttgt gacagtacaa 8100
acatctcatc aaacaacctt ggaagaaggt gatgaatctg atacctggac cgtcaacagt 8160
accacaagat tcgaaagagt ggattctacc gataacagac attttaagct tctgcagctt 8220
agattagatt gctatgcttt ctttctaatg agcaagaagt aaaaaaagtt gtaatagaac 8280
aagaaaaatg aaactgaaac ttgagaaatt gaagaccgtt tattaactta aatatcaatg 8340
ggaggtcatc gaaagagaaa aaaatcaaaa aaaaaaattt tcaagaaaaa gaaacgtgat 8400
aaaaattttt attgcctttt tcgacgaaga aaaagaaacg aggcggtctc tttttttctt 8460
tccaaacctt tagtacgggt aattaacgac accctagagg aagaaagagg ggaaatttag 8520
tatgctgtgc ttgggtgttt tgaagtggta cggcgatgcg cggagtccga gaaaatctgg 8580
aagagtaaaa aaggagtaga aacattttga agctatggtg tgtgggaccg gtcgaggggg 8640
ggcccggtac ccagcttttg ttccctttag tgagggttaa ttccgagctt ggcgtaatca 8700
tggtcatagc tgtttcctgt gtgaaattgt tatccgctca caattccaca caacatagga 8760
gccggaagca taaagtgtaa agcctggggt gcctaatgag tgaggtaact cacattaatt 8820
gcgttgcgct cactgcccgc tttccagtcg ggaaacctgt cgtgccagct gcattaatga 8880
atcggccaac gcgcggggag aggcggtttg cgtattgggc gctcttccgc ttcctcgctc 8940
actgactcgc tgcgctcggt cgttcggctg cggcgagcgg tatcagctca ctcaaaggcg 9000
gtaatacggt tatccacaga atcaggggat aacgcaggaa agaacatgtg agcaaaaggc 9060
cagcaaaagg ccaggaaccg taaaaaggcc gcgttgctgg cgtttttcca taggctcggc 9120
cccccctgacg agcatcacaa aaatcgacgc tcaagtcaga ggtggcgaaa cccgacagga 9180
ctataaagat accaggcgtt tccccctgga agctccctcg tgcgctctcc tgttccgacc 9240
ctgccgctta ccggatacct gtccgccttt ctcccttcgg gaagcgtggc gctttctcaa 9300
tgctcacgct gtaggtatct cagttcggtg taggtcgttc gctccaagct gggctgtgtg 9360
cacgaacccc ccgttcagcc cgaccgctgc gccttatccg gtaactatcg tcttgagtcc 9420
aacccggtaa gacacgactt atcgccactg gcagcagcca ctggtaacag gattagcaga 9480
gcgaggtatg taggcggtgc tacagagttc ttgaagtggt ggcctaacta cggctacact 9540
agaaggacag tatttggtat ctgcgctctg ctgaagccag ttaccttcgg aaaaagagtt 9600
ggtagctctt gatccggcaa acaaaccacc gctggtagcg gtggtttttt tgtttgcaag 9660

cagcagatta cgcgcagaaa aaaaggatct caagaagatc ctttgatctt ttctacgggg 9720
tctgacgctc agtggaacga aaactcacgt taagggattt tggtcatgag attatcaaaa 9780
aggatcttca cctagatcct tttaaattaa aaatgaagtt ttaaatcaat ctaaagtata 9840
tatgagtaaa cttggtctga cagttaccaa tgcttaatca gtgaggcacc tatctcagcg 9900
atctgtctat ttcgttcatc catagttgcc tgactgcccg tcgtgtagat aactacgata 9960
cgggagggct taccatctgg ccccagtgct gcaatgatac cgcgagaccc acgctcaccg 10020
gctccagatt tatcagcaat aaaccagcca gccggaaggg ccgagcgcag aagtggtcct 10080
gcaactttat ccgcctccat ccagtctatt aattgttgcc gggaagctag agtaagtagt 10140
tcgccagtta atagtttgcg caacgttgtt gccattgcta caggcatcgt ggtgtcacgc 10200
tcgtcgtttg gtatggcttc attcagctcc ggttcccaac gatcaaggcg agttacatga 10260
tcccccatgt tgtgaaaaaa agcggttagc tccttcggtc ctccgatcgt tgtcagaagt 10320
aagttggccg cagtgttatc actcatggtt atggcagcac tgcataattc tcttactgtc 10380
atgccatccg taagatgctt ttctgtgact ggtgagtact caaccaagtc attctgagaa 10440
tagtgtatgc ggcgaccgag ttgctcttgc ccggcgtcaa tacgggataa taccgcgcca 10500
catagcagaa ctttaaaagt gctcatcatt ggaaaacgtt cttcggggcg aaaactctca 10560
aggatcttac cgctgttgag atccagttcg atgtaaccca ctcgtgcacc caactgatct 10620
tcagcatctt ttactttcac cagcgtttct gggtgagcaa aaacaggaag gcaaaatgcc 10680
gcaaaaaagg gaataagggc gacacggaaa tgttgaatac tcatactctt cctttttcaa 10740
tattattgaa gcatttatca gggttattgt ctcatgagcg gatacatatt tgaatgtatt 10800
tagaaaaata aacaaatagg ggttccgcgc acatttcccc gaaaagtgcc acctgacgtc 10860
taagaaacca ttattatcat gacattaacc tataaaaata ggcgtatcac gaggcccttt 10920
cgtctcgcgc gtttcggtga tgacggtgaa aacctctgac acatgcagct cccggagacg 10980
gtcacagctt gtctgtaagc ggatgccggg agcagacaag cccgtcaggg cgcgtcagcg 11040
ggtgttggcg ggtgtcgggg ctggcttaac tatgcggcat cagagcagat tgtactgaga 11100
gtgcaccata tcgactacgt cgtaaggccg tttctgacag agtaaaattc ttgagggaac 11160
tttcaccatt atgggaaatg gttcaagaag gtattgactt aaactccatc aaatggtcag 11220
gtcattgagt gtttttttatt tgttgtattt tttttttttt agagaaaatc ctccaatatc 11280
aaattaggaa tcgtagtttc atgattttct gttacaccta actttttgtg tggtgccctc 11340
ctccttgtca atattaatgt taaagtgcaa ttcttttttcc ttatcacgtt gagccattag 11400
tatcaatttg cttacctgta ttcctttact atcctccttt ttctccttct tgataaatgt 11460
atgtagattg cgtatatagt ttcgtctacc ctatgaacat attccatttt gtaatttcgt 11520
gtcgtttcta ttatgaattt catttataaa gtttatgtac gaattctatc cttttgttgt 11580

ttccgggtgt acaatatgga cttcctcttt tctggcaacc aaacccatac atcgggattc 11640
ctataatacc ttcgttggtc tccctaacat gtaggtggcg gaggggagat atacaataga 11700
acagatacca gacaagacat aatgggctaa acaagactac accaattaca ctgcctcatt 11760
gatggtggta cataacgaac taatactgta gccctagact tgatagccat catcatatcg 11820
aagtttcact accctttttc catttgccat ctattgaagt aataataggc gcatgcaact 11880
tcttttcttt ttttttcttt tctctctccc ccgttgttgt ctcaccatat ccgcaatgac 11940
aaaaaaatga tggaagacac taaaggaaaa aattaacgac aaagacagca ccaacagatg 12000
tcgttgttcc agagctgatg aggggtatct cgaagcacac gaaacttttt ccttccttca 12060
ttcacgcaca ctactctcta atgagcaacg gtatacggcc ttccttccag ttacttgaat 12120
ttgaaataaa aaaagtttgc tgtcttgcta tcaagtataa atagacctgc aattattaat 12180
cttttgtttc ctcgtcattg ttctcgttcc ctttcttcct tgtttctttt tctgcacaat 12240
atttcaagct ataccaagca tacaatcaac tccagctgca ttaaaatgag taatcctcaa 12300
aaagctctaa acgactttct gtccagtgaa tctgttcata cacatgatag ttctaggaaa 12360
caatctaata agcagtcatc cgacgaagga cgctcttcat cacaaccttc acatcatcac 12420
tctggtggta ctaacaacaa taataacaat aataataata ataataacag taacaacaac 12480
aacaacggca acgatggggg aaatgatgac gactatgatt atgaaatgca agattataga 12540
ccttctccgc aaagtgcgcg gcctactccc acgtatgttc cacaatattc tgtagaaagt 12600
gggactgctt tcccgattca agaggttatt cctagcgcat acattaacac acaagatata 12660
aaccataaag ataacggtcc gccgagtgca agcagtaata gagcattcag gcctagaggg 12720
cagaccacag tgtcggccaa cgtgcttaac attgaagatt tttacaaaaa tgcagacgat 12780
gcgcatacca tcccggagtc acatttatcg agaaggagaa gtaggtcgag ggctacgagt 12840
aatgctgggc acagtgccaa tacaggcgcc acgaatggca ggactactgg tgcccaaact 12900
aatatggaaa gcaatgaatc accacgtaac gtccccatta tggtgaagcc aaagacatta 12960
taccagaacc ctcaaacacc tacagtcttg ccctccacat accatccaat taataaatgg 13020
tcttccgtca aaaacactta tttgaaggaa tttttagccg agtttatggg aacaatggtt 13080
atgattattt tcggtagtgc tgttgtttgt caggtcaatg ttgctgggaa aatacagcag 13140
gacaatttca acgtggcttt ggataacctt aacgttaccg ggtcttctgc agaaacgata 13200
gacgctatga agagtttaac atccttggtt tcatccgttg cgggcggtac ctttgatgat 13260
gtggcattgg gctgggctgc tgccgtggtg atgggctatt tctgcgctgg tggtagtgcc 13320
atctcaggtg ctcatttgaa tccgtctatt acattagcca atttggtgta tagaggtttt 13380
cccctgaaga aagttcctta ttactttgct ggacaattga tcggtgcctt cacaggcgct 13440

```
ttgatcttgt ttatttggta caaaagggtg ttacaagagg catatagcga ttggtggatg  13500
aatgaaagtg ttgcgggaat gttttgcgtt tttccaaagc cttatctaag ttcaggacgg  13560
caattttttt ccgaattttt atgtggagct atgttacaag caggaacatt tgcgctgacc  13620
gatccttata cgtgtttgtc ctctgatgtt ttcccattga tgatgtttat tttgattttc  13680
attatcaatg cttccatggc ttatcagaca ggtacagcaa tgaatttggc tcgtgatctg  13740
ggcccacgtc ttgcactata tgcagttgga tttgatcata aaatgctttg ggtgcatcat  13800
catcatttct tttgggttcc catggtaggc ccatttattg gtgcgttaat gggggggttg  13860
gtttacgatg tctgtattta tcagggtcat gaatctccag tcaactggtc tttaccagtt  13920
tataaggaaa tgattatgag agcctggttt agaaggcctg gttggaagaa gagaaataga  13980
gcaagaagaa catcggacct gagtgacttc tcatacaata acgatgatga tgaggaattt  14040
ggagaaagaa tggctcttca aaagacaaag accaagtcat ctatttcaga caacgaaaat  14100
gaagcaggag aaaagaaagt gcaatttaaa tctgttcagc gcggcaaaag aacgtttgg   14159
```

<210> 35
<211> 31
<212> DNA
<213> Artificial

<220>
<223> Primer FDHuf

<400> 35
tcgaagactc cgaatgaaaa agacatgcca g 31

<210> 36
<211> 33
<212> DNA
<213> Artificial

<220>
<223> Primer FDHur

<400> 36
tccggatacc aagttcattt tcaatacacc cca 33

<210> 37
<211> 27
<212> DNA
<213> Artificial

<220>
<223> Primer FDHdf

<400> 37
atgcatgcag aatggttctt atgccac 27

<210> 38
<211> 30

<212> DNA
<213> Artificial

<220>
<223> Primer FDHdr

<400> 38
gaagacagtt ctgttattaa cgacgagcca 30

<210> 39
<211> 5577
<212> DNA
<213> Artificial

<220>
<223> pRN621

<400> 39

tctgtgcggt atttcacacc gcatacaggt ggcacttttc ggggaaatgt gcgcggaacc 60
cctatttgtt tatttttcta aatacattca aatatgtatc cgctcatgag acaataaccc 120
tgataaatgc ttcaataata gcacgtgagg agggccacca tggccaagtt gaccagtgcc 180
gttccggtgc tcaccgcgcg cgacgtcgcc ggagcggtcg agttctggac cgaccggctc 240
gggttctccc gggacttcgt ggaggacgac ttcgccggtg tggtccggga cgacgtgacc 300
ctgttcatca gcgcggtcca ggaccaggtg gtgccggaca acaccctggc ctgggtgtgg 360
gtgcgcggcc tggacgagct gtacgccgag tggtcggagg tcgtgtccac gaacttccgg 420
gacgcctccg ggccggccat gaccgagatc ggcgagcagc cgtgggggcg ggagttcgcc 480
ctgcgcgacc cggccggcaa ctgcgtgcac ttcgtggccg aggagcagga ctgacacgtg 540
ctaaaacttc atttttaatt taaaaggatc taggtgaaga tcctttttga taatctcatg 600
accaaaatcc cttaacgtga gttttcgttc cactgagcgt cagaccccgt agaaaagatc 660
aaaggatctt cttgagatcc tttttttctg cgcgtaatct gctgcttgca aacaaaaaaa 720
ccaccgctac cagcggtggt ttgtttgccg gatcaagagc taccaactct ttttccgaag 780
gtaactggct tcagcagagc gcagatacca aatactgtcc ttctagtgta gccgtagtta 840
ggccaccact tcaagaactc tgtagcaccg cctacatacc tcgctctgct aatcctgtta 900
ccagtggctg ctgccagtgg cgataagtcg tgtcttaccg ggttggactc aagacgatag 960
ttaccggata aggcgcagcg gtcgggctga acggggggtt cgtgcacaca gcccagcttg 1020
gagcgaacga cctacaccga actgagatac ctacagcgtg agctatgaga aagcgccacg 1080
cttcccgaag ggagaaaggc ggacaggtat ccggtaagcg gcagggtcgg aacaggagag 1140
cgcacgaggg agcttccagg gggaaacgcc tggtatcttt atagtcctgt cgggtttcgc 1200
cacctctgac ttgagcgtcg atttttgtga tgctcgtcag gggggcggag cctatggaaa 1260
aacgccagca acgcggcctt tttacggttc ctggcctttt gctggccttt tgctcacatg 1320

ttctttcctg cgttatcccc tgattctgtg gataaccgta ttaccgcctt tgagtgagct 1380

gataccgctc gccgcagccg aacgaccgag cgcagcgagt cagtgagcga ggaagcggaa 1440

gagcgcccaa tacgcaaacc gcctctcccc gcgcgttggc cgattcatta atgcagctgg 1500

cacgacaggt ttcccgactg gaaagcgggc agtgagcgca acgcaattaa tgtgagttag 1560

ctcactcatt aggcacccca ggctttacac tttatgcttc cggctcgtat gttgtgtgga 1620

attgtgagcg gataacaatt tcacacagga aacagctatg accatgatta cgccaagcta 1680

tttaggtgac actatagaat actcaagcta tgcatcaagc ttggtaccga gctcggatcc 1740

actagtaacg gccgccagtg tgctggaatt cgcccttcga agactccgaa tgaaaaagac 1800

atgccagtaa taaaactatt ttgatgttat gcggaatata ctattcttgg attattcact 1860

gttaactaaa agttggagaa atcactctgc actgtcaatc attgaaaaaa agaacatata 1920

aaagggcaca aaattgagtc ttttttaatg agttcttgct gaggaaagtt tagttaatat 1980

atcatttacg taaaatatgc atattcttgt attgtgcttt ttttattcat tttaagcagg 2040

aacaatttac aagtattgca acgctaatca aatcaaaata acagctgaaa attaatatgt 2100

cgaagggaaa ggttttgctg gttctttacg aaggtggtaa gcatgctgaa gagcaggaaa 2160

agttattggg gtgtattgaa aatgaacttg gtatccggag ctcgtacgtt cgaacttaag 2220

gcctcgtccc cgccgggtca cccggccagc gacatggagg cccagaatac cctccttgac 2280

agtcttgacg tgcgcagctc aggggcatga tgtgactgtc gcccgtacat ttagcccata 2340

catccccatg tataatcatt tgcatccata cattttgatg gccgcacggc gcgaagcaaa 2400

aattacggct cctcgctgca gacctgcgag cagggaaacg ctcccctcac agacgcgttg 2460

aattgtcccc acgccgcgcc cctgtagaga aatataaaag gttaggattt gccactgagg 2520

ttcttctttc atatacttcc ttttaaaatc ttgctaggat acagttctca catcacatcc 2580

gaacataaac aaccgggtag cccagaacga cgcccggtcg agatccgtcc cgccaccgcc 2640

gccgacatgg cggcggtctg cgacatcgtc aatcactaca tcgagacgag cacggtcaac 2700

ttccgtacgg agccgcagac tccgcaggag tggatcgacg acctggagcg cctccaggac 2760

cgctacccct ggctcgtcgc cgaggtggag ggcgtcgtcg ccggcatcgc ctacgccggc 2820

ccctggaagg cccgcaacgc ctacgactgg accgtcgagt cgacggtgta cgtctcccac 2880

cggcaccagc ggctcggact gggctccacc ctctacaccc acctgctgaa gtccatggag 2940

acccagggct tcaagagcgt ggtcgccgtc atcggactgc ccaacgaccc gagcgtgcgc 3000

ctgcacgagg cgctcggata caccgcgcgc gggacgctgc gggcagccgg ctacaagcac 3060

gggggctggc acgacgtggg gttctggcag cgcgacttct agctgccggc cccgccccgc 3120

cccgtccggc ccgtcacaca gatctaatca atactgacaa taaaaagatt cttgttttca 3180

agaacttgtc atttgtatag tttttttata ttgtagttgt tctattttaa tcaaatgtta 3240

gcgtgattta tattttttt cgcctcgaca tcatctgccc agatgcgaag ttaagtgcgc 3300
agaaagtaat atcatgcgtc aatcgtatgt gaatgctggt cgctatactg ctgtcgattc 3360
gatactaacg ccgccatcca gtgtcgacgg atcctaggtg tacagggccc aagggcgaat 3420
tctgcagata tccatcacac tggcggccgc tcgagcatgc atgcagaatg gttcttatgc 3480
caccagagct tatggacaga agaaataaga gtgattatga gtatttgtga gcagaagttt 3540
tccggtctcc ttttgttctt gttttggcgt attctccact attcgtccat agcacattta 3600
taccttagct aaatattttg taaagcaaaa ttttcgttat ctcttaaaaa atagaagagc 3660
ggtttattaa tatcaaataa ttgaaactgc tgatatggta gctatataca aaatctgctg 3720
tcaaaatttg gcagtaaacg atcttcacgg tagcggttca aataaagagg aaaagtcttt 3780
ctcccttact gtttttctgg aatttggctc gtcgttaata acagaactgt cttcgaaggg 3840
cgaattctgc agatatccat cacactggcg gccgctcgag catgcatcta gagggcccaa 3900
ttcgccctat agtgagtcgt attacaattc actggccgtc gttttacaac gtcgtgactg 3960
ggaaaaccct ggcgttaccc aacttaatcg ccttgcagca catccccctt tcgccagctg 4020
gcgtaatagc gaagaggccc gcaccgatcg cccttcccaa cagttgcgca gcctatacgt 4080
acggcagttt aaggtttaca cctataaaag agagagccgt tatcgtctgt ttgtggatgt 4140
acagagtgat attattgaca cgccggggcg acggatggtg atccccctgg ccagtgcacg 4200
tctgctgtca gataaagtct cccgtgaact ttacccggtg gtgcatatcg gggatgaaag 4260
ctggcgcatg atgaccaccg atatggccag tgtgccggtc tccgttatcg gggaagaagt 4320
ggctgatctc agccaccgcg aaaatgacat caaaaacgcc attaacctga tgttctgggg 4380
aatataaatg tcaggcatga gattatcaaa aaggatcttc acctagatcc ttttcacgta 4440
gaaagccagt ccgcagaaac ggtgctgacc ccggatgaat gtcagctact gggctatctg 4500
gacaagggaa aacgcaagcg caaagagaaa gcaggtagct tgcagtgggc ttacatggcg 4560
atagctagac tgggcggttt tatggacagc aagcgaaccg gaattgccag ctggggcgcc 4620
ctctggtaag gttgggaagc cctgcaaagt aaactggatg gctttctcgc cgccaaggat 4680
ctgatggcgc aggggatcaa gctctgatca agagacagga tgaggatcgt ttcgcatgat 4740
tgaacaagat ggattgcacg caggttctcc ggccgcttgg gtggagaggc tattcggcta 4800
tgactgggca caacagacaa tcggctgctc tgatgccgcc gtgttccggc tgtcagcgca 4860
ggggcgcccg gttctttttg tcaagaccga cctgtccggt gccctgaatg aactgcaaga 4920
cgaggcagcg cggctatcgt ggctggccac gacgggcgtt ccttgcgcag ctgtgctcga 4980
cgttgtcact gaagcgggaa gggactggct gctattgggc gaagtgccgg ggcaggatct 5040
cctgtcatct caccttgctc ctgccgagaa agtatccatc atggctgatg caatgcggcg 5100

gctgcatacg cttgatccgg ctacctgccc attcgaccac caagcgaaac atcgcatcga 5160
gcgagcacgt actcggatgg aagccggtct tgtcgatcag gatgatctgg acgaagagca 5220
tcaggggctc gcgccagccg aactgttcgc caggctcaag gcgagcatgc ccgacggcga 5280
ggatctcgtc gtgacccatg gcgatgcctg cttgccgaat atcatggtgg aaaatggccg 5340
cttttctgga ttcatcgact gtggccggct gggtgtggcg gaccgctatc aggacatagc 5400
gttggctacc cgtgatattg ctgaagagct tggcggcgaa tgggctgacc gcttcctcgt 5460
gctttacggt atcgccgctc ccgattcgca gcgcatcgcc ttctatcgcc ttcttgacga 5520
gttcttctga attattaacg cttacaattt cctgatgcgg tattttctcc ttacgca 5577

<210> 40
<211> 5830
<212> DNA
<213> Artificial

<220>
<223> pRN622

<400> 40

tatttcacac cgcatacagg tggcactttt cggggaaatg tgcgcggaac ccctatttgt 60

ttatttttct aaatacattc aaatatgtat ccgctcatga gacaataacc ctgataaatg 120

cttcaataat agcacgtgag gagggccacc atggccaagt tgaccagtgc cgttccggtg 180

ctcaccgcgc gcgacgtcgc cggagcggtc gagttctgga ccgaccggct cgggttctcc 240

cgggacttcg tggaggacga cttcgccggt gtggtccggg acgacgtgac cctgttcatc 300

agcgcggtcc aggaccaggt ggtgccggac aacaccctgg cctgggtgtg ggtgcgcggc 360

ctggacgagc tgtacgccga gtggtcggag gtcgtgtcca cgaacttccg ggacgcctcc 420

gggccggcca tgaccgagat cggcgagcag ccgtgggggc gggagttcgc cctgcgcgac 480

ccggccggca actgcgtgca cttcgtggcc gaggagcagg actgacacgt gctaaaactt 540

catttttaat ttaaaaggat ctaggtgaag atcctttttg ataatctcat gaccaaaatc 600

ccttaacgtg agttttcgtt ccactgagcg tcagaccccg tagaaaagat caaaggatct 660

tcttgagatc ctttttttct gcgcgtaatc tgctgcttgc aaacaaaaaa accaccgcta 720

ccagcggtgg tttgtttgcc ggatcaagag ctaccaactc tttttccgaa ggtaactggc 780

ttcagcagag cgcagatacc aaatactgtc cttctagtgt agccgtagtt aggccaccac 840

ttcaagaact ctgtagcacc gcctacatac ctcgctctgc taatcctgtt accagtggct 900

gctgccagtg gcgataagtc gtgtcttacc gggttggact caagacgata gttaccggat 960

aaggcgcagc ggtcgggctg aacggggggt tcgtgcacac agcccagctt ggagcgaacg 1020

acctacaccg aactgagata cctacagcgt gagctatgag aaagcgccac gcttcccgaa 1080

gggagaaagg cggacaggta tccggtaagc ggcagggtcg gaacaggaga gcgcacgagg 1140

gagcttccag ggggaaacgc ctggtatctt tatagtcctg tcgggtttcg ccacctctga 1200

cttgagcgtc gatttttgtg atgctcgtca ggggggcgga gcctatggaa aaacgccagc 1260

aacgcggcct ttttacggtt cctgggcttt tgctggcctt ttgctcacat gttctttcct 1320

gcgttatccc ctgattctgt ggataaccgt attaccgcct ttgagtgagc tgataccgct 1380

cgccgcagcc gaacgaccga gcgcagcgag tcagtgagcg aggaagcgga agagcgccca 1440

atacgcaaac cgcctctccc cgcgcgttgg ccgattcatt aatgcagctg gcacgacagg 1500

tttcccgact ggaaagcggg cagtgagcgc aacgcaatta atgtgagtta gctcactcat 1560

taggcacccc aggctttaca ctttatgctt ccggctcgta tgttgtgtgg aattgtgagc 1620

ggataacaat ttcacacagg aaacagctat gaccatgatt acgccaagct atttaggtga 1680

cactatagaa tactcaagct atgcatcaag cttggtaccg agctcggatc cactagtaac 1740

ggccgccagt gtgctggaat tcgccctttc gaagactccg aatgaaaaag acatgccagt 1800

aataaaaata attgatgtta tgcggaatat actattcttg gattattcac tgttaactaa 1860

aagttggaga aatcactctg cactgtcaat cattgaaaaa aagaacatat aaaagggcac 1920

aaaatcgagt ctttttaat gagttcttgc tgaggaaaat ttagttaata tatcatttac 1980

ataaaacatg catattattg tgttgtactt tctttattca ttttaagcag gaataattac 2040

aagtattgca acgctaatca aatcgaaata acagctgaaa attaatatgt cgaagggaaa 2100

ggttttgctg gttctttatg aaggtggtaa gcatgctgaa gagcaggaaa agttattggg 2160

gtgtattgaa aatgaacttg gtatccggag ctcgtacgtt cgaacttaag gcctcgtccc 2220

cgccgggtca cccggccagc gacatggagg cccagaatac cctccttgac agtcttgacg 2280

tgcgcagctc aggggcatga tgtgactgtc gcccgtacat ttagcccata catccccatg 2340

tataatcatt tgcatccata cattttgatg gccgcacggc gcgaagcaaa aattacggct 2400

cctcgctgca gacctgcgag cagggaaacg ctcccctcac agacgcgttg aattgtcccc 2460

acgccgcgcc cctgtagaga aatataaaag gttaggattt gccactgagg ttcttctttc 2520

atatacttcc ttttaaaatc ttgctaggat acagttctca catcacatcc gaacataaac 2580

aaccatgggt aaggaaaaga ctcacgtttc gaggccgcga ttaaattcca acatggatgc 2640

tgatttatat gggtataaat gggctcgcga taatgtcggg caatcaggtg cgacaatcta 2700

tcgattgtat gggaagcccg atgcgccaga gttgtttctg aaacatggca aaggtagcgt 2760

tgccaatgat gttacagatg agatggtcag actaaactgg ctgacggaat ttatgcctct 2820

tccgaccatc aagcatttta tccgtactcc tgatgatgca tggttactca ccactgcgat 2880

ccccggcaaa acagcattcc aggtattaga agaatatcct gattcaggtg aaaatattgt 2940

tgatgcgctg gcagtgttcc tgcgccggtt gcattcgatt cctgtttgta attgtccttt 3000

taacagcgat cgcgtatttc gtctcgctca ggcgcaatca cgaatgaata acggtttggt 3060
tgatgcgagt gattttgatg acgagcgtaa tggctggcct gttgaacaag tctggaaaga 3120
aatgcataag cttttgccat tctcaccgga ttcagtcgtc actcatggtg atttctcact 3180
tgataacctt atttttgacg aggggaaatt aataggttgt attgatgttg gacgagtcgg 3240
aatcgcagac cgataccagg atcttgccat cctatggaac tgcctcggtg agttttctcc 3300
ttcattacag aaacggcttt ttcaaaaata tggtattgat aatcctgata tgaataaatt 3360
gcagtttcat ttgatgctcg atgagttttt ctaatcagta ctgacaataa aaagattctt 3420
gttttcaaga acttgtcatt tgtatagttt ttttatattg tagttgttct attttaatca 3480
aatgttagcg tgatttatat ttttttttcgc ctcgacatca tctgcccaga tgcgaagtta 3540
agtgcgcaga aagtaatatc atgcgtcaat cgtatgtgaa tgctggtcgc tatactgctg 3600
tcgattcgat actaacgccg ccatccagtg tcgacggatc ctaggtgtac agggcccaaa 3660
agggcgaatt ctgcagatat ccatcacact ggcggccgct cgagcatgca gaatggttct 3720
tatgccacca gagcttatgg acagaagaaa taagagtgat tatgagtatt tgtgagcaga 3780
agttttccgg tctccttttg ttcttgtttt ggcgtattct ccactattcg tccatagcac 3840
atttatacct tagctaaata ttttgtaaag caaaattttc gttatctctt aaaaaataga 3900
agagcggttt attaatatca aataattgaa actgctgata tggtagctat atacaaaatc 3960
tgctgtcaaa atttggcagt aaacgatctt cacggtagcg gttcaaataa agaggaaaag 4020
tccttctccc ttactgtttt tctggaattt ggctcgtcgt taataacaga actgtcttca 4080
agggcgaatt ctgcagatat ccatcacact ggcggccgct cgagcatgca tctagagggc 4140
ccaattcgcc ctatagtgag tcgtattaca attcactggc cgtcgtttta caacgtcgtg 4200
actgggaaaa ccctggcgtt acccaactta atcgccttgc agcacatccc cctttcgcca 4260
gctggcgtaa tagcgaagag gcccgcaccg atcgcccttc ccaacagttg cgcagcctat 4320
acgtacggca gtttaaggtt tacacctata aaagagagag ccgttatcgt ctgtttgtgg 4380
atgtacagag tgatattatt gacacgccgg ggcgacggat ggtgatcccc ctggccagtg 4440
cacgtctgct gtcagataaa gtctcccgtg aactttaccc ggtggtgcat atcggggatg 4500
aaagctggcg catgatgacc accgatatgg ccagtgtgcc ggtctccgtt atcggggaag 4560
aagtggctga tctcagccac cgcgaaaatg acatcaaaaa cgccattaac ctgatgttct 4620
ggggaatata aatgtcaggc atgagattat caaaaaggat cttcacctag atccttttca 4680
cgtagaaagc cagtccgcag aaacggtgct gaccccggat gaatgtcagc tactgggcta 4740
tctggacaag ggaaaacgca agcgcaaaga gaaagcaggt agcttgcagt gggcttacat 4800
ggcgatagct agactgggcg gttttatgga cagcaagcga accggaattg ccagctgggg 4860
cgccctctgg taaggttggg aagccctgca aagtaaactg gatggctttc tcgccgccaa 4920

ggatctgatg gcgcagggga tcaagctctg atcaagagac aggatgagga tcgtttcgca 4980

tgattgaaca agatggattg cacgcaggtt ctccggccgc ttgggtggag aggctattcg 5040

gctatgactg ggcacaacag acaatcggct gctctgatgc cgccgtgttc cggctgtcag 5100

cgcaggggcg cccggttctt tttgtcaaga ccgacctgtc cggtgccctg aatgaactgc 5160

aagacgaggc agcgcggcta tcgtggctgg ccacgacggg cgttccttgc gcagctgtgc 5220

tcgacgttgt cactgaagcg ggaagggact ggctgctatt gggcgaagtg ccggggcagg 5280

atctcctgtc atctcacctt gctcctgccg agaaagtatc catcatggct gatgcaatgc 5340

ggcggctgca tacgcttgat ccggctacct gcccattcga ccaccaagcg aaacatcgca 5400

tcgagcgagc acgtactcgg atggaagccg gtcttgtcga tcaggatgat ctggacgaag 5460

agcatcaggg gctcgcgcca gccgaactgt tcgccaggct caaggcgagc atgcccgacg 5520

gcgaggatct cgtcgtgacc catggcgatg cctgcttgcc gaatatcatg gtggaaaatg 5580

gccgcttttc tggattcatc gactgtggcc ggctgggtgt ggcggaccgc tatcaggaca 5640

tagcgttggc tacccgtgat attgctgaag agcttggcgg cgaatgggct gaccgcttcc 5700

tcgtgcttta cggtatcgcc gctcccgatt cgcagcgcat cgccttctat cgccttcttg 5760

acgagttctt ctgaattatt aacgcttaca atttcctgat gcggtatttt ctccttacgc 5820

atctgtgcgg 5830

<210> 41
<211> 7690
<212> DNA
<213> Artificial

<220>
<223> pRN558

<400> 41

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accataattc cgttttaaga gcttggtgag cgctaggagt cactgccagg tatcgtttga 240

acacggcatt agtcagggaa gtcataacac agtcctttcc cgcaattttc tttttctatt 300

actcttggcc tcctctagta cactctatat ttttttatgc ctcggtaatg attttcattt 360

ttttttttcc acctagcgga tgactctttt tttttcttag cgattggcat tatcacataa 420

tgaattatac attatataaa gtaatgtgat ttcttcgaag aatatactaa aaaatgagca 480

ggcaagataa acgaaggcaa agatgacaga gcagaaagcc ctagtaaagc gtattacaaa 540

tgaaaccaag attcagattg cgatctcttt aaagggtggt cccctagcga tagagcactc 600

gatcttccca gaaaaagagg cagaagcagt agcagaacag gccacacaat cgcaagtgat 660

taacgtccac acaggtatag ggtttctgga ccatatgata catgctctgg ccaagcattc 720

cggctggtcg ctaatcgttg agtgcattgg tgacttacac atagacgacc atcacaccac 780

tgaagactgc gggattgctc tcggtcaagc ttttaaagag gccctactgg cgcgtggagt 840

aaaaaggttt ggatcaggat ttgcgccttt ggatgaggca ctttccagag cggtggtaga 900

tctttcgaac aggccgtacg cagttgtcga acttggtttg caaagggaga aagtaggaga 960

tctctcttgc gagatgatcc cgcattttct tgaaagcttt gcagaggcta gcagaattac 1020

cctccacgtt gattgtctgc gaggcaagaa tgatcatcac cgtagtgaga gtgcgttcaa 1080

ggctcttgcg gttgccataa gagaagccac ctcgcccaat ggtaccaacg atgttccctc 1140

caccaaaggt gttcttatgt agtgacaccg attatttaaa gctgcagcat acgatatata 1200

tacatgtgta tatatgtata cctatgaatg tcagtaagta tgtatacgaa cagtatgata 1260

ctgaagatga caaggtaatg catcattcta tacgtgtcat tctgaacgag gcgcgctttc 1320

ctttttcctt tttgcttttt cttttttttt ctcttgaact cgacggatca tatgcggtgt 1380

gaaataccgc acagatgcgt aaggagaaaa taccgcatca ggaaattgta aacgttaata 1440

ttttgttaaa attcgcgtta aatttttgtt aaatcagctc attttttaac caataggccg 1500

aaatcggcaa aatcccttat aaatcaaaag aatagaccga gatagggttg agtgttgttc 1560

cagtttggaa caagagtcca ctattaaaga acgtggactc caacgtcaaa gggcgaaaaa 1620

ccgtctatca gggcgatggc ccactacgtg aaccatcacc ctaatcaagt tttttggggt 1680

cgaggtgccg taaagcacta aatcggaacc ctaaagggag cccccgattt agagcttgac 1740

ggggaaagcc ggcattgcga ataccgcttc cacaaacatt gctcaaaagt atctctttgc 1800

tatatatctc tgtgctatat ccctatataa cctacccatc cacctttcgc tccttgaact 1860

tgcatctaaa ctcgacctct acatttttta tgtttatctc tagtattact ctttagacaa 1920

aaaaattgta gtaagaacta ttcatagagt gaatcgaaaa caatacgaaa atgtaaacat 1980

ttcctatacg tagtatatag agacaaaata gaagaaaccg ttcataattt tctgaccaat 2040

gaagaatcat caacgctatc actttctgtt cacaaagtat gcgcaatcca catcggtata 2100

gaatataatc ggggatgcct ttatcttgaa aaaatgcacc cgcagcttcg ctagtaatca 2160

gtaaacgcgg gaagtggagt caggcttttt ttatggaaga gaaaatagac accaaagtag 2220

ccttcttcta accttaacgg acctacagtg caaaaagtta tcaagagact gcattataga 2280

gcgcacaaag gagaaaaaaa gtaatctaag atgctttgtt agaaaaatag cgctctcggg 2340

atgcattttt gtagaacaaa aaagaagtat agattctttg ttggtaaaat agcgctctcg 2400

cgttgcattt ctgttctgta aaaatgcagc tcagattctt tgtttgaaaa attagcgctc 2460

tcgtcgcgtt gcatttttgt tttacaaaaa tgaagcacag attcttcgtt ggtaaaatag 2520

cgctttcgcg ttgcatttct gttctgtaaa aatgcagctc agattctttg tttgaaaaat 2580
tagcgctctc gcgttgcatt tttgttctac aaaatgaagc acagatgctt cgttaacaaa 2640
gatatgctat tgaagtgcaa gatggaaacg cagaaaatga accggggatg cgacgtgcaa 2700
gattacctat gcaatagatg caatagtttc tccaggaacc gaaatacata cattgtcttc 2760
cgtaaagcgc tagactatat attattatac aggttcaaat atactatctg tttcagggaa 2820
aactcccagg ttcggatgtt caaaattcaa tgatgggtaa caagtacgat cgtaaatctg 2880
taaaacagtt tgtcggatat taggctgtat ctcctcaaag cgtattcgaa tatcattgag 2940
aagctgcagc gtcacatcgg ataataatga tggcagccat tgtagaagtg ccttttgcat 3000
ttctagtctc tttctcggtc tagctagttt tactacatcg cgaagataga atcttagatc 3060
acactgcctt tgctgagctg gatcaataga gtaacaaaag agtggtaagg cctcgttaaa 3120
ggacaaggac ctgagcggaa gtgtatcgta cagtagacgg agtatctagt atagtctata 3180
gtccgtggaa ttaattctca tctttgacag cttatcatcg ataatccgga gctagcatgc 3240
ggccgccagt gtgatggata tctgcagaat tcgccctttt aagcttcgta cgtgtggaag 3300
aacgattaca acaggtgttg tcctctgagg acataaaata cacaccgaga ttcatcaact 3360
cattgctgga gttagcatat ctacaattgg gtgaaatggg gagcgatttg caggcatttg 3420
ctcggcatgc cggtagaggt gtggtcaata agagcgacct catgctatac ctgagaaagc 3480
aacctgacct acaggaaaga gttactcaag aataagaatt ttcgttttaa aacctaagag 3540
tcactttaaa atttgtatac acttattttt tttataactt atttaataat aaaaatcata 3600
aatcataaga aattcgcgcg cttaagcagc ttcaccagcc tttctagcca aagattgagc 3660
catcttttca gcggtagcca aagcagaaga ggtcataatg tccaagttac cagcgtaagc 3720
tggcaagtag tgagcagcac cttcaacttc caaccaaaca gcggtcttca aaccagagaa 3780
ttgaccaaca cctggcaagt taactggctt gtcttgtggg ataacttcga attgaactct 3840
ttgcttcaat ctgtaacctg gaacgtatgc ttgaacagct tcagccattt cgttgattga 3900
agcttcgatg tcgtcttgag atgcttcgtc agacaaaacg taaacggtgt ctctcatcat 3960
caatggtggt tcagctgggt tcaaaacgat gatagcctta cccttagcag caccaccaac 4020
aacttcgata gccctagagg tggtttcggt gaattcgtcg atgttagctc tggtacctgg 4080
accagcagac ttagaagcga tagaagcgat gatttcagcg tagtgaactc tagcaactct 4140
tgaaacagca gcaaccattg ggatggtagc ttgaccacca caggtaacca tgttaacgtt 4200
taattggtca acgttagctt ccaagttaac aactggaaca cagtatggac cgatagcagc 4260
tggggtcaag tcgatcaatc tgatgtctgg cttagcttct ctcaaagcag cgtcgttctt 4320
aacgtgagca ccagcagagg tagcgtcgaa aacgatgtcg atgtcagcga attctggcat 4380

gttcatcaaa ccgataacac cttcgtgggt ggtagcaaca cccattcttc tagctctagc 4440
caaaccgtca gattgtgggt cgataccaac cataacagcc atttccaagt gttgaccgtg 4500
tcttaggatc ttgatcatca agtcagtacc gatgttacca gaaccgatga tagcaacctt 4560
tctcttagac atctgcagtc tagatatatt tgttgtaaaa agtagataat tacttccttg 4620
atgatctgta aaaaagagaa aaagaaagca tctaagaact tgaaaaacta cgaattagaa 4680
aagaccaaat atgtatttct tgcattgacc aatttatgca agtttatata tatgtaaatg 4740
taagtttcac gaggttctac taaactaaac cacccccttg gttagaagaa aagagtgtgt 4800
gagaacaggc tgttgttgtc acacgattcg gacaattctg tttgaaagag agagagtaac 4860
agtacgatcg aacgaacttt gctctggaga tcacagtggg catcatagca tgtggtacta 4920
aaccctttcc cgccattcca gaaccttcga ttgcttgtta caaaacctgt gagccgtcgc 4980
taggaccttg ttgtgtgacg aaattggaag ctgcaatcaa taggaagaca ggaagtcgag 5040
cgtgtctggg ttttttcagt tttgttcttt ttgcaaacaa atcacgagcg acggtaattt 5100
ctttctcgat aagaggccac gtgctttatg agggtaacat caattcaaga aggagggaaa 5160
cacttccttt ttctggccct gataatagta tgagggtgaa gccaaaataa aggattcgcg 5220
cccaaatcgg catctttaaa tgcaggtatg cgatagttcc tcactctttc cttactcacg 5280
agtaattctt gcaaatgcct attatgcaga tgttataata tctgtgcgtc ttgagttgaa 5340
gagctcgaga ctagtggatc ccccgggctg caggaattcg atatcaagct tatcgatacc 5400
gtcgacctcg agggggggcc cggtacccag cttttgttcc ctttagtgag ggttaattcc 5460
gagcttggcg taatcatggt catagctgtt tcctgtgtga aattgttatc cgctcacaat 5520
tccacacaac ataggagccg gaagcataaa gtgtaaagcc tggggtgcct aatgagtgag 5580
gtaactcaca ttaattgcgt tgcgctcact gcccgctttc cagtcgggaa acctgtcgtg 5640
ccagctgcat taatgaatcg gccaacgcgc ggggagaggc ggtttgcgta ttgggcgctc 5700
ttccgcttcc tcgctcactg actcgctgcg ctcggtcgtt cggctgcggc gagcggtatc 5760
agctcactca aaggcggtaa tacggttatc cacagaatca ggggataacg caggaaagaa 5820
catgtgagca aaaggccagc aaaaggccag gaaccgtaaa aaggccgcgt tgctggcgtt 5880
tttccatagg ctcggccccc ctgacgagca tcacaaaaat cgacgctcaa gtcagaggtg 5940
gcgaaacccg acaggactat aaagatacca ggcgttcccc cctggaagct ccctcgtgcg 6000
ctctcctgtt ccgaccctgc cgcttaccgg atacctgtcc gcctttctcc cttcgggaag 6060
cgtggcgctt tctcaatgct cacgctgtag gtatctcagt tcggtgtagg tcgttcgctc 6120
caagctgggc tgtgtgcacg aaccccccgt tcagcccgac cgctgcgcct tatccggtaa 6180
ctatcgtctt gagtccaacc cggtaagaca cgacttatcg ccactggcag cagccactgg 6240
taacaggatt agcagagcga ggtatgtagg cggtgctaca gagttcttga agtggtggcc 6300

taactacggc tacactagaa ggacagtatt tggtatctgc gctctgctga agccagttac 6360

cttcggaaaa agagttggta gctcttgatc cggcaaacaa accaccgctg gtagcggtgg 6420

tttttttgtt tgcaagcagc agattacgcg cagaaaaaaa ggatctcaag aagatccttt 6480

gatcttttct acggggtctg acgctcagtg gaacgaaaac tcacgttaag ggattttggt 6540

catgagatta tcaaaaagga tcttcaccta gatcctttta aattaaaaat gaagttttaa 6600

atcaatctaa agtatatatg agtaaacttg gtctgacagt taccaatgct taatcagtga 6660

ggcacctatc tcagcgatct gtctatttcg ttcatccata gttgcctgac tgcccgtcgt 6720

gtagataact acgatacggg agggcttacc atctggcccc agtgctgcaa tgataccgcg 6780

agacccacgc tcaccggctc cagatttatc agcaataaac cagccagccg gaagggccga 6840

gcgcagaagt ggtcctgcaa ctttatccgc ctccatccag tctattaatt gttgccggga 6900

agctagagta agtagttcgc cagttaatag tttgcgcaac gttgttgcca ttgctacagg 6960

catcgtggtg tcacgctcgt cgtttggtat ggcttcattc agctccggtt cccaacgatc 7020

aaggcgagtt acatgatccc ccatgttgtg aaaaaaagcg gttagctcct tcggtcctcc 7080

gatcgttgtc agaagtaagt tggccgcagt gttatcactc atggttatgg cagcactgca 7140

taattctctt actgtcatgc catccgtaag atgcttttct gtgactggtg agtactcaac 7200

caagtcattc tgagaatagt gtatgcggcg accgagttgc tcttgcccgg cgtcaatacg 7260

ggataatacc gcgccacata gcagaacttt aaaagtgctc atcattggaa aacgttcttc 7320

ggggcgaaaa ctctcaagga tcttaccgct gttgagatcc agttcgatgt aacccactcg 7380

tgcacccaac tgatcttcag catcttttac tttcaccagc gtttctgggt gagcaaaaac 7440

aggaaggcaa aatgccgcaa aaaagggaat aagggcgaca cggaaatgtt gaatactcat 7500

actcttcctt tttcaatatt attgaagcat ttatcagggt tattgtctca tgagcggata 7560

catatttgaa tgtatttaga aaaataaaca aataggggtt ccgcgcacat ttccccgaaa 7620

agtgccacct gacgtcttat tatcatgaca ttaacctata aaaataggcg tatcacgagg 7680

ccctttcgtc 7690

<210> 42
<211> 9416
<212> DNA
<213> Artificial

<220>
<223> pRN595

<400> 42

gacgaaaggg cctcgtgata cgcctatttt tataggttaa tgtcatgata ataagacgtc 60

aggtggcact tttcggggaa atgtgcgcgg aacccctatt tgtttatttt tctaaataca 120

ttcaaatatg tatccgctca tgagacaata accctgataa atgcttcaat aatattgaaa 180
aaggaagagt atgagtattc aacatttccg tgtcgccctt attccctttt ttgcggcatt 240
ttgccttcct gtttttgctc acccagaaac gctggtgaaa gtaaaagatg ctgaagatca 300
gttgggtgca cgagtgggtt acatcgaact ggatctcaac agcggtaaga tccttgagag 360
ttttcgcccc gaagaacgtt ttccaatgat gagcactttt aaagttctgc tatgtggcgc 420
ggtattatcc cgtattgacg ccgggcaaga gcaactcggt cgccgcatac actattctca 480
gaatgacttg gttgagtact caccagtcac agaaaagcat cttacggatg gcatgacagt 540
aagagaatta tgcagtgctg ccataaccat gagtgataac actgcggcca acttacttct 600
gacaacgatc ggaggaccga aggagctaac cgcttttttt cacaacatgg gggatcatgt 660
aactcgcctt gatcgttggg aaccggagct gaatgaagcc ataccaaacg acgagcgtga 720
caccacgatg cctgtagcaa tggcaacaac gttgcgcaaa ctattaactg gcgaactact 780
tactctagct tcccggcaac aattaataga ctggatggag gcggataaag ttgcaggacc 840
acttctgcgc tcggcccttc cggctggctg gtttattgct gataaatctg gagccggtga 900
gcgtgggtct cgcggtatca ttgcagcact ggggccagat ggtaagccct cccgtatcgt 960
agttatctac acgacgggca gtcaggcaac tatggatgaa cgaaatagac agatcgctga 1020
gataggtgcc tcactgatta agcattggta actgtcagac caagtttact catatatact 1080
ttagattgat ttaaaacttc atttttaatt taaaaggatc taggtgaaga tcctttttga 1140
taatctcatg accaaaatcc cttaacgtga gttttcgttc cactgagcgt cagaccccgt 1200
agaaaagatc aaaggatctt cttgagatcc tttttttctg cgcgtaatct gctgcttgca 1260
aacaaaaaaa ccaccgctac cagcggtggt ttgtttgccg gatcaagagc taccaactct 1320
ttttccgaag gtaactggct tcagcagagc gcagatacca aatactgtcc ttctagtgta 1380
gccgtagtta ggccaccact tcaagaactc tgtagcaccg cctacatacc tcgctctgct 1440
aatcctgtta ccagtggctg ctgccagtgg cgataagtcg tgtcttaccg ggttggactc 1500
aagacgatag ttaccggata aggcgcagcg gtcgggctga acggggggtt cgtgcacaca 1560
gcccagcttg gagcgaacga cctacaccga actgagatac ctacagcgtg agcattgaga 1620
aagcgccacg cttcccgaag ggagaaaggc ggacaggtat ccggtaagcg gcagggtcgg 1680
aacaggagag cgcacgaggg agcttccagg gggaaacgcc tggtatcttt atagtcctgt 1740
cgggtttcgc cacctctgac ttgagcgtcg atttttgtga tgctcgtcag gggggcggag 1800
cctatggaaa aacgccagca acgcggcctt tttacggttc ctggcctttt gctggccttt 1860
tgctcacatg ttctttcctg cgttatcccc tgattctgtg gataaccgta ttaccgcctt 1920
tgagtgagct gataccgctc gccgcagccg aacgaccgag cgcagcgagt cagtgagcga 1980
ggaagcggaa gagcgcccaa tacgcaaacc gcctctcccc gcgcgttggc cgattcatta 2040

atgcagctgg cacgacaggt ttcccgactg gaaagcgggc agtgagcgca acgcaattaa 2100
tgtgagttac ctcactcatt aggcacccca ggctttacac tttatgcttc cggctcctat 2160
gttgtgtgga attgtgagcg gataacaatt tcacacagga aacagctatg accatgatta 2220
cgccaagctc ggaattaacc ctcactaaag ggaacaaaag ctgggtaccg ggccccccct 2280
cgaggtcgac ggtatcgata agcttgatat cgaattcctg cagcccgggg gatccactag 2340
tctcgagctc ttcaactcaa gacgcacaga tattataaca tctgcataat aggcatttgc 2400
aagaattact cgtgagtaag gaaagagtga ggaactatcg catacctgca tttaaagatg 2460
ccgatttggg cgcgaatcct ttattttggc ttcaccctca tactattatc agggccagaa 2520
aaaggaagtg tttccctcct tcttgaattg atgttaccct cataaagcac gtggcctctt 2580
atcgagaaag aaattaccgt cgctcgtgat ttgtttgcaa aaagaacaaa actgaaaaaa 2640
cccagacacg ctcgacttcc tgtcttccta ttgattgcag cttccaattt cgtcacacaa 2700
caaggtccta gcgacggctc acaggttttg taacaagcaa tcgaaggttc tggaatggcg 2760
ggaaagggtt tagtaccaca tgctatgatg cccactgtga tctccagagc aaagttcgtt 2820
cgatcgtact gttactctct ctctttcaaa cagaattgtc cgaatcgtgt gacaacaaca 2880
gcctgttctc acacactctt ttcttctaac caagggggtg gtttagttta gtagaacctc 2940
gtgaaactta catttacata tatataaact tgcataaatt ggtcaatgca agaaatacat 3000
atttggtctt ttctaattcg tagtttttca agttcttaga tgctttcttt ttctcttttt 3060
tacagatcat caaggaagta attatctact ttttacaaca aatatatcta gaaaatggct 3120
gttaccaacg ttgctgaatt gaacgctttg gttgaaaggg ttaagaaggc tcaaagagaa 3180
tacgcttctt tcacccaaga acaagttgac aagatcttca gagctgctgc tttggctgct 3240
gctgacgcta gaatcccatt ggctaagatg gctgttgctg aatctggtat gggtatcgtt 3300
gaagacaagg ttatcaagaa ccacttcgct tctgaataca tctacaacgc ttacaaggac 3360
gaaaagacct gtggtgtttt gtcagaagac gacaccttcg gtaccatcac catcgctgaa 3420
ccaatcggta tcatctgtgg tatcgttcca accaccaacc caacctctac cgctatcttc 3480
aagtctttga tctctttgaa gaccagaaac gctatcatct tctctccaca cccaagagct 3540
aaagacgcta ccaacaaggc tgctgacatc gttttgcaag ctgctatcgc tgctggtgct 3600
ccaaaggact tgatcggttg gatcgaccaa ccatctgttg aattgtctaa cgctttgatg 3660
caccacccag acatcaactt gatcttggct accggtggtc caggtatggt taaggctgct 3720
tactcttctg gtaagccagc tatcggtgtt ggtgctggta acaccccagt tgttatcgac 3780
gaaaccgctg acatcaagag agctgttgct tctgttttga tgtctaagac cttcgacaac 3840
ggtgttatct gtgcttctga acaatctgtt gttgttgttg actctgttta cgacgctgtt 3900

agagaaagat tcgctaccca cggtggttac ttgttgcaag gtaaggaatt gaaggctgtt 3960
caagacgtta tcttgaagaa cggtgctttg aacgctgcta tcgttggtca accagcttac 4020
aagatcgctg aattagctgg tttctctgtt ccagaaaaca ccaagatctt gatcggtgaa 4080
gttaccgttg ttgacgaatc tgaaccattc gctcacgaaa agttgtctcc aaccttggct 4140
atgtacagag ctaaggactt cgaagacgct gttgaaaaag ctgaaaagtt ggttgctatg 4200
ggtggtattg gtcacacctc ttgtttgtac accgaccaag acaaccaacc agctagagtt 4260
tcttacttcg gtcaaaagat gaagaccgct agaatcttga tcaacacccc agcttctcaa 4320
ggtggtatcg gtgacttgta caacttcaag ttggctccat ctttgacctt gggttgtggt 4380
tcttggggtg gtaactctat ctctgaaaac gttggtccaa agcacttgat caacaagaag 4440
accgttgcta agagagctga aaacatgttg tggcacaagt tgccaaaatc tatctacttc 4500
agaagaggtt ctttgccaat cgctttggac gaagttatca ccgacggtca caagagagct 4560
ttgatcgtta ccgacagatt cttgttcaac aacggttacg ctgaccaaat cacctctgtt 4620
ttgaaggctg ctggtgttga aaccgaagtt ttcttcgaag ttgaagctga cccaaccttg 4680
tctatcgtta gaaagggtgc tgaattggct aactctttca agccagacgt tatcatcgct 4740
ttgggtggtg gttctccaat ggacgctgct aagatcatgt gggttatgta cgaacaccca 4800
gaaacccact tcgaagaatt ggctttgaga ttcatggaca tcagaaagag aatctacaag 4860
ttcccaaaga tgggtgttaa ggctaagatg atcgctgtta ccaccacctc tggtaccggt 4920
tctgaagtta ccccattcgc tgttgttacc gacgacgcta ccggtcaaaa gtacccattg 4980
gctgactacg ctttgacccc agacatggct atcgttgacg ctaacttggt tatggacatg 5040
ccaaagtctt tgtgtgcttt cggtggtttg gacgctgtta cccacgctat ggaagcttac 5100
gtttctgttt tggcttctga attctctgac ggtcaagctt tgcaagcttt gaagttgttg 5160
aaggaatact tgccagcttc ttaccacgaa ggttctaaga acccagttgc tagagaaaga 5220
gttcactctg ctgctaccat cgctggtatc gctttcgcta acgctttctt gggtgtttgt 5280
cactctatgg ctcacaagtt gggttctcaa ttccacatcc cacacggttt ggctaacgct 5340
ttgttgatct gtaacgttat cagatacaac gctaacgaca acccaaccaa gcaaaccgct 5400
ttctctcaat acgacagacc acaagctaga agaagatacg ctgaaatcgc tgaccacttg 5460
ggtttgtctg ctccaggtga cagaaccgct gcaaagatcg aaaagttgtt ggcttggttg 5520
gaaaccttga aggctgaatt gggtatccca aagtctatca gagaagctgg tgttcaagaa 5580
gctgacttct tggctaacgt tgacaagttg tctgaagacg ctttcgacga ccaatgtacc 5640
ggtgctaacc caagataccc attgatctct gaattgaagc aaatcttgtt ggacacctac 5700
tacggtagag actacgttga aggtgaaacc gctgctaaga aggaagctgc tccagctaag 5760
gctgaaaaga aggctaagaa gtctgcttag cttaagcgcg cgaatttctt atgatttatg 5820

atttttatta ttaaataagt tataaaaaaa ataagtgtat acaaatttta aagtgactct 5880

taggttttaa aacgaaaatt cttattcttg agtaactctt tcctgtaggt caggttgctt 5940

tctcaggtat agcatgaggt cgctcttatt gaccacacct ctaccggcat gccgagcaaa 6000

tgcctgcaaa tcgctcccca tttcacccaa ttgtagatat gctaactcca gcaatgagtt 6060

gatgaatctc ggtgtgtatt ttatgtcctc agaggacaac acctgttgta atcgttcttc 6120

cacacgtacg aagcttaaaa gggcgaattc tgcagatatc catcacactg gcggccgcat 6180

gctagctccg gattatcgat gataagctgt caaagatgag aattaattcc acggactata 6240

gactatacta gatactccgt ctactgtacg atacacttcc gctcaggtcc ttgtccttta 6300

acgaggcctt accactcttt tgttactcta ttgatccagc tcagcaaagg cagtgtgatc 6360

taagattcta tcttcgcgat gtagtaaaac tagctagacc gagaaagaga ctagaaatgc 6420

aaaaggcact tctacaatgg ctgccatcat tattatccga tgtgacgctg cagcttctca 6480

atgatattcg aatacgcttt gaggagatac agcctaatat ccgacaaact gttttacaga 6540

tttacgatcg tacttgttac ccatcattga attttgaaca tccgaacctg ggagttttcc 6600

ctgaaacaga tagtatattt gaacctgtat aataatatat agtctagcgc tttacggaag 6660

acaatgtatg tatttcggtt cctggagaaa ctattgcatc tattgcatag gtaatcttgc 6720

acgtcgcatc cccggttcat ttctgcgtt tccatcttgc acttcaatag catatctttg 6780

ttaacgaagc atctgtgctt cattttgtag aacaaaaatg caacgcgaga gcgctaattt 6840

ttcaaacaaa gaatctgagc tgcattttta cagaacagaa atgcaacgcg aaagcgctat 6900

tttaccaacg aagaatctgt gcttcatttt tgtaaaacaa aaatgcaacg cgacgagagc 6960

gctaattttt caaacaaaga atctgagctg catttttaca gaacagaaat gcaacgcgag 7020

agcgctattt taccaacaaa gaatctatac ttcttttttg ttctacaaaa atgcatcccg 7080

agagcgctat ttttctaaca aagcatctta gattactttt tttctccttt gtgcgctcta 7140

taatgcagtc tcttgataac tttttgcact gtaggtccgt taaggttaga agaaggctac 7200

tttggtgtct attttctctt ccataaaaaa agcctgactc cacttcccgc gtttactgat 7260

tactagcgaa gctgcgggtg cattttttca agataaaggc atccccgatt atattctata 7320

ccgatgtgga ttgcgcatac tttgtgaaca gaaagtgata gcgttgatga ttcttcattg 7380

gtcagaaaat tatgaacggt ttcttctatt ttgtctctat atactacgta taggaaatgt 7440

ttacattttc gtattgtttt cgattcactc tatgaatagt tcttactaca atttttttgt 7500

ctaaagagta atactagaga taaacataaa aaatgtagag gtcgagttta gatgcaagtt 7560

caaggagcga aaggtggatg ggtaggttat atagggatat agcacagaga tatatagcaa 7620

agagatactt ttgagcaatg tttgtggaag cggtattcgc aatgccggct ttccccgtca 7680

agctctaaat cgggggctcc ctttagggtt ccgatttagt gctttacggc acctcgaccc 7740
caaaaaactt gattagggtg atggttcacg tagtgggcca tcgccctgat agacggtttt 7800
tcgccctttg acgttggagt ccacgttctt taatagtgga ctcttgttcc aaactggaac 7860
aacactcaac cctatctcgg tctattcttt tgatttataa gggattttgc cgatttcggc 7920
ctattggtta aaaaatgagc tgatttaaca aaaatttaac gcgaatttta acaaaatatt 7980
aacgtttaca atttcctgat gcggtatttt ctccttacgc atctgtgcgg tatttcacac 8040
cgcatatgat ccgtcgagtt caagagaaaa aaaaagaaaa agcaaaaaga aaaaaggaaa 8100
gcgcgcctcg ttcagaatga cacgtataga atgatgcatt accttgtcat cttcagtatc 8160
atactgttcg tatacatact tactgacatt cataggtata catatataca catgtatata 8220
tatcgtatgc tgcagcttta aataatcggt gtcactacat aagaacacct ttggtggagg 8280
gaacatcgtt ggtaccattg ggcgaggtgg cttctcttat ggcaaccgca agagccttga 8340
acgcactctc actacggtga tgatcattct tgcctcgcag acaatcaacg tggagggtaa 8400
ttctgctagc ctctgcaaag ctttcaagaa aatgcgggat catctcgcaa gagagatctc 8460
ctactttctc cctttgcaaa ccaagttcga caactgcgta cggcctgttc gaaagatcta 8520
ccaccgctct ggaaagtgcc tcatccaaag gcgcaaatcc tgatccaaac ctttttactc 8580
cacgcgccag tagggcctct ttaaaagctt gaccgagagc aatcccgcag tcttcagtgg 8640
tgtgatggtc gtctatgtgt aagtcaccaa tgcactcaac gattagcgac cagccggaat 8700
gcttggccag agcatgtatc atatggtcca gaaaccctat acctgtgtgg acgttaatca 8760
cttgcgattg tgtggcctgt tctgctactg cttctgcctc tttttctggg aagatcgagt 8820
gctctatcgc taggggacca ccctttaaag agatcgcaat ctgaatcttg gtttcatttg 8880
taatacgctt tactagggct ttctgctctg tcatctttgc cttcgtttat cttgcctgct 8940
catttttttag tatattcttc gaagaaatca cattacttta tataatgtat aattcattat 9000
gtgataatgc caatcgctaa gaaaaaaaaa gagtcatccg ctaggtggaa aaaaaaaaat 9060
gaaaatcatt accgaggcat aaaaaaatat agagtgtact agaggaggcc aagagtaata 9120
gaaaaagaaa attgcgggaa aggactgtgt tatgacttcc ctgactaatg ccgtgttcaa 9180
acgatacctg gcagtgactc ctagcgctca ccaagctctt aaaacggaat tatggtgcac 9240
tctcagtaca atctgctctg atgccgcata gttaagccag ccccgacacc cgccaacacc 9300
cgctgacgcg ccctgacggg cttgtctgct cccggcatcc gcttacagac aagctgtgac 9360
cgtctccggg agctgcatgt gtcagaggtt ttcaccgtca tcaccgaaac gcgcga 9416

<210> 43
<211> 9352
<212> DNA

<213> Artificial

<220>
<223> pRN596

<400> 43

```
gacgaaaggg cctcgtgata cgcctatttt tataggttaa tgtcatgata ataagacgtc     60
aggtggcact tttcggggaa atgtgcgcgg aacccctatt tgtttatttt tctaaataca    120
ttcaaatatg tatccgctca tgagacaata accctgataa atgcttcaat aatattgaaa    180
aaggaagagt atgagtattc aacatttccg tgtcgccctt attccctttt ttgcggcatt    240
ttgccttcct gtttttgctc acccagaaac gctggtgaaa gtaaaagatg ctgaagatca    300
gttgggtgca cgagtgggtt acatcgaact ggatctcaac agcggtaaga tccttgagag    360
ttttcgcccc gaagaacgtt ttccaatgat gagcactttt aaagttctgc tatgtggcgc    420
ggtattatcc cgtattgacg ccgggcaaga gcaactcggt cgccgcatac actattctca    480
gaatgacttg gttgagtact caccagtcac agaaaagcat cttacggatg gcatgacagt    540
aagagaatta tgcagtgctg ccataaccat gagtgataac actgcggcca acttacttct    600
gacaacgatc ggaggaccga aggagctaac cgcttttttt cacaacatgg gggatcatgt    660
aactcgcctt gatcgttggg aaccggagct gaatgaagcc ataccaaacg acgagcgtga    720
caccacgatg cctgtagcaa tggcaacaac gttgcgcaaa ctattaactg gcgaactact    780
tactctagct tcccggcaac aattaataga ctggatggag gcggataaag ttgcaggacc    840
acttctgcgc tcggcccttc cggctggctg gtttattgct gataaatctg gagccggtga    900
gcgtgggtct cgcggtatca ttgcagcact ggggccagat ggtaagccct cccgtatcgt    960
agttatctac acgacgggca gtcaggcaac tatggatgaa cgaaatagac agatcgctga   1020
gataggtgcc tcactgatta agcattggta actgtcagac caagtttact catatatact   1080
ttagattgat ttaaaacttc atttttaatt taaaaggatc taggtgaaga tcctttttga   1140
taatctcatg accaaaatcc cttaacgtga gttttcgttc cactgagcgt cagaccccgt   1200
agaaaagatc aaaggatctt cttgagatcc tttttttctg cgcgtaatct gctgcttgca   1260
aacaaaaaaa ccaccgctac cagcggtggt ttgtttgccg gatcaagagc taccaactct   1320
ttttccgaag gtaactggct tcagcagagc gcagatacca aatactgtcc ttctagtgta   1380
gccgtagtta ggccaccact tcaagaactc tgtagcaccg cctacatacc tcgctctgct   1440
aatcctgtta ccagtggctg ctgccagtgg cgataagtcg tgtcttaccg ggttggactc   1500
aagacgatag ttaccggata aggcgcagcg gtcgggctga acggggggtt cgtgcacaca   1560
gcccagcttg gagcgaacga cctacaccga actgagatac ctacagcgtg agcattgaga   1620
aagcgccacg cttcccgaag ggagaaaggc ggacaggtat ccggtaagcg gcagggtcgg   1680
aacaggagag cgcacgaggg agcttccagg ggggaacgcc tggtatcttt atagtcctgt   1740
```

cgggtttcgc cacctctgac ttgagcgtcg atttttgtga tgctcgtcag gggggccgag 1800
cctatggaaa aacgccagca acgcggcctt tttacggttc ctggcctttt gctggccttt 1860
tgctcacatg ttctttcctg cgttatcccc tgattctgtg gataaccgta ttaccgcctt 1920
tgagtgagct gataccgctc gccgcagccg aacgaccgag cgcagcgagt cagtgagcga 1980
ggaagcggaa gagcgcccaa tacgcaaacc gcctctcccc gcgcgttggc cgattcatta 2040
atgcagctgg cacgacaggt ttcccgactg gaaagcgggc agtgagcgca acgcaattaa 2100
tgtgagttac ctcactcatt aggcacccca ggctttacac tttatgcttc cggctcctat 2160
gttgtgtgga attgtgagcg gataacaatt tcacacagga aacagctatg accatgatta 2220
cgccaagctc ggaattaacc ctcactaaag ggaacaaaag ctgggtaccg ggccccccct 2280
cgaggtcgac ggtatcgata agcttgatat cgaattcctg cagcccgggg gatccactag 2340
tctcgagctc ttcaactcaa gacgcacaga tattataaca tctgcataat aggcatttgc 2400
aagaattact cgtgagtaag gaaagagtga ggaactatcg catacctgca tttaaagatg 2460
ccgatttggg cgcgaatcct ttattttggc ttcaccctca tactattatc agggccagaa 2520
aaaggaagtg tttccctcct tcttgaattg atgttaccct cataaagcac gtggcctctt 2580
atcgagaaag aaattaccgt cgctcgtgat ttgtttgcaa aaagaacaaa actgaaaaaa 2640
cccagacacg ctcgacttcc tgtcttccta ttgattgcag cttccaattt cgtcacacaa 2700
caaggtccta gcgacggctc acaggttttg taacaagcaa tcgaaggttc tggaatggcg 2760
ggaaagggtt tagtaccaca tgctatgatg cccactgtga tctccagagc aaagttcgtt 2820
cgatcgtact gttactctct ctctttcaaa cagaattgtc cgaatcgtgt gacaacaaca 2880
gcctgttctc acacactctt ttcttctaac caagggggtg gtttagttta gtagaacctc 2940
gtgaaactta catttacata tatataaact tgcataaatt ggtcaatgca agaaatacat 3000
atttggtctt ttctaattcg tagtttttca agttcttaga tgctttcttt ttctcttttt 3060
tacagatcat caaggaagta attatctact ttttacaaca aatatatcta gaaaatgtct 3120
acccaacaaa ccatgaccgt tgacgaacac atcaaccaat tagttagaaa ggctcaagtt 3180
gctttgaagg aatacttgaa gccagaatac acccaagaaa agatcgacta catcgttaag 3240
aaggcttctg ttgctgcttt ggaccaacac tgtgctttgg ctgctgctgc tgttgaagaa 3300
accggtagag gtatcttcga agacaaggct accaagaaca tcttcgcttg tgaacacgtt 3360
acccacgaaa tgagacacgc taagaccgtt ggtatcatca acgttgaccc attgtacggt 3420
atcaccgaaa tcgctgaacc agttggtgtt gtttgtggtg ttaccccagt taccaaccca 3480
acctctaccg ctatcttcaa gtctttgatc tctatcaaga ccagaaaccc aatcgttttc 3540
tctttccacc catctgcttt gaagtgttct attatggctg ctaaaatcgt tagagacgct 3600
gctatcgctg ctggtgctcc agaaaactgt atccaatgga tcgaattcgg tggtatcgaa 3660

gcttctaaca agttgatgaa ccacccaggt gttgctacta tcttggctac cggtggtaac 3720

gctatggtta aggctgcata ctcttctggt aagccagctt tgggtgttgg tgctggtaac 3780

gttccaacct acatcgaaaa gacctgtaac atcaagcaag ctgctaacga cgttgttatg 3840

tctaagtctt tcgacaacgg tatgatctgt gcttctgaac aagctgctat catcgacaag 3900

gaaatctacg accaagttgt tgaagaaatg aagaccttgg gtgcttactt catcaacgaa 3960

gaagaaaagg ctaagttgga aaagttcatg ttcggtgtta acgcttactc tgctgacgtt 4020

aacaacgcta gattgaaccc aaagtgtcca ggtatgtctc cacaatggtt cgctgaacaa 4080

gttggtatca aggtaccaga agactgtaac atcatctgtg ctgtttgtaa ggaagttggt 4140

ccaaacgaac cattgaccag agaaaagttg tctccagttt tggctatctt gaaagctgaa 4200

aacacccaag acggtatcga caaggctgaa gctatggttg aatttaacgg tagaggtcac 4260

tctgctgcta tccactctaa cgacaaggct gttgttgaaa agtacgcttt gaccatgaag 4320

gcttgtagaa tcttgcacaa caccccatct tctcaaggtg gtatcggttc tatctacaac 4380

tacatctggc catctttcac cttgggttgt ggttcttacg gtggtaactc tgtttctgct 4440

aacgttacct accacaactt gttgaacatc aagagattgg ctgacagaag aaacaacttg 4500

caatggttca gagttccacc aaagatcttc ttcgaaccac actctatcag atacttggct 4560

gaattgaagg aattgtctaa gatcttcatc gtttctgaca gaatgatgta caagttgggt 4620

tacgttgaca gagttatgga cgttttgaag agaagatcta acgaagttga aatcgaaatc 4680

ttcatcgacg ttgaaccaga cccatctatc caaaccgttc aaaagggttt ggctgttatg 4740

aacaccttcg gtccagacaa catcatcgct atcggtggtg gttctgctat ggacgctgct 4800

aagatcatgt ggttgttgta cgaacaccca gaagctgact tcttcgctat gaagcaaaag 4860

ttcatcgact tgagaaagag agctttcaag ttcccaacca tgggtaagaa ggctagattg 4920

atctgtatcc caaccacctc tggtaccggt tctgaagtta ccccattcgc tgttatctct 4980

gaccacgaaa ccggtaagaa gtacccattg gctgactact ctttgacccc atctgttgct 5040

atcgttgacc caatgttcac catgtctttg ccaaagagag ctatcgctga caccggtttg 5100

gacgttttgg ttcacgctac cgaagcttac gtttctgtta tggctaacga atacaccgac 5160

ggtttggcta gagaagctgt taagttggtt tttgaaaact tgttgaagtc ttacaacggt 5220

gacttggaag ctagagaaaa gatgcacaac gctgctacca tcgctggtat ggctttcgct 5280

tctgctttct tgggtatgga ccactctatg gctcacaagg ttggtgctgc tttccacttg 5340

ccacacggta gatgtgttgc tgttttgttg ccacacgtta tcagatacaa cggtcaaaag 5400

ccaagaaagt tggctatgtg gccaaagtac aacttctaca aggctgacca aagatacatg 5460

gaattggctc aaatggttgg tttgaagtgt aacaccccag ctgaaggtgt tgaagctttc 5520

gctaaggctt gtgaagaatt gatgaaggct accgaaacca tcaccggttt caagaaggct 5580
aacatcgacg aagctgcttg gatgtctaag gttccagaaa tggctttgtt ggctttcgaa 5640
gaccaatgtt ctccagctaa cccaagagtt ccaatggtta aggacatgga aaagatcttg 5700
aaggctgctt actacccaat cgcttagctt aagcgcgcga atttcttatg atttatgatt 5760
tttattatta aataagttat aaaaaaaata agtgtataca aattttaaag tgactcttag 5820
gttttaaaac gaaaattctt attcttgagt aactctttcc tgtaggtcag gttgctttct 5880
caggtatagc atgaggtcgc tcttattgac cacacctcta ccggcatgcc gagcaaatgc 5940
ctgcaaatcg ctccccattt cacccaattg tagatatgct aactccagca atgagttgat 6000
gaatctcggt gtgtatttta tgtcctcaga ggacaacacc tgttgtaatc gttcttccac 6060
acgtacgaag ctaaaagggc gaattctgca gatatccatc acactggcgg ccgcatgcta 6120
gctccggatt atcgatgata agctgtcaaa gatgagaatt aattccacgg actatagact 6180
atactagata ctccgtctac tgtacgatac acttccgctc aggtccttgt cctttaacga 6240
ggccttacca ctcttttgtt actctattga tccagctcag caaaggcagt gtgatctaag 6300
attctatctt cgcgatgtag taaaactagc tagaccgaga aagagactag aaatgcaaaa 6360
ggcacttcta caatggctgc catcattatt atccgatgtg acgctgcagc ttctcaatga 6420
tattcgaata cgctttgagg agatacagcc taatatccga caaactgttt tacagattta 6480
cgatcgtact tgttacccat cattgaattt tgaacatccg aacctgggag ttttccctga 6540
aacagatagt atatttgaac ctgtataata atatatagtc tagcgcttta cggaagacaa 6600
tgtatgtatt tcggttcctg gagaaactat tgcatctatt gcataggtaa tcttgcacgt 6660
cgcatccccg gttcattttc tgcgtttcca tcttgcactt caatagcata tctttgttaa 6720
cgaagcatct gtgcttcatt ttgtagaaca aaaatgcaac gcgagagcgc taatttttca 6780
aacaaagaat ctgagctgca tttttacaga acagaaatgc aacgcgaaag cgctatttta 6840
ccaacgaaga atctgtgctt catttttgta aaacaaaaat gcaacgcgac gagagcgcta 6900
atttttcaaa caaagaatct gagctgcatt tttacagaac agaaatgcaa cgcgagagcg 6960
ctattttacc aacaaagaat ctatacttct tttttgttct acaaaaatgc atcccgagag 7020
cgctattttt ctaacaaagc atcttagatt actttttttc tcctttgtgc gctctataat 7080
gcagtctctt gataactttt tgcactgtag gtccgttaag gttagaagaa ggctactttg 7140
gtgtctattt tctcttccat aaaaaaagcc tgactccact tcccgcgttt actgattact 7200
agcgaagctg cgggtgcatt ttttcaagat aaaggcatcc ccgattatat tctataccga 7260
tgtggattgc gcatactttg tgaacagaaa gtgatagcgt tgatgattct tcattggtca 7320
gaaaattatg aacggtttct tctattttgt ctctatatac tacgtatagg aaatgtttac 7380
attttcgtat tgttttcgat tcactctatg aatagttctt actacaattt ttttgtctaa 7440

agagtaatac tagagataaa cataaaaaat gtagaggtcg agtttagatg caagttcaag 7500

gagcgaaagg tggatgggta ggttatatag ggatatagca cagagatata tagcaaagag 7560

atacttttga gcaatgtttg tggaagcggt attcgcaatg ccggctttcc ccgtcaagct 7620

ctaaatcggg ggctcccttt agggttccga tttagtgctt tacggcacct cgaccccaaa 7680

aaacttgatt agggtgatgg ttcacgtagt gggccatcgc cctgatagac ggtttttcgc 7740

cctttgacgt tggagtccac gttctttaat agtggactct tgttccaaac tggaacaaca 7800

ctcaacccta tctcggtcta ttcttttgat ttataaggga ttttgccgat ttcggcctat 7860

tggttaaaaa atgagctgat ttaacaaaaa tttaacgcga attttaacaa aatattaacg 7920

tttacaattt cctgatgcgg tattttctcc ttacgcatct gtgcggtatt tcacaccgca 7980

tatgatccgt cgagttcaag agaaaaaaaa agaaaaagca aaaagaaaaa aggaaagcgc 8040

gcctcgttca gaatgacacg tatagaatga tgcattacct tgtcatcttc agtatcatac 8100

tgttcgtata catacttact gacattcata ggtatacata tatacacatg tatatatatc 8160

gtatgctgca gctttaaata atcggtgtca ctacataaga acacctttgg tggagggaac 8220

atcgttggta ccattgggcg aggtggcttc tcttatggca accgcaagag ccttgaacgc 8280

actctcacta cggtgatgat cattcttgcc tcgcagacaa tcaacgtgga gggtaattct 8340

gctagcctct gcaaagcttt caagaaaatg cgggatcatc tcgcaagaga gatctcctac 8400

tttctccctt tgcaaaccaa gttcgacaac tgcgtacggc ctgttcgaaa gatctaccac 8460

cgctctggaa agtgcctcat ccaaaggcgc aaatcctgat ccaaaccttt ttactccacg 8520

cgccagtagg gcctctttaa aagcttgacc gagagcaatc ccgcagtctt cagtggtgtg 8580

atggtcgtct atgtgtaagt caccaatgca ctcaacgatt agcgaccagc cggaatgctt 8640

ggccagagca tgtatcatat ggtccagaaa ccctatacct gtgtggacgt taatcacttg 8700

cgattgtgtg gcctgttctg ctactgcttc tgcctctttt tctgggaaga tcgagtgctc 8760

tatcgctagg ggaccaccct ttaaagagat cgcaatctga atcttggttt catttgtaat 8820

acgctttact agggctttct gctctgtcat ctttgccttc gtttatcttg cctgctcatt 8880

ttttagtata ttcttcgaag aaatcacatt actttatata atgtataatt cattatgtga 8940

taatgccaat cgctaagaaa aaaaaagagt catccgctag gtggaaaaaa aaaaatgaaa 9000

atcattaccg aggcataaaa aaatatagag tgtactagag gaggccaaga gtaatagaaa 9060

aagaaaattg cgggaaagga ctgtgttatg acttccctga ctaatgccgt gttcaaacga 9120

tacctggcag tgactcctag cgctcaccaa gctcttaaaa cggaattatg gtgcactctc 9180

agtacaatct gctctgatgc cgcatagtta agccagcccc gacacccgcc aacacccgct 9240

gacgcgccct gacgggcttg tctgctcccg gcatccgctt acagacaagc tgtgaccgtc 9300

tccgggagct gcatgtgtca gaggttttca ccgtcatcac cgaaacgcgc ga 9352

<210> 44
<211> 29
<212> DNA
<213> Artificial

<220>
<223> Primer GPD2uf

<400> 44
ggtaccagat cttttgcggc gaggtgccg 29

<210> 45
<211> 42
<212> DNA
<213> Artificial

<220>
<223> Primer GPD2ur

<400> 45
tctagactta aggaatgtgt atcttgttaa tcttctgaca gc 42

<210> 46
<211> 28
<212> DNA
<213> Artificial

<220>
<223> Primer GPD2df

<400> 46
ctcgagatag tctacaacaa cgtccgca 28

<210> 47
<211> 39
<212> DNA
<213> Artificial

<220>
<223> Primer GPD2dr

<400> 47
ccatggagat ctgcagtgaa aaagctcgaa gaaacagct 39

<210> 48
<211> 4397
<212> DNA
<213> Artificial

<220>
<223> pRN594

<400> 48

```
gacgacggcg acctggcggg cttcgtggtc gtctcgtact ccggctggaa ccgccggctg     60
accgtcgagg acatcgaggt cgccccggag caccgggggc acggggtcgg gcgcgcgttg    120
```

atggggctcg cgacggagtt cgcccgcgag cggggcgccg ggcacctctg gctggaggtc 180
accaacgtca acgcaccggc gatccacgcg taccggcgga tggggttcac cctctgcggc 240
ctggacaccg ccctgtacga cggcaccgcc tcggacggcg agcaggcgct ctacatgagc 300
atgccctgcc cctagtactg acaataaaaa gattcttgtt ttcaagaact tgtcatttgt 360
atagtttttt tatattgtag ttgttctatt ttaatcaaat gttagcgtga tttatatttt 420
ttttcgcctc gacatcatct gcccagatgc gaagttaagt gcgcagaaag taatatcatg 480
cgtcaatcgt atgtgaatgc tggtcgctat actgctgtcg attcgatact aacgccgcca 540
tccagtgtcg acggatccta ggtgtacagg gcccaaaagg gcgaattctg cagatatcca 600
tcacactggc ggccgctcga gatagtctac aacaacgtcc gcatggaaga cctaccggag 660
atgattgaag agctagacat cgatgacgaa tagacactct cccccccct cccccctctga 720
tctttcctgt tgcctctttt tcccccaacc aatttatcat tatacacaag ttctacaact 780
actactagta acattactac agttattata attttctatt ctctttttct ttaagaatct 840
atcattaacg ttaatttcta tatatacata actaccatta tacacgctat tatcgtttac 900
atatcacatc accgttaatg aaagatacga caccctgtac actaacacaa ttaaataatc 960
gccataacct tttctgttat ctatagccct taaagctgtt tcttcgagct tttcactgc 1020
agatctccat ggcgatgcct gcttgccgaa tatcatggtg gaaaatggcc gcttttctgg 1080
attcatcgac tgtggccggc tgggtgtggc ggaccgctat caggacatag cgttggctac 1140
ccgtgatatt gctgaagagc ttggcggcga atgggctgac cgcttcctcg tgctttacgg 1200
tatcgccgct cccgattcgc agcgcatcgc cttctatcgc cttcttgacg agttcttctg 1260
aattgaaaaa ggaagagtat gagtattcaa catttccgtg tcgcccttat tcccttttt 1320
gcggcatttt gccttcctgt ttttgctcac ccagaaacgc tggtgaaagt aaaagatgct 1380
gaagatcagt tgggtgcacg agtgggttac atcgaactgg atctcaacag cggtaagatc 1440
cttgagagtt ttcgccccga agaacgtttt ccaatgatga gcacttttaa agttctgcta 1500
tgtggcgcgg tattatcccg tattgacgcc gggcaagagc aactcggtcg ccgcatacac 1560
tattctcaga atgacttggt tgagtactca ccagtcacag aaaagcatct tacggatggc 1620
atgacagtaa gagaattatg cagtgctgcc ataaccatga gtgataacac tgcggccaac 1680
ttacttctga caacgatcgg aggaccgaag gagctaaccg cttttttgca caacatgggg 1740
gatcatgtaa ctcgccttga tcgttgggaa ccggagctga atgaagccat accaaacgac 1800
gagcgtgaca ccacgatgcc tgtagcaatg gcaacaacgt tgcgcaaact attaactggc 1860
gaactactta ctctagcttc ccggcaacaa ttaatagact ggatggaggc ggataaagtt 1920
gcaggaccac ttctgcgctc ggcccttccg gctggctggt ttattgctga taaatctgga 1980

gccggtgagc gtgggtctcg cggtatcatt gcagcactgg ggccagatgg taagccctcc 2040
cgtatcgtag ttatctacac gacggggagt caggcaacta tggatgaacg aaatagacag 2100
atcgctgaga taggtgcctc actgattaag cattggtaac tgtcagacca agtttactca 2160
tatatacttt agattgattt aaaacttcat ttttaattta aaaggatcta ggtgaagatc 2220
ctttttgata atctcatgac caaaatccct taacgtgagt tttcgttcca ctgagcgtca 2280
gaccccgtag aaaagatcaa aggatcttct tgagatcctt tttttctgcg cgtaatctgc 2340
tgcttgcaaa caaaaaaacc accgctacca gcggtggttt gtttgccgga tcaagagcta 2400
ccaactcttt ttccgaaggt aactggcttc agcagagcgc agataccaaa tactgttctt 2460
ctagtgtagc cgtagttagg ccaccacttc aagaactctg tagcaccgcc tacatacctc 2520
gctctgctaa tcctgttacc agtggctgct gccagtggcg ataagtcgtg tcttaccggg 2580
ttggactcaa gacgatagtt accggataag gcgcagcggt cgggctgaac ggggggttcg 2640
tgcacacagc ccagcttgga gcgaacgacc tacaccgaac tgagatacct acagcgtgag 2700
ctatgagaaa gcgccacgct tcccgaaggg agaaaggcgg acaggtatcc ggtaagcggc 2760
agggtcggaa caggagagcg cacgagggag cttccagggg gaaacgcctg gtatctttat 2820
agtcctgtcg ggtttcgcca cctctgactt gagcgtcgat ttttgtgatg ctcgtcaggg 2880
gggcggagcc tatggaaaaa cgccagcaac gcggcctttt tacggttcct ggccttttgc 2940
tggccttttg ctcacatgtt ctttcctgcg ttatcccctg attctgtgga taaccgtatt 3000
accgcctttg agtgagctga taccgctcgc cgcagccgaa cgaccgagcg cagcgagtca 3060
gtgagcgagg aagcggaaga gcgcccaata cgcaaaccgc ctctccccgc gcgttggccg 3120
attcattaat gcagctggca cgacaggttt cccgactgga aagcgggcag tgagcgcaac 3180
gcaattaatg tgagttagct cactcattag gcaccccagg ctttacactt tatgcttccg 3240
gctcgtatgt tgtgtggaat tgtgagcgga taacaatttc acacaggaaa cagctatgac 3300
catgattacg ccaagctatt taggtgacac tatagaatac tcaagctatg catcaagctt 3360
ggtaccagat cttttgcggc gaggtgccga tgggttgctg aggggaagag tgtttagctt 3420
acggacctat tgccattgtt attccgatta atctattgtt cagcagctct tctctaccct 3480
gtcattctag tatttttttt tttttttttt ggttttactt ttttttcttc ttgccttttt 3540
ttcttgttac tttttttcta gttttttttc cttccactaa gctttttcct tgatttatcc 3600
ttgggttctt ctttctactc ctttagattt ttttttata tattaatttt taagtttatg 3660
tattttggta gattcaattc tctttccctt tccttttcct tcgctcccct tccttatcaa 3720
tgcttgctgt cagaagatta acaagataca cattccttaa ggcctcgtcc ccgccgggtc 3780
acccggccag cgacatggag gcccagaata ccctccttga cagtcttgac gtgcgcagct 3840
caggggcatg atgtgactgt cgcccgtaca tttagcccat acatccccat gtataatcat 3900

ttgcatccat acattttgat ggccgcacgg cgcgaagcaa aaattacggc tcctcgctgc 3960

agacctgcga gcagggaaac gctcccctca cagacgcgtt gaattgtccc cacgccgcgc 4020

ccctgtagag aaatataaaa ggttaggatt tgccactgag gttcttcttt catatacttc 4080

cttttaaaat cttgctagga tacagttctc acatcacatc cgaacataaa caaccatgta 4140

aaatgaccac tcttgacgac acggcttacc ggtaccgcac cagtgtcccg gggacgccg 4200

aggccatcga ggcactggat gggtccttca ccaccgacac cgtcttccgc gtcaccgcca 4260

ccggggacgg cttcaccctg cgggaggtgc cggtggaccc gcccctgacc aaggtgttcc 4320

ccgacgacga atcggacgac gaatcggacg ccggggagga cggcgacccg gactcccgga 4380

cgttcgtcgc gtacggg 4397

<210> 49
<211> 367
<212> PRT
<213> Escherichia coli

<400> 49

Met Asp Arg Ile Ile Gln Ser Pro Gly Lys Tyr Ile Gln Gly Ala Asp
1 5 10 15

Val Ile Asn Arg Leu Gly Glu Tyr Leu Lys Pro Leu Ala Glu Arg Trp
20 25 30

Leu Val Val Gly Asp Lys Phe Val Leu Gly Phe Ala Gln Ser Thr Val
35 40 45

Glu Lys Ser Phe Lys Asp Ala Gly Leu Val Val Glu Ile Ala Pro Phe
50 55 60

Gly Gly Glu Cys Ser Gln Asn Glu Ile Asp Arg Leu Arg Gly Ile Ala
65 70 75 80

Glu Thr Ala Gln Cys Gly Ala Ile Leu Gly Ile Gly Gly Gly Lys Thr
85 90 95

Leu Asp Thr Ala Lys Ala Leu Ala His Phe Met Gly Val Pro Val Ala
100 105 110

Ile Ala Pro Thr Ile Ala Ser Thr Asp Ala Pro Cys Ser Ala Leu Ser
115 120 125

Val Ile Tyr Thr Asp Glu Gly Glu Phe Asp Arg Tyr Leu Leu Leu Pro
130 135 140

```
Asn Asn Pro Asn Met Val Ile Val Asp Thr Lys Ile Val Ala Gly Ala
145                 150                 155                 160

Pro Ala Arg Leu Leu Ala Ala Gly Ile Gly Asp Ala Leu Ala Thr Trp
                165                 170                 175

Phe Glu Ala Arg Ala Cys Ser Arg Ser Gly Ala Thr Thr Met Ala Gly
            180                 185                 190

Gly Lys Cys Thr Gln Ala Ala Leu Ala Leu Ala Glu Leu Cys Tyr Asn
        195                 200                 205

Thr Leu Leu Glu Glu Gly Glu Lys Ala Met Leu Ala Ala Glu Gln His
    210                 215                 220

Val Val Thr Pro Ala Leu Glu Arg Val Ile Glu Ala Asn Thr Tyr Leu
225                 230                 235                 240

Ser Gly Val Gly Phe Glu Ser Gly Gly Leu Ala Ala Ala His Ala Val
                245                 250                 255

His Asn Gly Leu Thr Ala Ile Pro Asp Ala His His Tyr Tyr His Gly
            260                 265                 270

Glu Lys Val Ala Phe Gly Thr Leu Thr Gln Leu Val Leu Glu Asn Ala
        275                 280                 285

Pro Val Glu Glu Ile Glu Thr Val Ala Ala Leu Ser His Ala Val Gly
    290                 295                 300

Leu Pro Ile Thr Leu Ala Gln Leu Asp Ile Lys Glu Asp Val Pro Ala
305                 310                 315                 320

Lys Met Arg Ile Val Ala Glu Ala Ala Cys Ala Glu Gly Glu Thr Ile
                325                 330                 335

His Asn Met Pro Gly Gly Ala Thr Pro Asp Gln Val Tyr Ala Ala Leu
            340                 345                 350

Leu Val Ala Asp Gln Tyr Gly Gln Arg Phe Leu Gln Glu Trp Glu
        355                 360                 365
```

<210> 50
<211> 1120
<212> DNA
<213> Artificial

<220>

<223> nucleotide seqeunce encoding E.coli gldA codon-optimised for yeast

<400> 50

```
ctgcagaaaa tggacagaat catccaatct ccaggtaagt acatccaagg tgctgacgtt      60
atcaacagat tgggtgaata cttgaagcca ttggctgaaa gatggttggt tgttggtgac     120
aagttcgttt tgggtttcgc tcaatctacc gttgaaaagt ctttcaagga cgctggtttg     180
gttgttgaaa tcgctccatt cggtggtgaa tgttctcaaa acgaaatcga cagattgaga     240
ggtatcgctg aaaccgctca atgtggtgct atcttgggta tcggtggtgg taagaccttg     300
gacaccgcta aggctttggc tcacttcatg ggtgttccag ttgctatcgc tccaaccatc     360
gcttctaccg acgctccatg ttctgctttg tctgttatct acaccgacga aggtgaattc     420
gacagatact tgttgttgcc aaacaaccca aacatggtta tcgttgacac caagatcgtt     480
gctggtgctc cagctagatt gttggcagct ggtatcggtg acgctttggc tacctggttc     540
gaagctagag cttgttctag atctggtgct accaccatgg ctggtggtaa gtgtacccaa     600
gctgctttgg ctttggctga attgtgttac aacaccttgt tggaagaagg tgaaaaggct     660
atgttggctg ctgaacaaca cgttgttacc ccagctttgg aaagagttat cgaagctaac     720
acctacttgt ctggtgttgg tttcgaatct ggtggtttgg ctgctgctca cgctgttcac     780
aacggtttga ccgctatccc agacgctcac cactactacc acggtgaaaa ggttgctttc     840
ggtaccttga cccaattggt tttggaaaac gctccagttg aagaaatcga aaccgttgct     900
gctttgtctc acgctgttgg tttgccaatc accttggctc aattggacat caaggaagac     960
gttccagcta agatgagaat cgttgctgaa gctgcttgtg ctgaaggtga aaccatccac    1020
aacatgccag gtggtgctac cccagaccaa gtttacgctg ctttgttggt tgctgaccaa    1080
tacggtcaaa gattcctaca agaatgggaa taaggcgcgc                          1120
```

<210> 51
<211> 1949
<212> DNA
<213> Artificial

<220>
<223> pRNgldA

<400> 51

```
actagtaaat gtgtggggaa gcgggtaagc tgccacagca attaatgcac aacatttaac     60
ctacattctt ccttatcgga tcctcaaaac ccttaaaaac atatgcctca ccctaacata    120
ttttccaatt aaccctcaat atttctctgt cacccggcct ctattttcca ttttcttctt    180
tacccgccac gcgttttttt ctttcaaatt tttttcttcc ttcttctttt tcttccacgt    240
cctcttgcat aaataaataa accgttttga aaccaaactc gcctctctct ctccttttttg   300
aaatattttt gggtttgttt gatcctttcc ttcccaatct ctcttgttta atatatattc    360
```

atttatatca cgctctcttt ttatcttcct ttttttcctc tctcttgtat tcttccttcc 420

cctttctact caaaccaaga agaaaaagaa aaggtcaatc tttgttaaag aataggatct 480

tctactacat cagcttttag atttttcacg cttactgctt ttttcttccc aagatcgaaa 540

atttactgaa ttaactgcag aaaatggaca gaatcatcca atctccaggt aagtacatcc 600

aaggtgctga cgttatcaac agattgggtg aatacttgaa gccattggct gaaagatggt 660

tggttgttgg tgacaagttc gttttgggtt tcgctcaatc taccgttgaa aagtctttca 720

aggacgctgg tttggttgtt gaaatcgctc cattcggtgg tgaatgttct caaaacgaaa 780

tcgacagatt gagaggtatc gctgaaaccg ctcaatgtgg tgctatcttg ggtatcggtg 840

gtggtaagac cttggacacc gctaaggctt tggctcactt catgggtgtt ccagttgcta 900

tcgctccaac catcgcttct accgacgctc catgttctgc tttgtctgtt atctacaccg 960

acgaaggtga attcgacaga tacttgttgt tgccaaacaa cccaaacatg gttatcgttg 1020

acaccaagat cgttgctggt gctccagcta gattgttggc agctggtatc ggtgacgctt 1080

tggctacctg gttcgaagct agagcttgtt ctagatctgg tgctaccacc atggctggtg 1140

gtaagtgtac ccaagctgct ttggctttgg ctgaattgtg ttacaacacc ttgttggaag 1200

aaggtgaaaa ggctatgttg gctgctgaac aacacgttgt taccccagct ttggaaagag 1260

ttatcgaagc taacacctac ttgtctggtg ttggtttcga atctggtggt ttggctgctg 1320

ctcacgctgt tcacaacggt ttgaccgcta tcccagacgc tcaccactac taccacggtg 1380

aaaaggttgc tttcggtacc ttgacccaat tggttttgga aaacgctcca gttgaagaaa 1440

tcgaaaccgt tgctgctttg tctcacgctg ttggtttgcc aatcaccttg gctcaattgg 1500

acatcaagga agacgttcca gctaagatga gaatcgttgc tgaagctgct tgtgctgaag 1560

gtgaaaccat ccacaacatg ccaggtggtg ctaccccaga ccaagtttac gctgctttgt 1620

tggttgctga ccaatacggt caaagattcc tacaagaatg ggaataaggc gcgccccttt 1680

tcctttgtcg atatcatgta attagttatg tcacgcttac attcacgccc tcctcccaca 1740

tccgctctaa ccgaaaagga aggagttaga caacctgaag tctaggtccc tatttatttt 1800

ttttaatagt tatgttagta ttaagaacgt tatttatatt tcaaattttt cttttttttc 1860

tgtacaaacg cgtgtacgca tgtaacatta tactgaaaac cttgcttgag aaggttttgg 1920

gacgctcgaa ggcttcctag gcgtacgtt 1949

<210> 52
<211> 552
<212> PRT
<213> Citrobacter freundii

<400> 52

Met Ser Gln Phe Phe Phe Asn Gln Arg Thr His Leu Val Ser Asp Val

1 5 10 15

Ile Asp Gly Thr Ile Ile Ala Ser Pro Trp Asn Asn Leu Ala Arg Leu
20 25 30

Glu Ser Asp Pro Ala Ile Arg Ile Val Val Arg Arg Asp Leu Asn Lys
35 40 45

Asn Asn Val Ala Val Ile Ser Gly Gly Gly Ser Gly His Glu Pro Ala
50 55 60

His Val Gly Phe Ile Gly Lys Gly Met Leu Thr Ala Ala Val Cys Gly
65 70 75 80

Asp Val Phe Ala Ser Pro Ser Val Asp Ala Val Leu Thr Ala Ile Gln
85 90 95

Ala Val Thr Gly Glu Ala Gly Cys Leu Leu Ile Val Lys Asn Tyr Thr
100 105 110

Gly Asp Arg Leu Asn Phe Gly Leu Ala Ala Glu Lys Ala Arg Arg Leu
115 120 125

Gly Tyr Asn Val Glu Met Leu Ile Val Gly Asp Asp Ile Ser Leu Pro
130 135 140

Asp Asn Lys His Pro Arg Gly Ile Ala Gly Thr Ile Leu Val His Lys
145 150 155 160

Ile Ala Gly Tyr Phe Ala Glu Arg Gly Tyr Asn Leu Ala Thr Val Leu
165 170 175

Arg Glu Ala Gln Tyr Ala Ala Asn Asn Thr Phe Ser Leu Gly Val Ala
180 185 190

Leu Ser Ser Cys His Leu Pro Gln Glu Ala Asp Ala Ala Pro Arg His
195 200 205

His Pro Gly His Ala Glu Leu Gly Met Gly Ile His Gly Glu Pro Gly
210 215 220

Ala Ser Val Ile Asp Thr Gln Asn Ser Ala Gln Val Val Asn Leu Met
225 230 235 240

Val Asp Lys Leu Met Ala Ala Leu Pro Glu Thr Gly Arg Leu Ala Val
245 250 255

Met Ile Asn Asn Leu Gly Gly Val Ser Val Ala Glu Met Ala Ile Ile
260 265 270

Thr Arg Glu Leu Ala Ser Ser Pro Leu His Pro Arg Ile Asp Trp Leu
275 280 285

Ile Gly Pro Ala Ser Leu Val Thr Ala Leu Asp Met Lys Ser Phe Ser
290 295 300

Leu Thr Ala Ile Val Leu Glu Glu Ser Ile Glu Lys Ala Leu Leu Thr
305 310 315 320

Glu Val Glu Thr Ser Asn Trp Pro Thr Pro Val Pro Pro Arg Glu Ile
325 330 335

Ser Cys Val Pro Ser Ser Gln Arg Ser Ala Arg Val Glu Phe Gln Pro
340 345 350

Ser Ala Asn Ala Met Val Ala Gly Ile Val Glu Leu Val Thr Thr Thr
355 360 365

Leu Ser Asp Leu Glu Thr His Leu Asn Ala Leu Asp Ala Lys Val Gly
370 375 380

Asp Gly Asp Thr Gly Ser Thr Phe Ala Ala Gly Ala Arg Glu Ile Ala
385 390 395 400

Ser Leu Leu His Arg Gln Gln Leu Pro Leu Asp Asn Leu Ala Thr Leu
405 410 415

Phe Ala Leu Ile Gly Glu Arg Leu Thr Val Val Met Gly Gly Ser Ser
420 425 430

Gly Val Leu Met Ser Ile Phe Phe Thr Ala Ala Gly Gln Lys Leu Glu
435 440 445

Gln Gly Ala Ser Val Ala Glu Ser Leu Asn Thr Gly Leu Ala Gln Met
450 455 460

Lys Phe Tyr Gly Gly Ala Asp Glu Gly Asp Arg Thr Met Ile Asp Ala
465 470 475 480

Leu Gln Pro Ala Leu Thr Ser Leu Leu Thr Gln Pro Gln Asn Leu Gln
485 490 495

Ala Ala Phe Asp Ala Ala Gln Ala Gly Ala Glu Arg Thr Cys Leu Ser
500 505 510

Ser Lys Ala Asn Ala Gly Arg Ala Ser Tyr Leu Ser Ser Glu Ser Leu
515 520 525

Leu Gly Asn Met Asp Pro Gly Ala His Ala Val Ala Met Val Phe Lys
530 535 540

Ala Leu Ala Glu Ser Glu Leu Gly
545 550

<210> 53
<211> 1678
<212> DNA
<213> Artificial

<220>
<223> nucleotide sequence encoding the C. freundii dhaK codon-optimised for yeast

<400> 53

```
tctagaaaaa tgtctcaatt cttcttcaac cagagaaccc acttggtttc tgacgttatc     60
gacggtgcta tcatcgcttc accatggaac aatttggcta gattggaatc tgacccagct    120
atcagaatcg ttgttagaag agacttgaac aagaacaacg ttgctgttat ctctggtggt    180
ggttctggtc acgaaccagc tcacgttggt ttcatcggta agggtatgtt gaccgctgct    240
gtttgtggtg acgttttcgc ttctccatct gttgacgctg ttttgactgc tatccaagct    300
gttaccggtg aagctggttg tttgttgatc gttaagaact acaccggtga cagattgaac    360
ttcggtttgg ctgctgaaaa ggctagaaga ttgggttaca acgttgaaat gttgatcgtt    420
ggtgacgaca tctctttgcc agacaacaag cacccaagag gtatcgctgg taccatcttg    480
gttcacaaga tcgctggtta cttcgctgaa agaggttaca acttagctac cgttttgaga    540
gaagctcaat acgctgcttc taacaccttc tctttgggtg ttgctttgtc ttcttgtcac    600
ttgccacaag aaaccgacgc tgctccaaga caccacccag gtcacgctga attgggtatg    660
ggtatccacg gtgaaccagg tgcttctgtt atcgacaccc aaaactctgc tcaagttgtt    720
aacttgatgg ttgacaagtt gttggctgct ttgccagaaa ccggtagatt ggctgttatg    780
atcaacaact tgggtggtgt ttctgttgct gaaatggcta tcatcaccag agaattggct    840
tcttctccat tgcactcaag aatcgactgg ttgatcggtc cagcttcttt ggtaaccgct    900
ttggacatga agggtttctc tttgaccgct atcgttttgg aagaatctat cgaaaaggct    960
ttgttgaccg aagttgaaac ctctaactgg ccaaccccag ttccaccaag agaaatcacc   1020
tgtgttgttt cttctcacgc ttctgctaga gttgaattcc aaccatctgc taacgctttg   1080
gttgctggta tcgttgaatt ggttaccgct accttgtctg acttggaaac ccacttgaac   1140
gctttggacg ctaaggttgg tgacggtgac accggttcta ccttcgctgc tgctgctaga   1200
```

```
gaaatcgctt ctttgttgca cagacaacaa ttgccattga acaacttggc taccttgttc   1260
gctttgatcg gtgaaagatt gaccgttgtt atgggtggtt cttctggtgt tttgatgtct   1320
atcttcttca ccgctgctgg tcaaaagttg gaacaaggtg ctaacgttgt tgaagctttg   1380
aacaccggtt tggctcaaat gaagttctac ggtggtgctg acgaaggtga cagaaccatg   1440
atcgacgctt tgcaaccagc tttgacctct ttgttggctc aaccaaagaa cttgcaagct   1500
gctttcgacg ctgctcaagc tggtgctgaa agaacctgtt tgtcttctaa ggctaacgct   1560
ggtagagctt cttacttgtc ttctgaatct ttgttgggta acatggaccc aggtgctcaa   1620
agattggcta tggttttcaa ggctttggct gaatctgaat tgggttaata aggtcgac     1678
```

<210> 54
<211> 2951
<212> DNA
<213> Artificial

<220>
<223> pRNdhaK

<400> 54

ggatccacta gtaacggccg ccagtgtgct ggaattcgcc cttctcgagc ttaagacgcg 60

tttcttcttc agattccctc atggagaaag tgcggcagat gtatatgaca gagtcgccag 120

tttccaagag actttattca ggcacttcca tgataggcaa gagagaagac ccagagatgt 180

tgttgtccta gttacacatg gtatttattc cagagtattc ctgatgaaat ggtttagatg 240

gacatacgaa gagtttgaat cgtttaccaa tgttcctaac gggagcgtaa tggtgatgga 300

actggacgaa tccatcaata gatacgtcct gaggaccgtg ctacccaaat ggactgattg 360

tgagggagac ctaactacat agtgtttaaa gattacggat atttaactta cttagaataa 420

tgccattttt ttgagttata ataatcctac gttagtgtga gcgggattta aactgtgagg 480

accttaatac attcagacac ttctgcggta tcaccctact tattcccttc gagattatat 540

ctaggaaccc atcaggttgg tggaagatta cccgttctaa gacttttcag cttcctctat 600

tgatgttaca cctggacacc ccttttctgg catccagttt ttaatcttca gtggcatgtg 660

agattctccg aaattaatta aagcaatcac acaattctct cggataccac ctcggttgaa 720

actgacaggt ggtttgttac gcatgctaat gcaaaggagc ctatatacct ttggctcggc 780

tgctgtaaca gggaatataa agggcagcat aatttaggag tttagtgaac ttgcaacatt 840

tactattttc ccttcttacg taaatatttt tctttttaat tctaaatcaa tctttttcaa 900

tttttttgttt gtattctttt cttgcttaaa tctataacta caaaaaacac atacataaat 960

ctagaaaaat gtctcaattc ttcttcaacc agagaaccca cttggtttct gacgttatcg 1020

acggtgctat catcgcttca ccatggaaca atttggctag attggaatct gacccagcta 1080

tcagaatcgt tgttagaaga gacttgaaca agaacaacgt tgctgttatc tctggtggtg 1140

gttctggtca cgaaccagct cacgttggtt tcatcggtaa gggtatgttg accgctgctg 1200
tttgtggtga cgttttcgct tctccatctg ttgacgctgt tttgactgct atccaagctg 1260
ttaccggtga agctggttgt ttgttgatcg ttaagaacta caccggtgac agattgaact 1320
tcggtttggc tgctgaaaag gctagaagat tgggttacaa cgttgaaatg ttgatcgttg 1380
gtgacgacat ctctttgcca gacaacaagc acccaagagg tatcgctggt accatcttgg 1440
ttcacaagat cgctggttac ttcgctgaaa gaggttacaa cttagctacc gttttgagag 1500
aagctcaata cgctgcttct aacaccttct ctttgggtgt tgctttgtct tcttgtcact 1560
tgccacaaga aaccgacgct gctccaagac accacccagg tcacgctgaa ttgggtatgg 1620
gtatccacgg tgaaccaggt gcttctgtta tcgacaccca aaactctgct caagttgtta 1680
acttgatggt tgacaagttg ttggctgctt tgccagaaac cggtagattg gctgttatga 1740
tcaacaactt gggtggtgtt tctgttgctg aaatggctat catcaccaga gaattggctt 1800
cttctccatt gcactcaaga atcgactggt tgatcggtcc agcttctttg gtaaccgctt 1860
tggacatgaa gggtttctct ttgaccgcta tcgttttgga agaatctatc gaaaaggctt 1920
tgttgaccga agttgaaacc tctaactggc caaccccagt tccaccaaga gaaatcacct 1980
gtgttgtttc ttctcacgct tctgctagag ttgaattcca accatctgct aacgctttgg 2040
ttgctggtat cgttgaattg gttaccgcta ccttgtctga cttggaaacc cacttgaacg 2100
ctttggacgc taaggttggt gacggtgaca ccggttctac cttcgctgct gctgctagag 2160
aaatcgcttc tttgttgcac agacaacaat tgccattgaa caacttggct accttgttcg 2220
ctttgatcgg tgaaagattg accgttgtta tgggtggttc ttctggtgtt ttgatgtcta 2280
tcttcttcac cgctgctggt caaaagttgg aacaaggtgc taacgttgtt gaagctttga 2340
acaccggttt ggctcaaatg aagttctacg gtggtgctga cgaaggtgac agaaccatga 2400
tcgacgcttt gcaaccagct ttgacctctt tgttggctca accaaagaac ttgcaagctg 2460
ctttcgacgc tgctcaagct ggtgctgaaa gaacctgttt gtcttctaag gctaacgctg 2520
gtagagcttc ttacttgtct tctgaatctt tgttgggtaa catggaccca ggtgctcaaa 2580
gattggctat ggttttcaag gctttggctg aatctgaatt gggttaataa ggtcgagaca 2640
aatcgctctt aaatatatac ctaaagaaca ttaaagctat attataagca aagatacgta 2700
aattttgctt atattattat acacatatca tatttctata tttttaagat ttggttatat 2760
aatgtacgta atgcaaagga aataaatttt atacattatt gaacagcgtc caagtaacta 2820
cattatgtgc actaatagtt tagcgtcgtg aagactttat tgtgtcgcga aaagtaaaaa 2880
ttttaaaaat tagagcacct tgaacttgcg aaaaaggttc tcatcaactg tttaaaacgt 2940
acgaagctta a 2951

<210> 55
<211> 14200
<212> DNA
<213> Artificial

<220>
<223> pRN957

<400> 55

gacgaaaggg cctcgtgata cgcctatttt tataggttaa tgtcatgata ataagacgtc 60
aggtggcact tttcggggaa atgtgcgcgg aacccctatt tgtttatttt tctaaataca 120
ttcaaatatg tatccgctca tgagacaata accctgataa atgcttcaat aatattgaaa 180
aaggaagagt atgagtattc aacatttccg tgtcgccctt attccctttt ttgcggcatt 240
ttgccttcct gtttttgctc acccagaaac gctggtgaaa gtaaaagatg ctgaagatca 300
gttgggtgca cgagtgggtt acatcgaact ggatctcaac agcggtaaga tccttgagag 360
ttttcgcccc gaagaacgtt ttccaatgat gagcactttt aaagttctgc tatgtggcgc 420
ggtattatcc cgtattgacg ccgggcaaga gcaactcggt cgccgcatac actattctca 480
gaatgacttg gttgagtact caccagtcac agaaaagcat cttacggatg gcatgacagt 540
aagagaatta tgcagtgctg ccataaccat gagtgataac actgcggcca acttacttct 600
gacaacgatc ggaggaccga aggagctaac cgcttttttt cacaacatgg gggatcatgt 660
aactcgcctt gatcgttggg aaccggagct gaatgaagcc ataccaaacg acgagcgtga 720
caccacgatg cctgtagcaa tggcaacaac gttgcgcaaa ctattaactg gcgaactact 780
tactctagct tcccggcaac aattaataga ctggatggag gcggataaag ttgcaggacc 840
acttctgcgc tcggcccttc cggctggctg gtttattgct gataaatctg gagccggtga 900
gcgtgggtct cgcggtatca ttgcagcact ggggccagat ggtaagccct cccgtatcgt 960
agttatctac acgacgggca gtcaggcaac tatggatgaa cgaaatagac agatcgctga 1020
gataggtgcc tcactgatta agcattggta actgtcagac caagtttact catatatact 1080
ttagattgat ttaaaacttc atttttaatt taaaaggatc taggtgaaga tcctttttga 1140
taatctcatg accaaaatcc cttaacgtga gttttcgttc cactgagcgt cagaccccgt 1200
agaaaagatc aaaggatctt cttgagatcc tttttttctg cgcgtaatct gctgcttgca 1260
aacaaaaaaa ccaccgctac cagcggtggt ttgtttgccg gatcaagagc taccaactct 1320
ttttccgaag gtaactggct tcagcagagc gcagatacca aatactgtcc ttctagtgta 1380
gccgtagtta ggccaccact tcaagaactc tgtagcaccg cctacatacc tcgctctgct 1440
aatcctgtta ccagtggctg ctgccagtgg cgataagtcg tgtcttaccg ggttggactc 1500
aagacgatag ttaccggata aggcgcagcg gtcgggctga acggggggtt cgtgcacaca 1560
gcccagcttg gagcgaacga cctacaccga actgagatac ctacagcgtg agcattgaga 1620

aagcgccacg cttcccgaag ggagaaaggc ggacaggtat ccggtaagcg gcagggtcgg 1680
aacaggagag cgcacgaggg agcttccagg ggggaacgcc tggtatcttt atagtcctgt 1740
cgggtttcgc cacctctgac ttgagcgtcg atttttgtga tgctcgtcag gggggccgag 1800
cctatggaaa aacgccagca acgcggcctt tttacggttc ctggcctttt gctggccttt 1860
tgctcacatg ttctttcctg cgttatcccc tgattctgtg gataaccgta ttaccgcctt 1920
tgagtgagct gataccgctc gccgcagccg aacgaccgag cgcagcgagt cagtgagcga 1980
ggaagcggaa gagcgcccaa tacgcaaacc gcctctcccc gcgcgttggc cgattcatta 2040
atgcagctgg cacgacaggt ttcccgactg gaaagcgggc agtgagcgca acgcaattaa 2100
tgtgagttac ctcactcatt aggcacccca ggctttacac tttatgcttc cggctcctat 2160
gttgtgtgga attgtgagcg gataacaatt tcacacagga aacagctatg accatgatta 2220
cgccaagctc ggaattaacc ctcactaaag ggaacaaaag ctgggtaccg ggccccccct 2280
cgaggtcgag cttaagacgc gtttcttctt cagattccct catggagaaa gtgcggcaga 2340
tgtatatgac agagtcgcca gtttccaaga gactttattc aggcacttcc atgataggca 2400
agagagaaga cccagagatg ttgttgtcct agttacacat ggtatttatt ccagagtatt 2460
cctgatgaaa tggtttagat ggacatacga agagtttgaa tcgtttacca atgttcctaa 2520
cgggagcgta atggtgatgg aactggacga atccatcaat agatacgtcc tgaggaccgt 2580
gctacccaaa tggactgatt gtgagggaga cctaactaca tagtgtttaa agattacgga 2640
tatttaactt acttagaata atgccatttt tttgagttat aataatccta cgttagtgtg 2700
agcgggattt aaactgtgag gaccttaata cattcagaca cttctgcggt atcaccctac 2760
ttattccctt cgagattata tctaggaacc catcaggttg gtggaagatt acccgttcta 2820
agacttttca gcttcctcta ttgatgttac acctggacac ccctttctg gcatccagtt 2880
tttaatcttc agtggcatgt gagattctcc gaaattaatt aaagcaatca cacaattctc 2940
tcggatacca cctcggttga aactgacagg tggtttgtta cgcatgctaa tgcaaaggag 3000
cctatatacc tttggctcgg ctgctgtaac agggaatata aagggcagca taatttagga 3060
gtttagtgaa cttgcaacat ttactatttt cccttcttac gtaaatattt ttcttttaa 3120
ttctaaatca atctttttca atttttgtt tgtattcttt tcttgcttaa atctataact 3180
acaaaaaaca catacataaa tctagaaaaa tgtctcaatt cttcttcaac cagagaaccc 3240
acttggtttc tgacgttatc gacggtgcta tcatcgcttc accatggaac aatttggcta 3300
gattggaatc tgacccagct atcagaatcg ttgttagaag agacttgaac aagaacaacg 3360
ttgctgttat ctctggtggt ggttctggtc acgaaccagc tcacgttggt ttcatcggta 3420
agggtatgtt gaccgctgct gtttgtggtg acgttttcgc ttctccatct gttgacgctg 3480

ttttgactgc tatccaagct gttaccggtg aagctggttg tttgttgatc gttaagaact 3540

acaccggtga cagattgaac ttcggtttgg ctgctgaaaa ggctagaaga ttgggttaca 3600

acgttgaaat gttgatcgtt ggtgacgaca tctctttgcc agacaacaag cacccaagag 3660

gtatcgctgg taccatcttg gttcacaaga tcgctggtta cttcgctgaa agaggttaca 3720

acttagctac cgttttgaga gaagctcaat acgctgcttc taacaccttc tctttgggtg 3780

ttgctttgtc ttcttgtcac ttgccacaag aaaccgacgc tgctccaaga caccacccag 3840

gtcacgctga attgggtatg ggtatccacg gtgaaccagg tgcttctgtt atcgacaccc 3900

aaaactctgc tcaagttgtt aacttgatgg ttgacaagtt gttggctgct ttgccagaaa 3960

ccggtagatt ggctgttatg atcaacaact tgggtggtgt ttctgttgct gaaatggcta 4020

tcatcaccag agaattggct tcttctccat tgcactcaag aatcgactgg ttgatcggtc 4080

cagcttcttt ggtaaccgct ttggacatga agggtttctc tttgaccgct atcgttttgg 4140

aagaatctat cgaaaaggct ttgttgaccg aagttgaaac ctctaactgg ccaaccccag 4200

ttccaccaag agaaatcacc tgtgttgttt cttctcacgc ttctgctaga gttgaattcc 4260

aaccatctgc taacgctttg gttgctggta tcgttgaatt ggttaccgct accttgtctg 4320

acttggaaac ccacttgaac gctttggacg ctaaggttgg tgacggtgac accggttcta 4380

ccttcgctgc tgctgctaga gaaatcgctt ctttgttgca cagacaacaa ttgccattga 4440

acaacttggc taccttgttc gctttgatcg gtgaaagatt gaccgttgtt atgggtggtt 4500

cttctggtgt tttgatgtct atcttcttca ccgctgctgg tcaaaagttg gaacaaggtg 4560

ctaacgttgt tgaagctttg aacaccggtt tggctcaaat gaagttctac ggtggtgctg 4620

acgaaggtga cagaaccatg atcgacgctt tgcaaccagc tttgacctct ttgttggctc 4680

aaccaaagaa cttgcaagct gctttcgacg ctgctcaagc tggtgctgaa agaacctgtt 4740

tgtcttctaa ggctaacgct ggtagagctt cttacttgtc ttctgaatct ttgttgggta 4800

acatggaccc aggtgctcaa agattggcta tggttttcaa ggctttggct gaatctgaat 4860

tgggttaata aggtcgagac aaatcgctct taaatatata cctaaagaac attaaagcta 4920

tattataagc aaagatacgt aaattttgct tatattatta tacacatatc atatttctat 4980

atttttaaga tttggttata taatgtacgt aatgcaaagg aaataaattt tatacattat 5040

tgaacagcgt ccaagtaact acattatgtg cactaatagt ttagcgtcgt gaagacttta 5100

ttgtgtcgcg aaaagtaaaa attttaaaaa ttagagcacc ttgaacttgc gaaaaaggtt 5160

ctcatcaact gtttaaaacg tacgcctagg aagccttcga gcgtcccaaa accttctcaa 5220

gcaaggtttt cagtataatg ttacatgcgt acacgcgttt gtacagaaaa aaaagaaaaa 5280

tttgaaatat aaataacgtt cttaatacta acataactat taaaaaaaat aaatagggac 5340

ctagacttca ggttgtctaa ctccttcctt ttcggttaga gcggatgtgg gaggagggcg 5400

tgaatgtaag cgtgacataa ctaattacat gatatcgaca aaggaaaagg ggcgcgcctt 5460

attcccattc ttgtaggaat ctttgaccgt attggtcagc aaccaacaaa gcagcgtaaa 5520

cttggtctgg ggtagcacca cctggcatgt tgtggatggt ttcaccttca gcacaagcag 5580

cttcagcaac gattctcatc ttagctggaa cgtcttcctt gatgtccaat tgagccaagg 5640

tgattggcaa accaacagcg tgagacaaag cagcaacggt ttcgatttct tcaactggag 5700

cgttttccaa aaccaattgg gtcaaggtac cgaaagcaac cttttcaccg tggtagtagt 5760

ggtgagcgtc tgggatagcg gtcaaaccgt tgtgaacagc gtgagcagca gccaaaccac 5820

cagattcgaa accaacacca gacaagtagg tgttagcttc gataactctt tccaaagctg 5880

gggtaacaac gtgttgttca gcagccaaca tagccttttc accttcttcc aacaaggtgt 5940

tgtaacacaa ttcagccaaa gccaaagcag cttgggtaca cttaccacca gccatggtgg 6000

tagcaccaga tctagaacaa gctctagctt cgaaccaggt agccaaagcg tcaccgatac 6060

cagctgccaa caatctagct ggagcaccag caacgatctt ggtgtcaacg ataaccatgt 6120

ttgggttgtt tggcaacaac aagtatctgt cgaattcacc ttcgtcggtg tagataacag 6180

acaaagcaga acatggagcg tcggtagaag cgatggttgg agcgatagca actggaacac 6240

ccatgaagtg agccaaagcc ttagcggtgt ccaaggtctt accaccaccg atacccaaga 6300

tagcaccaca ttgagcggtt tcagcgatac ctctcaatct gtcgatttcg ttttgagaac 6360

attcaccacc gaatggagcg atttcaacaa ccaaaccagc gtccttgaaa gacttttcaa 6420

cggtagattg agcgaaaccc aaaacgaact tgtcaccaac aaccaaccat ctttcagcca 6480

atggcttcaa gtattcaccc aatctgttga taacgtcagc accttggatg tacttacctg 6540

gagattggat gattctgtcc attttctgca gttaattcag taaattttcg atcttgggaa 6600

gaaaaaagca gtaagcgtga aaaatctaaa agctgatgta gtagaagatc ctattcttta 6660

acaaagattg accttttctt tttcttcttg gtttgagtag aaaggggaag gaagaataca 6720

agagagagga aaaaaaggaa gataaaaaga gagcgtgata taaatgaata tatattaaac 6780

aagagagatt gggaaggaaa ggatcaaaca aacccaaaaa tatttcaaaa aggagagaga 6840

gaggcgagtt tggtttcaaa acggtttatt tatttatgca agaggacgtg gaagaaaaag 6900

aagaaggaag aaaaaaattt gaaagaaaaa aacgcgtggc gggtaaagaa gaaaatggaa 6960

aatagaggcc gggtgacaga gaaatattga gggttaattg gaaaatatgt tagggtgagg 7020

catatgtttt taagggtttt gaggatccga taaggaagaa tgtaggttaa atgttgtgca 7080

ttaattgctg tggcagctta cccgcttccc cacacattta ctagtctcga gctcttcaac 7140

tcaagacgca cagatattat aacatctgca taataggcat ttgcaagaat tactcgtgag 7200

taaggaaaga gtgaggaact atcgcatacc tgcatttaaa gatgccgatt tgggcgcgaa 7260

tcctttattt tggcttcacc ctcatactat tatcagggcc agaaaaagga agtgtttccc 7320
tccttcttga attgatgtta ccctcataaa gcacgtggcc tcttatcgag aaagaaatta 7380
ccgtcgctcg tgatttgttt gcaaaaagaa caaaactgaa aaaacccaga cacgctcgac 7440
ttcctgtctt cctattgatt gcagcttcca atttcgtcac acaacaaggt cctagcgacg 7500
gctcacaggt tttgtaacaa gcaatcgaag gttctggaat ggcgggaaag ggtttagtac 7560
cacatgctat gatgcccact gtgatctcca gagcaaagtt cgttcgatcg tactgttact 7620
ctctctcttt caaacagaat tgtccgaatc gtgtgacaac aacagcctgt tctcacacac 7680
tcttttcttc taaccaaggg ggtggtttag tttagtagaa cctcgtgaaa cttacattta 7740
catatatata aacttgcata aattggtcaa tgcaagaaat acatatttgg tcttttctaa 7800
ttcgtagttt ttcaagttct tagatgcttt ctttttctct tttttacaga tcatcaagga 7860
agtaattatc tactttttac aacaaatata tctagaaaat ggctgttacc aacgttgctg 7920
aattgaacgc tttggttgaa agggttaaga aggctcaaag agaatacgct tctttcaccc 7980
aagaacaagt tgacaagatc ttcagagctg ctgctttggc tgctgctgac gctagaatcc 8040
cattggctaa gatggctgtt gctgaatctg gtatgggtat cgttgaagac aaggttatca 8100
agaaccactt cgcttctgaa tacatctaca acgcttacaa ggacgaaaag acctgtggtg 8160
ttttgtcaga agacgacacc ttcggtacca tcaccatcgc tgaaccaatc ggtatcatct 8220
gtggtatcgt tccaaccacc aacccaacct ctaccgctat cttcaagtct ttgatctctt 8280
tgaagaccag aaacgctatc atcttctctc cacacccaag agctaaagac gctaccaaca 8340
aggctgctga catcgttttg caagctgcta tcgctgctgg tgctccaaag gacttgatcg 8400
gttggatcga ccaaccatct gttgaattgt ctaacgcttt gatgcaccac ccagacatca 8460
acttgatctt ggctaccggt ggtccaggta tggttaaggc tgcttactct tctggtaagc 8520
cagctatcgg tgttggtgct ggtaacaccc cagttgttat cgacgaaacc gctgacatca 8580
agagagctgt tgcttctgtt ttgatgtcta agaccttcga caacggtgtt atctgtgctt 8640
ctgaacaatc tgttgttgtt gttgactctg tttacgacgc tgttagagaa agattcgcta 8700
cccacggtgg ttacttgttg caaggtaagg aattgaaggc tgttcaagac gttatcttga 8760
agaacggtgc tttgaacgct gctatcgttg gtcaaccagc ttacaagatc gctgaattag 8820
ctggtttctc tgttccagaa aacaccaaga tcttgatcgg tgaagttacc gttgttgacg 8880
aatctgaacc attcgctcac gaaaagttgt ctccaacctt ggctatgtac agagctaagg 8940
acttcgaaga cgctgttgaa aaagctgaaa agttggttgc tatgggtggt attggtcaca 9000
cctcttgttt gtacaccgac caagacaacc aaccagctag agtttcttac ttcggtcaaa 9060
agatgaagac cgctagaatc ttgatcaaca ccccagcttc tcaaggtggt atcggtgact 9120
tgtacaactt caagttggct ccatctttga ccttgggttg tggttcttgg ggtggtaact 9180

ctatctctga aaacgttggt ccaaagcact tgatcaacaa gaagaccgtt gctaagagag 9240
ctgaaaacat gttgtggcac aagttgccaa aatctatcta cttcagaaga ggttctttgc 9300
caatcgcttt ggacgaagtt atcaccgacg gtcacaagag agctttgatc gttaccgaca 9360
gattcttgtt caacaacggt tacgctgacc aaatcacctc tgttttgaag gctgctggtg 9420
ttgaaaccga agttttcttc gaagttgaag ctgacccaac cttgtctatc gttagaaagg 9480
gtgctgaatt ggctaactct ttcaagccag acgttatcat cgctttgggt ggtggttctc 9540
caatggacgc tgctaagatc atgtgggtta tgtacgaaca cccagaaacc cacttcgaag 9600
aattggcttt gagattcatg gacatcagaa agagaatcta caagttccca aagatgggtg 9660
ttaaggctaa gatgatcgct gttaccacca cctctggtac cggttctgaa gttacccccat 9720
tcgctgttgt taccgacgac gctaccggtc aaaagtaccc attggctgac tacgctttga 9780
ccccagacat ggctatcgtt gacgctaact tggttatgga catgccaaag tctttgtgtg 9840
ctttcggtgg tttggacgct gttacccacg ctatggaagc ttacgtttct gttttggctt 9900
ctgaattctc tgacggtcaa gctttgcaag ctttgaagtt gttgaaggaa tacttgccag 9960
cttcttacca cgaaggttct aagaacccag ttgctagaga aagagttcac tctgctgcta 10020
ccatcgctgg tatcgctttc gctaacgctt tcttgggtgt ttgtcactct atggctcaca 10080
agttgggttc tcaattccac atcccacacg gtttggctaa cgctttgttg atctgtaacg 10140
ttatcagata caacgctaac gacaacccaa ccaagcaaac cgctttctct caatacgaca 10200
gaccacaagc tagaagaaga tacgctgaaa tcgctgacca cttgggtttg tctgctccag 10260
gtgacagaac cgctgcaaag atcgaaaagt tgttggcttg gttggaaacc ttgaaggctg 10320
aattgggtat cccaaagtct atcagagaag ctggtgttca agaagctgac ttcttggcta 10380
acgttgacaa gttgtctgaa gacgctttcg acgaccaatg taccggtgct aacccaagat 10440
acccattgat ctctgaattg aagcaaatct tgttggacac ctactacggt agagactacg 10500
ttgaaggtga aaccgctgct aagaaggaag ctgctccagc taaggctgaa aagaaggcta 10560
agaagtctgc ttagcttaag cgcgcgaatt tcttatgatt tatgattttt attattaaat 10620
aagttataaa aaaaataagt gtatacaaat tttaaagtga ctcttaggtt ttaaaacgaa 10680
aattcttatt cttgagtaac tctttcctgt aggtcaggtt gctttctcag gtatagcatg 10740
aggtcgctct tattgaccac acctctaccg gcatgccgag caaatgcctg caaatcgctc 10800
cccatttcac ccaattgtag atatgctaac tccagcaatg agttgatgaa tctcggtgtg 10860
tattttatgt cctcagagga caacacctgt tgtaatcgtt cttccacacg tacgaagctt 10920
aaaagggcga attctgcaga tatccatcac actggcggcc gcatgctagc tccggattat 10980
cgatgataag ctgtcaaaga tgagaattaa ttccacggac tatagactat actagatact 11040

ccgtctactg tacgatacac ttccgctcag gtccttgtcc tttaacgagg ccttaccact 11100
cttttgttac tctattgatc cagctcagca aaggcagtgt gatctaagat tctatcttcg 11160
cgatgtagta aaactagcta gaccgagaaa gagactagaa atgcaaaagg cacttctaca 11220
atggctgcca tcattattat ccgatgtgac gctgcagctt ctcaatgata ttcgaatacg 11280
ctttgaggag atacagccta atatccgaca aactgtttta cagatttacg atcgtacttg 11340
ttacccatca ttgaattttg aacatccgaa cctgggagtt ttccctgaaa cagatagtat 11400
atttgaacct gtataataat atatagtcta gcgctttacg gaagacaatg tatgtatttc 11460
ggttcctgga gaaactattg catctattgc ataggtaatc ttgcacgtcg catccccggt 11520
tcattttctg cgtttccatc ttgcacttca atagcatatc tttgttaacg aagcatctgt 11580
gcttcatttt gtagaacaaa aatgcaacgc gagagcgcta atttttcaaa caaagaatct 11640
gagctgcatt tttacagaac agaaatgcaa cgcgaaagcg ctattttacc aacgaagaat 11700
ctgtgcttca tttttgtaaa acaaaaatgc aacgcgacga gagcgctaat tttcaaaca 11760
aagaatctga gctgcatttt tacagaacag aaatgcaacg cgagagcgct attttaccaa 11820
caaagaatct atacttcttt tttgttctac aaaaatgcat cccgagagcg ctatttttct 11880
aacaaagcat cttagattac tttttttctc ctttgtgcgc tctataatgc agtctcttga 11940
taacttttttg cactgtaggt ccgttaaggt tagaagaagg ctactttggt gtctattttc 12000
tcttccataa aaaaagcctg actccacttc ccgcgtttac tgattactag cgaagctgcg 12060
ggtgcatttt ttcaagataa aggcatcccc gattatattc tataccgatg tggattgcgc 12120
atactttgtg aacagaaagt gatagcgttg atgattcttc attggtcaga aaattatgaa 12180
cggtttcttc tattttgtct ctatatacta cgtataggaa atgtttacat tttcgtattg 12240
ttttcgattc actctatgaa tagttcttac tacaattttt ttgtctaaag agtaatacta 12300
gagataaaca taaaaaatgt agaggtcgag tttagatgca agttcaagga gcgaaaggtg 12360
gatgggtagg ttatataggg atatagcaca gagatatata gcaaagagat acttttgagc 12420
aatgtttgtg gaagcggtat tcgcaatgcc ggctttcccc gtcaagctct aaatcggggg 12480
ctccctttag ggttccgatt tagtgcttta cggcacctcg accccaaaaa acttgattag 12540
ggtgatggtt cacgtagtgg gccatcgccc tgatagacgg tttttcgccc tttgacgttg 12600
gagtccacgt tctttaatag tggactcttg ttccaaactg gaacaacact caaccctatc 12660
tcggtctatt cttttgattt ataagggatt ttgccgattt cggcctattg gttaaaaaat 12720
gagctgattt aacaaaaatt taacgcgaat tttaacaaaa tattaacgtt tacaatttcc 12780
tgatgcggta ttttctcctt acgcatctgt gcggtatttc acaccgcata tgatccgtcg 12840
agttcaagag aaaaaaaaag aaaaagcaaa aagaaaaaag gaaagcgcgc ctcgttcaga 12900
atgacacgta tagaatgatg cattaccttg tcatcttcag tatcatactg ttcgtataca 12960

tacttactga cattcatagg tatacatata tacacatgta tatatatcgt atgctgcagc 13020
tttaaataat cggtgtcact acataagaac acctttggtg gagggaacat cgttggtacc 13080
attgggcgag gtggcttctc ttatggcaac cgcaagagcc ttgaacgcac tctcactacg 13140
gtgatgatca ttcttgcctc gcagacaatc aacgtggagg gtaattctgc tagcctctgc 13200
aaagctttca agaaaatgcg ggatcatctc gcaagagaga tctcctactt tctccctttg 13260
caaaccaagt tcgacaactg cgtacggcct gttcgaaaga tctaccaccg ctctggaaag 13320
tgcctcatcc aaaggcgcaa atcctgatcc aaaccttttt actccacgcg ccagtagggc 13380
ctctttaaaa gcttgaccga gagcaatccc gcagtcttca gtggtgtgat ggtcgtctat 13440
gtgtaagtca ccaatgcact caacgattag cgaccagccg gaatgcttgg ccagagcatg 13500
tatcatatgg tccagaaacc ctatacctgt gtggacgtta atcacttgcg attgtgtggc 13560
ctgttctgct actgcttctg cctctttttc tgggaagatc gagtgctcta tcgctagggg 13620
accacccttt aaagagatcg caatctgaat cttggtttca tttgtaatac gctttactag 13680
ggctttctgc tctgtcatct ttgccttcgt ttatcttgcc tgctcatttt ttagtatatt 13740
cttcgaagaa atcacattac tttatataat gtataattca ttatgtgata atgccaatcg 13800
ctaagaaaaa aaaagagtca tccgctaggt ggaaaaaaaa aaatgaaaat cattaccgag 13860
gcataaaaaa atatagagtg tactagagga ggccaagagt aatagaaaaa gaaaattgcg 13920
ggaaaggact gtgttatgac ttccctgact aatgccgtgt tcaaacgata cctggcagtg 13980
actcctagcg ctcaccaagc tcttaaaacg gaattatggt gcactctcag tacaatctgc 14040
tctgatgccg catagttaag ccagccccga cacccgccaa cacccgctga cgcgccctga 14100
cgggcttgtc tgctcccggc atccgcttac agacaagctg tgaccgtctc cgggagctgc 14160
atgtgtcaga ggttttcacc gtcatcaccg aaacgcgcga 14200

<210> 56
<211> 11289
<212> DNA
<213> Artificial

<220>
<223> pRN958

<400> 56

gacgaaaggg cctcgtgata cgcctatttt tataggttaa tgtcatgata ataagacgtc 60

aggtggcact tttcggggaa atgtgcgcgg aacccctatt tgtttatttt tctaaataca 120

ttcaaatatg tatccgctca tgagacaata accctgataa atgcttcaat aatattgaaa 180

aaggaagagt atgagtattc aacatttccg tgtcgccctt attccctttt ttgcggcatt 240

ttgccttcct gtttttgctc acccagaaac gctggtgaaa gtaaaagatg ctgaagatca 300

gttgggtgca cgagtgggtt acatcgaact ggatctcaac agcggtaaga tccttgagag 360

ttttcgcccc gaagaacgtt ttccaatgat gagcactttt aaagttctgc tatgtggcgc 420

ggtattatcc cgtattgacg ccgggcaaga gcaactcggt cgccgcatac actattctca 480

gaatgacttg gttgagtact caccagtcac agaaaagcat cttacggatg gcatgacagt 540

aagagaatta tgcagtgctg ccataaccat gagtgataac actgcggcca acttacttct 600

gacaacgatc ggaggaccga aggagctaac cgcttttttt cacaacatgg gggatcatgt 660

aactcgcctt gatcgttggg aaccggagct gaatgaagcc ataccaaacg acgagcgtga 720

caccacgatg cctgtagcaa tggcaacaac gttgcgcaaa ctattaactg gcgaactact 780

tactctagct tcccggcaac aattaataga ctggatggag gcggataaag ttgcaggacc 840

acttctgcgc tcggcccttc cggctggctg gtttattgct gataaatctg gagccggtga 900

gcgtgggtct cgcggtatca ttgcagcact ggggccagat ggtaagccct cccgtatcgt 960

agttatctac acgacgggca gtcaggcaac tatggatgaa cgaaatagac agatcgctga 1020

gataggtgcc tcactgatta agcattggta actgtcagac caagtttact catatatact 1080

ttagattgat ttaaaacttc atttttaatt taaaaggatc taggtgaaga tcctttttga 1140

taatctcatg accaaaatcc cttaacgtga gttttcgttc cactgagcgt cagaccccgt 1200

agaaaagatc aaaggatctt cttgagatcc tttttttctg cgcgtaatct gctgcttgca 1260

aacaaaaaaa ccaccgctac cagcggtggt ttgtttgccg gatcaagagc taccaactct 1320

ttttccgaag gtaactggct tcagcagagc gcagatacca aatactgtcc ttctagtgta 1380

gccgtagtta ggccaccact tcaagaactc tgtagcaccg cctacatacc tcgctctgct 1440

aatcctgtta ccagtggctg ctgccagtgg cgataagtcg tgtcttaccg ggttggactc 1500

aagacgatag ttaccggata aggcgcagcg gtcgggctga acggggggtt cgtgcacaca 1560

gcccagcttg gagcgaacga cctacaccga actgagatac ctacagcgtg agcattgaga 1620

aagcgccacg cttcccgaag ggagaaaggc ggacaggtat ccggtaagcg gcagggtcgg 1680

aacaggagag cgcacgaggg agcttccagg ggggaacgcc tggtatcttt atagtcctgt 1740

cgggtttcgc cacctctgac ttgagcgtcg atttttgtga tgctcgtcag gggggccgag 1800

cctatggaaa aacgccagca acgcggcctt tttacggttc ctggcctttt gctggccttt 1860

tgctcacatg ttctttcctg cgttatcccc tgattctgtg gataaccgta ttaccgcctt 1920

tgagtgagct gataccgctc gccgcagccg aacgaccgag cgcagcgagt cagtgagcga 1980

ggaagcggaa gagcgcccaa tacgcaaacc gcctctcccc gcgcgttggc cgattcatta 2040

atgcagctgg cacgacaggt ttcccgactg gaaagcgggc agtgagcgca acgcaattaa 2100

tgtgagttac ctcactcatt aggcacccca ggctttacac tttatgcttc cggctcctat 2160

gttgtgtgga attgtgagcg gataacaatt tcacacagga aacagctatg accatgatta 2220

cgccaagctc ggaattaacc ctcactaaag ggaacaaaag ctgggtaccg ggccctagga 2280

agccttcgag cgtcccaaaa ccttctcaag caaggttttc agtataatgt tacatgcgta 2340

cacgcgtttg tacagaaaaa aaagaaaaat ttgaaatata aataacgttc ttaatactaa 2400

cataactatt aaaaaaaata aatagggacc tagacttcag gttgtctaac tccttccttt 2460

tcggttagag cggatgtggg aggagggcgt gaatgtaagc gtgacataac taattacatg 2520

atatcgacaa aggaaaaggg gcgcgcctta ttcccattct tgtaggaatc tttgaccgta 2580

ttggtcagca accaacaaag cagcgtaaac ttggtctggg gtagcaccac ctggcatgtt 2640

gtggatggtt tcaccttcag cacaagcagc ttcagcaacg attctcatct tagctggaac 2700

gtcttccttg atgtccaatt gagccaaggt gattggcaaa ccaacagcgt gagacaaagc 2760

agcaacggtt tcgatttctt caactggagc gttttccaaa accaattggg tcaaggtacc 2820

gaaagcaacc ttttcaccgt ggtagtagtg gtgagcgtct gggatagcgg tcaaaccgtt 2880

gtgaacagcg tgagcagcag ccaaaccacc agattcgaaa ccaacaccag acaagtaggt 2940

gttagcttcg ataactcttt ccaaagctgg ggtaacaacg tgttgttcag cagccaacat 3000

agccttttca ccttcttcca acaaggtgtt gtaacacaat tcagccaaag ccaaagcagc 3060

ttgggtacac ttaccaccag ccatggtggt agcaccagat ctagaacaag ctctagcttc 3120

gaaccaggta gccaaagcgt caccgatacc agctgccaac aatctagctg gagcaccagc 3180

aacgatcttg gtgtcaacga taaccatgtt tgggttgttt ggcaacaaca agtatctgtc 3240

gaattcacct tcgtcggtgt agataacaga caaagcagaa catggagcgt cggtagaagc 3300

gatggttgga gcgatagcaa ctggaacacc catgaagtga gccaaagcct tagcggtgtc 3360

caaggtctta ccaccaccga tacccaagat agcaccacat tgagcggttt cagcgatacc 3420

tctcaatctg tcgatttcgt tttgagaaca ttcaccaccg aatggagcga tttcaacaac 3480

caaaccagcg tccttgaaag acttttcaac ggtagattga gcgaaaccca aaacgaactt 3540

gtcaccaaca accaaccatc tttcagccaa tggcttcaag tattcaccca atctgttgat 3600

aacgtcagca ccttggatgt acttacctgg agattggatg attctgtcca ttttctgcag 3660

ttaattcagt aaattttcga tcttgggaag aaaaaagcag taagcgtgaa aaatctaaaa 3720

gctgatgtag tagaagatcc tattctttaa caaagattga ccttttcttt ttcttcttgg 3780

tttgagtaga aaggggaagg aagaatacaa gagagaggaa aaaaaggaag ataaaaagag 3840

agcgtgatat aaatgaatat atattaaaca agagagattg ggaaggaaag gatcaaacaa 3900

acccaaaaat atttcaaaaa ggagagagag aggcgagttt ggtttcaaaa cggtttattt 3960

atttatgcaa gaggacgtgg aagaaaaaga agaaggaaga aaaaaatttg aaagaaaaaa 4020

acgcgtggcg ggtaaagaag aaaatggaaa atagaggccg ggtgacagag aaatattgag 4080

ggttaattgg aaaatatgtt agggtgaggc atatgttttt aagggttttg aggatccgat 4140

aaggaagaat gtaggttaaa tgttgtgcat taattgctgt ggcagcttac ccgcttcccc 4200

acacatttac tagtctcgag ctcttcaact caagacgcac agatattata acatctgcat 4260

aataggcatt tgcaagaatt actcgtgagt aaggaaagag tgaggaacta tcgcatacct 4320

gcatttaaag atgccgattt gggcgcgaat cctttatttt ggcttcaccc tcatactatt 4380

atcagggcca gaaaaaggaa gtgtttccct ccttcttgaa ttgatgttac cctcataaag 4440

cacgtggcct cttatcgaga aagaaattac cgtcgctcgt gatttgtttg caaaaagaac 4500

aaaactgaaa aaacccagac acgctcgact tcctgtcttc ctattgattg cagcttccaa 4560

tttcgtcaca caacaaggtc ctagcgacgg ctcacaggtt ttgtaacaag caatcgaagg 4620

ttctggaatg gcgggaaagg gtttagtacc acatgctatg atgcccactg tgatctccag 4680

agcaaagttc gttcgatcgt actgttactc tctctctttc aaacagaatt gtccgaatcg 4740

tgtgacaaca acagcctgtt ctcacacact cttttcttct aaccaagggg gtggtttagt 4800

ttagtagaac ctcgtgaaac ttacatttac atatatataa acttgcataa attggtcaat 4860

gcaagaaata catatttggt cttttctaat tcgtagtttt tcaagttctt agatgctttc 4920

tttttctctt ttttacagat catcaaggaa gtaattatct actttttaca acaaatatat 4980

ctagaaaatg gctgttacca acgttgctga attgaacgct ttggttgaaa gggttaagaa 5040

ggctcaaaga gaatacgctt ctttcaccca agaacaagtt gacaagatct tcagagctgc 5100

tgctttggct gctgctgacg ctagaatccc attggctaag atggctgttg ctgaatctgg 5160

tatgggtatc gttgaagaca aggttatcaa gaaccacttc gcttctgaat acatctacaa 5220

cgcttacaag gacgaaaaga cctgtggtgt tttgtcagaa gacgacacct tcggtaccat 5280

caccatcgct gaaccaatcg gtatcatctg tggtatcgtt ccaaccacca acccaacctc 5340

taccgctatc ttcaagtctt tgatctcttt gaagaccaga aacgctatca tcttctctcc 5400

acacccaaga gctaaagacg ctaccaacaa ggctgctgac atcgttttgc aagctgctat 5460

cgctgctggt gctccaaagg acttgatcgg ttggatcgac caaccatctg ttgaattgtc 5520

taacgctttg atgcaccacc cagacatcaa cttgatcttg gctaccggtg gtccaggtat 5580

ggttaaggct gcttactctt ctggtaagcc agctatcggt gttggtgctg gtaacacccc 5640

agttgttatc gacgaaaccg ctgacatcaa gagagctgtt gcttctgttt tgatgtctaa 5700

gaccttcgac aacggtgtta tctgtgcttc tgaacaatct gttgttgttg ttgactctgt 5760

ttacgacgct gttagagaaa gattcgctac ccacggtggt tacttgttgc aaggtaagga 5820

attgaaggct gttcaagacg ttatcttgaa gaacggtgct ttgaacgctg ctatcgttgg 5880

tcaaccagct tacaagatcg ctgaattagc tggtttctct gttccagaaa acaccaagat 5940

cttgatcggt gaagttaccg ttgttgacga atctgaacca ttcgctcacg aaaagttgtc 6000

tccaaccttg gctatgtaca gagctaagga cttcgaagac gctgttgaaa aagctgaaaa 6060
gttggttgct atgggtggta ttggtcacac ctcttgtttg tacaccgacc aagacaacca 6120
accagctaga gtttcttact tcggtcaaaa gatgaagacc gctagaatct tgatcaacac 6180
cccagcttct caaggtggta tcggtgactt gtacaacttc aagttggctc catctttgac 6240
cttgggttgt ggttcttggg gtggtaactc tatctctgaa aacgttggtc caaagcactt 6300
gatcaacaag aagaccgttg ctaagagagc tgaaaacatg ttgtggcaca agttgccaaa 6360
atctatctac ttcagaagag gttctttgcc aatcgctttg gacgaagtta tcaccgacgg 6420
tcacaagaga gctttgatcg ttaccgacag attcttgttc aacaacggtt acgctgacca 6480
aatcacctct gttttgaagg ctgctggtgt tgaaaccgaa gtttcttcg aagttgaagc 6540
tgacccaacc ttgtctatcg ttagaaaggg tgctgaattg gctaactctt tcaagccaga 6600
cgttatcatc gctttgggtg gtggttctcc aatggacgct gctaagatca tgtgggttat 6660
gtacgaacac ccagaaaccc acttcgaaga attggctttg agattcatgg acatcagaaa 6720
gagaatctac aagttcccaa agatgggtgt taaggctaag atgatcgctg ttaccaccac 6780
ctctggtacc ggttctgaag ttaccccatt cgctgttgtt accgacgacg ctaccggtca 6840
aaagtaccca ttggctgact acgctttgac cccagacatg gctatcgttg acgctaactt 6900
ggttatggac atgccaaagt ctttgtgtgc tttcggtggt ttggacgctg ttacccacgc 6960
tatggaagct tacgtttctg ttttggcttc tgaattctct gacggtcaag ctttgcaagc 7020
tttgaagttg ttgaaggaat acttgccagc ttcttaccac gaaggttcta agaacccagt 7080
tgctagagaa agagttcact ctgctgctac catcgctggt atcgctttcg ctaacgcttt 7140
cttgggtgtt tgtcactcta tggctcacaa gttgggttct caattccaca tcccacacgg 7200
tttggctaac gctttgttga tctgtaacgt tatcagatac aacgctaacg acaacccaac 7260
caagcaaacc gctttctctc aatacgacag accacaagct agaagaagat acgctgaaat 7320
cgctgaccac ttgggtttgt ctgctccagg tgacagaacc gctgcaaaga tcgaaaagtt 7380
gttggcttgg ttggaaacct tgaaggctga attgggtatc ccaaagtcta tcagagaagc 7440
tggtgttcaa gaagctgact tcttggctaa cgttgacaag ttgtctgaag acgctttcga 7500
cgaccaatgt accggtgcta acccaagata cccattgatc tctgaattga agcaaatctt 7560
gttggacacc tactacggta gagactacgt tgaaggtgaa accgctgcta agaaggaagc 7620
tgctccagct aaggctgaaa agaaggctaa gaagtctgct tagcttaagc gcgcgaattt 7680
cttatgattt atgattttta ttattaaata agttataaaa aaaataagtg tatacaaatt 7740
ttaaagtgac tcttaggttt taaaacgaaa attcttattc ttgagtaact ctttcctgta 7800
ggtcaggttg ctttctcagg tatagcatga ggtcgctctt attgaccaca cctctaccgg 7860

catgccgagc aaatgcctgc aaatcgctcc ccatttcacc caattgtaga tatgctaact 7920
ccagcaatga gttgatgaat ctcggtgtgt attttatgtc ctcagaggac aacacctgtt 7980
gtaatcgttc ttccacacgt acgaagctta aaagggcgaa ttctgcagat atccatcaca 8040
ctggcggccg catgctagct ccggattatc gatgataagc tgtcaaagat gagaattaat 8100
tccacggact atagactata ctagatactc cgtctactgt acgatacact tccgctcagg 8160
tccttgtcct ttaacgaggc cttaccactc ttttgttact ctattgatcc agctcagcaa 8220
aggcagtgtg atctaagatt ctatcttcgc gatgtagtaa aactagctag accgagaaag 8280
agactagaaa tgcaaaaggc acttctacaa tggctgccat cattattatc cgatgtgacg 8340
ctgcagcttc tcaatgatat tcgaatacgc tttgaggaga tacagcctaa tatccgacaa 8400
actgttttac agatttacga tcgtacttgt tacccatcat tgaattttga acatccgaac 8460
ctgggagttt tccctgaaac agatagtata tttgaacctg tataataata tatagtctag 8520
cgctttacgg aagacaatgt atgtatttcg gttcctggag aaactattgc atctattgca 8580
taggtaatct tgcacgtcgc atccccggtt cattttctgc gtttccatct tgcacttcaa 8640
tagcatatct ttgttaacga agcatctgtg cttcattttg tagaacaaaa atgcaacgcg 8700
agagcgctaa tttttcaaac aaagaatctg agctgcattt ttacagaaca gaaatgcaac 8760
gcgaaagcgc tattttacca acgaagaatc tgtgcttcat tttgtaaaa caaaaatgca 8820
acgcgacgag agcgctaatt tttcaaacaa agaatctgag ctgcattttt acagaacaga 8880
aatgcaacgc gagagcgcta ttttaccaac aaagaatcta tacttctttt ttgttctaca 8940
aaaatgcatc ccgagagcgc tatttttcta acaaagcatc ttagattact ttttttctcc 9000
tttgtgcgct ctataatgca gtctcttgat aactttttgc actgtaggtc cgttaaggtt 9060
agaagaaggc tactttggtg tctattttct cttccataaa aaaagcctga ctccacttcc 9120
cgcgtttact gattactagc gaagctgcgg gtgcattttt tcaagataaa ggcatccccg 9180
attatattct ataccgatgt ggattgcgca tactttgtga acagaaagtg atagcgttga 9240
tgattcttca ttggtcagaa aattatgaac ggtttcttct attttgtctc tatatactac 9300
gtataggaaa tgtttacatt ttcgtattgt tttcgattca ctctatgaat agttcttact 9360
acaatttttt tgtctaaaga gtaatactag agataaacat aaaaaatgta gaggtcgagt 9420
ttagatgcaa gttcaaggag cgaaaggtgg atgggtaggt tatataggga tatagcacag 9480
agatatatag caaagagata cttttgagca atgtttgtgg aagcggtatt cgcaatgccg 9540
gctttccccg tcaagctcta aatcgggggc tccctttagg gttccgattt agtgctttac 9600
ggcacctcga ccccaaaaaa cttgattagg gtgatggttc acgtagtggg ccatcgccct 9660
gatagacggt ttttcgccct ttgacgttgg agtccacgtt ctttaatagt ggactcttgt 9720
tccaaactgg aacaacactc aaccctatct cggtctattc ttttgattta taagggattt 9780

tgccgatttc ggcctattgg ttaaaaaatg agctgattta acaaaaattt aacgcgaatt 9840

ttaacaaaat attaacgttt acaatttcct gatgcggtat tttctcctta cgcatctgtg 9900

cggtatttca caccgcatat gatccgtcga gttcaagaga aaaaaaaaga aaaagcaaaa 9960

agaaaaaagg aaagcgcgcc tcgttcagaa tgacacgtat agaatgatgc attaccttgt 10020

catcttcagt atcatactgt tcgtatacat acttactgac attcataggt atacatatat 10080

acacatgtat atatatcgta tgctgcagct ttaaataatc ggtgtcacta cataagaaca 10140

cctttggtgg agggaacatc gttggtacca ttgggcgagg tggcttctct tatggcaacc 10200

gcaagagcct tgaacgcact ctcactacgg tgatgatcat tcttgcctcg cagacaatca 10260

acgtggaggg taattctgct agcctctgca aagctttcaa gaaaatgcgg gatcatctcg 10320

caagagagat ctcctacttt ctccctttgc aaaccaagtt cgacaactgc gtacggcctg 10380

ttcgaaagat ctaccaccgc tctggaaagt gcctcatcca aaggcgcaaa tcctgatcca 10440

aacctttttta ctccacgcgc cagtagggcc tctttaaaag cttgaccgag agcaatcccg 10500

cagtcttcag tggtgtgatg gtcgtctatg tgtaagtcac caatgcactc aacgattagc 10560

gaccagccgg aatgcttggc cagagcatgt atcatatggt ccagaaaccc tatacctgtg 10620

tggacgttaa tcacttgcga ttgtgtggcc tgttctgcta ctgcttctgc ctctttttct 10680

gggaagatcg agtgctctat cgctagggga ccacccttta aagagatcgc aatctgaatc 10740

ttggtttcat ttgtaatacg ctttactagg gctttctgct ctgtcatctt tgccttcgtt 10800

tatcttgcct gctcattttt tagtatattc ttcgaagaaa tcacattact ttatataatg 10860

tataattcat tatgtgataa tgccaatcgc taagaaaaaa aaagagtcat ccgctaggtg 10920

gaaaaaaaaa aatgaaaatc attaccgagg cataaaaaaa tatagagtgt actagaggag 10980

gccaagagta atagaaaaag aaaattgcgg gaaaggactg tgttatgact tccctgacta 11040

atgccgtgtt caaacgatac ctggcagtga ctcctagcgc tcaccaagct cttaaaacgg 11100

aattatggtg cactctcagt acaatctgct ctgatgccgc atagttaagc cagccccgac 11160

acccgccaac acccgctgac gcgccctgac gggcttgtct gctcccggca tccgcttaca 11220

gacaagctgt gaccgtctcc gggagctgca tgtgtcagag gttttcaccg tcatcaccga 11280

aacgcgcga 11289

<210> 57
<211> 11102
<212> DNA
<213> Artificial

<220>
<223> pRN607

<400> 57

agtcgtgtct taccgggttg gactcaagac gatagttacc ggataaggcg cagcggtcgg 60
gctgaacggg gggttcgtgc acacagccca gcttggagcg aacgacctac accgaactga 120
gatacctaca gcgtgagcat tgagaaagcg ccacgcttcc cgaagggaga aaggcggaca 180
ggtatccggt aagcggcagg gtcggaacag gagagcgcac gagggagctt ccagggggga 240
acgcctggta tctttatagt cctgtcgggt ttcgccacct ctgacttgag cgtcgatttt 300
tgtgatgctc gtcagggggg ccgagcctat ggaaaaacgc cagcaacgcg gcctttttac 360
ggttcctggc cttttgctgg ccttttgctc acatgttctt tcctgcgtta tcccctgatt 420
ctgtggataa ccgtattacc gcctttgagt gagctgatac cgctcgccgc agccgaacga 480
ccgagcgcag cgagtcagtg agcgaggaag cggaagagcg cccaatacgc aaaccgcctc 540
tccccgcgcg ttggccgatt cattaatgca gctggcacga caggtttccc gactggaaag 600
cgggcagtga gcgcaacgca attaatgtga gttacctcac tcattaggca ccccaggctt 660
tacactttat gcttccggct cctatgttgt gtggaattgt gagcggataa caatttcaca 720
caggaaacag ctatgaccat gattacgcca agctcggaat taaccctcac taaagggaac 780
aaaagctggg tacgttttaa acagttgatg agaacctttt tcgcaagttc aaggtgctct 840
aatttttaaa atttttactt ttcgcgacac aataaagtct tcacgacgct aaactattag 900
tgcacataat gtagttactt ggacgctgtt caataatgta taaaatttat ttcctttgca 960
ttacgtacat tatataacca aatcttaaaa atatagaaat atgatatgtg tataataata 1020
taagcaaaat ttacgtatct ttgcttataa tatagcttta atgttcttta ggtatatatt 1080
taagagcgat ttgtctcgac ttacaaggcg ctttgaaccc ccttcaaaaa ctcacatagg 1140
cctactgcac caggatcttc tacttgagat gaatcgccga catacgaagc tctgccaaat 1200
ttggcctcga atgtagcagt ggatttagca ccttcctctg cagcttttac cgccttattg 1260
aaatccttag atgcagtaaa ttctttaacg aatggttcta aagcatcaat catggtggat 1320
gatcccttcc ttgcctttgt atatttgtat aaagtatcca atgcaattcc gagtgactta 1380
gccacaattt ccttagtgac gggttcatcc tttgatttac aaacctgaat taatccgtgt 1440
gaaaaacccg acaaaagaat agaatataaa ccaccagaag tacctcccat tgagccttca 1500
atgaaatctg aaatttgggc aaccgcctga gataatgagt ccttcgacag cttgtcaagg 1560
ttttcggtga tgcctttaac tcctgccact aaagtgtaac cacaatcacc atcaccaact 1620
tgattgtcta gttccgtaat gtgcggttcg ctcttgataa cttgttcagc accactcttc 1680
atccactcag caaacttgtc aaagtcatag gtaccgacgg cctttgctgt tacttcatta 1740
tgtaacaagt catcgttaac agatggggca gaagtctttt caaaatctgc aattggccag 1800
cccggtgcgt tcgtaaatgc gttcaacaag tctagtactg atttgatctc ctcaaaatca 1860
gattgcaaag cctttgtagc cttagtggcg tttagtaatg tgatactgaa cccattacca 1920

ttgaaggagg tcatcaatgt gccagcaatt gtttgaaccg gggttatgtt gtaattttcc 1980
tttaagaaat ccgtagtttt ggaagtgata gaactaataa caaaattaga aacaccgccg 2040
agattgttaa ctaacaagac aacttcatca tcttcatcaa actttacaaa agctctatcc 2100
ttatcgtttg gatccaatag ttttggtagc atatacttgg agatcaagtc ttcggtagaa 2160
ggaatagggt ctaaaacttt cacaccaggt tcgttatgaa tacccatacc caattccatt 2220
tgtttttcgt ttaattcact ttcgaatttc ctgccaggaa ctttacaatg gtctaaagaa 2280
gatccaatgg tcaccaaatt gtcgttgata attttagcca ctttagctgt accgtctaag 2340
ccatacttac tagaatattc ttctgcgaag gcacctacaa tcttatgaac caaaacggta 2400
cctgccaatg ctcttctacc aaccatacca cccttttctc tgccaactgc aacatcatca 2460
cctatgacag caacgcggca gttaataccc aaggctcttg ctctctcagc ggacagacca 2520
aaatgcaaaa catcacctgt gtagttcttc acaatcaata aaacgccaga cgcattttca 2580
ttgactaaac ggattgcatt taaaatctgt tttgttgaag gggatgcaaa aatttcgcca 2640
accacggcgc cactcaacat acccttacca atgaaaccgg cgtgtgtagg ttcatgtcca 2700
ctaccaccac cagaaattaa tgcgatcttg tcggaatcgg tctttctgaa gagaattttt 2760
tcttcaggga ccagcgtaat ggaggggtta gcaagggcaa accctttgag acttgaattg 2820
actggatctg tgacttcaaa cgatttagcg gacattttct agatttatgt atgtgttttt 2880
tgtagttata gatttaagca agaaaagaat acaaacaaaa aattgaaaaa gattgattta 2940
gaattaaaaa gaaaaatatt tacgtaagaa gggaaaatag taaatgttgc aagttcacta 3000
aactcctaaa ttatgctgcc ctttatattc cctgttacag cagccgagcc aaaggtatat 3060
aggctccttt gcattagcat gcgtaacaaa ccacctgtca gtttcaaccg aggtggtatc 3120
cgagagaatt gtgtgattgc tttaattaat ttcggagaat ctcacatgcc actgaagatt 3180
aaaaactgga tgccagaaaa ggggtgtcca ggtgtaacat caatagagga agctgaaaag 3240
tcttagaacg ggtaatcttc caccaacctg atgggttcct agatataatc tcgaagggaa 3300
taagtagggt gataccgcag aagtgtctga atgtattaag gtcctcacag tttaaatccc 3360
gctcacacta acgtaggatt attataactc aaaaaaatgg cattattcta agtaagttaa 3420
atatccgtaa tctttaaaca ctatgtagtt aggtctccct cacaatcagt ccatttgggt 3480
agcacggtcc tcaggacgta tctattgatg gattcgtcca gttccatcac cattacgctc 3540
ccgttaggaa cattggtaaa cgattcaaac tcttcgtatg tccatctaaa ccatttcatc 3600
aggaatactc tggaataaat accatgtgta actaggacaa caacatctct gggtcttctc 3660
tcttgcctat catggaagtg cctgaataaa gtctcttgga aactggcgac tctgtcatat 3720
acatctgccg cactttctcc atgagggaat ctgaagaaga aaccggttta tcattatcaa 3780

```
tactgccatt tcaaagaata cgtaaataat taatagtagt gattttccta actttattta   3840
gtcaaaaaat tagcctttta attctgctgt aacccgtaca tgcccaaaat agggggcggg   3900
ttacacagaa tatataacat cgtaggtgtc tgggtgaaca gtttattcct ggcatccact   3960
aaatataatg gagcccgctt tttaagctgg catccagaaa aaaaaagaat cccagcacca   4020
aaatattgtt ttcttcacca accatcagtt cataggtcca ttctcttagc gcaactacag   4080
agaacagggg cacaaacagg caaaaaacgg gcacaacctc aatggagtga tgcaacctgc   4140
ctggagtaaa tgatgacaca aggcaattga cccacgcatg tatctatctc attttcttac   4200
accttctatt accttctgct ctctctgatt tggaaaaagc tgaaaaaaaa ggttgaaacc   4260
agttccctga aattattccc ctacttgact aataagtata taaagacggt aggtattgat   4320
tgtaattctg taaatctatt tcttaaactt cttaaattct acttttatag ttagtctttt   4380
ttttagtttt aaaacaccaa gaacttagtt tcgaataaac acacataaag aattcaaaat   4440
gtcgctgatc agcatcctgt ctcccctaat tacttccgag ggcttagatt caagaatcaa   4500
accttcacca aaaaaggatg cctctactac cactaagcca tcactatgga aaactactga   4560
gttcaaattc tactacattg catttctggt cgtggttccc ttgatgttct atgctgggtt   4620
acaagctagt tcgccccgaaa atccaaacta tgcaagatac gaacgtctcc tatctcaagg   4680
ttggttattt ggcagaaaag tagacaatag tgattctcaa tataggtttt tcagggacaa   4740
ttttgcgcta ttgtcagttt taatgctagt ccacacttct ataaaacgca ttgtacttta   4800
ttcaacaaat atcactaaat tgaggtttga tctgatattt ggtttgatct tttagtggc    4860
cgctcatggt gtcaattcga taagaatttt agcccatatg ctaattttat atgccatcgc   4920
ccatgtacta aagaacttta gaagaatagc caccatcagc atttggattt atggtatttc   4980
tacgcttttt attaacgaca acttcagagc atatccattt ggtaatattt gctctttttt   5040
aagcccattg gaccattggt atagaggtat cattccaaga tgggatgtct tttcaattt    5100
tactcttttg agagtcttaa gttacaactt ggacttctta gagaggtggg agaatttaca   5160
aaagaagaaa agtccatcct atgaatcaaa agaagctaaa tcagccattt tgctcaatga   5220
acgtgctaga ttaactgctg cacaccccat acaggactac agcttaatga attatattgc   5280
atatgttact tacacgccac ttttcattgc cggccccatt ataacattca atgattatgt   5340
ttaccaatcg aaacatacct tgccatcaat aaatttcaaa ttcatttttt actatgcggt   5400
gagattcgtt attgctctct tatctatgga gttcatttta cactttctcc acgttgtggc   5460
aatctcaaaa accaaagcgt gggaaaatga cacacctttc cagatttcca tgattggctt   5520
atttaatttg aatattattt ggctaaaact actgattccg tggaggctgt ttaggctgtg   5580
ggctttgcta gacggaatcg atacacctga aaatatgatc aggtgtgttg ataacaatta   5640
cagttcacta gcattctgga gagcttggca tagaagctac aataagtggg ttgtccgtta   5700
```

catatatatt cctctaggtg gttcaaaaaa tagagttttg acatcactag cagtcttttc 5760

cttcgtagct atatggcatg acatcgaact aaagttatta ttatggggtt ggctaatagt 5820

tttgttcctc ttaccagaaa tttttgctac ccaaattttc tctcattata ccgacgcagt 5880

ctggtacaga cacgtttgcg ctgtcggtgc tgttttcaac atatgggtta tgatgatcgc 5940

taatcttttt ggattctgct tgggctctga cggtactaaa aaattactaa gcgatatgtt 6000

ctgtaccgta tctggtttca aatttgtaat tttggcaagc gttagtttat tcatcgcagt 6060

acaaataatg tttgaaatca gagaagaaga aaagaggcac ggaatttacc taaaatgctg 6120

aggatcccct tttcctttgt cgatatcatg taattagtta tgtcacgctt acattcacgc 6180

cctcctccca catccgctct aaccgaaaag gaaggagtta gacaacctga agtctaggtc 6240

cctatttatt ttttttaata gttatgttag tattaagaac gttatttata tttcaaattt 6300

ttcttttttt tctgtacaaa cgcgtgtacg catgtaacat tatactgaaa accttgcttg 6360

agaaggtttt gggacgctcg aaggcttcct agttctagag cggccgccac cgcggtggag 6420

ctccaattcg ccctatagtg agtcgtatta caattcactg gccgtcgttt tacaacgtcg 6480

tgactgggaa aaccctggcg ttacccaact taatcgcctt gcagcacatc cccctttcgc 6540

cagctggcgt aatagcgaag aggcccgcac cgatcgccct tcccaacagt tgcgcagcct 6600

gaatggcgaa tggcgcgacg cgccctgtag cggcgcatta agcgcggcgg gtgtggtggt 6660

tacgcgcagc gtgaccgcta cacttgccag cgccctagcg cccgctcctt tcgctttctt 6720

cccttccttt ctcgccacgt tcgccggtag tgttagacct gaacaaggtt tactaaaaat 6780

ccgtaaagaa cttcaattgt acgccaactt aaggcctcgt ccccgccggg tcacccggcc 6840

agcgacatgg aggcccagaa taccctcctt gacagtcttg acgtgcgcag ctcaggggca 6900

tgatgtgact gtcgcccgta catttagccc atacatcccc atgtataatc atttgcatcc 6960

atacattttg atggccgcac ggcgcgaagc aaaaattacg gctcctcgct gcagacctgc 7020

gagcagggaa acgctcccct cacagacgcg tgaattgtcc ccacgccgcg cccctgtaga 7080

gaaatataaa aggttaggat ttgccactga ggttcttctt tcatatactt ccttttaaaa 7140

tcttgctagg atacagttct cacatcacat ccgaacataa acaaccatgg gtaaaaagcc 7200

tgaactcacc gcgacgtctg tcgagaagtt tctgatcgaa aagttcgaca gcgtctccga 7260

cctgatgcag ctctcggagg gcgaagaatc tcgtgctttc agcttcgatg taggagggcg 7320

tggatatgtc ctgcgggtaa atagctgcgc cgatggtttc tacaaagatc gttatgttta 7380

tcggcacttt gcatcggccg cgctcccgat tccggaagtg cttgacattg gggaattcag 7440

cgagagcctg acctattgca tctcccgccg tgcacagggt gtcacgttgc aagacctgcc 7500

tgaaaccgaa ctgcccgctg ttctgcagcc ggtcgcggag gccatggatg cgatcgctgc 7560

ggccgatctt agccagacga gcgggttcgg cccattcgga ccgcaaggaa tcggtcaata 7620

cactacatgg cgtgatttca tatgcgcgat tgctgatccc catgtgtatc actggcaaac 7680

tgtgatggac gacaccgtca gtgcgtccgt cgcgcaggct ctcgatgagc tgatgctttg 7740

ggccgaggac tgccccgaag tccggcacct cgtgcacgcg gatttcggct ccaacaatgt 7800

cctgacggac aatggccgca taacagcggt cattgactgg agcgaggcga tgttcgggga 7860

ttcccaatac gaggtcgcca acatcttctt ctggaggccg tggttggctt gtatggagca 7920

gcagacgcgc tacttcgagc ggaggcatcc ggagcttgca ggatcgccgc ggctccgggc 7980

gtatatgctc cgcattggtc ttgaccaact ctatcagagc ttggttgacg gcaatttcga 8040

tgatgcagct tgggcgcagg gtcgatgcga cgcaatcgtc cgatccggag ccgggactgt 8100

cgggcgtaca caaatcgccc gcagaagcgc ggccgtctgg accgatggct gtgtagaagt 8160

actcgccgat agtggaaacc gacgccccag cactcgtccg agggcaaagg aataatcagt 8220

actgacaata aaaagattct tgttttcaag aacttgtcat ttgtatagtt tttttatatt 8280

gtagttgttc tattttaatc aaatgttagc gtgatttata tttttttttcg cctcgacatc 8340

atctgcccag atgcgaagtt aagtgcgcag aaagtaatat catgcgtcaa tcgtatgtga 8400

atgctggtcg ctatactgct gtcgattcga tactaacgcc gccatccagt gtcgacggat 8460

cctaggtgta cataaacttt ataaatgaaa ttcataatag aaacgacacg aaattacaaa 8520

atggaatatg ttcatagggt agacgaaact atatacgcaa tctacataca tttatcaaga 8580

aggagaaaaa ggaggatagt aaaggaatac aggtaagcaa attgatacta atggctcaac 8640

gtgataagga aaaagaattg cactttaaca ttaatattga caaggaggag ggcaccacac 8700

aaaaagttag gtgtaacaga aaatcatgaa actacgattc ctaatttgat attggaggat 8760

tttctctaaa aaaaaaaaaa tacaacaaat aaaaaacact caatgacctg accatttgat 8820

ggagtttaag tcaatacctt cttgaaccat ttcccataat ggtgaaagtt ccctcaagaa 8880

ttttactctg tcagaaacgg ccttacgacg tagtcgatat ggtgcactct cagtacaatc 8940

tgctctgatg ccgcatagtt aagccagccc cgacacccgc caacacccgc tgacgcgccc 9000

tgacgggctt gtctgctccc ggcatccgct tacagacaag ctgtgaccgt ctccgggagc 9060

tgcatgtgtc agaggttttc accgtcatca ccgaaacgcg cgagacgaaa gggcctcgtg 9120

atacgcctat ttttataggt taatgtcatg ataataatgg tttcttagga cggatcgctt 9180

gcctgtaact tacacgcgcc tcgtatcttt taatgatgga ataatttggg aatttactct 9240

gtgtttattt atttttatgt tttgtatttg gattttagaa agtaaataaa gaaggtagaa 9300

gagttacgga atgaagaaaa aaaaataaac aaaggtttaa aaaatttcaa caaaaagcgt 9360

actttacata tatatttatt agacaagaaa agcagattaa atagatatac attcgattaa 9420

cgataagtaa aatgtaaaat cacaggattt tcgtgtgtgg tcttctacac agacaagatg 9480

aaacaattcg gcattaatac ctgagagcag gaagagcaag ataaaaggta gtatttgttg 9540
gcgatccccc tagagtcttt tacatcttcg gaaaacaaaa actatttttt ctttaatttc 9600
tttttttact ttctattttt aatttatata tttatattaa aaaatttaaa ttataattat 9660
ttttatagca cgtgatgaaa aggacccagg tggcactttt cggggaaatg tgcgcggaac 9720
ccctatttgt ttatttttct aaatacattc aaatatgtat ccgctcatga gacaataacc 9780
ctgataaatg cttcaataat attgaaaaag gaagagtatg agtattcaac atttccgtgt 9840
cgcccttatt cccttttttg cggcattttg ccttcctgtt tttgctcacc cagaaacgct 9900
ggtgaaagta aaagatgctg aagatcagtt gggtgcacga gtgggttaca tcgaactgga 9960
tctcaacagc ggtaagatcc ttgagagttt tcgccccgaa gaacgttttc caatgatgag 10020
cacttttaaa gttctgctat gtggcgcggt attatcccgt attgacgccg ggcaagagca 10080
actcggtcgc cgcatacact attctcagaa tgacttggtt gagtactcac cagtcacaga 10140
aaagcatctt acggatggca tgacagtaag agaattatgc agtgctgcca taaccatgag 10200
tgataacact gcggccaact tacttctgac aacgatcgga ggaccgaagg agctaaccgc 10260
ttttttgcac aacatggggg atcatgtaac tcgccttgat cgttgggaac cggagctgaa 10320
tgaagccata ccaaacgacg agcgtgacac cacgatgcct gtagcaatgg caacaacgtt 10380
gcgcaaacta ttaactggcg aactacttac tctagcttcc cggcaacaat taatagactg 10440
gatggaggcg gataaagttg caggaccact tctgcgctcg gcccttccgg ctggctggtt 10500
tattgctgat aaatctggag ccggtgagcg tgggtctcgc ggtatcattg cagcactggg 10560
gccagatggt aagccctccc gtatcgtagt tatctacacg acggggagtc aggcaactat 10620
ggatgaacga aatagacaga tcgctgagat aggtgcctca ctgattaagc attggtaact 10680
gtcagaccaa gtttactcat atatacttta gattgattta aaacttcatt tttaatttaa 10740
aaggatctag gtgaagatcc tttttgataa tctcatgacc aaaatccctt aacgtgagtt 10800
ttcgttccac tgagcgtcag accccgtaga aaagatcaaa ggatcttctt gagatccttt 10860
ttttctgcgc gtaatctgct gcttgcaaac aaaaaaacca ccgctaccag cggtggtttg 10920
tttgccggat caagagctac caactctttt tccgaaggta actggcttca gcagagcgca 10980
gataccaaat actgtccttc tagtgtagcc gtagttaggc caccacttca agaactctgt 11040
agcaccgcct acatacctcg ctctgctaat cctgttacca gtggctgctg ccagtggcga 11100
ta 11102